(19) Europäisches Patentamt / European Patent Office / Office européen des brevets

(11) **EP 3 586 957 B2**

(12) **NEW EUROPEAN PATENT SPECIFICATION**
After opposition procedure

(45) Date of publication and mention
of the opposition decision:
**15.07.2026   Bulletin 2026/29**

(45) Mention of the grant of the patent:
**30.03.2022   Bulletin 2022/13**

(21) Application number: **18757450.4**

(22) Date of filing: **22.02.2018**

(51) International Patent Classification (IPC):
**B01J 20/28** (2006.01)      **A61F 13/53** (2006.01)
**A61F 13/534** (2006.01)      **A61F 13/535** (2006.01)
**A61F 13/537** (2006.01)      **A61L 15/24** (2006.01)
**A61L 15/60** (2006.01)      **B01J 20/26** (2006.01)
**B32B 5/24** (2006.01)      **B32B 5/30** (2006.01)
**B32B 27/00** (2006.01)      **B32B 27/30** (2006.01)
**C08F 20/06** (2006.01)      **C08J 5/04** (2006.01)

(52) Cooperative Patent Classification (CPC):
(C-Sets available)
**B32B 5/022; A61F 13/53; A61L 15/42; A61L 15/60;
B01J 20/267; B01J 20/28004; B01J 20/28035;
B32B 5/028; B32B 5/26; B32B 7/12; C08F 220/06;
C08J 5/04;** A61F 2013/530481; A61F 2013/530569;
A61F 2013/530583;                            (Cont.)

(86) International application number:
**PCT/JP2018/006580**

(87) International publication number:
**WO 2018/155591 (30.08.2018 Gazette 2018/35)**

(54) **ABSORBENT ARTICLE COMPRISING WATER-ABSORBING SHEET**

ABSORBIERENDER ARTIKEL ENTHALTEND WASSER-ABSORBIERENDE SCHICHT

ARTICLE ABSORBANT COMPRENANT FEUILLE ABSORBANT L'EAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **22.02.2017   JP 2017031612
12.05.2017   JP 2017095689
27.09.2017   JP 2017187090**

(43) Date of publication of application:
**01.01.2020   Bulletin 2020/01**

(73) Proprietor: **Nippon Shokubai Co., Ltd.
Osaka-shi, Osaka 541-0043 (JP)**

(72) Inventors:
• **TORII, Kazushi**
**Himeji-shi
Hyogo 671-1282 (JP)**
• **ISHIZAKI, Kunihiko**
**Himeji-shi
Hyogo 671-1282 (JP)**
• **HORIE, Kazushi**
**Himeji-shi
Hyogo 671-1282 (JP)**
• **HIRAOKA, Ryuichi**
**Himeji-shi
Hyogo 671-1282 (JP)**
• **KITANO, Takahiro**
**Himeji-shi
Hyogo 671-1282 (JP)**
• **NAKAJIMA, Yasuhisa**
**Himeji-shi
Hyogo 671-1282 (JP)**
• **IKEUCHI, Hiroyuki**
**Himeji-shi
Hyogo 671-1282 (JP)**
• **WADA, Katsuyuki**
**Himeji-shi
Hyogo 671-1282 (JP)**

(74) Representative: **Hoffmann Eitle
Patent- und Rechtsanwälte PartmbB
Arabellastraße 30
81925 München (DE)**

(56) References cited:
EP-A- 2 557 095      EP-A1- 2 669 318
EP-A- 2 740 449      EP-A1- 2 927 266
EP-A- 3 112 022      EP-A1- 3 437 732

**(Cont. next page)**

EP-A2- 0 761 241     WO-A1-2011/086844
WO-A1-2013/002387     WO-A1-2013/099634
WO-A1-2013/099634     WO-A1-2016/158975
WO-A1-2017/170605     DE-A1- 19 543 368
JP-A- 2001 115 042     JP-A- 2002 060 544
JP-A- 2003 290 290     JP-A- 2015 083 693
US-A1- 2014 058 346     US-B1- 6 586 549

- **Invoice regarding Evonik product SXM9280; batch X607605**
- **Invoice regarding Evonik product SXM9280 batch X604509**
- **Rai et al "Safety Evaluation of Disposable Baby Diapers Using Principles of Quantitative Risk Assessment", Journal of Toxicology and Environmental Health, Part A**
- **Frank, M.Superabsorbents, Ullmann's Encyclopaedia of Industrial Chemistry**
- **Experimental report on rework of examples of D2**
- **Analysis report on measurements of products reworked in D11**
- **Report on calculations based on values retrieved in D11 and D14**

- **Analysis report on measurements of retained samples of batchesX607605 and X604509**
- **Report on measurements of original batches X607605 and X604509**
- **Additional analysis report on measurements of products reworked in D11**
- **A copy of the patentee's letter dated 12 November 2020**

Remarks:
The file contains technical information submitted after the application was filed and not included in this specification

(52) Cooperative Patent Classification (CPC): (Cont.)
B01J 2220/68; B32B 2255/02; B32B 2262/0253;
B32B 2262/0276; B32B 2262/04; B32B 2262/067;
B32B 2307/718; B32B 2307/726; B32B 2307/728;
B32B 2307/732; B32B 2555/00; B32B 2555/02

C-Sets
C08F 220/06, C08F 222/102

**Description**

Technical Field

The present invention relates to an absorbent article comprising a water-absorbing sheet containing a particulate water-absorbing agent which is suitable for a hygienic material such as a disposable diaper. In the water-absorbing sheet for use in the present invention, a specific particulate water-absorbing agent suitable for the water-absorbing sheet is sandwiched between two base materials.

Background Art

Water-absorbing resin (super absorbent polymer [SAP]) is a water-swellable, water-insoluble polymer gelling agent. Water-absorbing resin is used in various applications including use in hygienic materials such as disposable diapers, sanitary napkins, and adult incontinence products, water retaining agents for soil for agricultural/horticultural use, and industrial waterproofing agents.

Various kinds of monomers and hydrophilic polymers have been proposed as a raw material for such a water-absorbing resin. In view of performance and cost, the most widely used of these is a polyacrylic acid (salt)-based water-absorbing resin in which acrylic acid and/or salt thereof is used as a monomer(s).

Disposable diapers, which are one of the main applications of water-absorbing resin, have undergone advances in performance. Along with these advances have come requirements for the water-absorbing resin to have a large number of functions (physical properties). Specific examples of the physical properties of the water-absorbing resin include not only merely a high fluid retention capacity but also gel strength, water-soluble content, water absorption speed, fluid retention capacity under pressure, liquid permeability, particle size distribution, urine resistance, antibacterial property, impact resistance (damage resistance), powder fluidity, deodorizing property, anti-coloring property (whiteness), low dustiness, and the like (Patent Literatures 1 through 13).

Patent Literature 1 discloses a water-absorbing resin powder in which both liquid permeability and water absorption speed are achieved by controlling gel-grinding energy (GGE), as well as a method of producing the water-absorbing resin powder.

Patent Literatures 2 and 3 disclose improving the fluid retention capacity under pressure (Patent Literature 2) or liquid permeability (Patent Literature 3) of a water-absorbing resin powder. Specifically, in a gel-crushing step (a production step of the water-absorbing resin powder) after polymerization, hydrogel is further crushed in a kneading manner with use of a specific form of gel-crushing device.

Patent Literatures 4 and 5 disclose a water-absorbing agent having reduced re-wet during actual use in an absorbent article, the water-absorbing agent being defined by gel capillary absorption (GCA), etc.

Patent Literature 6 discloses that a water-absorbing resin achieving both a high fluid retention capacity under load and a high water absorption speed (FSR) can be produced by carrying out surface-crosslinking on a water-absorbing crosslinked polymer having a -COOR content (charge density) of 12 mmol/g or more and a predetermined -COOH/-COOR molar ratio (where R is a hydrogen atom, metal atom, or ammonium).

Patent Literature 7 discloses a method of producing a water-absorbing resin, the method including reversed phase suspension polymerization of a water-soluble ethylenically unsaturated monomer, the reversed phase suspension polymerization being carried out in 2 or more stages, such that in at least one stage after the first stage, the polymerization reaction is carried out with addition of an aminocarboxylic acid-based compound. Patent Literature 7 discloses that this production method makes it possible to produce a water-absorbing resin which achieves both high fluid retention capacity and high water absorption speed.

Patent Literature 8 discloses a water-absorbing resin defined by diffusing absorbency under pressure (DAP). Patent Literature 9 discloses a water-absorbing resin defined by gel bed permeability (GBP) of a gel after free swelling. Patent Literature 10 discloses a water-absorbing resin defined by gel bed permeability (GBP) of a gel after swelling under a load of 0.3 psi.

Patent Literatures 40 through 42 disclose a water-absorbing resin defined by fluid retention capacity, demand wettablity 5 minutes (DW5min), and the like, as well as an absorbent body which uses the water-absorbing resin.

A main application of these water-absorbing resins is their use in hygienic materials such as disposable diapers and incontinence pads. Such hygienic materials are typically configured to include an absorbent body (absorbent layer) in which the water-absorbing resin is used in combination with a fiber material. Recently, in order to reduce the thickness of hygienic materials such as disposable diapers, there has been a tendency to reduce the amount of fiber material used. There have been proposed absorbent articles using a water-absorbing resin which satisfies specific parameters, the absorbent articles (disposable diapers) having a water-absorbing resin concentration of 30 mass% to 100 mass% (preferably 60 mass% to 100 mass%) (Patent Literatures 11 through 15). Furthermore, there have been proposed absorbent articles using a water-absorbing resin defined by a specific parameter (K(t)) which indicates suitability for a water-absorbing resin concentration of 90% to 100% (Patent Literatures 16 through 18). Furthermore, there have been proposed absorbent articles using a water-absorbing resin defined by a specific parameter (sphericity) which indicates suitability for a water-absorbing resin concentration of 75% or more (Patent Literatures 19 through 21). In Patent Literature 43, water-absorbing compositions are described, which comprise a mixture of a water-absorbent resin and an excessive-liquid permeation buffer. The water-absorbing compositions are incorporated in absorbent articles together with hydrophilic fibers. Patent Literature 44 claims an absorbent article comprising an absorbent, a liquid-permeable top sheet and a liquid-impermeable back sheet. The absorbent comprises a specific water-absorbing agent and hydrophilic fibers. The water-absorbing agent comprises a water-absorbing resin having a crosslinking structure, is surface-treated and meets certain properties concerning heat retention, centrifuge retention capacity, absorbency against a pressure of 2.0 kPa and saline flow conductivity (SFC).

In the production of these absorbent articles in a disposable diaper factory, typically an absorbent body will be produced by mixing water-absorbing resin with fiber material and shaping each individual absorbent body in accordance with the absorbent article type. The absorbent bodies are processed so as to have any of a variety of shapes in accordance with the purpose thereof (for example, as seen in a planar view, an hourglass shape, a wedge-like shape, or an elliptical shape). In a method of producing such absorbent bodies, because each absorbent body is shaped individually, the absorbent body can be processed so as to have a discretionarily selected shape. Furthermore, the amounts of fiber and water-absorbing resin can be easily adjusted by absorbent article type. For these reasons, such a method of producing absorbent bodies is currently a mainstream method for disposable diapers.

In recent times, however, there has also emerged production of disposable diapers which utilize another type of absorbent body. This absorbent body is obtained by preparing a long water-absorbing sheet constituted by a water-absorbing resin fixed between two base materials, and then cutting the sheet during a hygienic material production process (ordinarily, the sheet is cut to a rectangular shape measuring approximately 10 cm in width by tens of cm in length). By buying or producing a long continuous water-absorbing sheet, disposable diaper manufacturers can simplify the disposable diaper production process, and also reduce the thickness of the disposable diapers by not using pulp. The water-absorbing sheet is a long continuous sheet in which water-absorbing resin particles are sandwiched or fixed between two base materials (particularly nonwoven fabric base materials). The long continuous sheet is cut and then incorporated into a disposable diaper (Patent Literatures 22 through 39).

In contrast to conventional hygienic materials (disposable diapers), disposable diapers which use a water-absorbing sheet have only appeared recently. As such, there has been almost no development of a water-absorbing resin suitable for the water-absorbing sheet, and almost no proposals regarding parameters for such a water-absorbing resin. As a result, water-absorbing resins as disclosed in Patent Literatures 1-18, which are suited for conventional disposable diapers (utilizing individually shaped absorbent bodies), are being used as is in water-absorbing sheets as well.

Citation List

[Patent Literature]

[Patent Literature 1] International Publication, No. 2011/126079
[Patent Literature 2] International Publication, No. 2015/030129
[Patent Literature 3] International Publication, No. 2015/030130
[Patent Literature 4] International Publication, No. 2015/129917
[Patent Literature 5] International Publication, No. 2016/204302
[Patent Literature 6] European Patent Application Publication No. 0872491
[Patent Literature 7] International Publication, No. 2007/004529
[Patent Literature 8] European Patent Application Publication No. 0712659
[Patent Literature 9] International Publication, No. 2004/096304
[Patent Literature 10] International Publication, No. 2005/016393
[Patent Literature 11] U.S. Patent No. 5147342
[Patent Literature 12] U.S. Patent No. 5149335
[Patent Literature 13] European Patent Application Publication No. 0532002
[Patent Literature 14] U.S. Patent No. 5601542
[Patent Literature 15] U.S. Patent No. 5669894
[Patent Literature 16] International Publication, No. 2012/174026
[Patent Literature 17] International Publication, No. 2013/078109
[Patent Literature 18] International Publication, No. 2015/041784
[Patent Literature 19] International Publication, No. 2011/117187

[Patent Literature 20] International Publication, No. 2012/001117
[Patent Literature 21] International Publication, No. 2012/024445
[Patent Literature 22] International Publication, No. 2010/004894
[Patent Literature 23] International Publication, No. 2010/004895
[Patent Literature 24] Japanese Patent Application Publication, Tokukai, No. 2010-115406
[Patent Literature 25] International Publication, No. 2010/076857
[Patent Literature 26] International Publication, No. 2010/082373
[Patent Literature 27] International Publication, No. 2010/113754
[Patent Literature 28] International Publication, No. 2010/143635
[Patent Literature 29] International Publication, No. 2011/043256
[Patent Literature 30] International Publication, No. 2011/086841
[Patent Literature 31] International Publication, No. 2011/086842
[Patent Literature 32] International Publication, No. 2011/086843
[Patent Literature 33] International Publication, No. 2011/086844
[Patent Literature 34] International Publication, No. 2011/117997
[Patent Literature 35] International Publication, No. 2011/118409
[Patent Literature 36] International Publication, No. 2011/136087
[Patent Literature 37] International Publication, No. 2012/043546
[Patent Literature 38] International Publication, No. 2013/099634
[Patent Literature 39] International Publication, No. 2013/099635
[Patent Literature 40] International Publication, No. 2016/104962
[Patent Literature 41] International Publication, No. 2016/104374
[Patent Literature 42] International Publication, No. 2016/103872
[Patent Literature 43] U.S. Patent No. 6586549
[Patent Literature 44] U.S. Patent Application Publication No. 2014/0058346

Summary of Invention

Technical Problem

Patent Literatures 22 through 39, for example, disclose water-absorbing sheets arranged in various manners. However, despite ingenuity in arranging adhesives, nonwoven fabrics, and the like of these water-absorbing sheets, a sufficient water absorbent property for disposable diapers has not been obtained. For example, in comparison with conventional disposable diapers (in which individually shaped absorbent bodies each obtained by mixing a water-absorbing resin and fibers are used), long water-absorbing sheets and disposable diapers in which the long water-absorbing sheets and cutouts thereof are used can be easily produced. However, such long water-absorbing sheets still had room for improvement in terms of absorption performance (e.g., absorption speed, leakage, re-wet of disposable diapers).

It is an object of the present invention to provide a water-absorbing sheet suitable for a hygienic material such as a disposable diaper.

Solution to Problem

As a result of studying a water-absorbing sheet suitable for hygienic materials such as a disposable diaper, the inventors of the present invention found that none of the various water-absorbing sheets disclosed in Patent Literatures 21 through 38 bring about sufficient characteristics. Then, the inventors of the present invention reached the conclusion that water-absorbing resin particles (particulate water-absorbing agent), which conventionally have not been used, need to be used for a water-absorbing sheet. Then, the inventors of the present invention focused on such a novel parameter as a DRC index for a water-absorbing sheet, and attained the object by using a specific particulate water-absorbing agent in a water-absorbing sheet.

Specifically, the present invention provides an absorbent article as specified in claim 1.

In the present invention, it is intended that one or more of the characteristics above can be provided not only in combination disclosed clearly above but also in further combinations. Further embodiments and advantages of the present invention as defined in the appended claims will be recognized by a person skilled in the art through, as necessary, reading and understanding the detailed description below.

Advantageous Effects of Invention

In comparison with conventional water-absorbing sheets, the present invention can improve a re-wet and an absorption speed (liquid absorption speed) of a water-absorbing sheet used in a sanitary product such as a disposable diaper as an example of an absorbent article.

Brief Description of Drawings

Fig. 1 is a view schematically illustrating a part (flat-surface container) of a device for water-absorbing sheet evaluation on a flat surface (flat surface evaluation 1).
Fig. 2 is a view schematically illustrating a part (injection tube) of the device for the water-absorbing sheet evaluation on a flat surface (flat surface evaluation 1).
Fig. 3 is a view schematically illustrating water-absorbing sheet evaluation on a flat surface (flat surface evaluation 1).
Fig. 4 is a view schematically illustrating a part (curved-surface container) of a device for water-absorbing sheet evaluation on a curved surface (curved surface evaluation 1).
Fig. 5 is a view schematically illustrating a part (curved-surface injection tube) of a device for water-absorbing sheet evaluation on a curved surface (curved surface evaluation 1).
Fig. 6 is a view schematically illustrating water-absorbing sheet evaluation on a curved surface (curved surface evaluation 1).
Fig. 7 is a view schematically illustrating a part (liquid injection tube) of a device for the water-absorbing sheet evaluation on a flat surface (flat surface evaluation 2).
Fig. 8 is a view schematically illustrating water-absorbing sheet evaluation on a flat surface (flat surface evaluation 2).
Fig. 9 is a view schematically illustrating a part (curved-surface container) of a device for water-absorbing sheet evaluation on a curved surface (curved surface evaluation 2).
Fig. 10 is a view schematically illustrating a part (guide) of a device for water-absorbing sheet evaluation on a curved surface (curved surface evaluation 2).
Fig. 11 is a view schematically illustrating a part (guide) of a device for water-absorbing sheet evaluation on a curved surface (curved surface evaluation 2).
Fig. 12 is a view schematically illustrating water-absorbing sheet evaluation on a curved surface (curved surface evaluation 2).
Fig. 13 is a view schematically illustrating water-absorbing sheet evaluation on a curved surface (curved surface evaluation 2).
Fig. 14 is a view schematically illustrating a device for liquid flow evaluation of a water-absorbing sheet.
Fig. 15 is a view schematically illustrating liquid flow evaluation of a water-absorbing sheet.
Fig. 16 is a view schematically illustrating a part (flat-surface container) of a device for water-absorbing sheet evaluation on a flat surface (flat surface evaluation 3).
Fig. 17 is a view schematically illustrating water-absorbing sheet evaluation on a flat surface (flat surface evaluation 3).
Fig. 18 is a view schematically illustrating a measuring instrument for measuring DRC5min.
Fig. 19 is a cross-sectional view schematically illustrating a measuring device for use in measurement of a diffusing absorbency.
Fig. 20 is a cross-sectional view illustrating main components of the measuring device.
Fig. 21 is a view illustrating directions in which a physiological saline diffuses in the measuring device.
Fig. 22 is a graph plotting DRC5min and weight average particle diameters (D50) of particulate water-absorbing agents produced in Production Examples 1 through 23 and in Comparative Production Examples 1 through 8.

Description of Embodiments

The following description will discuss the best mode of the present invention. Throughout the present specification, any expression in a singular form should be understood to encompass the concept of its plural form unless particularly mentioned otherwise. Therefore, the article specifying a single form (e.g., "a", "an", "the") should be understood to encompass the concept of its plural form unless particularly mentioned otherwise. In addition, any term used in the present specification should be understood as ordinarily used in this technical field unless particularly mentioned otherwise. Therefore, unless defined otherwise, all of the technical terms and scientific terms used in the present specification mean as generally understood by a person skilled in the technical field to which the present invention belongs. If there is any conflict in meaning, the present specification (including the definitions) take priority.

[1] Definitions of terms

[1-1] "Water-absorbing resin"

The term "water-absorbing resin" as used in the present invention refers to a water-swellable, water-insoluble polymer gelling agent that satisfies the following physical properties. Specifically, the term "water-absorbing resin" refers to a polymer gelling agent that satisfies the following physical properties: CRC (centrifuge retention capacity) defined in ERT 441.2-02 as "water-swelling property" is 5 g/g or more, and Ext (extractable) defined in ERT470.2-02 as "water-insolubility" is 50 mass% or less.

The water-absorbing resin can be designed as appropriate according to its purpose of use, and is not limited to any particular design. The water-absorbing resin is preferably a hydrophilic crosslinked polymer that has been obtained by crosslinking and polymerizing unsaturated monomers each of which has a carboxyl group. The water-absorbing resin is not limited to a form in which the water-absorbing resin is wholly (that is, 100 mass%) a polymer, and can be a water-absorbing resin composition containing an additive and the like within a range in which the above-described physical properties (CRC and Ext) are satisfied.

The term "water-absorbing resin" as used in the present invention may refer to not only an end product but also an intermediate produced during a process of producing the water-absorbing resin (e.g., a crosslinked hydrogel polymer after polymerization, a dried polymer after drying, a water-absorbing resin powder before surface crosslinking, or the like). In addition, the water-absorbing resin and the water-absorbing resin composition described above will also be collectively referred to as "water-absorbing resin". Examples of forms the water-absorbing resin encompass a sheet form, a fiber form, a film form, a particulate form, and a gel form. The water-absorbing resin for use in the present invention is preferably a particulate water-absorbing resin.

[1-2] "Particulate water-absorbing agent"

The term "water-absorbing agent" as used in the present specification means a gelling agent which contains a water-absorbing resin as a main component and absorbs a water-based liquid. The term "particulate water-absorbing agent" as used in the present specification means a water-absorbing agent in the form of particles (powder), and the term "particulate water-absorbing agent" is used to refer to a single particle of the water-absorbing agent or an aggregate of a plurality of particles of the water-absorbing agent. The term "particulate" means having the form of particles. A particle is a small grain-shaped solid or liquid object with a measurable size (according to the Glossary of Technical Terms in Japanese Industrial Standards, fourth edition, page 2002). In the present specification, a particulate water-absorbing agent may be simply referred to as "water-absorbing agent".

Note that the "water-based liquid" is not limited to water. Examples of the water-based liquid encompass urine, blood, sweat, feces, waste fluid, moisture, vapor, ice, a mixture of water and an organic solvent and/or an inorganic solvent, rain water, and ground water. The water-based liquid is thus not limited to any particular one, provided that the water-based liquid contains water. Preferable examples encompass urine, menstrual blood, sweat, and other body fluids.

The particulate water-absorbing agent for use in accordance with the present invention is suitably used in a hygienic material for absorbing a water-based liquid. A water-absorbing resin serving as a polymer is contained as a main component in a particulate water-absorbing agent. Specifically, the particulate water absorbing agent contains the water-absorbing resin in an amount of preferably 60 mass% to 100 mass%, 70 mass% to 100 mass%, 80 mass% to 100 mass%, or 90 mass% to 100 mass%. The particulate water-absorbing agent optionally further contains, as a non-polymer, water and/or an additive (such as inorganic fine particles and polyvalent metal cations). A suitable moisture content is 0.2 mass% to 30 mass%. The scope of the particulate water-absorbing agent also encompasses a water-absorbing resin composition in which these components are integrated.

The water-absorbing agent contains the water-absorbing resin in an amount up to approximately 100 mass%, more preferably 99 mass%, further preferably 97 mass%, particularly preferably 95 mass% or 90 mass%. Preferably, the water-absorbing agent further contains a component(s) in an amount of 0 mass% to 10 mass% other than the water-absorbing resin. The water-absorbing agent particularly preferably further contains water and/or an additive (inorganic fine particles or polyvalent metal cations) described later.

Examples of the water-absorbing resin to be contained as a main component in the particulate water-absorbing agent encompass a polyacrylic acid (salt)-based resin, a polysulfonic acid (salt)-based resin, a maleic anhydride (salt)-based, a polyacrylamide-based resin, a polyvinyl alcohol-based resin, a polyethylene oxide-based resin, a polyaspartic acid (salt)-based resin, a polyglutamic acid (salt)-based resin, a polyalginic acid (salt)-based, a starch-based resin, and a cellulose-based resin. The water-absorbing resin is preferably a polyacrylic acid (salt)-based resin.

[1-3] "Polyacrylic acid (salt)"

The term "polyacrylic acid (salt)" as used in the present invention refers to polyacrylic acid and/or a salt thereof, and refers to a polymer that contains, as a main component, a repeating unit of acrylic acid and/or a salt thereof (hereinafter referred to as "acrylic acid (salt)") and that contains a graft component as an optional component. The polyacrylic acid can be obtained by hydrolysis of polyacrylamide, polyacrylonitrile, and the like. The polyacrylic acid is preferably obtained by polymerization of an acrylic acid (salt).

The term "main component" means that the acrylic acid (salt) is used (contained) in an amount of ordinarily 50 mol% to 100 mol%, preferably of 70 mol% to 100 mol%, more preferably of 90 mol% to 100 mol%, and even more preferably of substantially 100 mol%, relative to a total amount of monomers for use in polymerization (excluding an internal crosslinking agent).

[1-4] "EDANA" and "ERT"

The term "EDANA" is an acronym for the European Disposables and Nonwovens Associations. The term "ERT" is an acronym for EDANA Recommended Test Methods, which are European standard (de facto international standard) measuring methods for water-absorbing resin. For the present invention, physical properties of water-absorbing resin are measured in conformity with the ERT master copy (2002 revised version; known literature) unless otherwise specified.

[1-4-1] "CRC" (ERT 441.2-02)

The term "CRC" is an acronym for "centrifuge retention capacity", and means a fluid retention capacity without pressure (hereinafter referred to also as "fluid retention capacity") of a particulate water-absorbing agent or of a water-absorbing resin. Specifically, the CRC refers to a fluid retention capacity (unit: g/g) measured after 0.2 g of a particulate water-absorbing agent or a water-absorbing resin contained in a nonwoven fabric bag is immersed in a large excess of a 0.9 mass% aqueous sodium chloride solution for 30 minutes so as to be allowed to freely swell, and then the water-absorbing resin is drained in a centrifuge (250 G).

Note that the CRC of a crosslinked hydrogel polymer (hereinafter referred to as "gel CRC") is measured while the weight of a sample and the free swelling period are changed to 0.4 g and 24 hours, respectively. In calculation of numerical values in the measurement, the mass of a resin solid content of a crosslinked hydrogel polymer is used as the mass of the water-absorbing resin. In a case where each side of the crosslinked hydrogel polymer has a size of 5 mm or more, the crosslinked hydrogel polymer is, before the measurement, cut with use of scissors or the like so that the side has a size of 1 mm or less.

[1-4-2] "AAP" (ERT 442.2-02)

The term "AAP" is an acronym for "absorption against pressure", and means a fluid retention capacity under pressure of a particulate water-absorbing agent or a water-absorbing resin. Specifically, "AAP" refers to a fluid retention capacity (unit: g/g) measured after 0.9 g of particulate water-absorbing agent or water-absorbing resin has been swollen in a large excess of a 0.9 mass% aqueous sodium chloride solution for 1 hour under a load of 2.06 kPa (21 g/cm$^2$, 0.3 psi). Note that in some cases the measurement may be carried out under a load of 4.83 kPa (49 g/cm$^2$, 0.7 psi).

Note that ERT 442.2-02 uses the term "Absorption Under Pressure (AUP)", which refers to substantially the same thing as "AAP".

[1-4-3] "PSD" (ERT 420.2-02)

The term "PSD" is an acronym for "particle size distribution", and means a particle size distribution of a particulate water-absorbing agent or a water-absorbing resin. The particle size distribution is measured by sieve classification.

Note that the mass average particle diameter (D50) and the logarithmic standard deviation ($\sigma\zeta$) of a particle size distribution are measured according to a method similar to "(3) Mass-Average Particle Diameter (D50) and Logarithmic Standard Deviation ($\sigma\zeta$) of Particle Diameter Distribution", which is a method disclosed in U.S. Patent No. 7638570.

[1-4-4] "Moisture Content" (ERT 430.2-02)

The term "moisture content" means a moisture content of a water-absorbing resin. Specifically, a "moisture content" refers to a value (unit: mass%) calculated from a drying loss from drying 4.0 g of a water-absorbing resin at 105°C for 3 hours. Note that in some cases, measurement may be carried out while the amount of the water-absorbing resin and the drying temperature are changed to 1.0 g and 180°C, respectively.

[1-4-5] "Ext" (ERT 470.2-02)

The term "Ext" is an abbreviation for "Extractables", and means a water-soluble content (water-soluble component amount) of water-absorbing resin. Specifically, the "Ext" refers to the amount (unit: mass%) of a polymer dissolved in an aqueous solution after adding 1.0 g of water-absorbing resin to 200 ml of a 0.9 mass% aqueous sodium chloride solution and stirring the resulting mixture at 500 rpm for 16 hours. The amount of the dissolved polymer is measured by pH titration.

[1-5] "Dunk Retention Capacity 5minutes" (DRC5min)

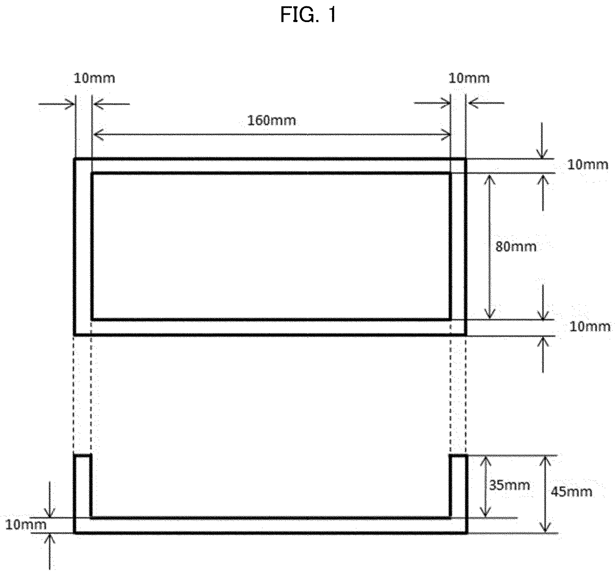

The term "DRC5min" means a fluid retention capacity without pressure for 5 minutes. Specifically, "DRC5min" refers to a fluid retention capacity (unit: g/g) after, as in the case of measurement of the AAP, 1.0 g of particulate water-absorbing agent or water-absorbing resin is dispersed uniformly in a cylindrical cell having a mesh at a bottom surface thereof (see Fig. 1) and then a resulting product is allowed to be in contact with a 0.9 mass% aqueous sodium chloride solution for 5 minutes so as to freely swell. A measuring method will be described in detail in Examples.

[1-5-1] "General index of DRC"

"General index of DRC" is defined by the following Formula (1).

$$\text{General index of DRC} = (K\text{-}DRC5min)/(D50/1000) \ldots \text{Formula (1)}$$

where K is any constant (e.g., 49). A proper value of K can be decided by producing various particulate water-absorbing agents, measuring DRC5min and D50, and determining whether or not a preferable particulate water-absorbing agent(s) was/were obtained. The general index of DRC is useful as an index (unit: dimensionless or g/g/mm) for determining a particulate water-absorbing agent having physical properties preferable for a water-absorbing sheet.

[1-5-2] "Index of DRC"

"Index of DRC" (also referred to as "DRC index" in the present specification) is defined by the following Formula (2).

$$\text{DRC index} = (49\text{-}DRC5min)/(D50/1000) \ldots \text{Formula (2)}$$

Formula (2) corresponds to a case where the value of K in the general index of DRC is 49. As in the case of the general index of DRC, the DRC index is useful as an index (unit: dimensionless or g/g/mm) for determining a particulate water-absorbing agent having physical properties preferable for a water-absorbing sheet.

[1-6] "Liquid permeability"

The term "liquid permeability" of a particulate water-absorbing agent or a water-absorbing resin as used in the present invention refers to flowability of a liquid passing through a space between particles of a swollen gel of a water-absorbing resin under load or without load. The "liquid permeability" is measured typically as a Saline Flow Conductivity (SFC) or Gel Bed Permeability (GBP).

The term "SFC" refers to liquid permeability of a 0.69 mass% aqueous sodium chloride solution in a particulate water-absorbing agent or in a water-absorbing resin under a load of 2.07 kPa, and is measured in conformity with the SFC test method disclosed in Patent Literature 15 (U.S. Patent No. 5669894).

The GBP is an under-load swelling GBP (Patent Literature 10) or a free swelling GBP (Patent Literature 9), and is evaluated as a liquid permeability with use of a 0.9 mass% aqueous sodium chloride solution under a load of 0.3 psi.

[1-7] "Non-uniformly pulverized shape"

The term "non-uniformly pulverized shape" indicates a crushed substance obtained by crushing a hydrogel of a crosslinked polymer during or after polymerization, or by crushing a dried material of such a hydrogel (preferably a dried material). This crushed substance is pulverized particles having non-uniform shape. The crushed substance is preferably a crushed substance obtained by aqueous solution polymerization. In a case where a pulverizing step is not carried out, in contrast, a non-uniformly pulverized shape cannot be achieved by spherical particles or by a granulated material of spherical particles which are typically obtained by, for example, reversed phase suspension polymerization or droplet polymerization which is carried out by spraying polymerizable monomers.

[1-8] "Moisture absorption fluidity"

The "moisture absorption fluidity" as used in the present invention evaluates blocking, caking, or powder fluidity of a particulate water-absorbing agent after the particulate water-absorbing agent was left to sit for 1 hour at an air temperature of 25°C and a relative humidity of 90%RH. The moisture absorption fluidity is determined by "Blocking Ratio" (B.R.) (also referred to as "moisture adsorption blocking ratio"). A method of calculating the moisture absorption fluidity will be described in detail in Examples. In simple terms, a moisture absorption fluidity is calculated as follows. A particulate water-absorbing agent is placed on a sieve, and classification is carried out. Then, the mass (W1 (g)) of the particulate water-absorbing agent remaining on the sieve and the mass (W2 (g)) of the particulate water-absorbing agent which has passed through the sieve are measured. Then, the moisture absorption fluidity is calculated according to the following Formula (3).

$$\text{Moisture absorption fluidity (B.R.) (mass\%)} = \{W1/(W1+W2)\} \times 100 \qquad \text{Formula (3)}$$

A measuring method will be described in detail in Examples.

[1-9] "Moisture absorption fluidity improving agent"

The term "moisture absorption fluidity improving agent" as used in the present invention refers to a compound or a composition which increases the fluidity of a particulate water-absorbing agent or a water-absorbing resin in a case where the moisture absorption fluidity improving agent is added to the particulate water-absorbing agent or the water-absorbing resin (B.R. is a method of evaluating moisture absorption fluidity, and a smaller value in B.R. means superior moisture absorption fluidity). Examples of the moisture absorption fluidity improving agent encompass, but are not limited to, silicon dioxide, hydrotalcite, phosphate, and aluminum salt.

[1-10] "Degradable soluble content"

The term "degradable soluble content" as used in the present invention refers to a water-soluble content measured by a water-soluble content (Ext) measuring method defined in ERT 470.2-02 in a case where a 0.90 mass% aqueous sodium chloride solution is changed to an aqueous solution (degradation test liquid) in which L-ascorbic acid is mixed with the 0.90 mass% aqueous sodium chloride solution, and the aqueous solution is allowed to stand still at 60°C for 2 hours, and then the aqueous solution is stirred for 1 hour.

[1-11] "Gel-grinding energy" (GGE)

The term "gel-grinding energy" as used in the present invention refers to mechanical energy per unit mass (unit mass of a crosslinked hydrogel polymer), the mechanical energy being necessary for a gel-crushing device to gel-crush a crosslinked hydrogel polymer. The gel-grinding energy does not include energy with which to heat or cool a jacket, or energy of water or steam to be introduced. Note that "gel-grinding energy" is abbreviated as "GGE". In a case where the gel-crushing device is driven by a three-phase alternating current power, the GGE is calculated based on the following Formula (4).

$$\text{GGE (J/g)} = \{\sqrt{3} \times \text{voltage} \times \text{electric current} \times \text{power factor} \times \text{motor efficiency}\} / \{\text{mass of crosslinked hydrogel polymer introduced into gel crusher per second}\} \qquad \text{Formula (4)}$$

The "power factor" and the "motor efficiency" are each a value which is unique to the gel-crushing device and changes depending on, for example, an operation condition of the gel-crushing device and which ranges from 0 to 1. These values can be known by, for example, making inquiries to a manufacturer of the device or the like. In a case where the gel-crushing device is driven by a single-phase alternating current power, GGE can be calculated by replacing "$\sqrt{3}$" with "1" in the above Formula (4). Note that a unit of a voltage is [V], a unit of an electric current is [A], and a unit of mass of a crosslinked hydrogel polymer is [g/s]. GGE is measured by the method disclosed in Patent Literature 1 (International Publication, No. 2011/126079).

Since the mechanical energy to be applied to the crosslinked hydrogel polymer is important in the present invention, the gel-grinding energy is preferably calculated by subtracting an electric current value of the gel-crushing device during idling from an electric current value of the gel-crushing device during gel-crushing. In a case where gel-crushing is carried out with use of a plurality of gel-crushing devices, in particular, a sum of electric current values of the plurality of gel-crushing devices during idling is large. It is therefore suitable to calculate the gel-grinding energy by subtracting the electric current values of the plurality of gel-crushing devices during idling from current values of the plurality of gel-crushing devices during gel-crushing. In this case, the gel-grinding energy is calculated by the following Formula (5). Note that this gel-grinding energy is denoted as GGE (2) to be distinguished from the GGE described earlier.

GGE (2) (J/g) = {√3 × voltage × (electric current during gel-crushing - electric current during idling) × power factor × motor efficiency} / {mass of crosslinked hydrogel polymer introduced into gel crusher per second}     Formula (5)

The "power factor" and the "motor efficiency" during gel-crushing are applied to the GGE (2). Since the electric current value during idling is small, the values of the power factor and the motor efficiency during idling are defined approximately as in the Formula (2). For example, in a case where an amount of the crosslinked hydrogel polymer to be continuously fed by a quantitative feeder is [t/hr], the "mass of crosslinked hydrogel polymer to be introduced into gel crusher per second" in each of Formulas (4) and (5) refers to a value obtained by converting [t/hr] into [g/s].

[1-12] "Circulation crushing ratio"

In the present invention, the "circulation crushing ratio" is defined by the following Formula (6).

Circulation crushing ratio (%) = (total amount of particulate water-absorbing agent or water-absorbing resin fed during crushing step) / (total amount of particulate water-absorbing agent or water-absorbing resin discharged during drying step)     Formula (6)

Note, however, that [total amount of particulate water-absorbing agent or water-absorbing resin fed during crushing step] is represented by the sum of [total amount of particulate water-absorbing agent or water-absorbing resin discharged during drying step] and [amount of classified polymer fed again during identical or different crushing step], and is defined by the amount of crushing with identical or different crusher. In the case of continuous crushing, [total amount of particulate water-absorbing agent or water-absorbing resin fed during crushing step] is defined by the amount of crushing (unit: kg/hr) during equilibrium. It should also be noted that in a small scale, the advantageous effects of the present invention may be slim. The circulation crushing ratio as defined in the present invention can be suitably applied to the above large scale (1 t/hr) or a larger scale. The circulation crushing ratio is measured based on the method disclosed in International Publication, No. 2011/034146.

[1-13] "GCA" (Gel Capillary Absorption)

A GCA evaluates a liquid absorbing ability during a 10-minute period during which there is a height difference of 10 cm between an upper surface of a glass filter and a meniscus at a lower part of a Marriott tube. GCA is measured by the method disclosed in Patent Literature 4 (International Publication, No. 2015/129917).

[1-14] "Surface tension"

A surface tension indicates, in a per-unit-area basis, work (free energy) necessary for increasing a surface area of a solid or a liquid. The surface tension as used in the present invention refers to a surface tension of an aqueous solution obtained by dispersing a particulate water-absorbing agent or a water-absorbing resin in a 0.90 mass% aqueous sodium chloride solution. The surface tension is measured by a method described in Examples.

[1-15] "Internal gas bubble ratio"

The term "true density" as used in the present invention means a density (unit: $g/cm^3$) of a sufficiently dry polyacrylic acid (salt)-based water-absorbing resin (having a moisture content of preferably less than 1 mass%, more preferably less than 0.5 mass%, and particularly preferably less than 0.1 mass%), the density being fixedly decided by a chemical composition (for example, repeating units of a polymer, minute raw materials such as a crosslinking agent, and graft component used optionally). Therefore, the true density of polyacrylic acid (salt)-based water-absorbing resin exhibited is substantially constant, although the true density may slightly vary due to its neutralization rate, the type of the salt of the neutralization (for example, sodium polyacrylate having a neutralization rate of 75 mol%), or the minute raw material.

In contrast, the term "apparent density" as used in the present invention means a density (unit: $g/cm^3$) in view of spaces present in particles of a polyacrylic acid (salt)-based water-absorbing resin (hereinafter such a space will be referred to as "internal gas bubble"). For example, a water-absorbing resin, which has been obtained by foaming polymerization or has been subjected to a granulation step, has internal spaces (internal gas bubbles) that are not connected to the outside. Therefore, in a case where the density of a water-absorbing resin is measured by dry density measurement, introduced gas cannot enter the internal gas bubbles. This causes a measured density to be an apparent density which is obtained from the volume including those of the internal gas bubbles.

In the present invention, an internal gas bubble ratio is obtained based on the following Formula (7).

$$\text{Internal gas bubble ratio (\%)} = \{(\text{true density } (g/cm^3)) - (\text{apparent density } (g/cm^3))\} / (\text{true density } (g/cm^3)) \times 100 \qquad \text{Formula (7)}$$

[1-16] "Bulk specific gravity"

A bulk specific gravity refers to a specific gravity when a container having a certain volume capacity is filled with a powder and the volume capacity is regarded as a volume. The bulk specific gravity is measured by a measuring method described in Examples.

[1-17] "Water-absorbing sheet"

The term "water-absorbing sheet" as used in the present invention refers to a sheet in which a water-absorbing resin is supported between two or more long base materials and which has water absorption performance (particularly 5 g/g or more in terms of CRC). Preferably an adhesive (more preferably a hot melt adhesive) is used for fixing the two base materials to each other and for fixing the base materials and the water-absorbing resin to each other. Alternatively, it is possible that any component other than a water-absorbing resin and an adhesive (such as a fiber component, an antibacterial agent, and a deodorant agent) can be supported between the base materials. Water-absorbing sheets can be produced one by one so that each water-absorbing sheet has a size suited for it purpose (e.g., a size of a disposable diaper). However, water-absorbing sheets are typically in the form of long continuous sheets, and distributed water-absorbing sheets are wound in the form a roll or are folded. Such water-absorbing sheets are cut in rectangle shapes or the like so as to be used as absorbent bodies of hygienic materials such as a disposable diaper.

Note that, as described in the present specification, water-absorbing sheets may have alignment properties in some cases. For example, in some cases, particulate water-absorbing agents used in respective surfaces of a water-absorbing sheet are different from each other. Therefore, a long water-absorbing sheet may be configured so that a specific surface can be identified. Examples of a method of identifying a specific surface encompass marking at least one surface (e.g., a surface that comes into contact with a liquid before any other surface when the water-absorbing sheet is used).

Note that according to disposable diapers each including a conventional high-concentration water-absorbing resin (e.g., including only a water-absorbing resin without any pulp), absorbent bodies which are individually shaped for corresponding disposable diapers are processed so as to be supported by water-absorbing resins. Therefore, such an absorbent layer is not a water-absorbing sheet as used in the present invention (particularly not a long water-absorbing sheet to be cut).

[1-18] Other

In the present specification, any range of "X to Y" means "X or more and Y or less". Unless otherwise specified, the unit of mass "t (ton)" means "metric ton", and "ppm" means "ppm by mass". Further, "... acid (salt)" means "... acid and/or salt thereof", and "(meth)acrylic" means "acrylic and/or methacrylic."

For convenience, "liter" may be referred to as "l" or "L", and "mass%" may be referred to as "wt%". Furthermore, in a case where trace components are measured, values equal to or less than a detection limit is indicated as N.D. (Non Detected).

[2] Physical properties of particulate water-absorbing agent

An aspect of the present invention provides an absorbent article as defined in claim 1.

In this Section [2], preferable physical property values satisfied by the particulate water-absorbing agent included in the absorbent article according to the present invention as defined in claim 1, including those of the physical properties (1) through (3) as recited in claim 1, will be described.

Note that a particulate water-absorbing agent described in this Section [2] may be referred to as "water-absorbing agent for use in the present invention".

[2-1] DRC index

As an example of a method of producing a particulate water-absorbing agent having excellent physical properties, there is Patent Literature 40 (Japanese Patent Application, Tokugan, No. 2016-194921) which has not been published, and it was found that such a particulate water-absorbing agent can be obtained by increasing a gel-grinding energy during gel-crushing. Specifically, a particulate water-absorbing agent of each of Production Examples 1, 2, 6, and 7 of the present specification in which a large gel-grinding energy was used exhibited a DRC5min higher than that of a particulate water-absorbing agent of Comparative Production Example 1 of the present specification in which a small gel-grinding energy was used. It was also found that even in a case where a large gel-grinding energy was used in gel-crushing, a large mass average particle diameter (D50) led to a small DRC5min (see: comparison between the particulate water-absorbing agents (2) and (3), comparison between the water-absorbing agents (6), (8), and (10), and comparison between the water-absorbing agents (7) and (9) of Tables 1 and 2 of the present specification described later). Between D50 and DRC5min, linearity is observed. This tendency is also observed in Table 2 of the present specification, showing DRC5min measured according to particle size fractions.

The inventors of the present invention found a DRC index represented by the Formula (a), as an index for determining a particulate water-absorbing agent having physical properties preferable for a water-absorbing sheet. This DRC index makes it easy to determine a particulate water-absorbing agent having excellent physical properties.

A requirement of a DRC index equal to or less than a specific value in a water-absorbing sheet presumably has the following technical meaning. In the present invention, a particulate water-absorbing agent having both a high fluid retention capacity and a high water absorption speed is also intended to be applied to a water-absorbing sheet. The numerator in the Formula (a) is (49-DRC5min). That is, a larger DRC5min leads to a smaller DRC index. The term "DRC5min" means a fluid retention capacity (unit: g/g) without pressure for 5 minutes. Therefore, a larger DRC5min can be said to reflect a high water absorption speed. This is also true even in a case where the constant K of the general index of DRC is a numerical value other than 49.

Meanwhile, the denominator of the Formula (a) is (D50/1000). D50 is a mass average particle diameter (defined by sieve classification) of a particulate water-absorbing agent, and the unit of D50 is ordinarily $\mu$m. Therefore, (D50/1000), which is the denominator of the Formula (a), is synonymous with the mass average particle diameter indicated by the unit [mm]. For example, "mass average particle diameter of 400 $\mu$m" of a particulate water-absorbing agent corresponds to "mass average particle diameter of 0.400 mm". It was thus found that a larger D50 leads to a smaller DRC5min. This is presumably because, in a case of groups of particles differing only in particle diameters but identical in mass, a group having a smaller particle diameter has a larger total specific surface area per unit mass and therefore absorbs water quickly. For example, if a particulate water-absorbing agent has a form of a perfect sphere, then reducing the D50 and particle diameter (r) of the particulate water-absorbing agent to half of the original values leads a specific surface area ($4\pi r^2$) of the particulate water-absorbing agent to be 4 times as much. In DRC index, this effect is presumably offset by dividing [DRC (g/g)] (which is a 5-minute absorption value) by [D50 ($\mu$m) / 1000] (=mass average particle size (unit: mm)). In addition, particles having a large particle size have an effect of diffusing a liquid between the particles. This needs to be taken into consideration in addition to a water absorption speed. In view of all of such various effects of particle diameter, and in a case where a centrifuge retention capacity (CRC) is 30 g/g to 50 g/g and where a mass average particle diameter (D50) is 200 $\mu$m to 600 $\mu$m, physical properties of a particulate water-absorbing agent suitable for a water-absorbing sheet can be reflected by dividing DRC by D50 to normalize the DRC. Note that more preferable CRC and D50 are in the ranges discussed in [2-2] and [2-3] described later. Note also that although the DRC index is a dimensionless index, the unit of DRC index would be [g/g/mm]. This is because the DRC index is calculated based on a 5-minute absorption value per average particle size (mm).

The DRC index calculated by the Formula (a) as recited in claim 1 is necessarily 34 or less, and a lower DRC index is more preferable. According to a preferred embodiment, the DRC index calculated by the Formula (a) is preferably any of the following values listed in the order from least preferable to most preferable: 33 or less, 32 or less, 31 or less, 30 or less, 29 or less, 28 or less, 27 or less, 26 or less, 25 or less, 24 or less, 23 or less, 22 or less, 21 or less, 20 or less, 19 or less, 18 or less, 17 or less, 16 or less, 15 or less, 14 or less, 13 or less, 12 or less, 11 or less, 10 or less, 9 or less, 8 or less, 7 or less, 6 or less, 5 or less, 4 or less, 3 or less, 2 or less, 1 or less, 0 or less, -10 or less, -20 or less, and -40 or less. The numerical value of the DRC index can be likewise calculated also in a case where K is other than 49. Although a lower limit value is preferably as low as possible, the lower limit value is preferably approximately -80 (negative 80), and more preferably approximately -60 (negative 60), from a viewpoint of a balance with other physical properties. Although a preferable range between the upper limit value and the lower limit value of the DRC index can be selected as appropriate from the ranges above, the preferable range is any of the following ranges listed in the order from least preferable to most preferable: -10 to 34, and 0 to 31.

A particulate water-absorbing agent defined by DRC5min (g/g), DRC index, or the like is suitable for a water-absorbing sheet. Such a particulate water-absorbing agent allows an absorbent article (i.e., sanitary product such as a disposable diaper in which a water-absorbing sheet is used) in actual use to have a reduced re-wet and an improved water absorption speed (liquid absorption speed). The DRC5min evaluates absorption performance for such a short period as 5 minutes, and is a method of properly evaluating water absorption speed of a water-absorbing sheet. The DRC5min therefore evaluates characteristics which cannot be properly evaluated by a conventionally known fluid retention capacity under pressure (AAP) for evaluating absorption performance of a saturated particulate water-absorbing sheet for 1 hour or by conventionally known FHA disclosed in U.S. Patent No. 7108916.

The GCA disclose in Patent Literature 4 (International Publication, No. 2015/129917) evaluates a liquid absorbing ability "under pressure" during a 10-minute period during which there is a height difference of 10 cm between an upper surface of a glass filter and a meniscus at a lower part of a Marriott tube. In contrast, the DRC5min is a parameter for evaluating absorption performance "without pressure" during such an even shorter period as 5 minutes. That is, the GCA and the DRC5min are different in measurement conditions, and are therefore parameters which cannot be used for an analogy therebetween. In addition, the DRC5min properly evaluates the performance of a particulate water-absorbing agent in a disposable diaper to absorb urine from pulp, and ultimately evaluates the performance to prevent rash or urine leakage.

[2-2] Centrifuge retention capacity (CRC)

The particulate water-absorbing agent for use in the present invention has a centrifuge retention capacity (CRC) of 30 g/g to 50 g/g. A lower limit value of CRC is preferably 31 g/g, more preferably 32 g/g, even more preferably 33 g/g, even more preferably 34 g/g, even more preferably 35 g/g, and most preferably 36 g/g. Meanwhile, an upper limit value of CRC is preferably 49 g/g, more preferably 48 g/g, even more preferably 47 g/g, even more preferably 46 g/g, even more preferably 45 g/g, even more preferably 44 g/g, even more preferably 43 g/g, even more preferably 42 g/g, even more preferably 41 g/g, even more preferably 40 g/g, even more preferably 39 g/g, and most preferably 38 g/g. Note that a combination of the upper limit value and the lower limit values can be selected as appropriate. For example, 30 g/g to 38 g/g, 36 g/g to 50 g/g, or 32 g/g to 42 g/g can be selected.

If the CRC is less than 30 g/g, then an absorption amount is small. This renders a particulate water-absorbing agent unsuitable as an absorbent body of a sanitary material such as a disposable diaper. If the CRC is more than 50 g/g, then a rate at which, for example, a body fluid such as urine or blood is absorbed decreases. This renders a particulate water-absorbing agent unsuitable for use in, for example, a disposable diaper having a high water absorption speed. Note that CRC can be controlled with use of, for example, an internal crosslinking agent and/or a surface-crosslinking agent.

[2-3] Particle size (particle size distribution, mass average particle diameter (D50), and logarithmic standard deviation ($\sigma\zeta$) of particle size distribution)

The particulate water-absorbing agent for use in the present invention has a mass average particle diameter (D50) of 200 $\mu$m to 600 pm. The mass average particle diameter (D50) is more preferably 200 pm to 550 pm, even more preferably 250 pm to 500 pm, and still more preferably 350 pm to 450 pm. The particulate water-absorbing agent contains particles with a particle diameter of less than 150 pm at a proportion of preferably 10 mass% or less, more preferably 5 mass% or less, even more preferably 1 mass% or less, and contains particles with a particle diameter of 850 pm or more at a proportion of preferably 5 mass% or less, more preferably 3 mass% or less, and even more preferably 1 mass% or less. A lower limit value of each of the proportions of such particles is preferably as low as possible and is desirably 0 mass%. Note, however, that a lower limit of each of the proportions of such particles can be approximately 0.1 mass%. The particulate water-absorbing agent has a logarithmic standard deviation ($\sigma\zeta$) of a particle size distribution which falls in a range of preferably 0.20 to 0.50, more preferably 0.25 to 0.40, and still more preferably 0.27 to 0.35. Note that these particle sizes are measured with use of a standard sieve in conformity with a measuring method disclosed in U.S. Patent No. 7638570 or EDANA ERT 420.2-02.

[2-4] Surface tension

The particulate water-absorbing agent for use in the present invention has a surface tension of preferably 65 mN/m or more, more preferably 66 mN/m or more, even more preferably 67 mN/m or more, even more preferably 69 mN/m or more, even more preferably 70 mN/m or more, even more preferably 71 mN/m or more, and most preferably 72 mN/m or more. In application of the particulate water-absorbing agent to a water-absorbing sheet, the effects of surface tension are more evident than in the case of conventional disposable diapers. Satisfying the above conditions of surface tension allows for a reduction in re-wet of a disposable diaper.

[2-5] Particle shape

The particle shape of the particulate water-absorbing agent for use in the present invention is preferably a non-uniformly pulverized shape. This is because: a particulate water-absorbing agent having a non-uniformly pulverized shape has a specific surface area larger than that of spherical particles obtained by a reversed phase suspension polymerization or a vapor phase polymerization so that the particulate water-absorbing agent has higher water absorption speed; and a particulate water-absorbing agent having a non-uniformly pulverized shape can be more easily fixed to a base material than in the case of spherical particles.

[2-6] Initial YI value

The particulate water-absorbing agent for use in the present invention has a YI value of preferably 0 to 17, more preferably 0 to 16, still more preferably 0 to 15, and most preferably 0 to 14.
According to a water-absorbing sheet for use in accordance with the present invention, color of a particulate water-absorbing agent is easily reflected on color of the entirety of the water-absorbing sheet. Therefore, causing the particulate water-absorbing agent to satisfy the YI values above makes it possible, when the particulate water-absorbing agent is used in combination with a hygienic material, to produce a disposable diaper which does not cause a user to have a feeling of a foreign body due to coloration. For measurement of color, the method disclosed in International Publication, No. 2009/005114 (method of measuring Lab value, YI value, WB value, and the like) can be used.

[2-7] YI value after coloration test

After a colorations acceleration test (maintained for 1 week in an atmosphere of 70°C and 65 RH%), the particulate water-absorbing agent for use in the present invention has a YI value of preferably 0 to 35, more preferably 0 to 30, even more preferably 0 to 25, and most preferably 0 to 22.
In a case where the particulate water-absorbing agent satisfies any of the above YI values after the coloration test, it is possible to prevent a user from having a feeling of a foreign body due to the coloration of a particulate water-absorbing agent. This is true even after a water-absorbing sheet is produced, stocked, distributed, and even after purchased and stored by a consumer.

[2-8] Moisture absorption fluidity (B.R.)

The particulate water-absorbing agent for use in the present invention has a moisture absorption fluidity (B.R.) of preferably 50 mass% or less, more preferably 30 mass% or less, even more preferably 20 mass% or less, still more preferably 10 mass% or less, and most preferably 0 mass%. In a case where a particulate water-absorbing agent satisfies any of the above moisture absorption fluidities (B.R.), it is possible to decrease the adhesion of the particulate water-absorbing agent to equipment. If the moisture absorption fluidity (B.R.) is more than 50 mass%, then the particulate water absorbing agent is difficult to handle in humid conditions. This may pose a problem that, during production of a thin absorbent body for hygienic material, for example, the particulate water-absorbing agent aggregates in a transport pipe in a production plant and therefore the transport pipe clogs and/or the particulate water-absorbing agent cannot be uniformly mixed with hydrophilic fibers.

[2-9] Water-soluble content (Ext)

The particulate water-absorbing agent for use in the present invention has a water-soluble content (Ext) of preferably 25 mass% or less, more preferably 24 mass% or less, even more preferably 22 mass% or less, and most preferably 20 mass% or less. In a case where the particulate water-absorbing agent satisfies any of the above water-soluble contents (Ext), an absorbing ability (e.g., fluid retention capacity under pressure) of the particulate water-absorbing agent improves. Therefore, in a case where the particulate water-absorbing agent is used in a disposable diaper, performance can be improved (such as a reduction in re-wet). Although a lower limit value of the water-soluble content (Ext) is preferably 0 mass%, the lower limit value is preferably 2 mass%, more preferably 5 mass%, and even more preferably 10 mass%, from the viewpoint of a balance with a fluid retention capacity (such as CRC or DRC). Therefore, examples of a preferable range of the water-soluble content (Ext) encompass 2 mass% to 24 mass%, 5 mass% to 22 mass%, and 10 mass% to 20 mass%.

[2-10] Degradable soluble content

The particulate water-absorbing agent for use in the present invention has a degradable soluble content of preferably 30 mass% or less, more preferably 27 mass% or less, even more preferably 24 mass% or less, and most preferably 20 mass% or less. In a case where the particulate water-absorbing agent satisfies any of the above degradable soluble contents, urine resistance improves. Therefore, in a case where the particulate water-absorbing agent is used in a disposable diaper, problems caused by a body fluid such as urine can be prevented, such as gel deterioration, skin irritation, rash, and a decrease in odor-removing ability. Although a lower limit value of the degradable soluble content is preferably 0 mass%, the lower limit value is preferably 2 mass%, more preferably 5 mass%, and even more preferably 10 mass%, from the viewpoint of a balance with a fluid retention capacity (such as CRC or DRC). Therefore, examples of a preferable range of the degradable soluble content encompass 2 mass% to 27 mass%, 5 mass% to 24 mass%, and 10 mass% to 20 mass%.

[2-11] Fluid retention capacity under pressure (AAP 2.06 kPa) and fluid retention capacity under pressure (AAP 4.83 kPa)

The particulate water-absorbing agent for use in the present invention has a fluid retention capacity under pressure (AAP 2.06 kPa) of preferably 18 g/g or more, more preferably 22 g/g or more, even more preferably 24 g/g or more, even more preferably 26 g/g or more, even more preferably 28 g/g or more, and most preferably 30 g/g or more. An upper limit value of the fluid retention capacity under pressure (AAP 2.06 kPa) is preferably 40 g/g.

In a case where the particulate water-absorbing agent satisfies any of the above fluid retention capacities under pressure (AAP 2.06 kPa), a disposable diaper produced with the particulate water-absorbing agent can have a reduced re-wet. This makes it possible to prevent rash and urine leakage. If the AAP is less than 18 g/g, then the re-wet of a liquid when a pressure is applied to an absorbent body becomes large. This means that such a particulate water-absorbing agent is unsuitable as an absorbent body of a sanitary material such as a disposable diaper. Note that AAP can be controlled with use of particle size, surface-crosslinking agent, or the like.

Meanwhile, the fluid retention capacity under pressure (AAP 4.83 kPa) which is a measure under a heavy load is preferably 10 g/g or more, more preferably 15 g/g or more, and even more preferably 18 g/g or more. An upper limit value of the fluid retention capacity under pressure (AAP 4.83 kPa) is 40 g/g as in the case of the fluid retention capacity under pressure (AAP 2.06 kPa).

[2-12] Saline flow conductivity (SFC)

The particulate water-absorbing agent for use in the present invention has a saline flow conductivity (SFC) of preferably less than 30 ($\times 10^{-7} \cdot cm^3 \cdot s \cdot g^{-1}$), more preferably less than 25 ($\times 10^{-7} \cdot cm^3 \cdot s \cdot g^{-1}$), even more preferably less than 20 ($\times 10^{-7} \cdot cm^3 \cdot s \cdot g^{-1}$), and most preferably less than 15 ($\times 10^{-7} \cdot cm^3 \cdot s \cdot g^{-1}$).

[2-13] Free swelling GBP (F-GBP)

A free swelling GBP (F-GBP) is measured in conformity with Patent Literature 9 (International Publication, No. 2004/096304). The free swelling GBP can be measured after particle size cut is carried out (e.g., GBP of particles having a particle size of 300 $\mu$m to 600 $\mu$m, out of all of the particles, is measured). Alternatively, it is possible to omit particle size cut so that GBP of the particulate water-absorbing agent in its entirety is measured.

[2-14] Diffusing absorbency under pressure (DAP)

The particulate water-absorbing agent for use in the present invention has a diffusing absorbency under pressure (DAP) of preferably 16 g/g or more, more preferably 18 g/g or more, even more preferably 20 g/g or more, even more preferably 22 g/g or more, even more preferably 24 g/g or more, even more preferably 26 g/g or more, even more preferably 28 g/g or more, even more preferably 30 g/g or more, even more preferably 31 g/g or more, and most preferably 32 g/g or more. The diffusing absorbency under pressure (DAP) is preferably as high as possible. However, from the viewpoint of a balance with other physical properties and production cost, an upper limit value of the diffusing absorbency under pressure (DAP) is preferably 40 g/g.

Unlike the AAP (vertical diffusion of 0.9 g of particulate water-absorbing agent), the diffusing absorbency under pressure (DAP) evaluates an absorption performance under pressure (e.g., 2.06 kPa) with use of a horizontal diffusion at such a high concentration as 1.5 g of particulate water-absorbing agent. Therefore, a water-absorbing sheet, for which diffusion and absorption of a liquid (urine) in a planar direction are of greater importance, preferably has a diffusing absorbency falling within the above ranges. The diffusing absorbency is measured in conformity with Patent Literature 8 (European Patent Application Publication No. 0712659).

[2-15] Gel capillary absorption (GCA)

The particulate water-absorbing agent for use in the present invention has a gel capillary absorption (GCA) of preferably 27 g/g or more, more preferably 28 g/g or more, even more preferably 29 g/g or more, even more preferably 30 g/g or more, and most preferably 31 g/g or more. In a case where the particulate water-absorbing agent satisfies any of the above GCA, a disposable diaper produced with the particulate water-absorbing agent can have a reduced re-wet. This makes it possible to prevent rash and urine leakage. GCA can be defined by the method disclosed in Patent Literature 4 (International Publication, No. 2015/129917) or Patent Literature 5 (International Publication, No. 2016/204302).

[2-16] Internal gas bubble ratio

The particulate water-absorbing agent for use in the present invention has an internal gas bubble ratio of preferably 0.5% to 2.5%, more preferably 0.8% to 2.3%, and even more preferably 1.0% to 2.0%.
While it is not desirable to be bound by a theory, an internal gas bubble ratio falling within the ranges above makes it possible to achieve both a liquid permeability and a water absorption speed of a particulate water-absorbing agent. Although the internal gas bubble ratio can be controlled by, for example, gel-grinding energy in the production method of the present disclosure or an amount of increase in molecular weight of water-soluble content, it is alternatively possible to use (in combination), for example, foaming polymerization or foam during drying to control the internal gas bubble ratio.

[2-17] Increase in fine powder

An amount of increase in the particulate water-absorbing agent for use in the present invention means an amount of increase in particles having a particle diameter (defined by a standard sieve) of 150 $\mu$m or less during a damage resistance paint shaker test (damage test) described in Examples. The increase in fine powder, which is [amount (mass%) of particles of water-absorbing agent having passed through a 150-pm mesh after damage test - amount (mass%) of particles of water-absorbing agent having passed through a 150-$\mu$m mesh before damage test], is +5 mass% or less, preferably +4 mass% or less, more preferably +3 mass% or less, even more preferably +2 mass% or less, and still more preferably +1 mass% or less.

[2-18] Bulk specific gravity

The particulate water-absorbing agent for use in the present invention has a bulk specific gravity of preferably 0.57 g/cm$^3$ to 0.75 g/cm$^3$, more preferably 0.58 g/cm$^3$ to 0.74 g/cm$^3$, even more preferably 0.59 g/cm$^3$ to 0.73 g/cm$^3$, and most preferably 0.60 g/cm$^3$ to 0.72 g/cm$^3$. The bulk specific gravity can be measured by the method (e.g., ERT 460.2-02) defined by EDANA.

[2-19] Polymer structure of water-absorbing resin

The water-absorbing resin, which is a main component of the particulate water-absorbing agent for use in the present invention, is preferably a polyacrylic acid (salt)-based water-absorbing resin, from the viewpoint of physical properties and productivity of the particulate water-absorbing agent to be obtained.

[2-20] Preferable additive of particulate water-absorbing agent

As necessary, the particulate water-absorbing agent for use in the present invention can optionally contain at least one additive selected from the group consisting of (a) a polyvalent metal salt, (b) a cationic polymer, (c) a chelating agent, (d) an inorganic reducing agent, (e) an $\alpha$-hydroxycarboxylic acid compound, and (f) a moisture absorption fluidity improving agent. Among these additives, the particulate water-absorbing agent contains preferably at least one additive, more preferably at least two additives, and particularly preferably (c) a chelating agent and/or (f) a moisture absorption fluidity improving agent.
Respective amounts of additives used (added) are decided as appropriate according to a purpose of the additive, and is therefore not particularly limited. The respective amounts are each preferably not more than 3 parts by mass, more preferably not more than 1 part by mass, relative to 100 parts by mass of a water-absorbing resin powder.

(a) Polyvalent metal salt and/or (b) cationic polymer

In the present invention, the particulate water-absorbing agent preferably contains a polyvalent metal salt and/or a cationic polymer, from the viewpoint of improvement in water absorption speed, liquid permeability, moisture absorption fluidity, and the like of the particulate water-absorbing agent used in a water-absorbing sheet.

As the polyvalent metal salt and/or the cationic polymer, a compound and an amount used thereof disclosed in "[7] Polyvalent metal salt and/or cationic polymer" of International Publication, No. 2011/040530 can be applied to the present invention.

(c) Chelating agent

In order to achieve a preferable degradable soluble content and preferable coloration in the present invention, the particulate water-absorbing agent preferably contains a chelating agent from the viewpoint of, for example, prevention of deterioration.

Specifically, as the chelating agent, a compound and an amount used thereof disclosed in "[2] Chelating agent" of International Publication No. 2011/040530 can be applied to the present invention.

According to a preferred embodiment, the chelating agent is selected from the group consisting of iminodiacetic acid, hydroxyethyl iminodiacetic acid, nitrilotriacetic acid, nitrilotri propionic acid, ethylenediaminetetraacetic acid, hydroxy ethylenediamine triacetic acid, hexamethylenediamine tetraacetic acid, diethylenetriamine pentaacetic acid (DTPA), triethylenetetramine hexaacetic acid, trans-1,2-diaminocyclohexane tetraacetic acid, bis(2-hydroxyethyl)glycine, diaminopropanol tetraacetic acid, ethylenediamine-2-propionic acid, glycol ether diaminetetraacetic acid, bis(2-hydroxybenzyl)ethylenediamine diacetic acid, 3-hydroxy-2,2-iminodisuccinic acid, iminodisuccinic acid, methylglycine diacetic acid, ethylenediamine-N,N'-di(methylene phosphinic acid), ethylenediaminetetra(methylene phosphinic acid), nitriloacetic acid-di(methylene phosphinic acid), nitrilodiacetic acid-(methylene phosphinic acid), nitriloacetic acid-P-proprionic acid-methylene phosphonate, nitrilotris(methylene phosphonate), cyclohexanediaminetetra(methylene phosphonate), ethylenediamine-N,N'-diacetic acid-N,N'-di(methylene phosphonate), ethylenediamine-N,N'-di(methylene phosphonate), polymethylenediaminetetra(methylene phosphonate), diethylenetriaminepenta(methylene phosphonate), and 1-hydroxyethylidenediphosphonic acid. Among these, aminocarboxylic acid (salt) is preferable, and diethylenetriamine pentaacetic acid (DTPA) (salt) is particularly preferable. Adding any of the chelating agents above to a particulate water-absorbing agent allows for an improvement in urine resistance of the particulate water-absorbing agent.

(d) Inorganic reducing agent

In order to achieve a preferable degradable soluble content and preferable coloration in the present invention, the particulate water-absorbing agent preferably contains an inorganic reducing agent from the viewpoint of, for example, color (coloration prevention), deterioration prevention, and reduction in residual monomers in the water-absorbing resin to be obtained.

Specifically, as the inorganic reducing agent, a compound and an amount used thereof disclosed in "[3] Inorganic reducing agent" of International Publication No. 2011/040530 can be applied to the present invention.

(e) $\alpha$-hydroxycarboxylic acid compound

In order to achieve a preferable degradable soluble content and preferable coloration in the present invention, $\alpha$-hydroxycarboxylic acid such as lactic acid or citric acid is preferably added. Note that the "a-hydroxycarboxylic acid compound" is a carboxylic acid having a hydroxyl group in a molecule or is a salt thereof, and is a hydroxycarboxylic acid having a hydroxyl group at an alpha position.

Specifically, as the $\alpha$-hydroxycarboxylic acid compound, a compound and an amount used thereof disclosed in "[6] $\alpha$-hydroxycarboxylic acid compound" of International Publication No. 2011/040530 can be applied to the present invention.

(f) Fluidity improving agent

A production method preferably further includes the step of adding a moisture absorption fluidity improving agent in an amount of 0.01 parts by mass to 1.0 part by mass, preferably 0.02 parts by mass to 0.7 parts by mass, and even more preferably 0.03 parts by mass to 0.5 parts by mass, relative to 100 parts by mass of the particulate water-absorbing agent or the water-absorbing resin. In a case where the conditions above are satisfied, the moisture absorption fluidity of the particulate water-absorbing agent is improved. This makes it possible to decrease the adhesion of the particulate water-absorbing agent to equipment when an absorbent body is produced with use of the particulate water-absorbing agent and a fiber material.

The moisture absorption fluidity improving agent is preferably selected from the group consisting of silicon dioxide, hydrotalcite, phosphoric acid, and aluminum salt. Adding the moisture absorption fluidity improving agent improves the moisture absorption fluidity of the particulate water-absorbing agent. This makes it possible to decrease the adhesion of the particulate water-absorbing agent to equipment when an absorbent body is produced with use of the particulate water-absorbing agent and a fiber material.

(f-1) Multicomponent metal compound having hydrotalcite structure and containing divalent and trivalent metal cations (two kinds of metal cations) and a hydroxyl group

In the present invention, the particulate water-absorbing agent can contain, as a moisture absorption fluidity improving agent, a multicomponent metal compound having a hydrotalcite structure and containing divalent and trivalent metal cations (two kinds of metal cations) and a hydroxyl group. The multicomponent metal compound for use in the present invention leads to a decrease in water absorption performance such as AAP of the water-absorbing agent to be small. In addition, the multicomponent metal compound has a function of preventing moisture adsorption blocking.

The multicomponent metal compound for use in the present invention has a hydrotalcite-like structure which is represented by the following general formula and which is known as a structure of a layered compound:

$$[M_1{}^{2+}{}_{1-x}M_2{}^{3+}{}_x(OH^-)_2]^{x+}\cdot[(A^{n-})_{x/n}\cdot mH_2O]^{x-}$$

Where $M_1{}^{2+}$ represents a divalent metal cation, $M_2{}^{3+}$ represents a trivalent metal cation, $A^{n-}$ represents a n-valent anion, and $H_2O$ represents water.

Examples of the divalent metal cation encompass $Mg^{2+}$, $Fe^{2+}$, $Zn^{2+}$, $Ca^{2+}$, $Ni^{2+}$, $Co^{2+}$, and $Cu^{2+}$, and, from the viewpoint of heat resistance and the like, $Mg^{2+}$ is preferable. Examples of the trivalent metal cation encompass $Al^{3+}$, $Fe^{3+}$, and $Mn^{3+}$, and, from the viewpoint of heat resistance and the like, $Al^{3+}$ is preferable. Therefore, in a preferred embodiment of the multicomponent metal compound, a divalent metal cation is a magnesium cation, and a trivalent metal cation is an aluminum cation.

In regard to the proportion of the divalent metal cation and the trivalent metal cation in the general formula, x is preferably 0.2 to 0.75, more preferably 0.25 to 0.7, and even more preferably 0.25 to 0.5. Examples of the anion encompass $OH^-$, $F^-$, $Cl^-$, $Br^-$, $NO_3{}^-$, $CO_3{}^{2-}$, $SO_4{}^{2-}$, $Fe(CN)_6{}^{3-}$, $CH_3COO^-$, oxalate ion, and salicylate ion, and a preferable anion is carbonate anion. Furthermore, m is a real number greater than 0. It is preferable that $0 < m \leq 10$.

The multicomponent metal compound is not limited to any particular form, and can have a spherical form (including a powder form). The multicomponent metal compound can have a specific particle size. A volume average particle diameter can be 2 $\mu$m or less, 1.5 $\mu$m or less, or 1 $\mu$m or less. If the particle diameter is large, it is then necessary to add a large amount of the multicomponent metal compound in order to sufficiently obtain a dust reduction effect. This may impair the water absorption performance of a water-absorbing agent to be obtained. If the particle diameter is excessively small, then workability may decrease during the step of adding the multicomponent metal compound, and/or it may be impossible to obtain sufficient performance. Therefore, the volume average particle diameter can be 0.05 $\mu$m or more, 0.1 $\mu$m or more, or 0.3 $\mu$m or more. Note that the volume average particle diameter of the multicomponent metal compound can be measured by a "laser diffraction scattering method" (for example, measured using a particle size analyzer Microtrac MT3000II (product name) manufactured by NIKKISO CO., LTD.). The average particle diameter of the multicomponent metal compound adhering to the surface of a water-absorbing resin can be measured by a measuring method in which a scanning electron microscope (SEM) is used. This method will be described in Examples.

Furthermore, the multicomponent metal compound can further have an organic compound intercalated between layers thereof and/or can be surface-treated so that the mixability with a resin or the like improves.

Examples of a preferable structural formula of the multicomponent metal compound encompass $Mg_6Al_2(OH)_{16}CO_3\cdot4H_2O$, $Mg_4Al_2(OH)_{12}CO_3\cdot3H_2O$, $Mg_{4.5}Al_2(OH)_{13}CO_3\cdot3.5H_2O$. Specifically, examples of the multicomponent metal compound encompass DHT-4H and DHT-6 manufactured by Kyowa Chemical Industry Co., Ltd., STABIACE HT-1-NC and STABIACE HT-P manufactured by SAKAI CHEMICAL INDUSTRY CO., LTD.

Although a hydrotalcite compound (HT compound) may or may not be surface-treated, the HT compound is more preferably not surface-treated. Specific examples of a surface-treating agent for use in the surface treatment encompass the following (a) through (j).

(a) Higher fatty acids such as stearic acid, oleic acid, erucic acid, palmitic acid, and lauric acid.
(b) Metal salts such as lithium salt, sodium salt, and potassium salt of the higher fatty acids (a) above.
(c) Anionic surfactants such as sulfate ester salts of higher alcohols (e.g., stearyl alcohol and oleyl alcohol), sulfate ester salts of polyethylene glycol ether, amide-bound sulfate ester salt, ether-bound sulfonate, ester-bound sulfonate, amide-bound alkyl aryl sulfonate, and ether-bound alkyl aryl sulfonate.

(d) Phosphate esters such as: monoesters and diesters resulting from reaction between orthophosphoric acid and oleyl alcohol, stearyl alcohol, or the like; and mixtures of such monoesters and diesters which can be acid types, alkali metal salts, or amine salts.

(e) Silane coupling agents such as vinylethoxysilane, $\gamma$-methacryloxypropyl trimethoxy silane, vinyltris(2-methoxyethoxy) silane, and $\gamma$-aminopropyl trimethoxysilane.

(f) Titanium coupling agents such as isopropyltriisostearoyl titanate, isopropyltris(dioctylpyrophosphate)titanate, and isopropyl tridecyl benzenesulfonyl titanate.

(g) Alkali coupling agents such as acetoalkoxyaluminum diisopropylate.

(h) Ethanolamines such as monoethanolamine, diethanolamine, and triethanolamine.

(i) n-propanolamines such as n-propanolamine, di-n-propanolamine, and tri-n-propanolamine.

(j) Isopropanolamines such as monoisopropanolamine, diisopropanolamine, and triisopropanolamine.

Among these, ethanolamines such as monoethanolamine, diethanolamine, and triethanolamine are preferable.

(Amount of multicomponent metal compound added)

An amount of the multicomponent metal compound added is preferably 0.01 parts by mass to 5 parts by mass, more preferably 0.01 parts by mass to 4.5 parts by mass, even more preferably 0.1 parts by mass to 4.5 parts by mass, still more preferably 0.1 parts by mass to 4 parts by mass, and particularly preferably 0.15 parts by mass to 3.5 parts by mass, relative to 100 parts by mass of a polyacrylic acid (salt)-based water-absorbing resin powder.

Therefore, while the final amount of the multicomponent metal compound contained in the absorbing agent for use in the present invention is defined by the above ranges, the amount of the multicomponent metal compound contained in the absorbing agent is substantially 0.01 parts by mass to 5 parts by mass because the amount of the multicomponent metal compound added is small relative to the mass of the water-absorbing agent.

In order to sufficiently prevent moisture adsorption blocking only by addition of the multicomponent metal compound, the amount of the multicomponent metal compound to add is preferably not less than 0.1 parts by mass, and more preferably not less than 0.2 parts by mass. From the viewpoint of water absorption performance, the amount is preferably not more than 1 part by mass, more preferably not more than 0.8 parts by mass, even more preferably not more than 0.6 parts by mass, and particularly preferably not more than 0.4 parts by mass.

(f-2) Water-insoluble metal phosphate containing phosphate anion and divalent or trivalent metal cation

In the present invention, the particulate water-absorbing agent can contain, as a moisture absorption fluidity improving agent, a water-insoluble metal phosphate containing phosphate anions and divalent or trivalent metal cations. The water-insoluble metal phosphate for use in the present invention leads to a decrease in water absorption performance such as AAP of the water-absorbing agent to be small. In addition, the multicomponent metal compound has a function of preventing moisture adsorption blocking.

The water-insoluble metal phosphate used in the present invention contains phosphate anions and divalent or trivalent metal cations. Examples of the phosphate anion encompass phosphoric acid ion, pyrophosphoric acid ion, tripolyphosphoric acid ion, hexapolyphosphoric acid ion, pentapolyphosphoric acid ion, heptapolyphosphoric acid ion, trimetaphosphoric acid ion, tetramethphosphoric acid ion, hexametaphosphoric acid ion, dihydrogenphosphate ion, and hydrogenphosphate ion. Examples of the divalent or trivalent metal cation encompass calcium ion, magnesium ion, strontium ion, barium ion, zinc ion, iron ion, aluminum ion, titanium ion, zirconium ion, hafnium ion, tin ion, cerium ion, scandium ion, yttrium ion, and lanthanum ion. In particular, the divalent or trivalent metal cation can be calcium ion or aluminum ion, or can be calcium ion. Specific examples of a salt of calcium encompass monocalcium phosphate, calcium monohydrogen phosphate, dicalcium phosphate, tricalcium phosphate, hydroxyapatite, calcium pyrophosphate, and calcium dihydrogen pyrophosphate. One of these can be used alone, or two or more of these can be used in combination. Tricalcium phosphate can be used alone. Note that the term "water-insoluble" means that a substance is dissolved in an amount of less than 1 g, relative to 100 g of water at 25°C.

The water-insoluble metal phosphate used in the present invention has a crystallite diameter whose an upper limit value can be less than 0.15 $\mu$m, less than 0.13 $\mu$m, or less than 0.1 $\mu$m. A lower limit value of the crystallite diameter can be, although not particularly limited, 0.005 $\mu$m or more, or 0.01 $\mu$m or more from the viewpoint of workability during the step of adding the water-insoluble metal phosphate. Therefore, the upper limit value and the lower limit value of the crystallite diameter can be selected as appropriate from the ranges above. For example, the range between the upper limit value and the lower limit value can be, 0.005 $\mu$m or more but less than 0.15 $\mu$m, 0.01 $\mu$m or more but less than 0.15 $\mu$m, or 0.01 $\mu$m or more but less than 0.1 $\mu$m.

In the present invention, while the crystallite diameter of the water-insoluble metal phosphate contained in a particulate water-absorbing agent (end product) can satisfy the ranges above, the crystallite diameter of the water-insoluble metal phosphate before being added to the water-absorbing resin powder can satisfy the ranges above.

A method of controlling the crystallite diameter of the water-insoluble metal phosphate is not limited to any particular one, but can be any publicly known method. Furthermore, the water-insoluble metal phosphate can be a commercially available water-insoluble metal phosphate.

Note that the crystallite diameter of the water-insoluble metal phosphate can be measured by X-ray diffraction (XRD) described in Examples.

The water-insoluble metal phosphate used in the present invention has an average primary particle diameter whose an upper limit value can be less than 2.0 $\mu$m, less than 1.5 $\mu$m, or less than 1.0 $\mu$m. In a case where the average primary particle diameter is less than 2.0 $\mu$m, makes it possible to further reduce the moisture adsorption blocking property. A lower limit value of the average primary particle diameter can be, although not particularly limited, 0.005 $\mu$m or more, or 0.01 $\mu$m or more from the viewpoint of workability during the step of adding the water-insoluble metal phosphate. Therefore, the upper limit value and the lower limit value of the average primary particle diameter can be selected as appropriate from the ranges above. For example, the range between the upper limit value and the lower limit value can be, 0.005 $\mu$m or more but less than 2.0 $\mu$m, 0.01 $\mu$m or more but less than 1.5 $\mu$m, or 0.01 $\mu$m or more but less than 1.0 $\mu$m.

An amount of the water-insoluble metal phosphate added is preferably 0.01 parts by mass to 2 parts by mass, more preferably 0.01 parts by mass to 1 part by mass, even more preferably not less than 0.01 parts by mass but less than 1 part by mass, particularly preferably 0.05 parts by mass to 0.7 parts by mass, and most preferably 0.08 parts by mass to 0.6 parts by mass, relative to 100 parts by mass of the water-absorbing resin powder. In a case where the water-insoluble metal phosphate is added in an amount of not less than 0.01 parts by mass, a sufficient moisture adsorption blocking performance is obtained. In a case where the water-insoluble metal phosphate is added in an amount of not more than 2 parts by mass, the water absorption performance can be sufficiently maintained. The water-insoluble metal phosphate is preferably not added in an amount of more than 2 parts by mass because adding the water-insoluble metal phosphate in such an amount leads to an increase in cost for an increased amount of water-insoluble metal phosphate added, although a moisture adsorption blocking performance can be obtained.

Note that in an embodiment of the present invention, characteristically, the average primary particle diameter of the water-insoluble metal phosphate before the water-insoluble metal phosphate is added to a water-absorbing resin powder satisfies the ranges above.

[3] Water-absorbing sheet

In the present invention, a water-absorbing sheet is configured so that a particulate water-absorbing agent containing a water-absorbing resin as a main component is supported between two base materials including at least one water-permeable base material. The water-absorbing resin described in Section [2] above is used as at least part of the particulate water-absorbing agent sandwiched in the water-absorbing sheet. In this way, the object of the present invention is attained.

The following description will discuss the water-absorbing sheet containing the particulate water-absorbing agent.

[3-1] At least one water-permeable base material

Base materials used in the present invention can have any shape selected as appropriate and can be made of any material selected as appropriate, provided that: the base materials can be fixed so as to sandwich the particulate water-absorbing agent; and at least one of the base materials is water-permeable. Examples of the base materials encompass papers (sanitary papers such as tissue paper, toilet paper, and paper towel), a net, a nonwoven fabric, a fabric, and a film. Among these base materials, a nonwoven fabric is preferable. A water-permeable surface (surface made of a water-permeable base material) of a water-absorbing sheet is ordinarily used on a side where a liquid is discharged (e.g., a side closer to the skin in the case of a disposable diaper). The base material can have a pore(s) and/or a slit(s) for water-permeability, or can be subjected to a hydrophilization treatment. Base materials for an outermost layer and for an intermediate layer in the water-absorbing sheet for use in the present invention can be an identical (identical nonwoven fabrics) or can be different base materials. The water-permeable base material has a permeability coefficient (JIS A1218) of preferably $1 \times 10^{-5}$ cm/sec or more. The permeability coefficient is ordinarily $1 \times 10^{-5}$ cm/sec or more, preferably $1 \times 10^{-4}$ cm/sec or more, more preferably $1 \times 10^{-3}$ cm/sec or more, even more preferably $1 \times 10^{-2}$ cm/sec or more, and most preferably $1 \times 10^{-1}$ cm/sec or more.

In addition, a base material having a tensile strength of 20 kgf/5cm (200N/5cm) or more, preferably 30 kgf/5cm (300N/5cm) or more, and more preferably 50 kgf/5cm (500N/5cm) or more, is used on at least one surface (preferably on both surfaces).

The base material to be used has a thickness which is preferably as thin as possible, provided that the strength of a water-absorbing sheet is secured. The thickness per base material is selected as appropriate from the following ranges:

0.01 mm to 2 mm, 0.02 mm to 1 mm, 0.03 mm to 0.6 mm, and 0.05 mm to 0.5 mm. A mass per unit area per base material is preferably 5 g/m$^2$ to 300 g/m$^2$, more preferably 8 g/m$^2$ to 200 g/m$^2$, even more preferably 10 g/m$^2$ to 100 g/m$^2$, and still more preferably 11 g/m$^2$ to 50 g/m$^2$.

The base material has a water permeability index of preferably 20 to 100. The water permeability index can be determined by the method disclosed in Japanese Patent No. 5711974.

[3-2] Adhesive

[Hot melt adhesive]

For the water-absorbing sheet for use in the present invention, base materials can be firmly fixed, or the base materials and the particulate water-absorbing agent can be firmly fixed. Examples of methods for such firm fixing encompass (i) a method using bonding, (ii) a method using various binders which are dissolved or dispersed in water, a water-soluble polymer, or a solvent, and (iii) a method in which the base materials are heat-sealed at a melting point of a material for the base materials. Preferably, firm fixing is carried out with use of an adhesive.

An adhesive to be used can be a solution-based. However, in view of trouble involved in removing a solvent and of the problem of residual solvent, it is preferable to use a hot melt adhesive which yields high productivity and which is free of the problem of residual solvent. The hot melt adhesive used in the present invention can be contained in advance in a surface of a base material or in a surface of a particulate water-absorbing agent. Alternatively, it is possible to use a hot melt adhesive in the step of producing the water-absorbing sheet. A form and/or a melting point of the hot melt adhesive can be selected as appropriate. For example, the hot melt adhesive can have a form of particles, fibers, a net, or a film, or can have a form of a heat-melted liquid. The melting temperature or a softening point of the hot melt adhesive is 50°C to 200°C, preferably 60°C to 180°C. In a case where an adhesive having the form of particles is used, the particle diameter of the particulate adhesive is approximately 0.01 times to 2 times, 0.02 times to 1 time, or 0.05 times to 0.5 times as much as an average particle diameter of the particulate water-absorbing agent.

In production of the water-absorbing sheet for use in the present invention, a hot melt adhesive can be used, for example, by the following method. A water-absorbing sheet can be produced by uniformly dispersing a mixture of a particulate water-absorbing agent and a hot melt adhesive on a base material (e.g., nonwoven fabric), disposing another base material, and then pressure-bonding at a temperature around a melting temperature of the hot melt adhesive.

The hot melt adhesive used in the present invention can be selected as appropriate. Preferably, the hot melt adhesive, which can be used as appropriate, is at least one selected from, for example, an ethylene-vinyl acetate copolymer adhesive, a styrene elastomer adhesive, a polyolefin-based adhesive, and a polyester-based adhesive.

Specific examples of the polyolefin-based adhesive encompass polyethylene, polypropylene, and atactic polypropylene. Specific examples of the styrene elastomer adhesive encompass a styrene-isoprene block copolymer (SIS), a styrene-butadiene block copolymer (SBS), a styrene-isobutylene block copolymer (SIBS), and a styrene-ethylene-butylene-styrene block copolymer (SEBS). Specific examples of the polyester-based adhesive encompass copolymerized polyolefin, polyethylene terephthalate (PET), polybutylene terephthalate (PBT), and copolymerized polyester, Specific examples of the ethylene-vinyl acetate copolymer adhesive encompass an ethylene-vinyl acetate copolymer (EVA) adhesive, an ethylene-ethyl acrylate copolymer (EEA), and an ethylene-butyl acrylate copolymer (EBA).

The adhesive (e.g., hot melt adhesive) is contained in an amount of 0 times (not used) to 2.0 times, preferably 0.01 times to 2.0 times, more preferably 0.1 times to 1.0 time, even more preferably 0.11 times to 0.50 times, still more preferably 0.12 times to 0.30 times, and further preferably 0.14 times to 0.25 times as much as the amount (mass standard) of the water-absorbing resin contained. If the adhesive (particularly hot melt adhesive) is contained in an excessively large amount, then not only is it disadvantageous in terms of cost and the mass of a water-absorbing sheet (i.e., increase in mass of a disposable diaper), but it may also deteriorate the water absorption performance of the water-absorbing sheet due to restriction on swelling of the particulate water-absorbing agent.

[3-3] Amount of particulate water-absorbing agent used

An amount of the particulate water-absorbing agent used per unit area of the water-absorbing sheet (a total amount in a case where a plurality of particulate water-absorbing agents are used in combination) is 100 g/m$^2$ to 1000 g/m$^2$ or 130 g/m$^2$ to 950 g/m$^2$, preferably 150 g/m$^2$ to 900 g/m$^2$, more preferably 200 g/m$^2$ to 800 g/m$^2$, and even more preferably 220 g/m$^2$ to 700 g/m$^2$ per unit area of the water-absorbing sheet in order to achieve sufficient liquid absorption performance where the water-absorbing sheet is used for an absorbent article like in the claimed invention. If the amount of particulate water-absorbing agent used is small, then a hygienic material (disposable diaper) as an end product absorbs water insufficiently. If the amount of particulate water-absorbing agent used is excessively large, then there may be a problem of liquid diffusion, liquid permeability, or air permeability.

[3-4] Material of base material

The base material used in the present invention are, for example, those listed in Section [3-1] above, and is more preferably a nonwoven fabric. The nonwoven fabric used in the present invention is not limited to any particular one. From the viewpoint of liquid permeability and flexibility of the nonwoven fabric and from the viewpoint of strength of the nonwoven fabric used in a water-absorbing sheet, examples of the nonwoven fabric encompass nonwoven fabrics made of: polyolefin fibers such as polyethylene (PE) and polypropylene (PP); polyester fibers such as polyethylene terephthalate (PET), polytrimethylene terephthalate (PTT), and polyethylene naphthalate (PEN); polyamide fibers such as nylon; rayon fiber; and other synthetic fibers. Other examples of the nonwoven fabric encompass nonwoven fabrics produced by mixing, for example, cotton, silk, linen, and/or pulp (cellulose) fibers.

Among these nonwoven fabrics, a nonwoven fabric made of synthetic fibers is preferable, and nonwoven fabrics made of rayon fibers, polyolefin fibers, and polyester fibers are especially preferable, from the viewpoint of, for example, increasing the strength of a water-absorbing sheet. The nonwoven fabric can be made of one of these fibers or two or more of these fibers in combination.

More specifically, a spunbonded nonwoven fabric produced by fibers selected from the group consisting of polyolefin fibers, polyester fiber, and a mixture thereof are more preferable from the viewpoint of increasing a shape retaining property of the water-absorbing sheet and preventing the water-absorbing resin from falling off due to skipped pick. In addition, a spunlaced nonwoven fabric including rayon fibers as a main component is more preferable as a nonwoven fabric used in the present invention, from the viewpoint of further increasing liquid absorption performance and flexibility when the base material is formed. Among the spunbonded nonwoven fabrics, a spunbond-meltblown-spunbond (SMS) nonwoven fabric and a spunbond-meltblown-meltblown-spunbond (SMMS) nonwoven fabric, each of which has a multi-layer structure of polyolefin fibers, are more preferable, and an SMS nonwoven fabric and an SMMS nonwoven fabric each including polypropylene fibers as a main component are especially preferable. Meanwhile, as the spunlaced nonwoven fabric, those including rayon fibers as a main component and also including polyolefin fibers and/or polyester fibers as appropriate are preferable. Especially a rayon-PET nonwoven fabric and a rayon-PET-PE nonwoven fabric are more preferable. The nonwoven fabric can contain a small amount of pulp fibers, provided that the thickness of the water-absorbing sheet does not increase.

(Hydrophillicity)

The nonwoven fabric has a hydrophillicity of preferably 5 to 200, more preferably 8 to 150, even more preferably 10 to 100, and still more preferably 12 to 80, the hydrophillicity being measured according to the measuring method used for "hydrophillicity of nonwoven fabric" described above.

If the hydrophilicity of the nonwoven fabric is excessively low, then the liquid absorption performance of the water-absorbing sheet deteriorates. Meanwhile, if the hydrophilicity is higher than necessary, then the liquid absorption performance does not improve accordingly. Therefore, the nonwoven fabric desirably has a hydrophilicity to a proper extent.

The nonwoven fabric having such a hydrophilic property is not limited to any particular one. Among the nonwoven fabrics above, the rayon fiber, which itself is a material exhibiting proper hydrophillicity, can be selected. Alternatively, the nonwoven fabric can be obtained by subjecting a hydrophobic chemical fibers such as polyolefin fibers or polyester fibers to a hydrophilization treatment by a known method so as to impart proper hydrophillicity to the hydrophobic chemical fibers.

(Hydrophilization treatment)

The base material (particularly nonwoven fabric) used in the present invention is preferably a hydrophilic nonwoven fabric for increased water-permeability. While a base material (particularly nonwoven fabric) containing a hydrophilic fiber material such as cellulose is suitably used, it is alternatively possible to obtain the base material by hydrophilizing, with use of a hydrophilization agent such as a surfactant, a base material or fibers of which the base material is made.

Examples of the hydrophilization agent encompass: anionic surfactants such as aliphatic sulfonate and sulfate ester salt of higher alcohol; cationic surfactants such as quaternary ammonium salt; nonionic surfactants such as polyethylene glycol fatty acid ester, polyglyceryl fatty acid ester, and sorbitan fatty acid ester; silicone-based surfactants such as polyoxyalkylene-modified silicone; and stain release agents such as those containing a polyester-based resin, a polyamide-based resin, an acrylic resin, and a urethane-based resin. The hydrophilization agent is used in an amount of, for example, 0 weight% to 2 weight%, preferably 1 ppm to 1 weight%, relative to the weight of the nonwoven fabric.

[3-5] Arrangement of particulate water-absorbing agent

According to the present invention, the water-absorbing sheet is configured so that a particulate water-absorbing agent containing a water-absorbing resin as a main component is supported between two base materials including at least one water-permeable base material. The particulate water-absorbing agent is sandwiched between the two base materials.

In the present specification, a base material included in the water-absorbing sheet may be referred to as "layer". For example, in a case where the base materials included in the water-absorbing sheet consist only of a first base material and a second base material, the water-absorbing sheet has a structure of "two layers". Likewise, in a case where the water-absorbing sheet includes a first base material, one intermediate base material, and a second base material, the water-absorbing sheet has a structure of "three layers".

Note that a base material (layer), which comes into contact with a liquid first in a case where a water-absorbing sheet is used, will be referred to as "upper layer". For example, in a case where an absorbent body including the water-absorbing sheet is used for a disposable diaper, "upper layer" refers to a layer that is closest to a user of the disposable diaper. Note also that a layer on the opposite side from the upper layer will be referred to as "lower layer".

Note that the water-absorbing sheet for use in the present invention is configured so that the particulate water-absorbing agent is supported by the base materials (layers) described above. Therefore, according to the water-absorbing sheet having the two-layer structure, for example, a space sandwiched between the two base materials is not physically divided. However, a first particulate water-absorbing agent (provided in the vicinity of the first base material) supported by the first base material can be distinguished from a particulate water-absorbing agent (provided in the vicinity of the second base material) supported by the second base material.

Likewise, according to the water-absorbing sheet having the three-layer structure, it is also possible to distinguish between a first particulate water-absorbing agent and a second particulate water-absorbing agent. According to the water-absorbing sheet having the three-layer structure, the first particulate water-absorbing agent may be distributed in the vicinity of a surface of the intermediate base material facing the first base material and distributed in the intermediate base material. Likewise, the second particulate water-absorbing agent may be distributed in the vicinity of a surface of the intermediate base material facing the second base material and distributed in the intermediate base material.

The water-absorbing sheet can have two layers. Alternatively, the water-absorbing sheet can have a layered structure of three or more layers in which at least one intermediate base material is further contained between the two base materials so as to form a plurality of layers. A water-absorbing sheet having a multi-layer structure (e.g., three-layer structure) can have a new function by including different particulate water-absorbing agents for respective layers and different third components (e.g., an antibacterial agent or a deodorant) for respective layers. Additionally, the water-absorbing sheet having the multi-layer structure can be configured so that a larger amount of particulate water-absorbing agent is sandwiched than in the case of a water-absorbing sheet having a two-layer structure.

Therefore, the water-absorbing sheet for use in the present invention is preferably a water-absorbing sheet having a layered structure of three or more layers (e.g., three-layer structure). In the case of a layered structure of three or more layers (e.g., three-layer structure), a particulate water-absorbing agent is preferably provided as described in at least one of the following (i) and (ii): (i) in the first base material, in the intermediate base material, and in the vicinity of respective surfaces of the first base material and of the intermediate base material facing each other; and (ii) in the second base material, in the intermediate base material, and in the vicinity of respective surfaces of the second base material and of the intermediate base material facing each other. In addition, the particulate water-absorbing agent for use in the present invention is preferably provided at least as described in the above (ii) in the water-absorbing sheet.

In a case where a particulate water-absorbing agent is provided in a water-absorbing sheet having a layered structure of three or more layers (e.g., three-layer structure), it is possible to use identical particulate water-absorbing agents or different particulate water-absorbing agents. It is preferable to use different particulate water-absorbing agents for imparting a function to the water-absorbing sheet. Example of the case where different particulate water-absorbing agent are used encompass (i) a case where a particulate water-absorbing agent other than the particulate water-absorbing agent for use in the present invention is used in addition to the particulate water-absorbing agent for use in the present invention, (ii) a case where a plurality of kinds of particulate water-absorbing agents encompassed in the scope of the particulate water-absorbing agent for use in the present invention are used, (iii) a case where a plurality of kinds of particulate water-absorbing agents differing in particle shape (e.g., particles each having a non-uniformly pulverized shape and particles each having a spherical particle are used), and (iv) a case where any combination of the cases (i) through (iii) above is applied.

In a case where the water-absorbing sheet for use in the present invention has a layered structure of three or more layers (particularly three-layer structure), a ratio of the mass of the particulate water-absorbing agent on an upper-layer side to the mass of a particulate water-absorbing agent on a lower-layer side is preferably 5/95 to 50/50, more preferably 5/95 to 40/60, even more preferably 5/95 to 30/70, and still more preferably 5/95 to 20/80.

From the viewpoint of sufficiently exhibiting the absorption performance on the lower-layer side and preventing liquid leakage, the mass on the upper-layer side is preferably 5 or more (i.e., the mass on the lower-layer side is 95 or less) in the ratio. From the viewpoint of increasing dry feeling on the upper-layer side and decreasing the backflow after liquid absorption, the mass on the upper-layer side is preferably 50 or less (i.e., the mass on the lower-layer side is 50 or more) in the ratio.

[3-6] Other particulate water-absorbing agents (water-absorbing resins) used in combination

In a preferable aspect among aspects in which a plurality of water-absorbing resins are used in combination, for example, a particulate water-absorbing which is different in particle shape or water absorbent property from the particulate water-absorbing agent for use in the present invention is further provided. In a more preferred embodiment, for example, the particulate water-absorbing agent for use in the present invention is contained on the lower-layer side of the multi-layer structure, and a particulate water-absorbing agent which is different in particle shape or water absorbent property from the particulate water-absorbing agent on the lower-layer side is contained on the upper-layer side.

In a preferable example, a particulate water-absorbing agent satisfying the following conditions (1) through (3) is preferably provided on the upper-layer side and on the lower-layer side: (1) a centrifuge retention capacity (CRC) is 30 g/g to 50 g/g, (2) a mass average particle diameter (D50) is 200 $\mu$m to 600 $\mu$m, and (3) a DRC index is 43 or less.

In another preferable example, it is preferable to further provide, on the lower-layer side, a particulate water-absorbing agent satisfying the following condition (3a) in addition to the conditions (1) and (2) above: (3a) the DRC index is more than 0 but 43 or less. In this case, it is preferable to also provide, on the upper-layer side, a particulate water-absorbing agent satisfying the above conditions (1), (2), and (3a). Alternatively, it is also preferable to provide a particulate water-absorbing agent satisfying the above conditions (1) and (2) and the following condition (3b): (3b) the DRC index is more than 43 but 50 or less.

In addition, the particulate water-absorbing agent satisfying the above conditions (1), (2), and (3a) has a surface tension of preferably 65 or more.

Alternatively, it is possible to use the particulate water-absorbing agent for use in the present invention and another particulate water-absorbing agent in combination. Specifically, the scope of the term "combination" encompasses using a blend of the another particulate water-absorbing agent and the particulate water-absorbing agent for use in the present invention. In a case where the particulate water-absorbing agent for use in the present invention is used in a water-absorbing sheet having a layered structure of three or more layers (e.g., three-layer structure), the particulate water-absorbing agent is preferably used at least on the lower-layer side of the three-layer structure and more preferably used on the lower-layer side and on the upper-layer side, because the particulate water-absorbing agent for use in the present invention is excellent in terms of water absorption speed and in liquid diffusion.

[3-7] Shape of water-absorbing sheet

The water-absorbing sheet for use in the present invention is highly characteristic in that the water-absorbing sheet can be reduced in thickness. In view of the use in a disposable diaper, the thickness while the water-absorbing sheet is in a dry state is preferably 5 mm or less, more preferably 4 mm or less, even more preferably 3 mm or less, and particularly preferably 2 mm or less. A lower limit value of the thickness is 0.2 mm or more, preferably 0.3 mm or more, and even more preferably 0.5 mm in view of strength and of a diameter of the particulate water-absorbing agent.

Furthermore, for imparting liquid permeability, a diffusion property, and flexibility to the water-absorbing sheet, the surface (first base material and second base material) of the water-absorbing sheet can include an embossed region as appropriate. In addition, the particulate water-absorbing agent can be distributed all over the surface of the water-absorbing sheet, or a part of the water-absorbing sheet can be a non-present region in which the particulate water-absorbing agent is not present. In particular, the water-absorbing sheet preferably has the non-present region which longitudinally extends in a channel form (striped). The embossed region can be provided all over the surface of the water-absorbing sheet or can be provided on a part of the surface of the water-absorbing sheet.

In a case where the embossed region and/or the non-present region extend longitudinally on the water-absorbing sheet, it is possible to diffuse a liquid along a length of the water-absorbing sheet. This continuous embossed region and/or the particulate water-absorbing agent non-present region can serve as a pathway (liquid transporting pathway) in which a large amount of liquid flows. The embossed region and/or the particulate water-absorbing agent non-present region can each have a linear form, a curved form, or waved form.

The water-absorbing sheet for use in the present invention has a water absorption performance of preferably 2000 g/m$^2$ or more, more preferably 4000 g/m$^2$ or more, and even more preferably 6000 g/m$^2$ to 18000 g/m$^2$, in terms of CRC.

The base material of the water-absorbing sheet for use in accordance with the present invention has a peel strength of ordinarily 0.05 N/7cm to 3.0 N/7cm, preferably 0.1 N/7cm to 2.5 N/7cm, more preferably 0.15 N/7cm to 2.0 N/7cm, and even more preferably 0.2 N/7cm to 1.5 N/7cm. If the peel strength of the base material is more than 3.0 N/7cm, then adhesion of the absorbent layer becomes excessively strong. This prevents the effect of adding a certain amount of water-absorbing resin having specific water absorption performance.

[3-8] Method of producing water-absorbing sheet

A method of producing the water-absorbing sheet for use in the present invention is not limited to any particular one, provided that a sheet can be formed. For example, methods described below can be applied. In addition to the methods disclosed in Patent Literatures 16 through 39, the water-absorbing sheet production methods disclosed in the following literature, for example, can be referenced as appropriate as method of producing the water-absorbing sheet: Japanese Patent Application Publication, Tokukai, No. 2008-183159, Japanese Patent Application Publication, Tokukai, No. 2002-345883, Japanese Patent Application Publication, Tokukaihei, No. 6-315501, Japanese Patent Application Publication, Tokukaihei, No. 6-190003, Japanese Patent Application Publication, Tokukaihei, No. 6-190002, Japanese Patent Application Publication, Tokukaihei, No. 6-190001, Japanese Patent Application Publication, Tokukaihei, No. 2-252558, Japanese Patent Application Publication, Tokukaihei, No. 2-252560, and Japanese Patent Application Publication, Tokukaihei, No. 2-252561.

(a) A particulate water-absorbing agent (preferably and an adhesive) is uniformly dispersed on a base material (e.g., nonwoven fabric) so as to be provided in a space of the base material. Then, another base material (e.g., nonwoven fabric) is placed onto the base material, and is then bonded (particularly pressure-bonded at a temperature around a melting temperature of the adhesive) to the base material.

(b) A particulate water-absorbing agent (preferably and an adhesive) is dispersed on a base material (e.g., nonwoven fabric) so as to be provided in a space of the base material. Then, the base material is allowed to pass through a heating furnace so that the particulate water-absorbing agent is fixed to the base material but not to such an extent that a powder is scattered. Another base material (e.g., nonwoven fabric) is placed onto the base material and is pressure-bonded to the base material.

(c) A particulate water-absorbing agent is uniformly dispersed on a base material (e.g., nonwoven fabric which can be melt-coated with an adhesive) so as to be provided in a space of the base material. Another nonwoven fabric melt-coated with an adhesive is placed onto the base material so as to face the dispersed water-absorbing resin layer, and is then pressured with use of a roll press machine or the like. The base material and the another base material are heated as needed so as to be bonded together.

Note that the term "provided in the space of the base material" used in describing the production method means that the particulate water-absorbing agent is intended to enter gaps between the fibers by which the base material is constituted. This can also be expressed as "entangling the particulate water-absorbing agent with the fibers of the base material".

By producing a water-absorbing sheet by any of the methods (a) through (c) above, for example, it is possible to produce a water-absorbing sheet having a structure in which an absorbent layer containing a water-absorbing resin and an adhesive is sandwiched between two nonwoven fabrics. Among these methods, the methods (a) and (c) are more preferable from the viewpoint of their simplicity and high production efficiency.

Alternatively, it is possible to produce a water-absorbing sheet by any combination of the methods (a) through (c) exemplified above. Alternatively, for the purpose of improving texture of the water-absorbing sheet and improving liquid absorption performance, it is possible to subject the surface of the water-absorbing sheet to embossing, for example, after the production of the water-absorbing sheet or at the time of the pressure-bonding during production of the water-absorbing sheet.

By carrying out the above method (c) on both sides of an intermediate base material, for example, it is possible to produce a water-absorbing sheet having three-layer structure in which a particulate water-absorbing agent is present (i) in a first base material, in the intermediate base material, and in the vicinity of respective surfaces of the first base material and of the intermediate base material facing each other; and (ii) in a second base material, in the intermediate base material, and in the vicinity of respective surfaces of the second base material and of the intermediate base material facing each other.

The water-absorbing sheet for use in the present invention can contain an additive(s) such as a deodorant agent, fibers, an antibacterial agent, and/or a gel stabilizing agent as appropriate (in an amount of 0 mass% to 100 mass%, preferably 0 mass% to 50 mass%, and even more preferably 0 mass% to 10 mass%, relative to the mass of the particulate water-absorbing agent).

The water-absorbing sheet produced is designed to have a proper length and a proper width according to an absorbent article. For example, the width is 10 to 3 m, preferably 10 cm to 1 m. In a case of a long water-absorbing sheet, the water-absorbing sheet is produced to have length of several tens of m to several thousands of m, ordinarily 10 m or more, preferably 100 m or more, more preferably 500 m or more, and particularly preferably 1000 m or more. Furthermore, the water-absorbing sheet produced is cut transversely and, as necessary, longitudinally, according to the purpose of the water-absorbing sheet (i.e., the size of a hygienic material for which to use the water-absorbing sheet).

[4] Method of producing particulate water-absorbing agent

A method of producing a particulate water-absorbing agent for use in accordance with the present invention may be a publicly known method or a combination of publicly known methods. The method of producing a particulate water-absorbing agent is not particularly limited provided that the method satisfies parameters specified in the present invention.

The following description will discuss production steps [4-1] through [4-9] as an example of the method of producing a particulate water-absorbing agent used for a water-absorbing sheet included in the absorbent article of the present invention.

[4-1] Step of preparing aqueous monomer solution

This step is a step of preparing an aqueous solution containing a monomer (e.g., an acrylic acid (salt)) as a main component (this solution is hereinafter referred to as an "aqueous monomer solution"). It is also possible to use a monomer slurry liquid to the extent that a water-absorbing resin to be produced will not have degraded water absorption performance. For convenience of description, however, this section describes an aqueous monomer solution.

The term "main component" means that the acrylic acid (salt) is used (contained) in an amount of ordinarily 50 mol% or more, preferably 70 mol% or more, more preferably 90 mol% or more (with an upper limit value of 100 mol%), per a total amount of monomers used for a polymerization reaction of a water-absorbing resin (excluding an internal crosslinking agent).

(Acrylic acid)

For the present invention, it is preferable that an acrylic acid and/or an acrylic acid salt (hereinafter referred to as "acrylic acid (salt)") is used as a monomer from the viewpoint of physical properties of a particulate water-absorbing agent to be produced and productivity.

The "acrylic acid" may be a publicly-known acrylic acid, and may contain, as a polymerization inhibitor, preferably a methoxyphenol, more preferably p-methoxyphenol, in an amount of preferably 200 ppm or less, more preferably 10 ppm to 160 ppm, even more preferably 20 ppm to 100 ppm, from the viewpoint of polymerizability of the acrylic acid and the color of a particulate water-absorbing agent to be produced. An impurity in the acrylic acid for the present invention may be a compound disclosed in U.S. Patent Application Publication No. 2008/0161512.

The "acrylic acid salt" is produced by neutralizing the above acrylic acid with a basic composition below. The acrylic acid salt may be a commercially available acrylic acid salt (for example, sodium acrylate) or may be produced by neutralizing an acrylic acid in a plant for producing a particulate water-absorbing agent.

(Basic composition)

In the present invention, the term "basic composition" refers to a composition containing a basic compound, such as a commercially available aqueous sodium hydroxide solution.

Specific examples of the basic compound encompass a carbonate or bicarbonate of an alkali metal, a hydroxide of an alkali metal, ammonia, and organic amine. Among these, the basic compound preferably has strong basicity in view of physical properties of a particulate water-absorbing agent to be obtained. That is, the basic compound is preferably a hydroxide of alkali metal, such as sodium hydroxide, potassium hydroxide, or lithium hydroxide, and is more preferably sodium hydroxide.

(Neutralization)

In the present invention, neutralization can be neutralization of an acrylic acid (before polymerization), neutralization of a crosslinked hydrogel polymer obtained by crosslinking and polymerizing an acrylic acid (after polymerization) (hereinafter referred to as "later neutralization"), or a combination of the neutralization of an acrylic acid and the neutralization of a crosslinked hydrogel polymer obtained by crosslinking and polymerizing an acrylic acid. These neutralizations are not limited to any particular type, and can be of a continuous type or a batch type. Among these, a continuous type is preferable from the viewpoint of production efficiency and the like.

Note that with regard to conditions such as a neutralization apparatus, a neutralization temperature, and a retention time, the conditions disclosed in International Publication No. 2009/123197 and U.S. Patent Application Publication No. 2008/0194863 can be applied to the present invention.

A neutralization rate in the present invention is preferably 10 mol% to 90 mol%, more preferably 40 mol% to 85 mol%, even more preferably 50 mol% to 80 mol%, and particularly preferably 60 mol% to 75 mol% per an acid group of a monomer. At a neutralization rate of less than 10 mol%, a fluid retention capacity may be lowered significantly. Meanwhile, in a case where the neutralization rate is higher than 90 mol%, it may not be possible to obtain a water-absorbing resin having a high fluid retention capacity under pressure.

The neutralization rate also applies to the later neutralization. The neutralization rate can also apply to a neutralization rate for a particulate water-absorbing agent which is an end product. Note that a neutralization rate of 75 mol% means a mixture of 25 mol% of an acrylic acid and 75 mol% of an acrylic acid salt. The mixture is referred to also as a partially neutralized acrylic acid.

(Other monomer(s))

In the present invention, "other monomer(s)" refers to a monomer(s) other than the acrylic acid (salt), and a particulate water-absorbing agent can be produced by using the other monomer(s) in combination with the acrylic acid (salt).

Examples of the other monomer(s) encompass an unsaturated monomer which is water-soluble or hydrophobic. Specifically, the compound disclosed in U.S. Patent Application Publication No. 2005/0215734 (except an acrylic acid) can be applied to the present invention.

(Internal crosslinking agent)

The compounds disclosed in US Patent No. 6241928 can be used as an internal crosslinking agent usable in the present invention. One of the compounds or two or more of the compounds is/are to be selected in view of reactivity.

From the viewpoint of, for example, the water absorption performance of a water-absorbing resin to be produced, the internal crosslinking agent is preferably a compound having two or more polymerizable unsaturated groups, more preferably a compound that is pyrolytic at a drying temperature below, even more preferably a compound having a (poly) alkylene glycol structural unit and two or more polymerizable unsaturated groups.

The polymerizable unsaturated groups are preferably an allyl group or a (meth)acrylate group, more preferably a (meth)acrylate group. The (poly)alkylene glycol structural unit is preferably polyethylene glycol. The n number of the (poly) alkylene glycol is preferably 1 to 100, more preferably 6 to 50.

Therefore, in the present invention, preferably (poly)alkylene glycol di(meth)acrylate or (poly)alkylene glycol tri(meth)acrylate is to be used, and more preferably (poly)ethylene glycol di(meth)acrylate is to be used.

The internal crosslinking agent is to be used in an amount of preferably 0.0001 mol% to 10 mol%, more preferably 0.001 mol% to 1 mol% relative to a total amount of monomers. In a case where the amount used falls within the above ranges, a desired water-absorbing resin can be obtained. Note that in a case where the amount used is excessively small, gel strength tends to be lowered and consequently there tends to be an increase in water-soluble content. In a case where the used amount is excessively large, fluid retention capacity tends to be lowered. Therefore, the amount used that is excessively large or excessively small is not preferable.

For the present invention, the following method is preferably used: An aqueous monomer solution to which a certain amount of internal crosslinking agent has been added in advance is prepared. Then, the aqueous monomer solution is simultaneously subjected to polymerization and to a crosslinking reaction. Alternatively, other than the above method, examples of a possible method encompass a method in which an internal crosslinking agent is added during or after the polymerization so that postcrosslinking is carried out, a method in which radical crosslinking is carried out with use of a radical polymerization initiator, and a method in which radiation crosslinking is carried out with use of active energy rays such as an electron ray and an ultraviolet ray. Alternatively, these methods may be used in combination.

(Other substances added to aqueous monomer solution)

The present invention may include adding any substance below to the aqueous monomer solution during the preparation thereof from the viewpoint of improved physical properties for a water-absorbing resin to be produced.

Specifically, a hydrophilic polymer such as starch, a starch derivative, cellulose, a cellulose derivative, polyvinyl alcohol, polyacrylic acid (salt), and crosslinked polyacrylic acid (salt) can be added in an amount of preferably 50 mass% or less, more preferably 20 mass% or less, even more preferably 10 mass% or less, especially even more preferably 5 mass% or less (with a lower limit value of 0 mass%). Alternatively, a carbonate, an azo compound, a foaming agent for air bubbles or the like, a surfactant, a chelating agent, a chain transfer agent, and/or the like can be added in an amount of preferably 5 mass% or less, more preferably 1 mass% or less, even more preferably 0.5 mass% or less (with a lower limit value of 0 mass%).

The above substances are not necessarily added to the aqueous monomer solution, but can be added during the polymerization, or can be added both to the aqueous monomer solution and during the polymerization.

In a case where a water-soluble resin or a water-absorbing resin is used as the hydrophilic polymer, a graft polymer or a water-absorbing resin composition (for example, a polymer produced from starch and an acrylic acid or a polymer produced from PVA and an acrylic acid) can be obtained. These polymers and water-absorbing resin compositions are also encompassed in the scope of the present invention.

(Monomer component concentration)

The above various substances are added during the step of preparing the aqueous monomer solution. The aqueous monomer solution may contain a monomer component at any concentration. The concentration is, however, within a range of preferably 10 mass% to 80 mass%, more preferably 20 mass% to 75 mass%, even more preferably 30 mass% to 70 mass%, from the viewpoint of physical properties of a water-absorbing resin to be produced.

In a case where aqueous solution polymerization or reversed phase suspension polymerization is employed, a solvent other than water can be used in combination as necessary. In such a case, the type of the solvent used is not limited to any particular one.

The "monomer component concentration" is a value determined by Formula (8) below. The mass of the aqueous monomer solution does not include the mass of a graft component, water-absorbing resin, or a hydrophobic solvent used in reversed phase suspension polymerization.

$$\text{Monomer component concentration (mass\%)} = \text{(mass of monomer component)} / \text{(mass of aqueous monomer solution)} \times 100 \tag{8}$$

[4-2] Polymerization step

This step is a step of polymerizing an acrylic acid (salt)-based aqueous monomer solution obtained in the step of preparing the aqueous monomer solution, so that a crosslinked hydrogel polymer (hereinafter referred to as "hydrogel") is obtained.

(Polymerization initiator)

The polymerization initiator usable in the present invention is selected as appropriate in accordance with a form of polymerization or the like and is not limited to any particular one. Examples of the polymerization initiator encompass a pyrolytic polymerization initiator, a photolytic polymerization initiator, and a redox-type polymerization initiator that contains a reducing agent for facilitating decomposition of any of those polymerization initiators. Specifically, used as the polymerization initiator is one of the polymerization initiators disclosed in U.S. Patent No. 7265190, or a compound of two or more of the polymerization initiators disclosed in U.S. Patent No. 7265190. Further, the polymerization initiator is preferably a peroxide or an azo compound, more preferably a peroxide, and even more preferably a persulfate, from the viewpoint of the handleability of the polymerization initiator and the physical properties of the particulate water-absorbing agent or the water-absorbing resin.

The amount of the polymerization initiator to be used ranges from preferably 0.001 mol% to 1 mol%, and more preferably 0.001 mol% to 0.5 mol%, relative to the amount of monomers. The amount of the reducing agent to be used ranges from preferably 0.0001 mol% to 0.02 mol%, relative to the amount of monomers.

A polymerization reaction can be carried out by, instead of using the polymerization initiator, irradiating a monomer with an active energy ray such as a radial ray, an electron ray, or an ultraviolet ray. Alternatively, any of these active energy rays can be used in combination with a polymerization initiator.

(Form of polymerization)

Polymerization to be applied to the present invention is not limited to any particular form. From the viewpoint of a water absorbent property, ease of control of polymerization, and the like, preferable examples of the polymerization encompass spray droplet polymerization, aqueous solution polymerization, and reversed phase suspension polymerization, more preferable examples of the polymerization encompass aqueous solution polymerization and reverse phase suspension polymerization, and even more preferable examples of the polymerization encompass aqueous solution polymerization. Among these, continuous aqueous solution polymerization is particularly preferable. The continuous aqueous solution polymerization can be any one of continuous belt polymerization and continuous kneader polymerization.

Specific examples of the form of continuous belt polymerization encompass those disclosed in US Patent No. 4893999, US Patent No. 6241928, and U.S. Patent Application Publication No. 2005/0215734. Specific examples of the form of continuous kneader polymerization encompass those disclosed in US Patent No. 6987151 and US Patent No. 6710141. In a case where these forms of continuous aqueous solution polymerization are employed, it is possible to improve efficiency with which a water-absorbing resin is produced.

Preferable examples of the form of the continuous aqueous solution polymerization encompass "high-temperature-initiating polymerization" and "high-concentration polymerization". The "high-temperature-initiating polymerization" is a form of polymerization in which polymerization is started while a temperature of an aqueous monomer solution is preferably 30°C or higher, more preferably 35°C or higher, even more preferably 40°C or higher, and especially even more preferably 50°C or higher (upper limit value: boiling point). The "high-concentration polymerization" is a form of polymerization in which polymerization is carried out while a monomer concentration is preferably 30 mass% or more, more preferably 35 mass% or more, even more preferably 40 mass% or more, and especially even more preferably 45 mass% or more (upper limit value: saturating concentration). Alternatively, it is possible to use these forms of polymerization in combination.

In the present invention, polymerization can be carried out in an air atmosphere. From the viewpoint of color of a water-absorbing resin to be obtained, polymerization is to be carried out preferably in an atmosphere of inert gas such as nitrogen or argon. In such a case, an oxygen concentration is preferably controlled to be, for example, 1 volume% or less. Note that dissolved oxygen in an aqueous monomer solution is also preferably substituted with inert gas (e.g., dissolved oxygen: less than 1 mg/l).

In the present invention, alternatively, it is possible to carry out foaming polymerization in which polymerization is carried out while gas bubbles (particularly the inert gas or the like) are dispersed into an aqueous monomer solution.

In the present invention, alternatively, it is possible to increase a solid content concentration during polymerization. A degree of increase in solid content as an index of an increase in such a solid content concentration can be defined by the following Formula (9). Note that the degree of increase in solid content concentration is preferably 1 mass% or more, and more preferably 2 mass% or more.

Degree (mass%) of increase in solid content = (solid content concentration in hydrogel after polymerization) - (solid content concentration in aqueous monomer solution) $\qquad$ (9)

Where the solid content concentration in an aqueous monomer solution is a value that can be obtained by the following Formula (10) and where components in a polymerization system are an aqueous monomer solution, a graft component, a water-absorbing resin and other solid matters (e.g., water-insoluble fine particles and the like), and therefore exclude a hydrophobic solvent in reverse phase suspension polymerization.

Solid content concentration (mass%) in aqueous monomer solution = {mass of (monomer component + graft component + water-absorbing resin + other solid matters)} / (mass of components in polymerization system) × 100 $\qquad$ (10)

[4-3] Gel-crushing step

This step is a step of gel-crushing a hydrogel, which has been obtained by the polymerization step, with use of, for example, a kneader, a screw extruder such as a meat chopper, or a gel-crusher such as a cutter mill in order to obtain a hydrogel in the form of particles (hereinafter referred to as "particulate hydrogel"). In a case where the polymerization step is carried out through kneader polymerization, such a step is equivalent to a combination of the polymerization step and the gel-crushing step which are carried out simultaneously. In a case where a particulate hydrogel is directly obtained through a polymerization process such as vapor phase polymerization or reverse phase suspension polymerization, the gel-crushing step may not be carried out.

With regard to gel-crushing conditions and forms other than above described, the following conditions can be preferably applied to the present invention in order to obtain a particulate water-absorbing agent for use in the present invention more easily.

[4-3-1] Gel CRC

In order for a particulate water-absorbing agent for use in the present invention to be obtained more easily, CRC of a hydrogel before gel-crushing (referred to as "gel CRC") is preferably 33 g/g or more. A gel CRC before gel-crushing of less than 10 g/g or of more than 45 g/g is not preferable because it becomes difficult to control the particle shape and the particle size distribution during the gel-crushing. In order to achieve such a gel CRC, an added amount of crosslinking agent during polymerization, polymerization concentration, or the like may be controlled as appropriate. Note that it is a well-known fact that a particulate water-absorbing agent or a water-absorbing resin preferably has a high gel CRC. It was, however, found in the present invention that a gel CRC of more than 45 g/g makes it difficult to control the particle shape and the particle size distribution.

[4-3-2] Gel-grinding energy (GGE)

In order for a particulate water-absorbing agent for use in the present invention to be obtained more easily, the upper limit value of the gel grinding energy (GGE) for gel-crushing of a hydrogel is preferably 60 J/g or less, more preferably 50 J/g or less, even more preferably 40 J/g or less, and the lower limit value of the gel-grinding energy (GGE) is preferably 15 J/g or more, more preferably 17 J/g or more, even more preferably 20 J/g or more, still even more preferably 23 J/g or more, still even more preferably 25 J/g or more, still even more preferably 29 J/g or more, and most preferably 34 J/g or more. For example, in the present invention, the gel-grinding energy (GGE) for gel-crushing of a hydrogel is preferably 29 J/g to 60 J/kg, more preferably 29 J/g to 50 J/g, even more preferably 29 J/g to 40 J/kg. Alternatively, for example, in the present invention, the gel-grinding energy (GGE) for gel-crushing of a hydrogel is preferably 34 J/g to 60 J/kg, more preferably 34 J/g to 50 J/g, even more preferably 34 J/g to 40 J/kg. By controlling the GGE within the above range, it is possible to perform gel-crushing while applying adequate shearing and compressive forces to the hydrogel. It is noted that the gel-grinding energy (GGE) includes the energy that the gel-crusher consumes in the idle state.

A gel-grinding energy (2) (GGE (2), referred to also as a net gel grinding energy), which excludes the energy that the gel-crusher consumes in the idle state, has an upper limit value of preferably 40 J/g or less, more preferably 38 J/g or less, even more preferably 35 J/g or less and a lower limit value of preferably 9 J/g or more, more preferably 12 J/g or more, even more preferably 15 J/g or more, and even more preferably 19 J/g or more. For example, in the present invention, the gel-grinding energy (2) (GGE (2)) for gel-crushing of a hydrogel is preferably 15 J/g to 40 J/kg, more preferably 15 J/g to 38 J/g, and even more preferably 15 J/g to 35 J/kg. Alternatively, for example, in the present invention, the gel-grinding energy (2) (GGE (2)) for gel-crushing of a hydrogel is preferably 19 J/g to 40 J/kg, more preferably 19 J/g to 38 J/g, and even more preferably 19 J/g to 35 J/kg. By controlling the GGE within the above range, it is possible to perform gel-crushing while applying adequate shearing and compressive forces to the hydrogel.

[4-3-3] Moisture content

In order for a particulate water-absorbing agent for use in the present invention to be obtained more easily, the moisture content of a hydrogel for use in the present invention is 50 mass% or more, preferably 52 mass% or more. A particulate water-absorbing agent having excellent physical properties can be obtained by increasing the water content in the hydrogel to be subjected to gel-crushing. The moisture content can be measured by a method as described in the Examples.

[4-4] Drying step

This step is a step of drying the particulate hydrogel, which has been obtained by the polymerization step and/or the gel-crushing step, until a desired resin solid content is attained, so as to obtain a dried polymer. The resin solid content is calculated from drying loss (a change in mass after heating 1 g of the water-absorbing resin at 180°C for three hours). The resin solid content is preferably 80 mass% or more, more preferably in a range of 85 mass% to 99 mass%, even more preferably in a range of 90 mass% to 98 mass%, and especially even more preferably in a range of 92 mass% to 97 mass%.

A drying method of drying the particulate hydrogel is not particularly limited. Examples of the drying method encompass thermal drying, hot air drying, drying under reduced pressure, fluidized bed drying, infrared drying, microwave drying, drum dryer drying, drying by azeotropic dehydration with a hydrophobic organic solvent, and high humidity drying by use of high temperature water vapor. The drying method is, among others, preferably hot air drying, more preferably band drying, in which hot air drying is performed on a through-flow belt, from the viewpoint of drying efficiency.

From the viewpoint of the color of a water-absorbing resin to be produced and drying efficiency, hot air drying is performed at a drying temperature (temperature of hot air) of preferably 120°C to 250°C, more preferably 150°C to 200°C. Drying conditions other than the drying temperature (e.g., the air velocity of hot air and the drying time) can be set as appropriate in accordance with moisture content of the particulate hydrogel to be dried, total mass of the particulate hydrogel to be dried, and a desired resin solid content. In the case of band drying, the various conditions disclosed in, for example, International Publication No. 2006/100300, International Publication No. 2011/025012, International Publication No. 2011/025013, and International Publication No. 2011/111657 can be applied as necessary.

Setting the drying temperature and the drying time to be within these ranges makes it possible to obtain a water-absorbing resin whose CRC (centrifuge retention capacity), water-soluble content (Ext), and color are within a desired range.

[4-5] Pulverizing step and classification step

This step is a step of pulverizing (pulverization step) the dried polymer obtained in the drying step and adjusting (classification step) the particle size of a resulting pulverized polymer to be a particle size within a certain range so that a water-absorbing resin powder is obtained (for convenience, water-absorbing resin in a powder form before being subjected to surface crosslinking is referred to as "water-absorbing resin powder").

An apparatus used in the pulverization step of the present disclosure can be, for example, a high-speed crusher such as a roll mill, a hammer mill, a screw mill, and a pin mill; a vibrating mill; a knuckle-type crusher; a cylindrical mixer; and the like. These apparatuses can be used in combination according to need.

A particle size adjusting method in the classification step of the present disclosure is not limited to a particular one and can be, for example, sieve classification with use of a JIS standard sieve (JIS Z8801-1 (2000)), airflow classification, or the like. Note that the particle size of water-absorbing resin is not limited to being adjusted during the pulverization step and classification step, but may alternatively be adjusted as appropriate during the polymerization step (in particular, in reversed phase suspension polymerization or spray droplet polymerization) or other steps (for example, a granulation step or a fine powder recycling step).

The particle size of water-absorbing resin powder obtained in the present invention is suitably adjusted to fall within the above ranges of the particle size of the particulate water-absorbing agent.

The above particle sizes apply not only to water-absorbing resin subsequent to surface crosslinking (for convenience, hereinafter referred to also as "water-absorbing resin particle(s)"), but also to the particulate water-absorbing agent as a final product. Therefore, it is preferable to subject the water-absorbing resin particles to surface crosslinking (surface-crosslinking step) so that the particle size falling within the above described range is maintained, and it is more preferable to carry out particle size adjustment by carrying out a sizing step subsequent to the surface-crosslinking step.

[4-5-1] Circulation crushing ratio

In a case where the particulate water-absorbing agent for use in the present invention has a circulation crushing ratio of less than 1.10, the liquid permeability (e.g., SFC) of the particulate water-absorbing agent will be deteriorated and there will be a significantly increased amount of fine powder after the particulate water-absorbing agent is damaged. Therefore, the circulation crushing ratio of less than 1.10 is not preferable. The increase of fine powder after the damage is defined by a measuring method described in the Examples. Even in a case where the amount of fine powder (for example, fine powder that is passed through a JIS standard sieve having a mesh size of 150 $\mu$m) is small immediately after production of the particulate water-absorbing agent, fine powder is produced due to process damage during the production of disposable diapers so as to cause an adverse effect, such as a decrease in liquid permeability, when the disposable diapers are actually used. Therefore, the circulation crushing ratio of less than 1.10 is not preferable.

In the present invention, the circulation crushing ratio is 1.10 or more, preferably 1.15 or more, more preferably 1.20 or more, even more preferably 1.30 or more, especially even more preferably 1.35 or more, and most preferably 1.40 or more, from the viewpoint of damage resistance. From the viewpoint of water absorption speed (e.g., FSR), the circulation crushing ratio has an upper limit value of 1.50 or less, preferably 1.40 or less, more preferably 1.35 or less, even more preferably 1.30 or less, yet even more preferably 1.25 or less, still even more preferably 1.20 or less, and especially even more preferably 1.15 or less.

That is, in a preferred embodiment of the present invention, the circulation crushing ratio is in a range of 1.10 to 1.50, preferably in a range of 1.15 to 1.40, even more preferably in a range of 1.30 to 1.35. These conditions are preferable because satisfying these conditions improves the water absorption speed of the particulate water-absorbing agent (e.g., DRC5min) as well as significantly decreasing fine powder resulting from process damage during the production of disposable diapers.

[4-6] Surface-crosslinking step

This step is a step of forming a portion with a higher crosslinking density in a surface layer (that is, a portion of the water-absorbing resin powder which portion is up to several tens of micrometers deep from the surface) of the water-absorbing resin powder produced through the above steps. This step includes a mixing step, a heat treatment step, and optionally a cooling step.

In the surface-crosslinking step, a water-absorbing resin (water-absorbing resin particles) can be obtained which has been surface-crosslinked by radical crosslinking on the surface of the water-absorbing resin powder, surface polymerization on the surface of the water-absorbing resin powder, crosslinking reaction with a surface-crosslinking agent, or the like.

(Surface-crosslinking agent)

A surface-crosslinking agent used in the present invention is not limited to any particular one. Examples of the surface-crosslinking agent encompass an organic surface-crosslinking agent and an inorganic surface-crosslinking agent. Among others, an organic surface-crosslinking agent that is reactive with a carboxyl group is preferable, from the viewpoint of the physical properties of a water-absorbing resin and the handleability of the surface-crosslinking agent. For example, one of the surface-crosslinking agents disclosed in U.S. Patent No. 7183456 can be used, or two or more of the surface-crosslinking agents disclosed in U.S. Patent No. 7183456 can be used. Specifically, examples of the surface-crosslinking agent encompass a polyhydric alcohol compound, an epoxy compound, a haloepoxy compound, a polyamine compound, a condensed product with a haloepoxy compound of the polyamine compound, an oxazoline compound, an oxazolidinone compound, a polyvalent metal salt, an alkylene carbonate compound, a cyclic urea compound, and the like.

An amount of the surface-crosslinking agent used (or a total amount used in a case where a plurality of surface-crosslinking agents are used) is preferably 0.01 parts by mass to 10 parts by mass, more preferably 0.01 parts by mass to 5 parts by mass, relative to 100 parts by mass of the water-absorbing resin powder. The surface-crosslinking agent is preferably added as an aqueous solution. In such a case, an amount of water used is preferably 0.1 parts by mass to 20 parts by mass, more preferably 0.5 parts by mass to 10 parts by mass, relative to 100 parts by mass of the water-absorbing resin powder. In a case where a hydrophilic organic solvent is used according to need, an amount of the hydrophilic organic solvent used is preferably not more than 10 parts by mass, and more preferably not more than 5 parts by mass, relative to 100 parts by mass of the water-absorbing resin powder.

It is possible to mix additives, which are added in a remoistening step described below, with the surface-crosslinking agent (aqueous solution) by adding each of the additives in a range of equal to or less than 5 parts by mass. Alternatively, it is possible to add the additives to the water-absorbing resin powder and the surface-crosslinking agent in a different mixing step described below.

(Mixing step)

This step is a step of mixing the water-absorbing resin powder and the surface-crosslinking agent. A method of mixing the surface-crosslinking agent is not limited to a particular one and can be a method in which a surface-crosslinking agent solution is prepared in advance, and the surface-crosslinking agent solution is mixed with the water-absorbing resin powder preferably by spraying or dropping the surface-crosslinking agent solution onto the water-absorbing resin powder, more preferably by spraying the surface-crosslinking agent solution onto the water-absorbing resin powder.

The above mixing may be performed with use of any device. The device is preferably a high-speed stirring mixer, more preferably a high-speed stirring continuous mixer.

(Heat treatment step)

This step is a step of heating a mixture, which has been obtained in the mixing step, so as to cause crosslinking reaction on a surface of the water-absorbing resin powder.

An apparatus for performing the crosslinking reaction is not limited to any particular one, and can be preferably a paddle dryer. A reaction temperature in the crosslinking reaction is set as appropriate according to a type of a used surface-crosslinking agent, and is preferably 50°C to 300°C, and more preferably 100°C to 200°C.

(Cooling step)

This step is an optional step which is carried out after the heat treatment step if needed.

An apparatus for carrying out the cooling is not limited to a particular one and is preferably an apparatus whose specification is identical with that of an apparatus used in the heat treatment step, and more preferably a paddle dryer. This is because such an apparatus can be used as a cooling apparatus by replacing a heating medium with a refrigerant. Note that, according to need, the water-absorbing resin particles obtained in the heat treatment step are force-cooled in the cooling step to a temperature preferably of 40°C to 80°C, and more preferably of 50°C to 70°C.

[4-7] Step of adding additive

This step is a step of adding, to the water-absorbing resin particles obtained in the surface-crosslinking step, at least one additive selected from the group consisting of a polyvalent metal salt, a cationic polymer, a chelating agent, an inorganic reducing agent, $\alpha$-hydroxycarboxylic acid compound and a moisture absorption fluidity improving agent, each of which are described in [2-20]. The additive is not limited to being added after the surface-crosslinking step (in particular, the remoistening step (a step of adding water in a small amount of approximately 0.1 mass% to 20 mass%)), but may be added in any step (e.g., the polymerization step, the gel-crushing step, the surface-crosslinking step, the granulation step, the fine powder recycling step, or a transportation step) provided that a certain effect is exhibited. That is, the particulate water-absorbing agent in the present invention may optionally contain a component(s) described in [2-20], other than the water-absorbing resin.

Note that in a case where the additive is added in the form of aqueous solution or slurry liquid, the water-absorbing resin particles are swollen by water again. Therefore, this step is also referred to as "remoistening step". Further, as described above, the additive can be mixed with the water-absorbing resin powder simultaneously with the surface-crosslinking agent (aqueous solution).

According to a preferred embodiment, the method of producing a particulate water-absorbing agent include a step of adding the chelating agent in an amount of 0.001 parts by mass to 0.2 parts by mass, preferably 0.003 parts by mass to 0.1 parts by mass, more preferably 0.005 parts by mass to 0.06 parts by mass relative to 100 parts by mass of the particulate water-absorbing agent or the water-absorbing resin. Addition of the chelating agent to the particulate water-absorbing agent enables an improvement in urine resistance of the particulate water-absorbing agent.

[4-8] Step of adding another additive

In the present invention, an additive other than the above described additives can be added in order to give various functions to the water-absorbing resin to be obtained. Specifically, examples of such an additive encompass a surfactant, a compound having a phosphorus atom, an oxidizer, an organic reducing agent, water-insoluble inorganic fine particles, organic powder such as metallic soap, a deodorant agent, an antibacterial agent, pulp, thermoplastic fibers, and the like. Note that, as the surfactant, a compound disclosed in International Publication No. 2005/075070 can be applied to the present invention. Moreover, as the water-insoluble inorganic fine particles, a compound disclosed in "[5] Water-insoluble inorganic fine particles" of International Publication No. 2011/040530 can be applied to the present invention.

An amount of the additive used (added) is determined as appropriate according to a purpose of the additive, and is therefore not limited to a particular one. The amount used (added) of the additive is preferably not more than 3 parts by mass, and more preferably not more than 1 part by mass relative to 100 parts by mass of the water-absorbing resin powder. It is also possible to add the additive during a step other than the above step.

[4-9] Other steps

In the present invention, in addition to the above described steps, it is possible to carry out a granulation step, a sizing step, a fine powder removal step, a fine powder recycling step, and the like according to need. Moreover, it is possible to further carry out one or more of a transportation step, a storing step, a packing step, a reserving step, and the like. Note that the "sizing step" encompasses a fine powder removal step subsequent to the surface-crosslinking step and a step of carrying out classification and pulverization in a case where a water-absorbing resin is aggregated to have a size larger than an intended size. The "fine powder recycling step" encompasses an aspect in which fine powder itself is added as in the present invention, and also a step of adding the fine powder, in the form of a large hydrogel, during any of the steps for producing the water-absorbing resin.

[5] Optimum production method within the above-described method of producing particulate water-absorbing agent

According to conventional methods for producing a water-absorbing agent, control of particle shape and particle size distribution by gel-crushing with a high gel-grinding energy was difficult at a gel CRC of 33 or more. (This is because the gel has a decreased crosslinking density and softens at the gel CRC of 33 or more.) Through diligent study, however, the inventors of the present invention discovered that increasing the moisture content of the gel (reducing the solid content) so as to further lower the strength of the gel allows the particle shape and the particle size distribution to be controlled easily by gel-crushing, even in a case where the gel has a high CRC.

That is, while using the above-described production method, it is possible to obtain the particulate water-absorbing agent for use in the present invention more easily by controlling the gel CRC and the gel-grinding energy, further by controlling a circulation ratio in pulverization after drying, and further by preferably adding the chelating agent.

An example of the method of producing the particulate water-absorbing agent for use in the present invention is a production method of producing a particulate water-absorbing agent characterized by performing gel-crushing in such a manner that a gel-grinding energy (GGE) of 29 to 60 (or a gel-grinding energy (2) (GGE (2)) of 15 to 40) is applied to a hydrogel of a crosslinked polyacrylic acid polymer which has an average size per piece of the hydrogel of 3000 $\mu$m or more, a gel CRC of 33.0 g/g or more, and a moisture content of 50 mass% or more.

In the above production method, pulverization is performed at the above-described circulation ratio after drying, and preferably, the chelating agent and/or the moisture absorption fluidity improving agent is added.

In an aspect, the present invention provides a method of producing a particulate water-absorbing agent,

characterized by performing gel-crushing by applying, to a gel, an energy satisfying at least one of: (4) 29 J/g to 60 J/g, preferably 29 J/g to 55 J/g, more preferably 29 J/g to 50 J/gm, or 34 J/g to 60 J/g, preferably 34 J/g to 55 J/g, more preferably 34 J/g to 50 J/g as a gel-grinding energy (GGE); and (5) 15 J/g to 40 J/g, preferably 15 J/g to 38 J/g, more preferably 15 J/g to 35 J/g, or 19 J/g to 40 J/g, preferably 19 J/g to 38 J/g, more preferably 19 J/g to 35 J/g as a gel-grinding energy (2) (GGE (2)), the gel having the following features (1) through (3): (1) an average size of at least one side of the gel is 3000 $\mu$m or more, 5000 $\mu$m or more, 10 mm or more, 30 mm or more, 10 cm or more, 50 cm or more, or 100 cm or more; (2) the gel CRC of the gel is 33.0 g/g or more, 34.0 g/g or more, 35.0 g/g or more, 36.0 g/g or more, 37.0 g/g or more, 38.0 g/g or more, 39.0 g/g or more, or 40.0 g/g or more, and an upper limit value of the gel CRC is 45.0 g/g; and (3) the moisture content of the gel is 50 mass% or more, 51 mass% or more, 52 mass% or more, 53 mass% or more, 54 mass% or more, 55 mass% or more, 56 mass% or more, 57 mass% or more, 58 mass% or more, 59 mass% or more, 60 mass% or more, 61 mass% or more, 62 mass% or more, 63 mass% or more, 64 mass% or more, 65 mass% or more, 66 mass% or more, 67 mass% or more, 68 mass% or more, 69 mass% or more, or 70 mass% or more, and is 90 mass% or less.

Note that one side of a gel refers to a length (so called a long diameter) connecting between any two points that are the most distanced from each other and are both on the surface of the gel.

Conventional methods for producing a particulate water-absorbing agent do not involve crushing a high-CRC gel (having a gel CRC of 33 g/g or more) with use of a high gel-grinding energy (GGE of 18 J/g or more). In the present invention, a hydrogel after polymerization is subjected to crushing (gel-crushing) with use of a gel-grinding energy higher than that used in the conventional methods, so that the shapes of the particles of a particulate water-absorbing agent is controlled physically rather than chemically. This enables an increase in water absorption speed. This makes it possible to produce a particulate water-absorbing agent which achieves both a high fluid retention capacity and a high water absorption speed and further achieves a reduction in re-wet as compared with a conventional particulate water-absorbing agent.

According to conventional methods for producing a water-absorbing agent, it is difficult to perform, on a gel having a gel CRC of 33 or more, control of particle shape and particle size distribution by gel-crushing with use of a high gel-grinding energy, since the gel having a gel CRC of 33 or more has a decreased crosslinking density and is soft. Through diligent study, however, the inventors of the present invention discovered that increasing the moisture content of the gel (reducing the solid content) so as to further lower the strength of the gel allows the particle shape and the particle size distribution to be controlled easily by gel-crushing, even in a case where the gel has a high CRC.

According to a preferred embodiment, the method of producing a particulate water-absorbing agent is characterized in that (a) the gel-crushing is performed until a gel obtained has a particle diameter of 360 $\mu$m to 1500 $\mu$m, (b) the gel having a mass of 10 kg/m$^2$ to 50 kg/m$^2$ per unit area of band drying is dried for 10 minutes to 60 minutes at a drying temperature of 150°C to 200°C and an air velocity of hot air of 0.8 m/s to 2.5 m/s, preferably 0.003 m/s to 0.1 m/s, even more preferably 0.005 m/s to 0.06 m/s in a vertical direction (an up-and-down direction), and (c) the gel thus dried is subjected to a surface treatment. This allows producing a particulate water-absorbing agent which has characteristics such as (1) being less prone to undergo gel blocking (formation of an aggregate of particles of a particulate water-absorbing agent) even when the particulate water-absorbing agent absorbs liquid, (2) having an increased elastic modulus of swollen gel and an enhanced water absorbing power under load, and (3) having a good resistance to moisture adsorption blocking.

[6] Absorbent article

Further, an absorbent article of the present invention includes: the water-absorbing sheet for use in the present invention; a liquid-permeable sheet; and a liquid-impermeable sheet, the water-absorbing sheet being sandwiched between the liquid-permeable sheet and the liquid-impermeable sheet. Examples of the absorbent article encompass a disposable diaper, an incontinence pad, a sanitary napkin, a pet sheet, a drip sheet for food, a water blocking agent for power cables. Further, as the liquid-permeable sheet and the liquid-impermeable sheet, ones that are publicly known in the technical field of absorbent articles can be used without any particular limitation. The absorbent article can be produced by a publicly known method.

[7] Another particulate water-absorbing agent for use in accordance with the present invention suitable for production of water-absorbing sheet

The water-absorbing sheet for use in the present invention can be produced with use of another particulate water-absorbing agent below, together with the above-described particulate water-absorbing agent. For convenience, in order to distinguish the another particulate water-absorbing agent from the above-described particulate water-absorbing agent, the another particulate water-absorbing agent to be described below will be referred to as "water-absorbing agent 2." Note that the same descriptions as those given on the above-described particulate water-absorbing agent will be omitted.

The particulate water-absorbing agent 2 described in section [7] also satisfies the following physical properties: (i) a centrifuge retention capacity (CRC) of 30 g/g to 50 g/g, (ii) a mass average particle diameter (D50) of 200 $\mu$m to 600

$\mu$m, (iii) a DRC index defined by Formula (a) of 0 or more and 43 or less, and (iv) a surface tension of 65 or more. Therefore, the particulate water-absorbing agent has physical properties suitable for both the first particulate water-absorbing agent and the second particulate water-absorbing agent.

(Moisture absorption fluidity improving agent)

As a moisture absorption fluidity improving agent, the particulate water-absorbing agent 2 can contain: a multicomponent metal compound having a hydrotalcite structure and containing divalent and trivalent metal cations (two kinds of metal cations) and a hydroxyl group; and water-insoluble metal phosphate containing an anion of a phosphoric acid and a divalent or trivalent metal cation.

[7-1] Definitions of terms

(7-1-1) "Volume average particle diameter"

"Volume average particle diameter" refers to an average of volume-based particle diameters. A method of measuring a volume average particle diameter of a multicomponent metal compound will be described in detail in the Examples.

(7-1-2) "Crystallite diameter"

A crystallite refers to the largest aggregate that can be considered as a single crystal. Each crystal grain is constituted by a plurality of crystallites. A crystallite diameter indicates a diameter (size) of a crystallite. As the size of a crystallite decreases, the number of crystallites constituting each crystal grain increases and the number of diffraction gratings per crystallite decreases. As a crystallite diameter decreases, diffraction lines expand. International Publication No. 2015/152299 describes that a crystallite diameter of 0.15 $\mu$m or more prevents sufficiently decreasing the moisture adsorption blocking property. A measuring method will be described in detail in Examples.

(7-1-3) "Average primary particle diameter"

An average primary particle diameter of a water-insoluble metal phosphate used in the present invention refers to a specific surface area-equivalent diameter of the water-insoluble metal phosphate. A measuring method will be described in detail in Examples.

(7-1-4) "Diffusing absorbency"

A diffusing absorbency in the present disclosure refers to a physical property value for evaluating an absorption amount of a water-absorbing resin, and takes account of diffusion ability of a water-based liquid in a case where the water-absorbing resin has a high basis weight and is in a state where particles of the water-absorbing resin are in close contact due to an external force. The diffusing absorbency is calculated from a measured value obtained by measurement under certain conditions after an elapse of a certain period of time (e.g., after an elapse of 60 minutes or 10 minutes) from a start of adsorption. A measuring method will be described in detail in the Examples.

A diffusing absorbency after 60 minutes of the particulate water absorbing agent 2 for use in accordance with the present invention is preferably 18 g/g or more, more preferably 20 g/g or more, most preferably 22 g/g or more. Typically, a diffusing absorbency after 60 minutes of a water-absorbing agent which has been subjected to a surface-crosslinking treatment is 18 g/g or more. However, some water-absorbing agents, though not common, have a low diffusing absorbency after 60 minutes. A particulate water-absorbing agent having a low diffusing absorbency after 60 minutes has a degraded diffusion property in an absorbent body and may be unable to exhibit sufficient performance as an absorbent body, despite having an excellent DRC or an excellent DRC index. An upper limit of the diffusing absorbency after 60 minutes is not particularly limited but is typically approximately 40 g/g or less.

A diffusing absorbency after 10 minutes of the particulate water absorbing agent 2 for use in accordance with the present invention is preferably 7 g/g or more, more preferably 9 g/g or more, even more preferably 11 g/g or more, and most preferably 13 g/g or more. Typically, a diffusing absorbency after 10 minutes of a water-absorbing agent which has been subjected to a surface-crosslinking treatment is 7 g/g or more. However, some water-absorbing agents, though not common, have a low diffusing absorbency after 10 minutes. A particulate water-absorbing agent having a low diffusing absorbency after 10 minutes has a degraded diffusion property in an absorbent body and may be unable to exhibit sufficient performance as an absorbent body, despite having an excellent DRC or an excellent DRC index. An upper limit of the diffusing absorbency after 10 minutes is not particularly limited but is typically approximately 30 g/g or less.

(7-1-5) "Re-wet"

In the present invention, re-wet refers to an amount of liquid, which has been absorbed by an absorbent body, is released back due to a pressure applied to the absorbent body. A measuring method will be described in detail in the Examples.

(7-1-6) "DRC5min contribution rate"

"DRC5min contribution rate" refers to a total of ratios of particles that satisfy DRC5min conditions defined for respective particle diameter ranges among the particles constituting the particulate water-absorbing agent 2.
For example, among the particles constituting the particulate water-absorbing agent 2, in a case where

(1) a DRC5min of particles with a particle diameter of 850 $\mu$m to 600 $\mu$m is 24 g/g to 44 g/g,
(2) a DRC5min of particles with a particle diameter of 600 $\mu$m to 500 $\mu$m is 29 g/g to 46 g/g,
(3) a DRC5min of particles with a particle diameter of 500 $\mu$m to 425 $\mu$m is 32 g/g to 49 g/g,
(4) a DRC5min of particles with a particle diameter of 425 $\mu$m to 300 $\mu$m is 37 g/g to 53 g/g, and
(5) a DRC5min of particles with a particle diameter of 300 $\mu$m to 150 $\mu$m is 41 g/g to 60 g/g,

and the particles satisfying (1) account for 10%, the particles satisfying (2) account for 20%, the particles satisfying (3) account for 15%, the particles satisfying (4) account for 10%, and the particles satisfying (5) account for 5%, respectively, of the whole particles constituting the particulate water-absorbing agent 2, a DRC5min contribution rate is 60%.

(Substance(s) added to aqueous monomer solution)

The particulate water-absorbing agent 2 for use in accordance with the present invention may be configured such that, other than the above-described substances, $\alpha$-hydroxycarboxylic acid (salt) is added to the aqueous monomer solution during the preparation thereof from the viewpoint of improved physical properties for a water-absorbing resin to be produced.

($\alpha$-hydroxycarboxylic acid (salt))

Ordinarily, from the viewpoint of the water absorbent property, color (coloring prevention), and the like in the water-absorbing agent to be obtained, it is preferable to add $\alpha$-hydroxycarboxylic acid. Addition of the $\alpha$-hydroxycarboxylic acid reduces the molecular weight of a water-soluble component in a water-absorbing agent to be produced, and accordingly reduces stickiness and discomfort during use of the water-absorbing agent as a hygienic material. Note that "a-hydroxycarboxylic acid (salt)" is a carboxylic acid having a hydroxyl group in a molecule or is a salt thereof, and is a hydroxycarboxylic acid having a hydroxyl group at an alpha position or is a salt thereof.
Specifically, as the $\alpha$-hydroxycarboxylic acid (salt), a compound and an amount used thereof disclosed in "[6] $\alpha$-hydroxycarboxylic acid compound" of International Publication No. 2011/040530 can be applied to the present invention.
Hydroxycarboxylic acid is a carboxylic acid which includes a hydroxyl group in a molecule thereof. Examples of the hydroxycarboxylic acid encompass: an aliphatic hydroxy acid such as lactic acid, glycolic acid, malic acid, glycerinic acid, tartaric acid, citric acid, isocitric acid, mevalonic acid, chinic acid, shikimic acid, or $\beta$-hydroxypropionic acid; an aromatic hydroxy acid such as salicylic acid, creosotic acid, vanillin acid, syringic acid, resorcylic acid, pyrocatechuic acid, protocatechuic acid, gentisic acid, orsellinic acid, mandelic acid, gallic acid; or a salt thereof.
In a case where the $\alpha$-hydroxycarboxylic acid is a salt in the present invention, the salt is preferably a monovalent salt from the viewpoint of solubility in water, and an alkali metal salt such as lithium, potassium, or sodium, an ammonia salt, a monovalent amine salt, or the like is preferably used. In a case where $\alpha$-hydroxy polyvalent carboxylic acid is used as a salt, all carboxyl groups may be a salt, or only part of the carboxyl groups may be a salt.
"$\alpha$-hydroxycarboxylic acid (salt)" refers to $\alpha$-hydroxycarboxylic acid and/or a salt thereof. Likewise, " acid (salt)" refers to " acid" and/or a salt thereof. Specifically, malic acid (salt) refers to malic acid and/or a salt thereof, and lactic acid (salt) refers to lactic acid and/or a salt thereof.
The above substances are not necessarily added to the aqueous monomer solution, but can be added during the polymerization, or can be added both to the aqueous monomer solution and during the polymerization.
In a case where a water-soluble resin or a water-absorbing resin is used as the hydrophilic polymer, a graft polymer or a water-absorbing resin composition (for example, a polymer produced from starch and an acrylic acid or a polymer produced from PVA and an acrylic acid) can be obtained. These polymers and water-absorbing resin compositions are also encompassed in the scope of the present invention.

[7-2] Method of producing polyacrylic acid (salt)-based particulate water-absorbing agent 2

In a production process for producing the particulate water-absorbing agent 2 for use in accordance with the present invention, steps up to a classification step are identical to the classification step for the above-described particulate water-absorbing agent. With regard to gel-crushing conditions and forms other than those of the gel-crushing step for the above-described particulate water-absorbing agent, the disclosure of International Publication No. 2011/126079 can be preferably applied to the present invention.

The water-absorbing resin powder obtained in the present invention has a weight average particle diameter (D50) which ranges preferably from 200 μm to 600 μm, more preferably 200 μm to 550 μm, even more preferably 250 μm to 500 μm, and especially even more preferably 350 μm to 450 μm. The water-absorbing resin powder contains particles with a particle diameter of less than 150 μm at a proportion of preferably 10 weight% or less, more preferably 5 weight% or less, even more preferably 1 weight% or less, and contains particles with a particle diameter of 850 μm or more at a proportion of preferably 5 weight% or less, more preferably 3 weight% or less, and even more preferably 1 weight% or less. A lower limit value of each of the proportions of such particles is preferably as low as possible and is desirably 0 weight%. Note, however, that a lower limit of each of the proportions of such particles can be approximately 0.1 weight%. The water-absorbing resin powder has a logarithmic standard deviation (σζ) of a particle size distribution which falls in a range of preferably 0.20 to 0.50, more preferably 0.25 to 0.40, and still more preferably 0.27 to 0.35. Note that these particle sizes are measured with use of a standard sieve in conformity with a measuring method disclosed in U.S. Patent No. 7638570 or EDANA ERT 420.2-02.

The above particle sizes apply not only to water-absorbing resin subsequent to surface crosslinking (for convenience, hereinafter referred to also as "water-absorbing resin particle(s)"), but also to the particulate water-absorbing agent 2 as a final product. Therefore, it is preferable to subject the water-absorbing resin particles to surface crosslinking (surface-crosslinking step) so that the particle size falling within the above described range is maintained, and it is more preferable to carry out particle size adjustment by carrying out a sizing step subsequent to the surface-crosslinking step.

In a production process for producing the particulate water-absorbing agent 2 for use in accordance with the present invention, the surface-crosslinking step is identical to the surface-crosslinking step for the above-described particulate water-absorbing agent.

[7-3] Physical properties of particulate water-absorbing agent 2

In a case where the polyacrylic acid (salt)-based water-absorbing agent 2 produced by the method in accordance with the present disclosure is used for a sanitary material (especially a disposable diaper), it is desirable to control, apart from the CRC of (7-3-2) which is 30 g/g to 50 g/g, at least one of the physical properties of (7-3-1) to (7-3-10), preferably two or more of the physical properties, including the AAP, of (7-3-1) to (7-3-10); more preferably three or more of the physical properties, including the AAP, of (7-3-1) to (7-3-10); and most preferably all of the physical properties of (7-3-1) to (7-3-10), such that the physical properties each fall within a desired range. Having physical properties which do not satisfy the below ranges may prevent sufficiently achieving effects of the present invention and achieving sufficient performance in a high-concentration disposable diaper.

The polyacrylic acid (salt)-based particulate water-absorbing agent 2 produced by the method in accordance with the present disclosure is not limited to any particular shape of the particulates. The following description will discuss physical properties of the particulate water-absorbing agent 2 or the water-absorbing resin. The physical properties below are measured in accordance with EDANA method unless otherwise specified.

(7-3-1) DRC5min (Dunk Retention Capacity 5minutes)

The DRC5min of the particulate water-absorbing agent 2 for use in the present invention is not particularly limited, provided that a DRC index described later in section (7-5-2) is satisfied. It is preferable, however, that the DRC5min is 35 g/g or more, 38 g/g or more, or 40 g/g or more. An upper limit value of the DRC5min is not particularly limited but is ordinarily 60 g/g or less, or 55 g/g or less.

(7-3-2) CRC (Fluid retention capacity without pressure)

The particulate water-absorbing agent 2 for use in the present invention has a CRC (fluid retention capacity without pressure) of 30 g/g to 50 g/g, preferably 31 g/g to 50 g/g, 32 g/g to 50 g/g, 33 g/g to 50 g/g, 34 g/g to 50 g/g, 35 g/g to 50 g/g, 36 g/g to 50 g/g, 30 g/g to 49 g/g, 30 g/g to 48 g/g, 30 g/g to 47 g/g, 30 g/g to 46 g/g, 30 g/g to 45 g/g, 30 g/g to 44 g/g, 30 g/g to 43 g/g, 30 g/g to 42 g/g, 30 g/g to 41 g/g, 30 g/g to 40 g/g, 30 g/g to 39 g/g, or 30 g/g to 38 g/g.

If the CRC is less than 5 g/g, then an absorption amount is small. This renders a particulate water-absorbing agent unsuitable as an absorbent body of a sanitary material such as a disposable diaper. If the CRC is more than 70 g/g, then a rate at which, for example, a body fluid such as urine or blood is absorbed decreases. This renders a particulate water-absorbing agent unsuitable for use in, for example, a disposable diaper having a high water absorption speed. Note that CRC can be controlled with use of, for example, an internal crosslinking agent and/or a surface-crosslinking agent.

(7-3-3) Gel CRC

The CRC (gel CRC), before gel-crushing, of a hydrogel of the particulate water-absorbing agent 2 obtained by a production method in accordance with the present disclosure is identical to the CRC (gel CRC), before gel-crushing, of the hydrogel of the above-described particulate water-absorbing agent.

(7-3-4) Fluid retention capacity under pressure (AAP)

The particulate water-absorbing agent 2 for use in accordance with the present invention has a fluid retention capacity under pressure (AAP) of preferably 18 g/g or more, more preferably 22 g/g or more, even more preferably 24 g/g or more, especially even more preferably 26 g/g or more, especially still even more preferably 28 g/g or more, and most preferably 30 g/g or more. The upper limit value of the AAP is not limited to any particular value, but is preferably 40 g/g or less.

If the AAP is less than 18 g/g, then the re-wet of a liquid when a pressure is applied to an absorbent body becomes large. This means that such a particulate water-absorbing agent is unsuitable as an absorbent body of a sanitary material such as a disposable diaper. Note that AAP can be controlled with use of particle size, surface-crosslinking agent, or the like.

In a case where the particulate water-absorbing agent 2 satisfies the above condition, a disposable diaper produced with use of the particulate water-absorbing agent 2 has an excellent ability to absorb urine from pulp and can have a reduced re-wet. This makes it possible to prevent rash and urine leakage.

(7-3-5) Particle size (particle size distribution, weight average particle diameter (D50), and logarithmic standard deviation ($\sigma\zeta$) of particle size distribution)

The particulate water-absorbing agent 2 for use in the present invention has a particle size (a particle size distribution, a weight average particle diameter (D50), and a logarithmic standard deviation ($\sigma\zeta$) of the particle size distribution) which is controlled so as to be the same as the particle size of the water-absorbing resin powder before being subjected to surface crosslinking.

(7-3-6) Saline flow conductivity (SFC)

Saline flow conductivity (SFC) of the particulate water-absorbing agent 2 obtained by a production method in accordance with the present disclosure is identical to the saline flow conductivity (SFC) of the above-described particulate water-absorbing agent.

(7-3-7) Yellowness (YI value/Yellow Index)

The particulate water-absorbing agent 2 obtained by a production method in accordance with the present disclosure has yellowness (YI value/Yellow Index, see European Patent No. 942014 and European Patent No. 1108745) which is preferably 0 to 17, more preferably 0 to 16, even more preferably 0 to 15, and most preferably 0 to 14, and preferably has little yellowing. Examples of a method of measurement of color encompass the method disclosed in International Publication, No. 2009/005114 (method of measuring Lab value, YI value, WB value, and the like).

Causing the particulate water-absorbing agent 2 to satisfy the conditions above makes it possible, when the particulate water-absorbing agent 2 is used in combination with a hygienic material, to produce a disposable diaper which does not cause a user to have a feeling of a foreign body due to coloration.

According to a preferred embodiment, after a colorations acceleration test (maintained for 1 week at 70°C and 65 RH%), the particulate water-absorbing agent 2 for use in the present invention has a YI value of 35 or less, preferably 30 or less, more preferably 25 or less, and even more preferably 22 or less. Causing the particulate water-absorbing agent 2 to satisfy the conditions above makes it possible, when the particulate water-absorbing agent 2 is used in combination with a hygienic material, to produce a disposable diaper which does not cause a user to have a feeling of a foreign body due to coloration.

(7-3-8) Surface tension

The particulate water-absorbing agent 2 for use in accordance with the present invention has a surface tension (defined by a measuring method described in the Examples) of preferably 66 mN/m or more, more preferably 68 mN/m or more, even more preferably 70 mN/m or more, especially even more preferably 71 mN/m or more, and most preferably 72 mN/m or more. Further, the surface tension of the particulate water-absorbing agent 2 for use in the present invention does not undergo a substantial decrease caused by a surfactant or the like. 75 mN/m is ordinarily sufficient as an upper limit of the surface tension.

Satisfying the above conditions of surface tension allows for a reduction in re-wet of a disposable diaper.

(7-3-9) Particle shape

According to a preferred embodiment, the particle shape of the particulate water-absorbing agent 2 for use in the present invention is a non-uniformly pulverized shape. This is because: a particulate water-absorbing agent 2 having a non-uniformly pulverized shape has a specific surface area larger than that of spherical particles obtained by a reversed phase suspension polymerization or a vapor phase polymerization so that the particulate water-absorbing agent 2 has higher water absorption speed; and a particulate water-absorbing agent 2 having a non-uniformly pulverized shape can be more easily fixed to a pulp than in the case of spherical particles.

(7-3-10) GCA (Gel Capillary Absorption)

The value of GCA of the particulate water-absorbing agent 2 for use in the present invention is calculated by a method described in the Examples to be described later. A higher value of GCA indicates a better performance, and the value of GCA is preferably 27.0 g/g or more, more preferably 28 g/g or more, even more preferably 29 g/g or more, still even more preferably 30.0 g/g or more, and yet even more preferably 31 g/g. The GCA is preferably as high as possible. However, from the viewpoint of a balance with other physical properties, a preferable upper limit is ordinarily approximately 50.0 g/g.

In a case where the particulate water-absorbing agent 2 satisfies the above condition, a disposable diaper produced with the particulate water-absorbing agent 2 has an excellent ability to absorb urine and can have a reduced re-wet. This makes it possible to prevent rash and urine leakage.

(7-3-11) Increase in fine powder

According to a preferred embodiment, an amount of increase in particles having a particle diameter of 150 $\mu$m or less between before and after a damage resistance paint shaker test described in the Examples, among the particulate water-absorbing agent 2 for use in the present invention, is +5% or less, preferably +4% or less, more preferably +3% or less, even more preferably +2% or less, and still even more preferably +1% or less.

(7-3-12) Moisture adsorption blocking ratio (B.R.)

A specific method of measuring (evaluating) a moisture adsorption blocking ratio (B.R.) will be described later, but the moisture adsorption blocking ratio (B.R.) of the particulate water-absorbing agent 2 for use in the present invention is preferably 0 weight% to 50 weight%, more preferably 0 weight% to 40 weight%, even more preferably 0 weight% to 30 weight%, and most preferably 0 weight% to 10 weight%. If the moisture adsorption blocking ratio (B.R.) is more than 50 weight%, then the particulate water absorbing agent 2 is difficult to handle in humid conditions. This may pose a problem that, during production of a thin absorbent body for hygienic material, for example, the particulate water-absorbing agent 2 aggregates in a transport pipe in a production plant and therefore the transport pipe clogs and/or the particulate water-absorbing agent 2 cannot be uniformly mixed with hydrophilic fibers.

In a case where the condition above is satisfied, it becomes possible to decrease the adherence of the particulate water-absorbing agent 2 to equipment when an absorbent body is produced with use of the particulate water-absorbing agent 2 and a fiber material.

(7-3-13) Water-soluble content

According to a preferred embodiment, the particulate water-absorbing agent 2 for use in the present invention has a water-soluble content (Ext) of 25 weight% or less, preferably 24 weight% or less, more preferably 22 weight% or less, and even more preferably 20% weight% or less. In a case where the particulate water-absorbing agent 2 satisfies the above condition, an absorbing ability (e.g., fluid retention capacity under pressure) of the particulate water-absorbing agent 2 improves. Therefore, in a case where the particulate water-absorbing agent 2 is used in a disposable diaper, performance can be improved (such as a reduction in re-wet).

(7-3-14) Degradable soluble content

According to a preferred embodiment, the particulate water-absorbing agent 2 for use in the present invention has a degradable soluble content of 30 weight% or less, preferably 27 weight% or less, more preferably 24 weight% or less, and even more preferably 20% weight% or less. In a case where the particulate water-absorbing agent 2 satisfies the above condition, urine resistance improves. Therefore, in a case where the particulate water-absorbing agent 2 is used in a disposable diaper, problems caused by a body fluid such as urine can be prevented, examples of which encompass gel deterioration, skin irritation, rash, and a decrease in odor-removing ability.

(7-3-15) Gel-grinding energy (GGE)

A preferable upper limit value and a preferable lower limit value of the gel-grinding energy (GGE) of the particulate water-absorbing agent 2 obtained by a production method in accordance with the present disclosure are identical to the preferable upper limit value and the preferable lower limit value of the above-described gel-grinding energy (GGE) of a particulate water-absorbing agent. The gel-grinding energy (GGE) for gel-crushing of a hydrogel of the particulate water-absorbing agent 2 is 18 J/g to 60 J/kg, preferably 20 J/g to 50 J/kg, more preferably 25 J/g to 40 J/kg.

A preferable upper limit value and a preferable lower limit value of the gel-grinding energy (2) (GGE (2)) of the particulate water-absorbing agent 2 obtained by a production method in accordance with the present disclosure are identical to the preferable upper limit value and the preferable lower limit value of the gel-grinding energy (2) (GGE (2)) of the above-described particulate water-absorbing agent. The gel-grinding energy (2) (GGE (2)) for gel-crushing of a hydrogel of the particulate water-absorbing agent 2 is 9 J/g to 40 J/kg, preferably 12 J/g to 38 J/g, more preferably 15 J/g to 35 J/kg.

(7-3-16) Circulation crushing ratio

The circulation crushing ratio of the particulate water-absorbing agent 2 obtained by a production method in accordance with the present disclosure is identical to the circulation crushing ratio of the above-described particulate water-absorbing agent.

(7-3-17) Internal gas bubble ratio

The internal gas bubble ratio of the particulate water-absorbing agent 2 obtained by a production method in accordance with the present disclosure is identical to the internal gas bubble ratio of the above-described particulate water-absorbing agent. Controlling the internal gas bubble ratio to be within the above ranges allows obtaining a water-absorbing resin having the water absorption speed and liquid permeability defined in present invention.

(7-3-18) Bulk specific gravity

The bulk specific gravity of the particulate water-absorbing agent 2 obtained by a production method in accordance with the present disclosure is identical to the bulk specific gravity of the above-described particulate water-absorbing agent.

(7-3-19) Re-wet

The re-wet of an absorbent body produced with use of the particulate water-absorbing agent 2 for use in the present invention is preferably 14 g or less, more preferably 13.5 g or less, 13 g or less, 12.5 g or less, 12 g or less, 11.5 g or less, 11 g or less, 10.5 g or less, 10 g or less, 9.5 g or less, 9 g or less, 8.5 g or less, 8 g or less, 7.5 g or less, 7 g or less, 6.5 g or less, 6 g or less, 5.5 g or less, 5 g or less, 4.5 g or less, 4 g or less, 3.5 g or less, 3 g or less, or 2.5 g or less.

Other physical properties of the particulate water-absorbing agent 2 for use in the present invention are identical to those of the above-described particulate water-absorbing agent.

According to a preferred embodiment, the particulate water-absorbing agent 2 contains a polyacrylic acid (salt)-based water-absorbing resin as a main component, from the viewpoint of physical properties of a particulate water-absorbing agent 2 to be produced and productivity.

[7-4] Preferred embodiments

Preferred embodiments of the particulate water-absorbing agent 2 will be described below. It is to be understood that the embodiments described below are provided for better understanding of the present invention, and the scope of the present invention should not be limited to the descriptions below. It is therefore clear that a person skilled in the art can make modifications as appropriate within the scope of the present invention as defined in the claims in view of the descriptions in the present specification. It is also to be understood that each of the below embodiments of the present invention can be used individually or in combination with another/other embodiment(s).

(7-4-1) Method of determining whether or not fluid retention capacity and water absorption speed of particulate water-absorbing agent 2 are excellent

The present disclosure provides a method of determining whether or not a fluid retention capacity and a water absorption speed of the particulate water-absorbing agent 2 are both excellent. This method includes:

(1) a step of measuring a DRC5min of the water-absorbing agent;
(2) a step of measuring a weight average particle diameter (D50) of the water-absorbing agent; and
(3) a step of calculating, based on values measured in the steps (1) and (2),

General index of DRC (General index of DRC) = (K - DRC5min(g/g))/(D50($\mu$m)/ 1000)

where K is any constant,
the step (3) being carried out so that in a case where the general index of DRC is a certain value or less, it is determined that the water-absorbing agent has an intended fluid retention capacity and an intended water absorption speed.

This method allows a particulate water-absorbing agent 2, which has preferable physical properties, to be determined by measuring only the DRC5min and the weight average particle diameter (D50) of the water-absorbing agent. It is therefore also easy to optimize the step of producing a preferable particulate water-absorbing agent 2. As demonstrated in Examples, it is to be understood that a particulate water-absorbing agent 2 having excellent physical properties can be obtained by increasing a moisture content and a gel-grinding energy of a hydrogel.

The value of K in the general index of DRC is any constant. The value of K is, for example, 30, 35, 40, 45, 46, 47, 48, 49, 50, 51, 52, 53, 54, 55, 60, or 70. In a preferred embodiment, the value of K is 49, and this value is used for calculating the DRC index of the particulate water-absorbing agent 2 for use in the present invention.

With the present invention, it is possible to obtain a particulate water-absorbing agent 2 in which a high fluid retention capacity and a high water absorption speed are both achieved (i.e., the fluid retention capacity and the water absorption speed are both good). Even if an attempt is made to obtain a particulate water-absorbing agent 2 in which a high fluid retention capacity and a high water absorption speed are both achieved, a particulate water-absorbing agent 2 having a high water absorption speed cannot be easily obtained merely by using a raw material (hydrogel) having a high fluid retention capacity. This is because, without knowledge of a proper production process, a particulate water-absorbing agent having a low water absorption speed as demonstrated in Comparative Production Examples is obtained. The present disclosure provides a method of determining whether or not a fluid retention capacity and a water absorption speed of the particulate water-absorbing agent 2 are both good, so that it is possible to easily find conditions necessary for obtaining a particulate water-absorbing agent 2 in which a high fluid retention capacity and a high water absorption speed are both achieved.

(7-4-2) Characteristics of particulate water-absorbing agent 2

The particulate water-absorbing agent 2 for use in the present invention has:

g/g, a centrifuge retention capacity (CRC) of 30 g/g to 50
a weight average particle diameter (D50) of 200 $\mu$m to 600 $\mu$m, and
a DRC index of a specific value, namely, 43 or less, the DRC index being represented by the following Formula (11):

$$(\text{Index of DRC}) = (49 - \text{DRC5min}[g/g])/(D50[\mu m]/1000)$$

$$\dots \text{Formula (11)}.$$

Note that the DRC index are as described in Section [2-1]. Therefore, in this section, the detailed descriptions will be omitted.

In a case where the polyacrylic acid (salt)-based particulate water-absorbing agent 2 obtained according to the present disclosure is used for a sanitary material, particularly for a disposable diaper, the particulate water-absorbing agent 2 preferably has physical properties, AAP and B.R., which satisfy the conditions described in Section (7-3). It is desirable that, of the physical properties above, at least one, preferably two or more including AAP and/or B.R., more preferably three or more including AAP and/or B.R., and most preferably all of the physical properties are controlled to fall within certain ranges. If these physical properties do not satisfy the ranges, then there is a possibility that the effects of the present invention may not be sufficiently obtained, so that sufficient performance may not be exhibited in a high-concentration disposable diaper. According to the particulate water-absorbing agent 2 for use in the present invention, in which the DRC index is a specific value (namely, 43) or less, any or all of these physical properties can each fall within a desired range.

The particulate water-absorbing agent 2 for use in accordance with the present invention optionally contains at least one moisture absorption fluidity improving agent selected from the group consisting of: a multicomponent metal compound having a hydrotalcite structure and containing divalent and trivalent metal cations (two kinds of metal cations) and a hydroxyl group; and a water-insoluble metal phosphate containing a phosphate anion and a divalent or trivalent metal cation.

The present invention provides a particulate water-absorbing agent 2 for use in the absorbent article of the invention, which agent not only achieves both a high fluid retention capacity and a high water absorption speed (i.e., the fluid retention capacity and the water absorption speed are both good) but also achieves blocking prevention (moisture absorption fluidity) under highly humid conditions and/or high absorption amount under pressure. If the moisture absorption fluidity (B.R.) is more than 50 weight%, then the particulate water absorbing agent 2 is difficult to handle in humid conditions. This may pose a problem that, during production of a thin absorbent body for hygienic material, for example, the particulate water-absorbing agent aggregates in a transport pipe in a production plant and therefore the transport pipe clogs and/or the particulate water-absorbing agent cannot be uniformly mixed with hydrophilic fibers. If the absorption capacity under load (AAP) is less than 18 g/g, then the re-wet of a liquid when a pressure is applied to an absorbent body becomes large. This means that such a particulate water-absorbing agent is unsuitable as an absorbent body of a sanitary material such as a disposable diaper. According to the present disclosure, the particulate water-absorbing agent 2 can contain at least one moisture absorption fluidity improving agent selected from the group consisting of: a multicomponent metal compound having a hydrotalcite structure and containing divalent and trivalent metal cations (two kinds of metal cations) and a hydroxyl group; and a water-insoluble metal phosphate containing a phosphate anion and a divalent or trivalent metal cation. This allows the particulate water-absorbing agent 2 to be provided so as to achieve blocking prevention (moisture absorption fluidity) under highly humid conditions and/or high absorption amount under pressure.

A specific multicomponent metal compound having hydrotalcite structure and containing divalent and trivalent metal cations (two kinds of metal cations) and a hydroxyl group is as described above.

(Method of adding and mixing multicomponent metal compound)

The method of producing the water-absorbing agent for use in the present invention can include a step of adding a multicomponent metal compound (hereinafter referred to as "multicomponent metal compound addition step"). The multicomponent metal compound addition step is a step of adding a multicomponent metal compound to a water-absorbing resin powder. The multicomponent metal compound addition step is preferably carried out after a drying step, and more preferably carried out after a pulverizing step and a classification step. In addition, the multicomponent metal compound addition step is preferably carried out before and/or after a surface-crosslinking step (i.e., the surface-crosslinking step is carried out before and/or after the multicomponent metal compound addition step), and particularly preferably carried out after the surface-crosslinking step (i.e., the multicomponent metal compound is added to a surface-crosslinked water-absorbing resin powder). In addition, the multicomponent metal compound addition step can be carried out a plurality of times. In such a case, the multicomponent metal compound addition step is carried out at least once after a drying step, more preferably at least once after a pulverizing step and a classification step, even more preferably at least once before and/or after a surface-crosslinking step, and particularly preferably at least once after a surface-crosslinking step.

It is preferable to dry-mix the multicomponent metal compound and the water-absorbing resin powder for use in the present invention. Dry-mixing is preferable because dry-mixing leads to a reduction in the amount of dust of a water-absorbing agent to be produced. Dry-mixing means mixing substances without adding a liquid substance. Note that a mixture obtained by the dry-mixing can contain a liquid substance which is absorbed or retained by a multicomponent metal compound and/or by a water-absorbing resin powder to be dry-mixed. Specifically, the dry-mixing can be carried out in a form in which, for example, a multicomponent metal compound (containing a moisture absorption content and an organic compound retained between layers) and a water-absorbing resin powder (containing, for example, a dry residue, a moisture absorption content, and a surface-crosslinking agent and a solvent added during the surface-crosslinking agent addition step) can be contained in a mixture, but no further liquid substance is added.

After the multicomponent metal compound is added, it is preferable to sufficiently mix in the multicomponent metal compound so as to sufficiently obtain the effects of the multicomponent metal compound. Specific conditions of mixing can be decided as appropriate according to a device used, the amount of particulate water-absorbing resin powder and multicomponent metal compound mixed, or the like. Examples of a method which can be employed encompass: a method in which a multicomponent metal compound is mixed in by stirring with use of a Loedige mixer for approximately 30 seconds to 1 minute at a rotation speed of 300 rpm; and a method in which a multicomponent metal compound is mixed in by stirring with use of a paddle-type stirring device for 20 minutes to 1 hour at a rotation speed of 60 rpm. Other examples of the method encompass: a method in which a multicomponent metal compound is mixed in while vibration is provided; and a method in which a multicomponent metal compound is added while a water-absorbing resin powder is being stirred.

A specific water-insoluble metal phosphate containing phosphate anions and divalent or trivalent metal cations is as described above.

(Method of mixing water-insoluble metal phosphate)

A step of adding a water-insoluble metal phosphate is carried out after the drying step. The step of adding a water-insoluble metal phosphate (hereinafter referred to as "water-insoluble metal phosphate addition step") is carried out preferably after the pulverizing step and the classification step, more preferably before and/or after the surface-crosslinking step, and particularly preferably after the surface-crosslinking step. In addition, the step of adding a water-insoluble metal phosphate can be carried out a plurality of times. In such a case, the step of adding a water-insoluble metal phosphate is carried out at least once after the drying step, more preferably at least once after the pulverizing step and the classification step, even more preferably at least once before and/or after the surface-crosslinking step, and particularly preferably at least once after the surface-crosslinking step.

The water-insoluble metal phosphate for use in the present invention can be added to a water-absorbing resin powder while the water-insoluble metal phosphate is in the form of a slurry aqueous solution, or can be added while being in a particulate form. Note, however, that it is preferable that the water-insoluble metal phosphate is dry-mixed with a water-absorbing resin powder obtained in the drying step. The term "dry-mixing" means as described above. Note that a mixture obtained by the dry-mixing can contain a liquid substance which is absorbed or retained by a water-insoluble metal phosphate and/or by a water-absorbing resin powder to be dry-mixed. Specifically, the dry-mixing can be carried out in a form in which, for example, a water-insoluble metal phosphate and a water-absorbing resin powder (containing, for example, a dry residue, a moisture absorption content, and a surface-crosslinking and a solvent agent added during the surface-crosslinking agent addition step) can be contained in a mixture, but no further liquid substance is added.

After the water-insoluble metal phosphate is added to the water-absorbing resin powder, it is preferable to sufficiently mix the water-insoluble metal phosphate and the water-absorbing resin powder so as to sufficiently obtain the effects of the present invention. Specific conditions of mixing can be decided as appropriate according to a device used, the amount of particulate water-absorbing resin powder and water-insoluble metal phosphate mixed, or the like. Examples of a method which can be employed encompass: a method in which a water-insoluble metal phosphate is mixed in by stirring with use of a Loedige mixer for approximately 30 seconds to 1 minute at a rotation speed of 300 rpm; and a method in which a water-insoluble metal phosphate is mixed in by stirring with use of a paddle-type stirring device for 20 minutes to 1 hour at a rotation speed of 60 rpm. Alternatively, it is possible to employ a method in which a water-insoluble metal phosphate is added while a water-absorbing resin powder is being stirred.

Examples of a device for mixing a water-absorbing resin and a water-insoluble metal phosphate encompass a cylindrical mixer, a screw type mixer, a screw extruder, Turbulizer, a Nauter mixer, a V-shaped mixer, a ribbon mixer, a double-armed kneader, a fluidization mixer, an air mixer, a rotating disc mixer, a roll mixer, a tumbling mixer, and a Loedige mixer. Examples of a mixing method encompass a batch type method, a continuous type method, and a combination of a batch type method and a continuous type method. From the viewpoint of industrial production, continuous mixing is preferable.

Mixing conditions are preferably set so that the water-absorbing resin powder is not damaged. For example, a rotation speed of a stirring section of a mixing device is preferably 1 rpm to 3000 rpm, more preferably 2 rpm to 500 rpm, and even more preferably 5 rpm to 300 rpm. A rotation speed of 3000 rpm or less makes it unlikely for the water-absorbing resin powder to be crushed, so that it is possible to prevent deterioration of a water absorbent property. A rotation speed of 1 rpm or more allows for sufficient mixing, so that it is possible to suitably obtain the effect of reducing a moisture adsorption blocking property (i.e., the effect of improving moisture absorption fluidity).

A temperature of the water-absorbing resin used in the multicomponent metal compound addition step and the water-insoluble metal phosphate addition step is preferably room temperature to 200°C, more preferably 50°C to 200°C, and even more preferably 50°C to 100°C.

A mixing time is preferably 1 second to 20 minutes, more preferably 10 seconds to 10 minutes, and even more preferably 20 seconds to 5 minutes. A mixing time of 20 minutes or less can prevent the water-absorbing resin from being crushed.

Mixing conditions for obtaining the particulate water-absorbing agent 2 for use in the present invention are most preferably set so that a temperature of the water-absorbing resin powder is 50°C to 100°C, the rotation speed of the stirring section is 5 rpm to 300 rpm, and a mixing time is 20 seconds to 5 minutes. The particulate water-absorbing agent 2 after the mixing under the conditions above is excellent in handleability, and does not cause a problem of adhesion, aggregation, or the like. This makes it unnecessary to further carry out a drying step. In a case where a certain amount (e.g., the above addition amount) of water is left in the particulate water-absorbing agent 2 by drying the particulate water-absorbing agent 2 to a proper extent, it is possible to suppress electrification of the particulate water-absorbing agent 2 and to impart excellent impact resistance (abrasion resistance) to the particulate water-absorbing agent 2.

In the present invention, the following can be both used: a multicomponent metal compound having a hydrotalcite structure and containing divalent and trivalent metal cations (two kinds of metal cations) and a hydroxyl group; and a water-insoluble metal phosphate containing phosphate anions and divalent or trivalent metal cations. In a case where a plurality of kinds of additives are present, it is possible to add the additives together or individually. In a case where the additives are individually added, the additives can be added at intervals of, for example, 10 seconds, 30 minutes, 1 minute, 3 minutes, 5 minutes, 10 minutes, 15 minutes, 20 minutes, 30 minutes, 40 minutes, 50 minutes, 60 minutes, 90 minutes, 120 minutes, or more.

In a case where the multicomponent metal compound and the water-insoluble metal phosphate are both used, the multicomponent metal compound and the water-insoluble metal phosphate are added in a total amount of 0.01 parts by weight to 1.0 part by weight, preferably 0.02 parts by weight to 0.7 parts by weight, and even more preferably 0.03 parts by weight to 0.5 parts by weight, relative to 100 parts by weight of the particulate water-absorbing agent 2 or of the water-absorbing resin.

In an aspect of the present disclosure, an absorbent body including the particulate water-absorbing agent 2 is provided. According to the absorbent body, a feeling of a foreign body due to coloration is not given to a user, and the problems caused by a body fluid such as urine can be prevented, such as gel deterioration, skin irritation, rash, deteriorated odor-removing ability, skin rash, and urine leakage.

In an aspect, the present disclosure provides a sanitary product including the absorbent body. According to the sanitary product (such as a disposable diaper), a feeling of a foreign body due to coloration is not given to a user, and the problems caused by a body fluid such as urine can be prevented, such as gel deterioration, skin irritation, rash, deteriorated odor-removing ability, skin rash, and urine leakage.

In an aspect, the present disclosure can provide a particulate water-absorbing agent 2 which, unlike the conventional techniques, achieves both a high fluid retention capacity and a high water absorption speed and which achieves blocking prevention under highly humid conditions.

In an aspect, the present disclosure can provide a particulate water-absorbing agent 2 which, unlike the conventional techniques, achieves both a high fluid retention capacity and a high water absorption speed and which achieves high absorption capacity under load.

In an aspect, the present disclosure can provide a particulate water-absorbing agent 2 which, unlike the conventional techniques, achieves both a high fluid retention capacity and a high water absorption speed and which achieves high absorption capacity under load and blocking prevention under highly humid conditions.

(7-4-3) Method of producing particulate water-absorbing agent 2

In an aspect, the present disclosure provides a method of producing a particulate water-absorbing agent 2, characterized by performing gel-crushing by applying an energy to a gel, the gel having the following features (1) through (3):

(1) at least one side of the gel has an average size of 3000 μm or more, 5000 μm or more, 10 mm or more, 30 mm or more, 10 cm or more, 50 cm or more, or 100 cm or more; and
(2) a gel CRC is 33.0 g/g or more, 34.0 g/g or more, 35.0 g/g or more, 36.0 g/g or more, 37.0 g/g or more, 38.0 g/g or more, 39.0 g/g or more, or 40.0 g/g or more and has an upper limit value of 45.0 g/g,

the energy satisfying at least one of the following (3) and (4):

(3) having a gel-grinding energy (GGE) of 20 J/g to 60 J/g, preferably 24 J/g to 55 J/g, and more preferably 28 J/g to 50 J/g; and
(4) having a gel-grinding energy (2) (GGE (2)) of 9 J/g to 40 J/g, preferably 12 J/g to 38 J/g, and more preferably 15 J/g to 35 J/g.

According to a preferred embodiment, (5) a moisture content is 50 weight% or more, 51 weight% or more, 52 weight% or more, 53 weight% or more, 55 weight% or more, 60 weight% or more, or 70 weight% or more, and 90 weight% or less.

A typical production process of producing the particulate water-absorbing agent 2 for use in accordance with the present invention is described in the above descriptions of the steps in the method of producing the particulate water-absorbing agent.

(Method of producing water-absorbing agent)

In an aspect of the present disclosure, provided is a method of producing a water-absorbing agent having a centrifuge retention capacity (CRC) of 30 g/g or more, the method including: a polymerization step of polymerizing an aqueous monomer solution containing an acrylic acid (salt); a drying step; a surface-crosslinking step; and a step of adding an $\alpha$-hydroxycarboxylic acid (salt) before the drying step. While it is not desirable to be bound by a theory, in production of a water-absorbing agent having a high fluid retention capacity, addition of an $\alpha$-hydroxycarboxylic acid (salt) before the drying step reduces a molecular weight of a soluble component contained in a polymer to be generated. Thus, a reduction in molecular weight of a soluble component to be eluted in a case where a water-absorbing agent has swelled due to liquid absorption decreases a viscosity of the soluble component. This results in a reduction in stickiness and discomfort during use of the water-absorbing agent as a hygienic material.

Addition of an optional additive before the drying step may be referred to as "internal addition", and addition of an optional additive after the drying step may be referred to as "external addition". In production of an absorbing agent having a high fluid retention capacity, internal addition of an $\alpha$-hydroxycarboxylic acid (salt) achieves a reduction in molecular weight of a soluble component to be eluted in a case where a water-absorbing agent has swelled due to liquid absorption. This results in a reduction in stickiness, which leads to discomfort, during actual use of, for example, a disposable diaper.

The water-absorbing agent for use in the present invention can be configured such that from the viewpoint of, for example, color (coloration prevention) and deterioration prevention, an optional chelating agent (e.g., diethylenetriamine pentaacetic acid (DTPA), ethylenediaminetetra(methylene phosphinic acid) (EDTMP), or the like) is externally added. Furthermore, the water-absorbing agent for use in the present invention can be configured such that an $\alpha$-hydroxycarboxylic acid (salt) is internally added, and from the viewpoint of, for example, color (coloration prevention) and deterioration prevention, an optional chelating agent (e.g., diethylenetriamine pentaacetic acid (DTPA) or a salt thereof, ethylenediaminetetra(methylene phosphinic acid) (EDTMP) or a salt thereof, or the like) is externally added.

According to a preferred embodiment, the $\alpha$-hydroxycarboxylic acid (salt) is added before, during, or after the polymerization step. The $\alpha$-hydroxycarboxylic acid (salt) is more preferably added before or during the polymerization step. Specifically, the $\alpha$-hydroxycarboxylic acid (salt) is preferably added to the aqueous monomer solution which has not been polymerized. Alternatively, the $\alpha$-hydroxycarboxylic acid (salt) is preferably added to the aqueous monomer solution which has started to be polymerized, specifically, to the aqueous monomer solution which has been being polymerized for 2 minutes since the start of the polymerization. The $\alpha$-hydroxycarboxylic acid (salt) can be added at any point in time between the start of the polymerization and the end of the polymerization of the aqueous monomer solution (e.g., after a lapse of, for example, 1 minute, 2 minutes, 3 minutes, 5 minutes, 10 minutes, or 20 minutes, since the start of the polymerization).

According to another preferred embodiment, the method further includes a gel-crushing step after the polymerization step and before the drying step, the $\alpha$-hydroxycarboxylic acid (salt) being added before or during the gel-crushing step. The $\alpha$-hydroxycarboxylic acid (salt) is more preferably added during the gel-crushing step. Specifically, the $\alpha$-hydroxycarboxylic acid (salt) is added while a hydrogel to be obtained after the polymerization is being subjected to gel-crushing.

According to a further preferred embodiment, the $\alpha$-hydroxycarboxylic acid (salt) is added before or during the polymerization step.

[8] Yet another particulate water-absorbing agent suitable for production of water-absorbing sheet

In the water-absorbing sheet for use in the present invention, any of particulate water-absorbing agents described in, for example, Patent Literatures Japanese Patent Application Publication, Tokukaihei, No. 08-57311 and Japanese Translation of PCT International Application, Tokuhyo, No. 2009-531158 can also be suitably used. In the following description, such a water-absorbing agent will be referred to as a "particulate water-absorbing agent 3" so as to be distinguished from the particulate water-absorbing agents which have been discussed in Sections [2] and [7]. The particulate water-absorbing agent 3 satisfies the following conditions: (i) a centrifuge retention capacity (CRC) is 30 g/g to 50 g/g; (ii) a mass average particle diameter (D50) is 200 $\mu$m to 600 $\mu$m; and (iii) a DRC index defined by Formula (a) is more than 43 and 50 or less. Therefore, the particulate water-absorbing agent 3 has physical properties suitable for the first particulate water-absorbing agent.

Preferable physical properties of the particulate water-absorbing agent 3 and a method of producing the particulate water-absorbing agent 3 are as described in, for example, the aforementioned Patent Literatures Japanese Patent Application Publication, Tokukaihei, No. 08-57311 and Japanese Translation of PCT International Application, Tokuhyo, No. 2009-531158.

The particulate water-absorbing agent 3 which is used in the water-absorbing sheet for use in the present invention is preferably used in combination with the particulate water-absorbing agent described in Section [2] or the particulate water-absorbing agent described in Section [7]. In this case, the particulate water-absorbing agent 3 and the particulate water-absorbing agent described in Section [2] or the particulate water-absorbing agent described in Section [7] are preferably localized to respective surfaces of the water-absorbing sheet. In a case where such a water-absorbing sheet is incorporated in an absorbent article, the water-absorbing sheet is preferably provided so that a surface to which the particulate water-absorbing agent 3 is localized serves as an upper layer.

This is because the particulate water-absorbing agent 3 is superior in diffusion of a liquid and the particulate water-absorbing agent described in Section [2] or the particulate water-absorbing agent described in Section [7] is superior in absorption speed. In a case where a particulate water-absorbing agent sandwiched in a water-absorbing sheet is configured as described above, the particulate water-absorbing agent allows a liquid having come into contact with the water-absorbing sheet to be immediately diffused and allows the liquid thus diffused to be subsequently immediately absorbed. Thus, a water-absorbing sheet in which such a particulate water-absorbing agent is sandwiched can be a water-absorbing sheet having a reduced re-wet.

[9] Particulate water-absorbing agent used for water-absorbing sheet for use in the present invention

A water-absorbing sheet for use in the present invention can be a water-absorbing sheet produced with use of a particulate water-absorbing agent described below. In addition, the particulate water-absorbing agent can be used for a method of producing the water-absorbing sheet for use in the present invention.

[1] A particulate water-absorbing agent satisfying the following:

> g/g; a centrifuge retention capacity (CRC) is 30 g/g to 50
> a weight average particle diameter (D50) is 200 μm to 600 μm; and
> a DRC index defined by the following formula is 34 or less:

$$\text{(Index of DRC)} = (49 - \text{DRC5min}[g/g])/(D50[\mu m]/\, 1000).$$

The particulate water-absorbing agent described in [1] above is present as the second particulate water-absorbing agent included in the absorbent article according to the claimed invention.

[2] The particulate water-absorbing agent described in [1], in which the DRC index is 30 or less.

[3] The particulate water-absorbing agent described in [1] or [2], in which the DRC index is 20 or less.

[4] The particulate water-absorbing agent described in any one of [1] through [3], in which a saline flow conductivity (SFC) is 0 (x $10^{-7} \cdot cm^3 \cdot s \cdot g^{-1}$) or more and less than 30 (x $10^{-7} \cdot cm^3 \cdot s \cdot g^{-1}$).

[5] The particulate water-absorbing agent described in any one of [1] through [4], in which a surface tension is 66 mN/m or more.

[6] The particulate water-absorbing agent described in any one of [1] through [5], in which a particle shape is a non-uniformly pulverized shape.

[7] The particulate water-absorbing agent described in any one of [1] through [6], in which a moisture absorption fluidity (B.R.) is 50 weight% or less.

[8] The particulate water-absorbing agent described in any one of [1] through [7], in which a water-soluble content (Ext) is 25 weight% or less.

[9] The particulate water-absorbing agent described in any one of [1] through [8], in which a degradable soluble content is 30 weight% or less.

[10] The particulate water-absorbing agent described in any one of [1] through [9], in which a fluid retention capacity under pressure (AAP) is 18 g/g or more.

[11] The particulate water-absorbing agent described in any one of [1] through [10], in which a fluid retention capacity under pressure (AAP) is 26 g/g or more.

[12] The particulate water-absorbing agent described in any one of [1] through [11], in which an internal gas bubble ratio defined by the following formula is 0.5% to 2.5%:

$$\text{(Internal gas bubble ratio [\%])} = \{(\text{true density [g/cm}^3])$$

$$- (\text{apparent density [g/cm}^3])\} / (\text{true density [g/cm}^3]) \times 100$$

[13] The particulate water-absorbing agent described in any one of [1] through [12], in which a bulk specific gravity is 0.57 to 0.75.

[14] The particulate water-absorbing agent described in any one of [1] through [13], in which a diffusing absorbency after 60 minutes is 18 g/g or more.

[15] The particulate water-absorbing agent described in any one of [1] through [14], in which a diffusing absorbency after 10 minutes is 7 g/g or more.

[16] The particulate water-absorbing agent described in any one of [1] through [15], containing a polyacrylic acid (salt)-based water-absorbing resin as a main component.

[17] The particulate water-absorbing agent described in any one of [1] through [16], further containing at least one moisture absorption fluidity improving agent selected from the group consisting of: a multicomponent metal compound having a hydrotalcite structure and containing divalent and trivalent metal cations (two kinds of metal cations) and a hydroxyl group; and a water-insoluble metal phosphate containing a phosphate anion and a divalent or trivalent metal cation.

A water-absorbing sheet for use in the present invention can be a water-absorbing sheet produced with use of a particulate water-absorbing agent described below. In addition, the particulate water-absorbing agent can be used for a method of producing the water-absorbing sheet for use in the present invention.

[A1] A particulate water-absorbing agent having a centrifuge retention capacity (CRC) of 30 g/g to 50 g/g, satisfying at least one of the following (1) through (5):

(1) a DRC5min with a particle diameter of 850 $\mu$m to 600 $\mu$m is 24 g/g to 44 g/g;
(2) a DRC5min with a particle diameter of 600 $\mu$m to 500 $\mu$m is 29 g/g to 46 g/g;
(3) a DRC5min with a particle diameter of 500 $\mu$m to 425 $\mu$m is 32 g/g to 49 g/g;
(4) a DRC5min with a particle diameter of 425 $\mu$m to 300 $\mu$m is 37 g/g to 53 g/g; and
(5) a DRC5min with a particle diameter of 300 $\mu$m to 150 $\mu$m is 41 g/g to 60 g/g, and

containing particles so as to satisfy a DRC5min contribution rate of 60% or more, which is a total rate of particles satisfying the conditions (1) through (5) above and accounting relative to a total amount of the particulate water-absorbing agent.

[A2] The particulate water-absorbing agent described in [A1], in which the DRC5min contribution rate is 70% or more.

[A3] The particulate water-absorbing agent described in [A1] or [A2], in which the conditions (2) through (4) are all satisfied.

[A4] The particulate water-absorbing agent described in any one of [A1] through [A3], in which the conditions (1) through (5) are all satisfied.

[A5] The particulate water-absorbing agent described in any one of [A1] through [A4], in which a saline flow conductivity (SFC) is 0 ($\times 10^{-7} \cdot \text{cm}^3 \cdot \text{s} \cdot \text{g}^{-1}$) or more and less than 30 ($\times 10^{-7} \cdot \text{cm}^3 \cdot \text{s} \cdot \text{g}^{-1}$).

[A6] The particulate water-absorbing agent described in any one of [A1] through [A5], in which a surface tension is 66 mN/m or more.

[A7] The particulate water-absorbing agent described in any one of [A1] through [A6], in which a particle shape is a non-uniformly pulverized shape.

[A8] The particulate water-absorbing agent described in any one of [A1] through [A7], in which a moisture absorption fluidity (B.R.) is 50% or less.

[A9] The particulate water-absorbing agent described in any one of [A1] through [A8], in which a water-soluble content (Ext) is 25 weight% or less.

[A10] The particulate water-absorbing agent described in any one of [A1] through [A9], in which a degradable soluble content is 30 weight% or less.

[A11] The particulate water-absorbing agent described in any one of [A1] through [A10], in which a fluid retention capacity under pressure (AAP) is 18 g/g or more.

[A12] The particulate water-absorbing agent described in any one of [A1] through [A11], in which a fluid retention capacity under pressure (AAP) is 26 g/g or more.

[A13] The particulate water-absorbing agent described in any one of [A1] through [A12], in which an internal gas bubble ratio defined by the following formula is 0.5% to 2.5%:

(Internal gas bubble ratio [%]) = {(true density [g/cm$^3$]) - (apparent density [g/cm$^3$])} / (true density [g/cm$^3$]) $\times$ 100.

[A14] The particulate water-absorbing agent described in any one of [A1] through [A13], in which a bulk specific gravity is 0.57 to 0.75.

[A15] The particulate water-absorbing agent described in any one of [A1] through [A14], containing a polyacrylic acid (salt)-based water-absorbing resin as a main component.

[Examples]

The following description will discuss the present invention in greater detail on the basis of Production Examples, Examples and Comparative Examples. Note, however, that the present invention is not limited to the description thereof and that the present invention also encompasses in its scope as defined in the appended claims any Production Example or Example derived from an appropriate combination of technical means disclosed in different Production Examples and Examples.

Electric devices/apparatuses (including devices/apparatuses used to measure physical properties of a particulate water-absorbing agent) in Production Examples, Examples, and Comparative Examples each used a 200-V or 100-V electric power supply, unless otherwise specified. Further, the physical properties of a particulate water-absorbing agent for use in the present invention were measured at room temperature (20°C to 25°C) and at a relative humidity of 50% RH, unless otherwise specified.

[Measurements of physical properties of particulate water-absorbing agent]

The following description will discuss how physical properties of a particulate water-absorbing agent for use in accordance with the present invention were measured. Note that in a case where a target of measurement is not a particulate water-absorbing agent, that is, for example, in a case where a target of measurement is a particulate hydrogel, the term "particulate water-absorbing agent" in the description below should be replaced with "particulate hydrogel".

(a) Centrifuge retention capacity (CRC)

The centrifuge retention capacity (fluid retention capacity without pressure, CRC) of the particulate water-absorbing agent for use in accordance with the present invention was measured in conformity with an EDANA method (ERT 441.2-02).

(b) Fluid retention capacity under pressure (AAP)

The fluid retention capacity under pressure (AAP) of the particulate water-absorbing agent for use in accordance with the present invention was measured in conformity with an EDANA method (ERT 442.2-02).

(c) Particle size distribution (particle size distribution, mass average particle diameter (D50), and logarithmic standard deviation ($\sigma\zeta$) of particle size distribution)

The particle size distribution (particle size distribution, mass average particle diameter (D50), and logarithmic standard deviation ($\sigma\zeta$) of particle size distribution) of the particulate water-absorbing agent for use in accordance with the present invention was measured according to "(3) Mass-Average Particle Diameter (D50) and Logarithmic Standard Deviation ($\sigma\zeta$) of Particle Diameter Distribution", which is disclosed in columns 27 and 28 of U.S. Patent No. 7638570.

That is, 10.00 g of the particulate water-absorbing agent was classified by using JIS standard sieves (The IIDA TESTING SIEVE: 80 mm in inner diameter; JIS Z8801-1 (2000)) having respective mesh sizes of 850 $\mu$m, 710 $\mu$m, 600 $\mu$m, 500 $\mu$m, 425 $\mu$m, 300 $\mu$m, 212 $\mu$m, 150 $\mu$m, 106 $\mu$m, and 75 $\mu$m or sieves corresponding to the JIS standard sieves. After this classification, the mass of each sieve was measured, and the mass percentage (mass%) of particles having a particle diameter of less than 150 $\mu$m was calculated. Note that the "mass percentage of particles having a particle diameter of less than 150 $\mu$m" refers to a mass proportion (%) of particles capable of passing through a JIS standard sieve having a mesh size of 150 $\mu$m, relative to a whole of the water-absorbing agent.

Further, a graph of a residual percentage R of each particle size mentioned above was plotted on a logarithmic probability paper, and a particle diameter corresponding to R = 50 mass% was read as the mass average particle diameter (D50) from the graph. Note that the mass average particle diameter (D50) refers to a particle diameter corresponding to 50 mass% of the whole of the particulate water-absorbing agent. Furthermore, note that the logarithmic standard deviation ($\sigma\zeta$) of the particle size distribution is expressed by the Formula (h) and that a smaller value of the logarithmic standard

deviation ($\sigma\zeta$) of the particle size distribution indicates a narrower particle size distribution.

$$\sigma\zeta = 0.5 \times \ln(X2/X1) \ldots \text{Formula (c)}$$

where X1 represents a particle diameter when R = 84.1%, and X2 represents a particle diameter when R = 15.9%.

(d) Moisture content

The moisture content of the particulate water-absorbing agent for use in accordance with the present invention was measured in conformity with an EDANA method (ERT430.2-02). Note that for the present invention, in measurements of the moisture content of the particulate water-absorbing agent, the amount of the particulate water-absorbing agent (sample) was changed to 1.0 g, and the drying temperature was changed to 180°C.

Further, in measurements of the moisture content of a hydrogel having a relatively large water content (moisture content of 20 mass% or more), a drying time was changed to 24 hours.

(e) Saline flow conductivity (SFC)

The saline flow conductivity (SFC) of the particulate water-absorbing agent for use in accordance with the present invention was measured according to a measuring method disclosed in U.S. Patent No. 5669894.

(f) Dunk Retention Capacity 5minutes (DRC5min)

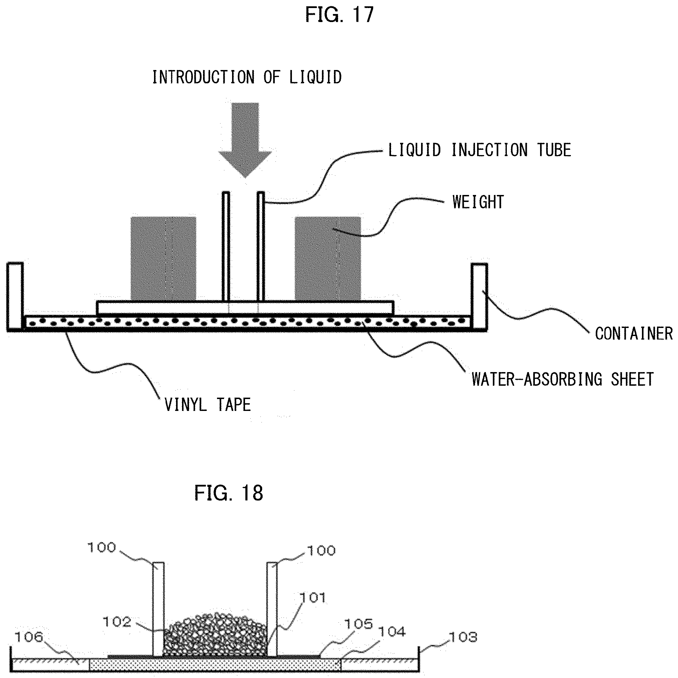

A device illustrated in Fig. 18 was used. In the device, a 400-mesh metal gauze 101 made of stainless steel (having a mesh size of 38 $\mu$m) was fused to the bottom of a plastic supporting cylinder 100 having an inner diameter of 60 mm. Then, 1.000 g $\pm$ 0.005 g of the particulate water-absorbing agent or a water-absorbing resin was spread out uniformly on the metal gauze, at room temperature (20°C to 25°C) and at a humidity of 50% RH. Then, the mass Wa (g) of this measuring device as a whole was measured.

A glass filter 104 having a diameter of 120 mm (manufactured by Sougo Rikagaku Glass Seisakusho Co., Ltd., fine pore diameter: 100 $\mu$m to 120 $\mu$m) was placed in a petri dish 103 having a circular or square shape whose bottom area was 400 cm$^2$. Then, 0.90-mass% saline 106 (23°C $\pm$ 0.5°C) was added in such an amount that the level of the 0.90-mass% saline was equal to the upper surface of the glass filter (a state in which liquid was slightly bulging due to its surface tension at the outer periphery of the glass filter or a state in which approximately 50% of the surface of the glass filter was covered by the liquid). On the saline and the glass filter, a sheet of filter paper 105 having a diameter of 110 mm (available from Advantec Toyo Kaisha, Ltd., product name: JIS P 3801 No. 2, with a thickness of 0.26 mm and a retaining particle diameter of 5 $\mu$m) was placed in such a manner that the entire surface of the sheet of filter paper was wet.

A whole of the measuring device described above was placed on the wet filter paper for absorption of the liquid (the temperature of the liquid was precisely controlled at 23°C $\pm$ 0.5°C during measurement). After exactly 5 minutes (300 seconds), the whole measuring device was lifted up and the mass Wb (g) of the whole measuring device was measured. Then, DRC5min (g/g) was calculated from Wa and Wb by the following Formula (f):

DRC5min (g/g) = {(Wb - Wa)/(mass of particulate water-absorbing agent)}　　　　　　　　　　Formula (f)

(g) Surface tension

Into a 100 ml beaker which had been sufficiently washed, 50 ml of physiological saline, which had been adjusted to 20°C, was put. Then, the surface tension of the physiological saline was measured with use of a surface tension meter (manufactured by KRUSS, K11 automatic surface tension meter). It was confirmed that a result of measurement with use of the surface tension meter was reasonable. That is, in this measurement, the surface tension needs to fall within a range of 71 mN/m to 75 mN/m.

Next, a fluorine resin rotor, which had been sufficiently washed and had a length of 25 mm, and 0.5 g of the particulate water-absorbing agent or the water-absorbing resin were put in the beaker containing the physiological saline whose temperature had been adjusted to 20°C and whose surface tension had been measured. Then, a resultant solution was stirred at 500 rpm for 4 minutes. After 4 minutes elapsed, the stirring was stopped. Thereafter, after sedimentation of the particulate water-absorbing agent or the water-absorbing resin which had absorbed water, the surface tension of a supernatant liquid was measured with similar procedures. Note that, in the present invention, a plate method using a platinum plate was employed, and the plate was sufficiently washed with deionized water and also cleaned with heat by the use of a gas burner before being used in each of the above measurements.

(h) Particle size (PSD) and logarithmic standard deviation ($\sigma\zeta$) of particle size distribution

The particle size (PSD) and the logarithmic standard deviation ($\sigma\zeta$) of particle size distribution of the particulate water-absorbing agent for use in accordance with the present invention were measured in conformity with a measuring method disclosed in U.S. Patent Application Publication No. 2006/204755.

That is, 10.00 g of the particulate water-absorbing agent was classified by using JIS standard sieves (The IIDA TESTING SIEVE: 80 mm in inner diameter; JIS Z8801-1 (2000)) having respective mesh sizes of 850 $\mu$m, 710 $\mu$m, 600 $\mu$m, 500 $\mu$m, 425 $\mu$m, 300 $\mu$m, 212 $\mu$m, 150 $\mu$m, 106 $\mu$m, and 75 $\mu$m or sieves corresponding to the JIS standard sieves. After this classification, the mass of each sieve was measured, and the mass percentage (mass%) of particles having a particle diameter of less than 150 $\mu$m was calculated. Note that the "mass percentage of particles having a particle diameter of less than 150 $\mu$m" refers to a mass proportion (%) of particles capable of passing through a JIS standard sieve having a mesh size of less than 150 $\mu$m, relative to a whole of the water-absorbing agent.

Further, a graph of a residual percentage R of each particle size mentioned above was plotted on a logarithmic probability paper, and a particle diameter corresponding to R = 50 mass% was read as the mass average particle diameter (D50) from the graph. Note that the mass average particle diameter (D50) refers to a particle diameter corresponding to 50 mass% of the whole of the particulate water-absorbing agent. Furthermore, note that the logarithmic standard deviation ($\sigma\zeta$) of the particle size distribution is expressed by the following Formula (h) and that a smaller value of the logarithmic standard deviation ($\sigma\zeta$) of the particle size distribution indicates a narrower particle size distribution.

$$\sigma\zeta = 0.5 \times \ln(X2/X1) \ldots \text{Formula (h)}$$

where X1 represents a particle diameter when R = 84.1%, and X2 represents a particle diameter when R = 15.9%.

(i) Moisture absorption fluidity (moisture adsorption blocking ratio) (B.R.; Blocking Ratio)

On an aluminum cup having a diameter of 52 mm, 2 g of the particulate water-absorbing agent or the water-absorbing resin was uniformly spread out and then left to stand still for one hour in a thermo-hygrostat (PLATINOUSLUCIFERPL-2G; manufactured by Tabai Espec Corp.) at a temperature of 25°C and at a relative humidity of 90%RH $\pm$ 5% RH. After one hour elapsed, the particulate water-absorbing agent or the water-absorbing resin in the aluminum cup was calmly transferred onto a JIS standard sieve (The IIDA TESTING SIEVE: 80 mm in inner diameter) having a mesh size of 2000 $\mu$m (JIS 8.6 mesh). The particulate water-absorbing agent or the water-absorbing resin was then classified at room temperature (20°C to 25°C) and at a relative humidity of 50% RH for 5 seconds, by using a Ro-Tap sieve shaker (ES-65 sieve shaker manufactured by Sieve Factory Iida Co., Ltd.; whose rotation speed was 230 rpm and number of impacts was 130 rpm). Thereafter, the mass (W1 (g)) of the particulate water-absorbing agent or the water-absorbing resin remaining on the JIS standard sieve and the mass (W2 (g)) of the particulate water-absorbing agent or the water-absorbing resin which had passed through the sieve were measured. Subsequently, the moisture absorption fluidity (moisture adsorption blocking ratio) was calculated by the following Formula (i). Note that a lower value of the blocking ratio means a better moisture absorption fluidity.

Moisture absorption fluidity (B.R.) (mass%) = {W 1 / (W 1 + W2)} $\times$ 100             Formula (i).

(j) Degradable soluble content

In a 250 ml plastic container in which a 35 mm rotor was placed and which had inner and outer lids, 200.0 g of an aqueous solution containing 0.05 mass% of L-ascorbic acid and 0.90 mass% of sodium chloride (degradation test liquid/ mixture of 0.10 g of L-ascorbic acid and 199.90 g of 0.90 mass% aqueous sodium chloride solution) was weighed and taken. Then, 1.00 g of the particulate water-absorbing agent or the water-absorbing resin was added to the aqueous solution, and the plastic container was sealed with the inner and outer lids. Thereafter, the plastic container was left to stand still for 2 hours, in a thermostat which had been adjusted to 60°C $\pm$ 2°C. After 2 hours elapsed, the plastic container was taken out from the thermostat and one-hour stirring using a stirrer (rotation speed: 500 rpm) was carried out at room temperature. A water-soluble content of the particulate water-absorbing agent or the water-absorbing resin was extracted by the above operation.

After the stirring, the extraction liquid was filtered with the use of a sheet of filter paper (manufactured by Advantec Toyo Kaisha, Ltd., product name: JIS P 3801 No. 2, with a thickness of 0.26 mm and a retaining particle diameter of 5 $\mu$m). Then, 50.0 g of a filtrate thus obtained was used as a liquid for measurement. Next, the liquid for measurement was titrated with a 0.1 N NaOH aqueous solution until the liquid had a pH of 10, and then titrated with a 0.1 N HCl aqueous solution until the liquid had a pH of 2.7. Respective titers in the above titration were determined as [NaOH] mL and [HCl] mL.

Further, similar operations were carried out on 200.0 g of only the degradation test liquid to which neither the particulate water-absorbing agent nor the water-absorbing resin was added, and blank titers ([b2NaOH] mL and [b2HCl] mL) were determined.

Subsequently, the degradable soluble content was calculated according to the following Equation (j-1) from titers and a monomer average molecular weight.

Degradable soluble content (mass%) = 0.1 × (monomer average molecular weight) × 200 × 100 × ([HCl] - [b2HCl])/1000/1.0/50.0          Formula (j-1)

Note that in a case where the monomer average molecular weight was unknown, the monomer average molecular weight was calculated by using a neutralization rate calculated by the following Formula (j-2).

Neutralization rate (mol%) = {1 - ([NaOH] - [b1NaOH]) / ([HCl] - [b1HCl])} × 100          Formula (j-2)

(k) Diffusing absorbency under pressure (DAP)

The diffusing absorbency of the particulate water-absorbing agent was measured according to a measuring method disclosed in Japanese Patent Application Publication, Tokukai, No. 2010-142808.

(1) Apparent density

The apparent density of water-absorbing resin powder was measured by dry density measurement (dry measurement at the volume of a certain mass of water-absorbing resin powder). In this measurement, moisture was further removed from the particulate water-absorbing agent and internal gas bubbles inside the powder were taken into consideration.

That is, 6.0 g of the particulate water-absorbing agent was weighed and taken in an aluminum cup having a bottom surface diameter of 5 cm. Then, the particulate water-absorbing agent was left to stand still in a windless dryer at 180° for 3 hours or longer and was sufficiently dried until the moisture content of the particulate water-absorbing agent was 1% or less. The apparent density (unit: g/cm$^3$) of 5.00 g of the water-absorbing agent thus dried was measured with use of helium gas, by using an automatic dry densimeter (Micromeritics Auto Pycnometer 1320, manufactured by Shimadzu Corporation). The measurement was repeated until identical measured values were obtained in two or more consecutive measurements.

(m) True density

The true density of the particulate water-absorbing agent for use in the present invention was determined by measuring the dry density of the particulate water-absorbing agent, in which closed-cells were destructed or turned into open-cells inside the particulate water-absorbing agent by pulverizing the particulate water-absorbing agent to fine powder which could pass through a JIS standard sieve having a mesh size of 45 μm.

Gas bubbles (closed-cells) contained in the particulate water-absorbing agent normally have a diameter of 1 μm to 300 μm. In pulverization, the water-absorbing resin starts breaking from portions near the closed cells. When the water-absorbing agent is pulverized until a particle diameter thereof reaches 45 μm or less, the water-absorbing agent thus pulverized has almost no closed cells. Therefore, the dry density of the water-absorbing agent having been pulverized to fine powder so as to have a particle diameter of 45 μm or less was regarded as a true density.

The true density was measured by using the water-absorbing agent pulverized to pass through the JIS standard sieve having a mesh size of 45 μm. Specifically, 15.0 g of the water-absorbing resin powder and 400 g of columnar porcelain balls (diameter: 13 mm, length: 13 mm) were placed in a ball mill pot (manufactured by TERAOKA, porcelain ball mill pot model No. 90, internal dimensions: 80 mm in diameter and 75 mm in height, and external dimensions: 90 mm in diameter and 110 mm in height). Then, the water-absorbing agent was pulverized to fine powder in the ball mill pot at 60 Hz for 2 hours. A water-absorbing agent obtained as a result was a water-absorbing agent 70 mass% or more of which would pass through a JIS standard sieve having a mesh size of 45 μm.

After the water-absorbing agent was classified by using a JIS standard sieve having a mesh size of 45 μm, a water-absorbing resin powder whose particle diameter was less than 45 μm was obtained. Then, 6.0 g of the water-absorbing resin powder was dried at 180 °C for 3 hours as in the case of "(I) Apparent density" described above. Thereafter, the dry density of the water-absorbing resin powder was measured. The dry density thus obtained was regarded as the "true density" of the present invention.

(n) Internal gas bubble ratio (closed-cell ratio)

The internal gas bubble ratio of the particulate water-absorbing agent was calculated, by the following Formula (n), from the apparent density (density $\rho 1$ [g/cm$^3$]) which was measured by the method described in the above "(l) Apparent density" and the true density (density $\rho 2$ [g/cm$^3$]) which was measured by the method described in the above "(m) True density".

$$\text{Internal gas bubble ratio (\%)} = (\rho 2 - \rho 1)/\rho 2 \times 100 \quad (n)$$

(o) Bulk specific gravity "Density" (ERT460.2-02)

The term "density" here refers to the bulk specific gravity of a water-absorbing agent. Note that the bulk specific gravity is measured in conformity with JIS K3362, with reference to ERT 460.2-02, in the present invention.

The bulk specific gravity was measured by use of a bulk specific gravity measuring device (manufactured by Kuramochi Scientific Instrument Seisakusho) in conformity with JIS K 3362. After 100.0 g of the particulate water-absorbing agent, which had been sufficiently stirred so as to avoid deviation of particle size, was placed in a funnel whose damper was closed, the damper was opened quickly so that the water-absorbing agent was dropped into a receiver having an internal capacity of 100 ml. Note that the weight (unit; g) of the receiver (this weight is referred to as "weight W9 (g)") was weighed in advance.

After part of the water-absorbing agent, which part was protruding from the top of the receiver, was removed by use of a glass rod, the weight (unit; g) of the receiver containing the water-absorbing agent (this weight is referred to as "weight W10 (g)") was accurately measured to the unit of 0.1 g, and the bulk specific gravity was calculated by the following Formula (o):

$$\text{Bulk specific gravity (g/cm}^3) = (W10 - W9)/100 \ldots$$

$$\text{Formula (o)}$$

Note that the above measurement was carried out at an ambient temperature of 24.2°C and at a relative humidity of 43%RH.

(p) Increase in fine powder from before to after damage (damage resistance)

An increase in fine powder (amount of increase in particles which pass through a 150-pm mesh) from before to after damage to the particulate water-absorbing agent for use in the present invention, which increase in fine powder is defined by a measuring method described later, is preferably 4 mass% or less, and more preferably 3.5 mass% or less. The particulate water-absorbing agent having the increase in fine powder in the above range causes no problem of deterioration of physical properties in actual use for disposable diaper production etc.

<Increase in fine powder after damaging>

A paint shaker test described below was carried out on the water-absorbing agent. An amount of increase in particles having a particle diameter of 150 $\mu$m or less from before to after the paint shaker test was measured by classification of the water-absorbing agent with use of a JIS standard sieve having a mesh size of 150 $\mu$m.

[Paint shaker test]

A paint shaker test (PS-test) is a test in which 10 g of glass beads having a diameter of 6 mm and 30 g of a water-absorbing resin are put in a glass container having a diameter of 6 cm and a height of 11 cm and then the glass container is attached to a paint shaker (Toyo Seiki Seisaku-sho, Ltd., product No. 488) and is shaken at 800 cycles/min (CPM) for 30 minutes. The details of a device for the paint shaker test are disclosed in Japanese Patent Application Publication, Tokukaihei, No. 9-235378.

After the glass container is shaken, the glass beads are removed with use of a JIS standard sieve having a mesh size of 2 mm, so that a damaged water-absorbing resin is obtained.

(q) Water-soluble content (Ext)

The water-soluble content (Ext) of the particulate water-absorbing agent for use in the present invention was measured in conformity with an EDANA method (ERT470.2-02).

(r) Surface area

The surface area of the particulate water-absorbing agent for use in the present invention can be measured by, for example, performing analysis using three-dimensional analysis software (e.g., high-speed three-dimensional analysis software TRI/3D-VOL-FCS64) on a result of measurement with use of a three-dimensional analysis apparatus using x-rays (e.g., Microfocus X-Ray CT System inspeXio SMX-225CT or inspeXio SMX-100CT manufactured by Shimadzu Corporation). When the surface area is measured, the above described internal gas bubble ratio and/or the like can be also measured simultaneously.

[Water-absorbing sheet evaluation]

Performance of a water-absorbing sheet for use in accordance with the present invention was evaluated by the following method.

(s-1) Flat surface evaluation 1

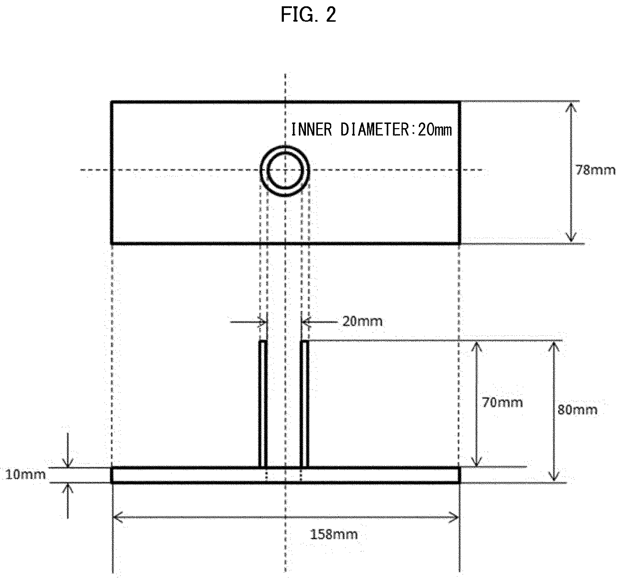

Flat surface evaluation of a water-absorbing sheet was carried out with use of a device illustrated in Figs. 1 and 2. A water-absorbing sheet was cut to have a size of 8 cm (length) × 16 cm (width). A resultant water-absorbing sheet was put in a plastic container, having an inner size of 8 cm (length) × 16 cm (width) × 3.5 cm (height) (Fig. 1), so that a bottom surface of the plastic container matched a bottom surface of the water-absorbing sheet substantially without a gap. A liquid injection tube (Fig. 2) was placed on the water-absorbing sheet, and weights, whose respective weights had been adjusted so that the weights would apply a pressure of 1.2 kPa, were placed on the liquid injection tube (the weights were placed so that the weights uniformly applied the pressure to the entire water-absorbing sheet). In this state, a certain amount (30 ml or 40ml) of a 0.9 mass% aqueous sodium chloride solution was introduced into the liquid injection tube. A time period from when the solution was introduced into the liquid injection tube to when the solution disappeared from the liquid injection tube was referred to as t1 (second). After it was made sure that the solution was taken in the water-absorbing sheet, the liquid injection tube was immediately removed, and the water-absorbing sheet was allowed to stand still for 10 minutes. After 10 minutes elapsed, the liquid injection tube was placed on the water-absorbing sheet again, and weights were placed on the liquid injection tube so that the weights applied a pressure similar to that applied in the first introduction of the solution. Subsequently, the second introduction of the solution was carried out (an amount of the solution was identical to that of the solution in the first introduction). A time period from when the solution was introduced into the liquid injection tube to when the solution disappeared from the liquid injection tube was referred to as t2 (second). After it was made sure that the solution was taken in the water-absorbing sheet, the liquid injection tube was immediately removed, and the water-absorbing sheet was allowed to stand still for 10 minutes. After 10 minutes elapsed, 30 sheets of filter paper (manufactured by ADVANTEC, model No. 2, obtained by cutting those having a size of 100 mm × 100 mm to have a size of 8 cm (length) × 7 cm (width)), whose mass had been measured in advance, were placed on a middle part of the water-absorbing sheet. A weight having a load of 4.8 kPa was further placed on the 30 sheets of filter paper, and then held for 10 seconds. After 10 seconds elapsed, the weight was removed, and a re-wet (g) was measured based on an increase in mass of the 30 sheets of filter paper.

(s-2) Curved surface evaluation 1

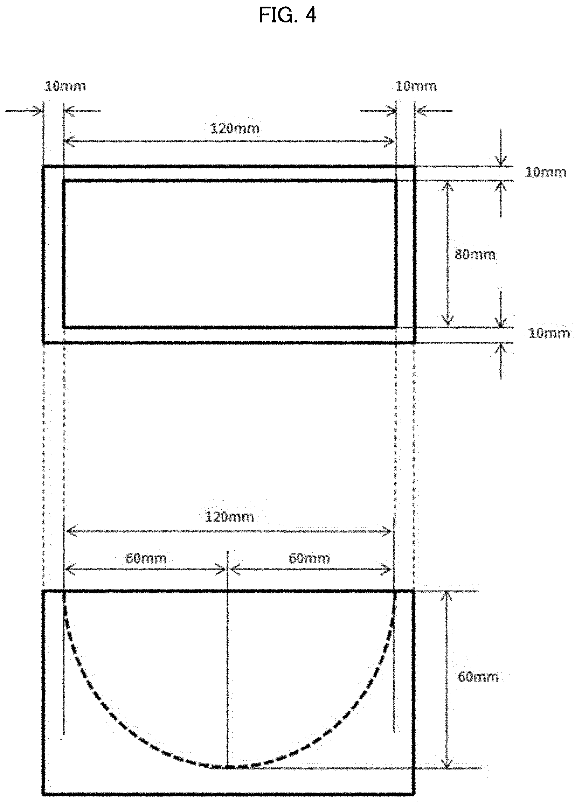

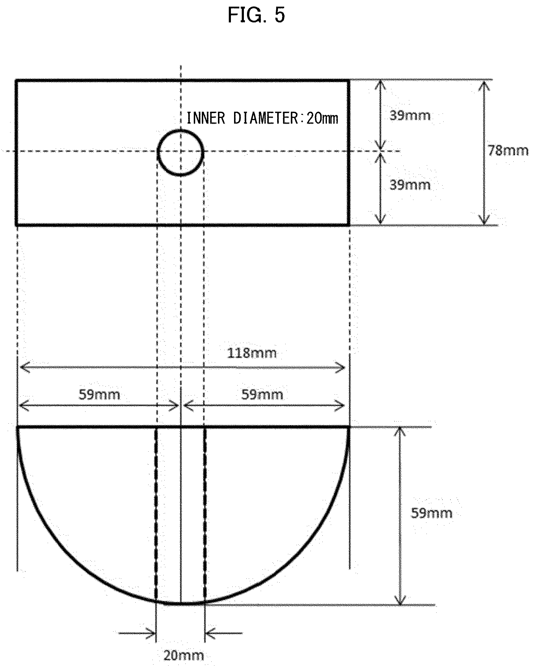

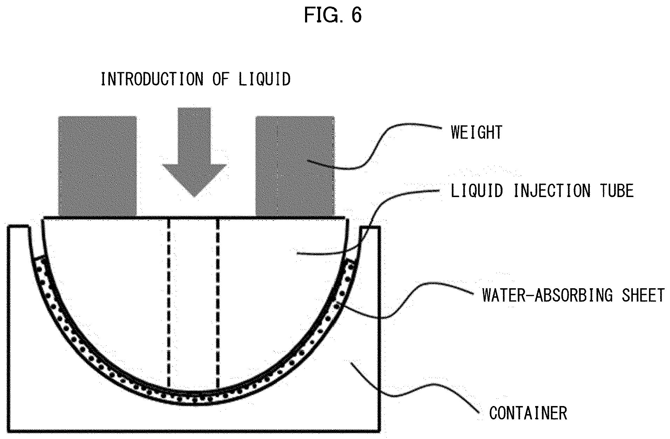

Curved surface evaluation of a water-absorbing sheet was carried out with use of a device illustrated in Figs. 4 and 5. A water-absorbing sheet was cut to have a size of 8 cm (length) × 16 cm (width). A resultant water-absorbing sheet was put in a container, having a curved surface (Fig. 4), so that a middle part of the curved surface of the container matched a middle part of the water-absorbing sheet. A liquid injection tube (Fig. 5) was placed on the water-absorbing sheet, and two weights, whose mass had been adjusted so that a total mass of the liquid injection tube and the two weights was 872 g, were placed on the liquid injection tube so that the two weights were symmetrically located with respect to a liquid introduction inlet and sandwiched the liquid introduction inlet therebetween (Fig. 6). In this state, a certain amount (30 ml or 40ml) of a 0.9 mass% aqueous sodium chloride solution was introduced into the liquid injection tube. A time period from when the solution was introduced into the liquid injection tube to when the solution disappeared from the liquid injection tube was referred to as t1 (second). After it was made sure that the solution was taken in the water-absorbing sheet, the liquid injection tube was immediately removed, and the water-absorbing sheet was allowed to stand still. After 10 minutes elapsed from the first introduction of the solution, the liquid injection tube was placed on the water-absorbing sheet again, and weights were placed on the liquid injection tube in a manner similar to that in the first introduction of the solution. Subsequently, the second introduction of the solution was carried out (an amount of the solution was identical to that of the solution in the first introduction). A time period from when the solution was introduced into the liquid injection tube to when the solution disappeared from the liquid injection tube was referred to as t2 (second). After it was made sure that the solution was taken in the water-absorbing sheet, the liquid injection tube was immediately removed, and the water-absorbing sheet was allowed to stand still. After 10 minutes elapsed from the second introduction of the solution, the water-absorbing sheet which had absorbed the solution was carefully taken out and then put in a plastic container having an inner size of 8 cm (length) × 16 cm (width) × 3.5 cm (height) (Fig. 1). On a middle part of the water-absorbing sheet, 30 sheets of filter paper (manufactured by ADVANTEC, model No. 2, obtained by cutting those having a size of 100 mm × 100 mm to have a size of 8 cm (length) × 7 cm (width)), whose mass had been measured in advance, were placed. A weight having a load of 4.8 kPa was further placed on the 30 sheets of filter paper, and then held for 10 seconds. After 10 seconds elapsed, the weight was removed, and a re-wet (g) was measured based on an increase in mass of the 30 sheets of filter paper.

(s-3) Flat surface evaluation 2

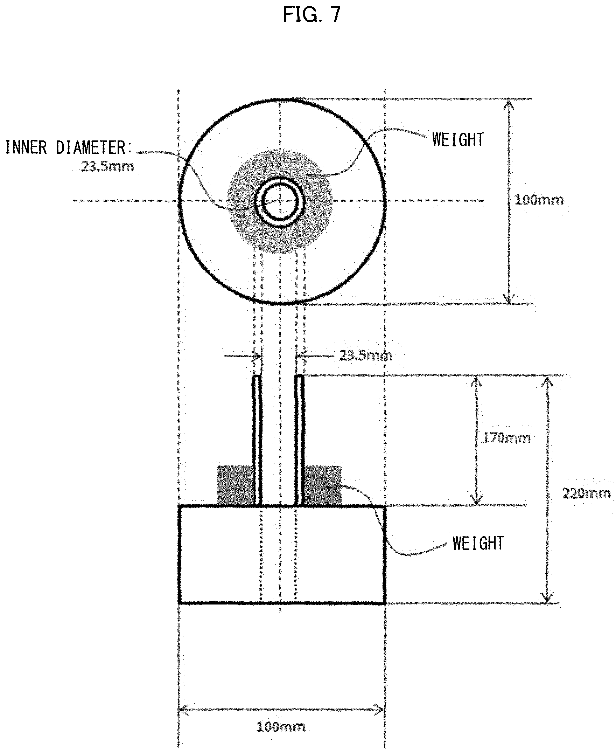

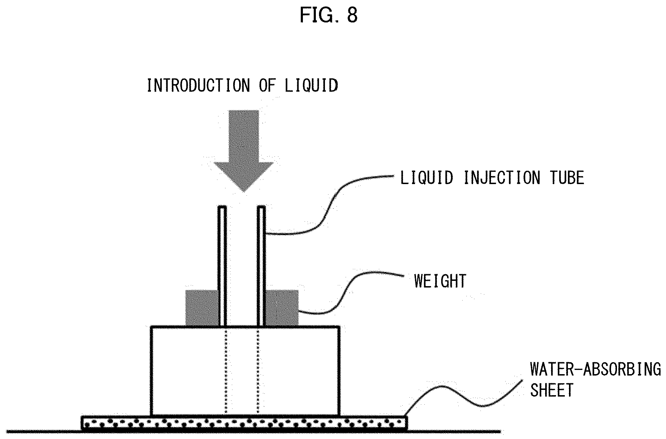

Flat surface evaluation of a water-absorbing sheet was carried out with use of a device illustrated in Fig. 7. A water-absorbing sheet was cut to have a size of 8 cm (length) × 16 cm (width). Subsequently, a liquid injection tube (Fig. 7, weighting 1000 g including a weight of a weight) was placed on the water-absorbing sheet (see Fig. 8). In this state, a certain amount (30 ml) of a 0.9 mass% aqueous sodium chloride solution was introduced into the liquid injection tube. A time period from when the solution was introduced into the liquid injection tube to when the solution disappeared from the liquid injection tube was referred to as t1 (second). After 80 seconds elapsed from introduction of the solution, the liquid injection tube was removed, and the water-absorbing sheet was allowed to stand still for 15 seconds. After 15 minutes elapsed, 10 sheets of filter paper (manufactured by ADVANTEC, model No. 2, obtained by cutting those having a size of 100 mm × 100 mm to have a size of 8 cm (length) × 7 cm (width)), whose mass had been measured in advance, were placed on a middle part of the water-absorbing sheet. A plastic plate (approximately 20 g) having an identical size (8 cm (length) × 7 cm (width)) was then placed on the 10 sheets of filter paper. A weight having a load of 4.8 kPa was further placed on the plastic plate, and then held for 10 seconds. After 10 seconds elapsed, the weight was removed, and a re-wet (g) was measured based on an increase in mass of the 10 sheets of filter paper.

(s-4) Curved surface evaluation 2

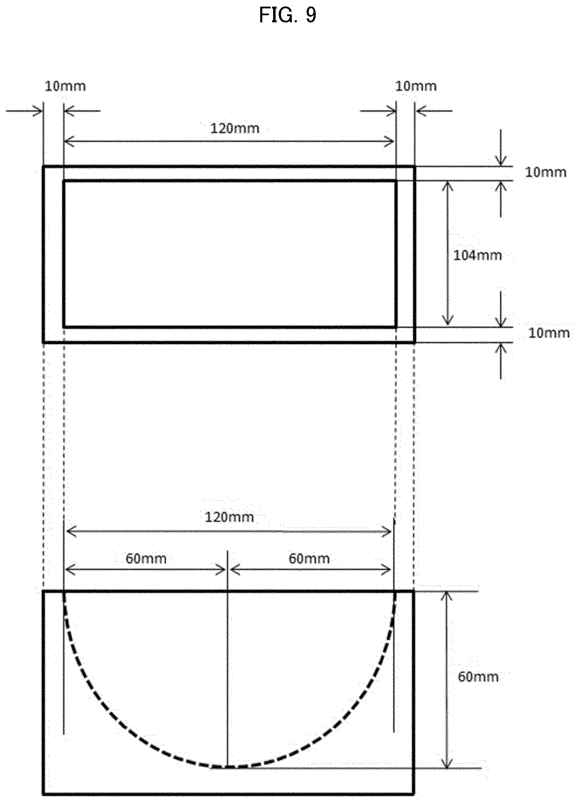

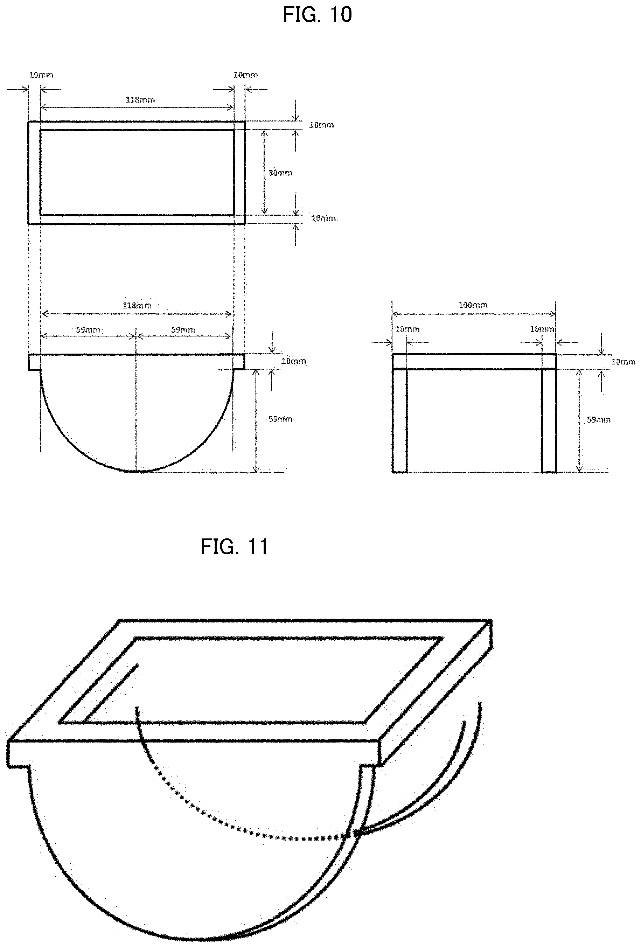

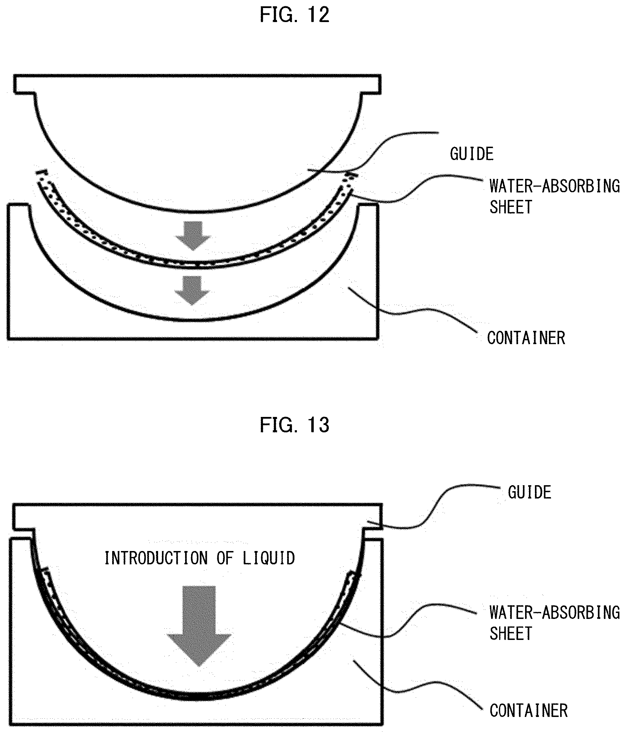

Curved surface evaluation of a water-absorbing sheet was carried out with use of a device illustrated in Figs. 9, 10, and 11 (Fig. 11 is a view comprehensibly illustrating a shape of the device illustrated in Fig. 10, and the device illustrated in Fig. 11 is identical to that illustrated in Fig. 10). A water-absorbing sheet was cut to have a size of 10 cm (length) × 16 cm (width). A resultant water-absorbing sheet was put in a container, having a curved surface (Fig. 9), so that a middle part of the curved surface of the container matched a middle part of the water-absorbing sheet. A guide for allowing edges of the water-absorbing sheet to be sandwiched between the guide and the container (Fig. 10) was placed on the water-absorbing sheet (see Fig. 12). In this state, a certain amount (30 ml or 40ml) of a 0.9 mass% aqueous sodium chloride solution was introduced, with use of a funnel, into the lowest and middle position on the curved surface (see Fig. 13). A time period from when the solution was introduced into a surface of the water-absorbing sheet to when the solution disappeared from the surface of the water-absorbing sheet was referred to as t1 (second). After 160 seconds elapsed from introduction of the solution, the guide was removed. Subsequently, the water-absorbing sheet was carefully taken out from such a curved-surface container so that gel which had absorbed the solution did not move, and then placed on a planar surface to stand still. The water-absorbing sheet was allowed to stand still for 50 seconds after the water-absorbing sheet was taken out from the curved-surface container. After 50 minutes elapsed, 10 sheets of filter paper (manufactured by ADVANTEC, model No. 2, obtained by cutting those having a size of 100 mm × 100 mm to have a size of 8 cm (length) × 7 cm (width)), whose mass had been measured in advance, were placed on a middle part of the water-absorbing sheet. A plastic plate (approximately 20 g) having an identical size (8 cm (length) × 7 cm (width)) was then placed on the 10 sheets of filter paper. A weight having a load of 4.8 kPa was further placed on the plastic plate, and then held for 10 seconds. After 10 seconds elapsed, the weight was removed, and a re-wet (g) was measured based on an increase in mass of the 10 sheets of filter paper.

(s-5) Liquid flow evaluation

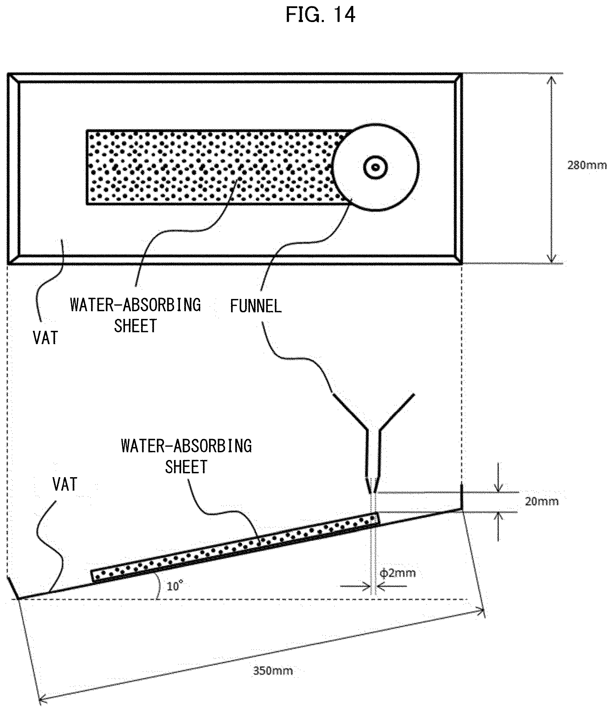

Liquid flow evaluation of a water-absorbing sheet was carried out with use of a device illustrated in Fig. 14. A water-absorbing sheet was cut to have a size of 8 cm (length) × 16 cm (width), and placed on a surface inclined at 10 degrees (see Fig. 14). A certain amount (15 ml) of a 0.9 mass% aqueous sodium chloride solution was introduced into a funnel illustrated in Fig. 14 (the funnel was set so that an outlet through which the solution was discharged was located 20 mm above from a middle position on an uppermost part of the water-absorbing sheet). The solution thus introduced was absorbed while the solution was flowing on the absorbing sheet, and the solution which had not been absorbed accumulated in a bottom part of a vat. An amount of the solution which accumulated in the bottom part was measured, and an amount obtained by subtracting the amount thus measured from 15 ml was regarded as an absorption amount.

(s-6) Flat surface evaluation 3

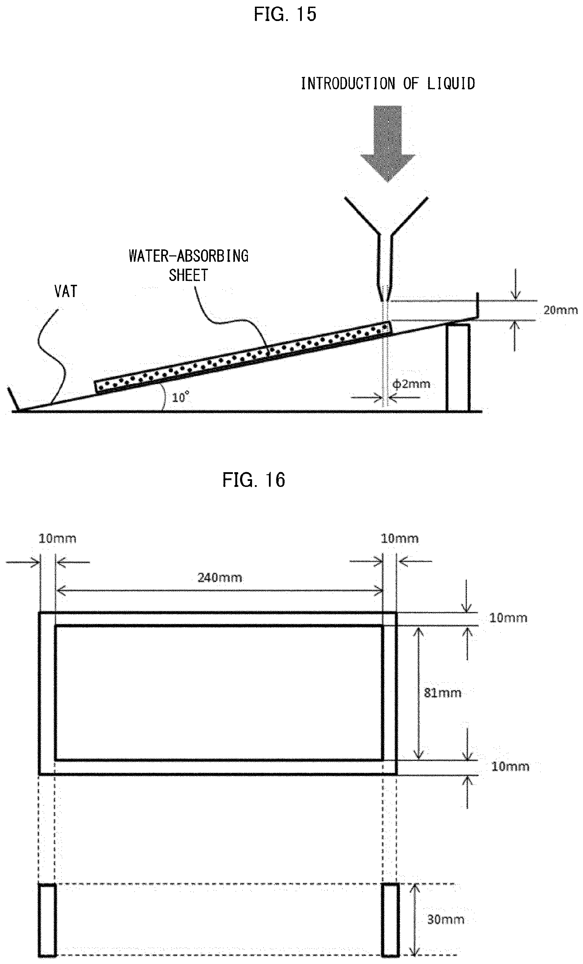

Flat surface evaluation of a water-absorbing sheet was carried out with use of a device illustrated in Figs. 2 and 16. A vinyl tape (manufactured by Nitto Denko Corporation, Nitto vinyl tape No. 21-100TM, 0.2 mm × 100 mm × 20 m, transparent) was attached to a bottom surface of a plastic container, having an inner size of 8.1 cm (length) × 24 cm (width) × 3 cm (height) (Fig. 16), so that the vinyl tape had no wrinkle. A water-absorbing sheet was cut to have a size of 8 cm (length) × 24 cm (width), and was put in the container so that a bottom surface of the water-absorbing sheet was bonded to the bottom surface (vinyl tape) of the container substantially without a gap.

In so doing, a simulated water-absorbing sheet can be prepared by, for example, dispersing a particulate water-absorbing agent on the vinyl tape and then placing nonwoven fabric on the particulate water-absorbing agent, and then flat surface evaluation can be carried out. Furthermore, the size of the plastic container (Fig. 16) and a size of a liquid injection tube (Fig. 2) can be also changed as appropriate. For example, it is considered to employ the size illustrated in Fig. 1 for measurement. Note, however, that, in Examples of the present invention, the devices illustrated in Figs. 2 and 16 and water-absorbing sheets prepared in Examples were used for evaluation.

Next, a liquid injection tube (Fig. 2) was placed on the water-absorbing sheet so that a middle part of the water-absorbing sheet matched a liquid introduction inlet, and weights, whose respective weights had been adjusted so that the weights would apply a pressure of 1.2 kPa, were placed on the liquid injection tube (the weights were placed so that the weights uniformly applied the pressure to the entire water-absorbing sheet, the pressure can be also changed as appropriate). In this state, a certain amount (30 ml or 40ml) of a 0.9 mass% aqueous sodium chloride solution was introduced into the liquid injection tube. A time period from when the solution was introduced into the liquid injection tube to when the solution disappeared from the liquid injection tube was referred to as t1 (second). After it was made sure that the solution was taken in the water-absorbing sheet, the liquid injection tube was immediately removed, and the water-absorbing sheet was allowed to stand still for 10 minutes. After 10 minutes elapsed, the liquid injection tube was placed on the water-absorbing sheet again, and weights were placed on the liquid injection tube so that the weights applied a pressure similar to that applied in the first introduction of the solution. Subsequently, the second introduction of the solution was carried out (an amount of the solution was identical to that of the solution in the first introduction). A time period from when the solution was introduced into the liquid injection tube to when the solution disappeared from the liquid injection tube was referred to as t2 (second). After it was made sure that the solution was taken in the water-absorbing sheet, the liquid injection tube was immediately removed, and the water-absorbing sheet was allowed to stand still for 10 minutes. After 10 minutes elapsed, the liquid injection tube was placed on the water-absorbing sheet again, and weights were placed on the liquid injection tube so that the weights applied a pressure similar to that applied in the second introduction of the solution. Subsequently, the third introduction of the solution was carried out (an amount of the solution was identical to that of the solution in the first introduction and the second introduction). A time period from when the solution was introduced into the liquid injection tube to when the solution disappeared from the liquid injection tube was referred to as t3 (second). After it was made sure that the solution was taken in the water-absorbing sheet, the liquid injection tube was immediately removed, and the water-absorbing sheet was allowed to stand still for 10 minutes. After 10 minutes elapsed, 30 sheets of filter paper (manufactured by ADVANTEC, model No. 2, obtained by cutting those having a size of 100 mm × 100 mm to have a size of 8 cm (length) × 7 cm (width), the size of the 30 sheets of filter paper can be also changed as appropriate), whose mass had been measured in advance, were placed on the middle part of the water-absorbing sheet. A weight having a load of 4.8 kPa was further placed on the 30 sheets of filter paper, and then held for 10 seconds. After 10 seconds elapsed, the weight was removed, and a re-wet (g) was measured based on an increase in mass of the 30 sheets of filter paper.

(t) Re-wet

There are known several methods for measuring a re-wet. The following measuring method is one example of the measuring methods, and how to measure a re-wet is not limited to such a measuring method.

A re-wet of an absorbent body which employed a particulate water-absorbing agent 2 for use in accordance with the present invention was measured by the following procedure.

In a 3-centimeter-deep rectangular resin tray having an inner size of 7.1 cm × 8.1 cm, 0.900 g of a particulate water-absorbing agent was uniformly dispersed. A top sheet having a size of 7 cm × 8 cm was then placed on the particulate water-absorbing agent so that the particulate water-absorbing agent did not move. An absorbent body thus obtained was regarded as a model absorbent body, and a re-wet of the absorbent body which absorbed a solution was measured. Note that a material of the resin tray is not limited to any particular one. Preferable examples of the material encompass ABS resin, acrylic resin, polypropylene, and Teflon (registered trademark) resin. Note also that, as the top sheet, a top sheet was used which had been taken out from a Mamy Poko (product name) tape type (size L, purchased in Japan in June 2014; number on the package bottom surface: 404088043) manufactured by Unicharm Corporation. The top sheet is, however, not limited to such a top sheet.

Into a middle part of the absorbent body, 32 ml of a 0.9 weight% aqueous sodium chloride solution was slowly introduced so that the solution did not cause the particulate water-absorbing agent to flow. Note, however, that introduction was ended within 10 seconds. After 5 minutes elapsed from start of the introduction, 20 sheets of filter paper (manufactured by ADVANTEC, model No. 2, obtained by cutting those having a size of 100 mm × 100 mm to have a size of 7 cm × 8 cm), whose weight had been measured in advance, were placed on the absorbent body. A weight having an identical size (having a bottom surface of 7 cm × 8 cm) (1200 g) was placed on the 20 sheets of filter paper. After 10 seconds elapsed, the weight and the 20 sheets of filter paper were removed. The weight of the 20 sheets of filter paper was measured, and then an amount (g) of the solution which had been absorbed by the 20 sheets of filter paper was determined by subtracting, from the weight thus measured, the weight of the 20 sheets of filter paper which had been measured in advance. The amount of the solution thus determined was regarded as a re-wet (g).

(u) Measurement of particle size of multicomponent metal compound (hydrotalcite) on water-absorbing agent

A particle size of a multicomponent metal compound (hydrotalcite) for use in accordance with the present invention was measured by measuring diameters, in a given direction, of 100 fine particles of the multicomponent metal compound which adhered to a surface of a water-absorbing resin and then calculating an average particle diameter. As a measuring device, a 3D real surface view microscope (manufactured by Keyence Corporation) was used.

Specifically, first, a water-absorbing agent, to which the multicomponent metal compound was added, was classified with use of JIS standard sieves (JIS Z8801-1(2000)) having respective mesh sizes of 600 $\mu$m and 300 $\mu$m or sieves corresponding to the JIS standard sieves so that the water-absorbing agent which had a particle diameter of 300 $\mu$m to 600 $\mu$m was taken out. On an electrically conductive carbon double-side tape for SEM (manufactured by Nisshin EM Co., Ltd.) having a size of 0.8 cm × 0.8 cm, approximately 0.05 g of the water-absorbing agent thus taken out was dispersed. The electrically conductive carbon double-side tape was then attached to an observation stage of the 3D real surface view microscope. Subsequently, an image of a surface of the water-absorbing agent was captured by the 3D real surface view microscope (detector; secondary electron detector, acceleration voltage; 1.7 kV, magnification; 5000 times). Thereafter, a diameter, in a given direction, of the multicomponent metal compound which adhered to the surface of the water-absorbing agent was measured, and then an average particle diameter was calculated. Note that, in a case where the water-absorbing agent was composed only of particles which passed through a sieve having a mesh size of 300 $\mu$m or particles which did not pass through a sieve having a mesh size of 600 $\mu$m, particles which were obtained by classifying the water-absorbing agent with use of a sieve having a mesh size in a range of 300 $\mu$m for an upper limit or a lower limit, for example, particles each having a diameter of 600 $\mu$m to 900 $\mu$m or 300 $\mu$m to 0 $\mu$m were alternatively used for measurement, as appropriate.

(v) Method of quantifying multicomponent metal compound (hydrotalcite) by X-ray diffraction

A hydrotalcite compound contained in a water-absorbing resin powder was qualified and quantified by powder X-ray diffraction (XRD) with use of a powder X-ray diffractometer (manufactured by Rigaku Corporation, product name: SmartLab). Measurement conditions are shown below.

X-ray source: Cu-K$\alpha$ X-ray ($\lambda$=0.15418 nm) / 45 kV / 200 mA

Scanning range: 2$\theta$=5° to 80°
Scanning speed: 3°/min

A glass sample folder having a 0.5-millimeter-deep depression was uniformly filled with a sample, and a surface of the sample with which the glass sample folder was filled was externally made flat with use of another glass plate. Next, the glass plate filled with the sample was set on the powder X-ray diffractometer, and an XRD pattern was obtained.

Whether or not the water-absorbing resin powder had the hydrotalcite compound was determined based on whether or not peaks of intense lines shown in the XRD pattern thus obtained included peaks of two intense lines peculiar to the hydrotalcite compound. Specifically, in a case where diffraction peaks were present at the following respective two diffraction angles (a) and (b), it was determined that the water-absorbing resin powder had the hydrotalcite compound.

$$(a) \quad 2\theta = 1 1.5° \pm 1.0°$$

$$(b) \quad 2\theta = 2 2.9° \pm 1.0°$$

Note that it was determined that the diffraction peak present at the diffraction angle (a) was based on a diffraction line corresponding to a (003) plane of the hydrotalcite compound. Note also that it was determined that the diffraction peak present at the diffraction angle (b) was based on a diffraction line corresponding to a (006) plane of the hydrotalcite compound.

An amount of hydrotalcite contained in the water-absorbing resin powder was calculated from diffraction peak intensity shown in the XRD pattern. Specifically, a water-absorbing resin powder containing a known amount of hydrotalcite was subjected to XRD measurement, and diffraction peak intensity at (a) $2\theta = 11.5° \pm 1.0°$ or (b) $2\theta = 22.9° \pm 1.0°$ in an XRD pattern was used to create a calibration curve. The calibration curve was regarded as an external standard, and an amount (mass%) of the hydrotalcite compound contained in the water-absorbing resin powder was determined.

(w) Measurement of crystallite diameter of water-insoluble metal phosphate

A crystallite diameter of water-insoluble metal phosphate was measured by powder X-ray diffraction (XRD) with use of a powder X-ray diffractometer (manufactured by Spectris Co., Ltd., product name: X'Pert PRO MPD). Measurement conditions are shown below.

X-ray source: Cu-K$\alpha$ X-ray ($\lambda$=0.15406 nm) / 45 kV / 40 mA

Scanning range: $2\theta$=20° to 40°

Step size: 0.017°
Scan step time: 50 seconds

A glass sample folder having a 0.5-millimeter-deep depression was uniformly filled with a sample, and a surface of the sample with which the glass sample folder was filled was externally made flat with use of another glass plate. Next, the glass plate filled with the sample was set on the powder X-ray diffractometer, and an XRD pattern was obtained.

The crystallite diameter of the water-insoluble metal phosphate was calculated by the Debye-Scherrer equation with use of a half-width of a diffraction peak having the highest relative intensity.

$$\text{Deby-Scherrer equation:} \quad d = 0.9 \times \lambda \div (B \times \cos\theta)$$

(d: crystallite diameter, $\lambda$: wavelength of X-ray, B: half-width of diffraction peak, $\theta$: diffraction angle $2\theta/\theta$)

The crystallite diameter of the water-insoluble metal phosphate on a particulate water-absorbing agent was determined by subjecting, to XRD measurement, the particulate water-absorbing agent to which the water-insoluble metal phosphate was added. Specifically, first, the particulate water-absorbing agent, to which the water-insoluble metal phosphate was added, was classified with use of a JIS standard sieve (JIS Z880 1-1(2000)) having a mesh size of 106 $\mu$m or a sieve corresponding to the JIS standard sieve so that 0.5 g of the particulate water-absorbing agent which had a particle diameter of 106 $\mu$m or less was taken out. Next, the particulate water-absorbing agent thus taken out was subjected to XRD measurement in a manner similar to that described above, and the crystallite diameter was calculated from an obtained diffraction peak.

(x) Measurement of average primary particle diameter of water-insoluble metal phosphate

An average primary particle diameter of water-insoluble phosphate used in the present invention refers to a specific surface area-equivalent diameter of the water-insoluble metal phosphate. The specific surface area-equivalent diameter indicates the following particle diameter. That is, a spherical particle having a specific surface area identical to a specific surface area, which is determined by the BET method, of a particle is assumed. In this case, a particle diameter of the spherical particle indicates the specific surface area-equivalent diameter. The specific surface area-based equivalent spherical diameter is calculated by the following equation.

$$D = \{6 / (Sg \times \rho)\}$$

where:

D represents a specific surface area-equivalent diameter (pm);
Sg represents a specific surface area ($m^2$/g); and
p represents absolute specific gravity (g/$cm^3$) of particles.

For the above measurement of the specific surface area, a device which measures a specific surface area by the nitrogen adsorption single point BET method can be used. Examples of the device encompass Macsorb HM model-1210 manufactured by Mountech Co., Ltd. A specific measuring method is as below.

First, a glass dedicated cell was filled with approximately 0.5 g of a measurement sample (hereinafter, an amount of the measurement sample, with which the glass dedicated cell was filled, will be expressed as "a (g)"). Next, the dedicated cell was set on a body of a measuring apparatus, dried and deaerated at 110°C for 60 minutes under a nitrogen atmosphere, and then cooled to a room temperature.

Subsequently, while the dedicated cell was being cooled with liquid nitrogen, a gas (a mixed gas of 30 volume% of nitrogen (primary) and 70 volume% of helium) for measurement was caused to flow into the dedicated cell at a flow rate of 25 ml/minute, and an amount (V ($cm^3$)) of the gas which adsorbed to the measurement sample was measured.

A value measured by the above operation was substituted into the following equation to calculate a specific surface area Sg ($m^2$/g) of the sample.

$$Sg = S / a = \{K \times (1 - P / P0) \times V\} / a$$

where:

S represents a total surface area ($m^2$) of a sample;
K represents a gas constant (4.29 in this measurement); and
P/P0 represents a relative pressure of an absorption gas and is 97% of a mixing ratio (0.29 in this measurement).

Note that, in the present disclosure, the following values were employed as absolute specific gravity.
Calcium phosphate: 3.1 (g/$cm^3$)
Aluminum phosphate: 2.6 (g/$cm^3$)
Apatite $\alpha$-TCP: 2.6 (g/$cm^3$)
Novaron AGZ010: 5.1 (g/$cm^3$)
Calcium phosphate TTCP: 3.1 (g/$cm^3$)
Aerosil 200CF: 2.2 (g/$cm^3$)

(y) Measurement of diffusing absorbency

A diffusing absorbency under pressure of a particulate water-absorbing agent was measured according to a measuring method disclosed in Japanese Patent Application Publication, Tokukai, No. 2010-142808. Specifically, the diffusing absorbency under pressure of the particulate water-absorbing agent was measured as below.

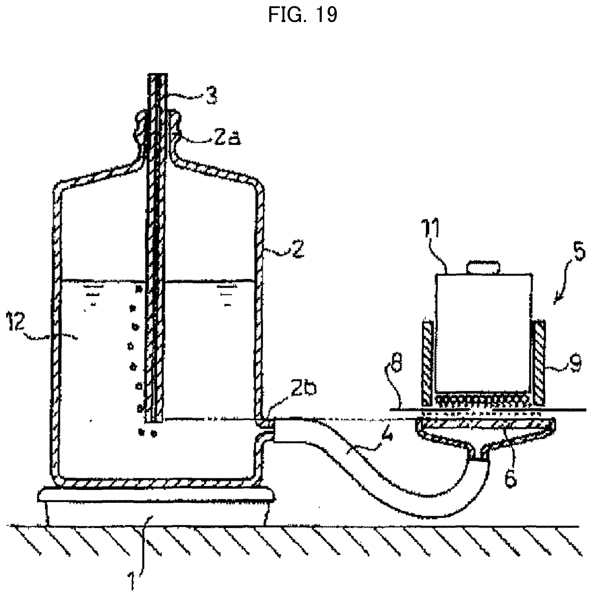

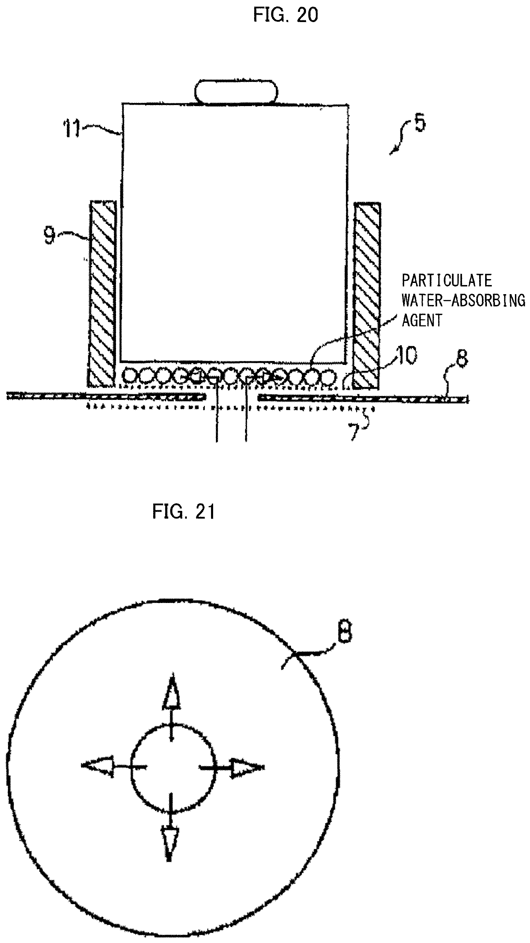

First, a measuring device used to measure the diffusing absorbency under pressure will be briefly described below with reference to Figs. 19 and 20.

As illustrated in Fig. 19, the measuring device includes: a balance 1; a container 2 which is placed on the balance 1 and which has a given capacity; an external air intake pipe 3; a duct 4; a glass filter 6; and a measuring section 5 which is placed on the glass filter 6. The container 2 has an opening 2a in its top, and has an opening 2b in its side surface. The external air intake pipe 3 is fitted into the opening 2a, and the duct 4 is attached to the opening 2b. A certain amount of physiological saline 12 is put in the container 2. A lower end of the external air intake pipe 3 is immersed in the physiological saline 12. The glass filter 6 is formed so as to have a diameter of 70 mm. The container 2 and the glass filter 6 are communicated with each other via the duct 4. The glass filter 6 is fixed so that an upper surface of the glass filter 6 is located at a position slightly higher than a position of the lower end of the external air intake pipe 3.

As illustrated in Fig. 20, the measuring section 5 includes a filter paper 7, a sheet 8, a supporting cylinder 9, a metal gauze 10 attached to a bottom of the supporting cylinder 9, and a weight 11. The measuring section 5 is configured such that the filter paper 7, the sheet 8, and the supporting cylinder 9 (that is, the metal gauze 10) are placed in this order on the glass filter 6 and that the weight 11 is placed inside the supporting cylinder 9, that is, on the metal gauze 10. The sheet 8 is made of polyethylene terephthalate (PET). The sheet 8 is formed in a ring shape so as to have, in its middle part, an opening having a diameter of 18 mm, and has a thickness of 0.1 mm. The supporting cylinder 9 is formed so as to have an inner diameter of 60 mm. The metal gauze 10 is made of stainless steel, and is a 400-mesh metal gauze (having a mesh size of 38 $\mu$m) according to a JIS standard. The metal gauze 10 is configured such that a certain amount of a particulate water-absorbing agent is uniformly spread on the metal gauze 10. A weight of the weight 11 is adjusted so that the weight 11 can uniformly apply a load of 20 g/cm$^2$ (1.96 kPa) to the metal gauze 10, that is, the particulate water-absorbing agent.

The diffusing absorbency under pressure was measured with use of the measuring device thus configured. The measuring method will be described below.

First, given preparation was made. For example, a certain amount of the physiological saline 12 was put in the container 2, and the external air intake pipe 3 was fitted into the container 2. Next, the filter paper 7 was placed on the glass filter 6. The sheet 8 was placed on the filter paper 7 so that the opening of the sheet 8 was located in a middle part of the glass filter 6. In parallel to those placing operations, 1.5 g of a particulate water-absorbing agent was uniformly spread inside the supporting cylinder 9, that is, on the metal gauze 10. The weight 11 was placed on the particulate water-absorbing agent.

Next, the metal gauze 10, that is, the supporting cylinder 9 inside which the particulate water-absorbing agent and the weight 11 were placed was placed on the sheet 8 so that a middle part of the metal gauze 10 matched the middle part of the glass filter 6.

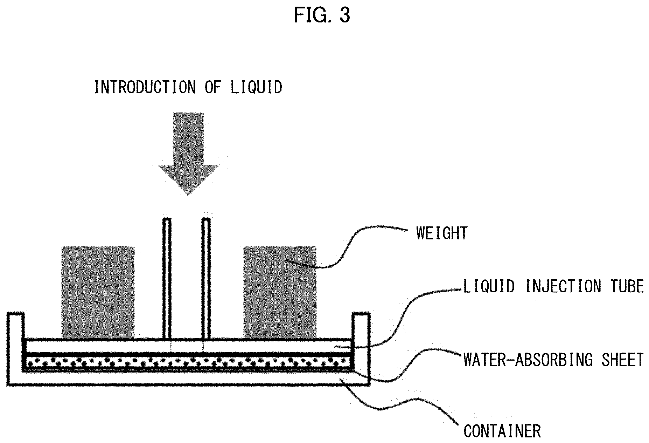

Subsequently, the particulate water-absorbing agent was caused to absorb the physiological saline 12 over 60 minutes from a time point when the supporting cylinder 9 was placed on the sheet 8. A weight W2 (g) of the physiological saline 12 thus absorbed was measured with use of the balance 1. Note that, as illustrated in Figs. 3 and 4, the physiological saline 12 passed through the opening of the sheet 8 and was then absorbed by the particulate water-absorbing agent while the physiological saline 12 was substantially uniformly diffusing in a horizontal direction (shown by an arrow in Figs. 20 and 21) of the particulate water-absorbing agent.

Thereafter, a diffusing absorbency under pressure (g/g) after 60 minutes elapsed from start of absorption was calculated by the following equation with use of the above weight W2.

Diffusing absorbency under pressure (g/g)=weight W2 (g)/weight (g) of a particulate water-absorbing agent

A value of a diffusing absorbency under pressure (g/g) after 10 minutes was calculated by changing the above liquid absorbing time from 60 minutes to 10 minutes.

[Particulate water-absorbing agent]

The following description will discuss a typical method of producing a particulate absorbing agent which can be used in a water-absorbing sheet for use in accordance with the present application, as well as typical production examples. However, the particulate water-absorbing agent and comparative particulate water-absorbing agents are not limited to a production method described in the present specification, and can be produced by a combination of publicly known methods as appropriate.

An aqueous monomer solution was obtained by dissolving 4.4 parts by weight of polyethylene glycol diacrylate (n=9) into 5500 parts by weight of a 38 weight% aqueous sodium acrylate solution (neutralization rate: 75 mol%). Next, a reaction solution thus obtained was deaerated for 30 minutes under a nitrogen gas atmosphere. Subsequently, the reaction solution was supplied into a twin-arm stainless steel kneader equipped with two sigma-type blades, an openable/closable lid, and a jacket. While the reaction solution was kept at 30°C, gas in a system was replaced with nitrogen gas. Then, while the reaction solution was stirred, 2.8 parts by weight of sodium persulfate and 0.12 parts by weight of L-ascorbic acid were added to the reaction solution. Polymerization started approximately 1 minute thereafter. The polymerization was carried out at a temperature of 30°C to 90°C. A particulate hydrogel was taken out from the kneader 60 minutes after start of the polymerization. The particulate hydrogel thus obtained had been grain refined such that the particulate hydrogel had a diameter of approximately 5 mm. The particulate hydrogel thus grain refined was spread on a 50-mesh metal gauze, and subjected to hot air drying at 160°C for 60 minutes. Next, a dried material thus obtained was pulverized with use of a roll mill (manufactured by Inoguchi Giken Ltd.; WML-type roll crusher) to obtain a water-absorbing resin powder (A) having a non-uniformly pulverized shape. The water-absorbing resin powder (A) having a non-uniformly pulverized shape was further classified with use of a metal gauze having a mesh size of 850 $\mu$m and a metal gauze having a mesh size of 180 $\mu$m to obtain a water-absorbing resin powder (A1), which was a fraction that passed through the metal gauze having a mesh size of 850 $\mu$m but did not pass through the metal gauze having a mesh size of 180 $\mu$m, and a water-absorbing resin powder (A2), which was a fraction that passed through the metal gauze having a mesh size of 180 $\mu$m. At this time, according to the water-absorbing resin powder (A), a proportion of particles having a particle diameter of less than

150 μm was 10.5 weight%. According to the water-absorbing resin powder (A1) obtained as a result of classification, it was possible to reduce, to 1.9 weight%, the proportion of the particles having a particle diameter of less than 150 μm.

Into a 5-liter mortar mixer (manufactured by Nishinihon Shikenki Seisakusho, a 5-liter container was kept warm in a bath at 80°C), 300 g of water-absorbing resin fine particles (A2) obtained above were put. While a stirring blade of the mortar mixer was rotated at a high speed with 60Hz/100V, 300 g of deionized water, heated to 90°C, was introduced at once into the mortar mixer. The water-absorbing resin fine particles (A2) and the deionized water were mixed together within 10 seconds. An entire content became a hydrogel granulated material having a particle diameter of approximately 3 mm to 10 mm. In the mortar mixer, the hydrogel granulated material was in a separated state, and was not likely to be kneaded by mixing with use of the stirring blade. After the hydrogel granulated material was stirred at a high speed for 1 minute in the mortar mixer, the hydrogel granulated material thus obtained in the separated state was spread on a 50-mesh metal gauze, and then subjected to hot air drying at 150°C for 60 minutes. Next, a dried granulated material thus obtained was pulverized with use of a roll mill to obtain a water-absorbing resin granulated product (A3'). The water-absorbing resin granulated product (A3') was further classified with use of a metal gauze having a mesh size of 850 μm and a metal gauze having a mesh size of 150 μm to obtain a water-absorbing resin granulated product (A3) which was a fraction that passed through the metal gauze having a mesh size of 850 μm but did not pass through the metal gauze having a mesh size of 150 μm. At this time, according to the water-absorbing resin granulated product (A3'), a proportion of particles having a particle diameter of less than 150 μm was 21.8 weight%. According to the water-absorbing resin granulated product (A3) obtained as a result of classification, it was possible to reduce, to 2.0 weight%, the proportion of the particles having a particle diameter of less than 150 μm.

Then, a surface-crosslinking agent solution, containing 0.025 parts by weight of ethyleneglycoldiglycidyl ether, 0.3 parts by weight of ethylene carbonate, 0.5 parts by weight of propylene glycol, and 2.0 parts by weight of deionized water, was mixed with 100 parts by weight of the water-absorbing resin granulated product (A3) obtained above. A resultant mixture was heat-treated at 200°C for 35 minutes to obtain a surface-crosslinked water-absorbing resin granulated product (A4). To 100 parts by weight of the surface-crosslinked water-absorbing resin granulated product (A4), 1 part by weight of a 1 weight% DTPA aqueous solution was added while being stirred. A resultant mixture was then mixed for 1 minute. Next, the mixture was left to sit for 30 minutes in a hot air dryer at 60°C, and then caused to pass through a metal gauze having a mesh size of 850 μm to obtain a water-absorbing resin granulated product (A4'). Subsequently, with 100 parts by weight of the water-absorbing resin granulated product (A4') thus obtained, 0.3 parts by weight of silicon dioxide (product name: Aerosil 200, manufactured by Nippon Aerosil Co., Ltd.) was mixed. Mixing was carried out in such a manner that 30 g of a water-absorbing resin was put in a 225-milliliter mayonnaise bottle together with silicon dioxide and then shaken for 3 minutes with use of a paint shaker (No.488/manufactured by Toyo Seiki Seisaku-sho, Ltd.) under a condition of 800(cycle/min(CPM)). As a result, a particulate water-absorbing agent (0) was obtained. Tables 3 and 4 show physical properties of the particulate water-absorbing agent (0).

[Production Example a]

First, there was prepared an aqueous monomer solution (a) containing 300 parts by mass of acrylic acid, 100 parts by mass of a 48 mass% aqueous sodium hydroxide solution, 0.94 parts by mass of polyethylene glycol diacrylate (average n number: 9), 16.4 parts by mass of a 0.1 mass% aqueous trisodium diethylenetriamine pentaacetate solution, and 314.3 parts by mass of deionized water.

Next, the aqueous monomer solution (a) whose temperature had been adjusted to 38°C was continuously fed by a metering pump, and then 150.6 parts by mass of a 48 mass% aqueous sodium hydroxide solution was further continuously line-mixed with the aqueous monomer solution (a). At this stage, the temperature of the aqueous monomer solution (a) was raised to 80°C due to heat of neutralization.

Subsequently, 14.6 parts by mass of a 4 mass% aqueous sodium persulfate solution was continuously line-mixed with the aqueous monomer solution (a), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (a) was obtained. The belt-shaped hydrogel (a) obtained was continuously cut at regular intervals in the width direction relative to the traveling direction of the polymerization belt so that the cut length was 300 mm. Thus, a strip-shaped hydrogel (a) was obtained. The strip-shaped hydrogel (a) had a CRC of 33.5 g/g and a moisture content of 50.5 mass% (a resin solid content of 49.5 weight%).

Note that "moisture content (%) = 100 - resin solid content (%)".

[Production Example b]

First, there was prepared an aqueous monomer solution (b) containing 300 parts by mass of acrylic acid, 100 parts by mass of a 48 mass% aqueous sodium hydroxide solution, 0.61 parts by mass of polyethylene glycol diacrylate (average n number: 9), 6.5 parts by mass of a 1.0 mass% aqueous pentasodium ethylenediamine tetra(methylene phosphonate) solution, and 346.1 parts by mass of deionized water.

Next, the aqueous monomer solution (b) whose temperature had been adjusted to 40°C was continuously fed by a metering pump, and then 150.6 parts by mass of a 48 mass% aqueous sodium hydroxide solution was further continuously line-mixed with the aqueous monomer solution (b). At this stage, the temperature of the aqueous monomer solution (b) was raised to 81°C due to heat of neutralization.

Subsequently, 14.6 parts by mass of a 4 mass% aqueous sodium persulfate solution was continuously line-mixed with the aqueous monomer solution (b), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (b) was obtained. The belt-shaped hydrogel (b) obtained was continuously cut at regular intervals in the width direction relative to the traveling direction of the polymerization belt so that the cut length was 300 mm. Thus, a strip-shaped hydrogel (b) was obtained. The strip-shaped hydrogel (b) had a CRC of 36.0 g/g and a moisture content of 51.9 mass% (a resin solid content of 48.1 weight%).

[Production Example c]

First, there was prepared an aqueous monomer solution (c) containing 300 parts by mass of acrylic acid, 100 parts by mass of a 48 mass% aqueous sodium hydroxide solution, 0.61 parts by mass of polyethylene glycol diacrylate (average n number: 9), 16.4 parts by mass of a 0.1 mass% aqueous pentasodium ethylenediamine tetra(methylene phosphonate) solution, and 274.4 parts by mass of deionized water.

Next, the aqueous monomer solution (c) whose temperature had been adjusted to 38°C was continuously fed by a metering pump, and then 150.6 parts by mass of a 48 mass% aqueous sodium hydroxide solution was further continuously line-mixed with the aqueous monomer solution (c). At this stage, the temperature of the aqueous monomer solution (c) was raised to 83°C due to heat of neutralization.

Subsequently, 14.6 parts by mass of a 4 mass% aqueous sodium persulfate solution was continuously line-mixed with the aqueous monomer solution (b), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (c) was obtained. The belt-shaped hydrogel (c) obtained was continuously cut at regular intervals in the width direction relative to the traveling direction of the polymerization belt so that the cut length was 300 mm. Thus, a strip-shaped hydrogel (c) was obtained. The strip-shaped hydrogel (c) had a CRC of 33.6 g/g and a moisture content of 46.9 mass% (a resin solid content of 53.1 weight%).

[Production Example d]

First, there was prepared an aqueous monomer solution (d) containing 300 parts by mass of acrylic acid, 100 parts by mass of a 48 mass% aqueous sodium hydroxide solution, 1.46 parts by mass of polyethylene glycol diacrylate (average n number: 9), 16.4 parts by mass of a 0.1 mass% aqueous trisodium diethylenetriamine pentaacetate solution, and 361 parts by mass of deionized water.

Next, the aqueous monomer solution (d) whose temperature had been adjusted to 42°C was continuously fed by a metering pump, and then 150.6 parts by mass of a 48 mass% aqueous sodium hydroxide solution was further continuously line-mixed with the aqueous monomer solution (d). At this stage, the temperature of the aqueous monomer solution (d) was raised to 81°C due to heat of neutralization.

Subsequently, 14.6 parts by mass of a 4 mass% aqueous sodium persulfate solution was continuously line-mixed with the aqueous monomer solution (b), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (d) was obtained. The belt-shaped hydrogel (d) obtained was continuously cut at regular intervals in the width direction relative to the traveling direction of the polymerization belt so that the cut length was 300 mm. Thus, a strip-shaped hydrogel (d) was obtained. The strip-shaped hydrogel (d) had a CRC of 33.3 g/g and a moisture content of 52.9 mass% (a resin solid content of 47.1 weight%).

[Production Example e]

First, there was prepared an aqueous monomer solution (e) containing 300 parts by mass of acrylic acid, 100 parts by mass of a 48 mass% aqueous sodium hydroxide solution, 0.61 parts by mass of polyethylene glycol diacrylate (average n number: 9), 6.5 parts by mass of a 1.0 mass% aqueous pentasodium ethylenediamine tetra(methylene phosphonate) solution, and 371.6 parts by mass of deionized water.

Next, the aqueous monomer solution (e) whose temperature had been adjusted to 42°C was continuously fed by a metering pump, and then 150.6 parts by mass of a 48 mass% aqueous sodium hydroxide solution was further continuously line-mixed with the aqueous monomer solution (e). At this stage, the temperature of the aqueous monomer solution (e) was raised to 81°C due to heat of neutralization.

Subsequently, 14.6 parts by mass of a 4 mass% aqueous sodium persulfate solution was continuously line-mixed with the aqueous monomer solution (b), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (e) was obtained. The belt-shaped hydrogel (e) obtained was continuously cut at regular intervals in the width direction relative to the traveling direction of the polymerization belt so that the cut length was 300 mm. Thus, a strip-shaped hydrogel (e) was obtained. The strip-shaped hydrogel (e) had a CRC of 36.7 g/g and a moisture content of 52.8 mass% (a resin solid content of 47.2 weight%).

[Table 1]

| | | Physical properties of hydrogel | |
|---|---|---|---|
| | | CRC (g/g) | Moisture content (wt%) |
| Production Example a | Hydrogel (a) | 33.5 | 50.5 |
| Production Example b | Hydrogel (b) | 36.0 | 51.9 |
| Production Example c | Hydrogel (c) | 33.6 | 46.9 |
| Production Example d | Hydrogel (d) | 33.3 | 52.9 |
| Production Example e | Hydrogel (e) | 36.7 | 52.8 |

[Production Example 1]

(Gel-crushing)

A particulate hydrogel (1) was obtained by feeding, to a screw extruder, the strip-shaped hydrogel (a), which had been obtained in Production Example a, and subjecting the strip-shaped hydrogel (a) to gel-crushing. The screw extruder includes a porous plate and a screw shaft. The porous plate is provided at a tip of the screw extruder and has a diameter of 100 mm, a pore diameter of 9.5 mm, 40 pores, an aperture ratio of 36.1%, and a thickness of 10 mm, and the screw shaft has an outer diameter of 86 mm.

The gel-crushing was carried out in Production Example 1, while the screw shaft of the screw extruder was being rotated at 130 rpm, by simultaneously feeding the strip-shaped hydrogel (a) and water vapor via respective different feed openings. Note that the strip-shaped hydrogel (a) was fed in an amount of 4640 g per minute and the water vapor was fed in an amount of 83 g per minute.

In Production Example 1, gel-grinding energy (GGE) was 26.9 J/g, and gel-grinding energy grinding energy (2) (GGE (2)) was 13.6 J/g. The hydrogel (a) which had not been subjected to the gel-crushing had a temperature of 80°C, and the particulate hydrogel (1) obtained after the gel-crushing had a temperature of 85°C.

The particulate hydrogel (1) obtained after the gel-crushing had a moisture content of 50.9 mass%, a mass average particle diameter (D50) of 994 $\mu$m, and a logarithmic standard deviation ($\sigma\zeta$) of a particle size distribution of 1.01. Table 2 shows gel-crushing conditions and physical properties of the particulate hydrogel (1).

(Drying)

Next, within one minute of the end of the gel-crushing, the particulate hydrogel (1) was placed on a through-flow belt of a continuous dryer of a through-flow belt type (the particulate hydrogel (1) having a temperature of 80°C at this stage). Then, the particulate hydrogel (1) was dried by causing hot air having 185°C to flow therethrough for 30 minutes. The hot air had an average air velocity of 1.0 m/s in the direction perpendicular to the traveling direction of the through-flow belt. The air velocity of the hot air was measured with use of a constant temperature thermal anemometer (Anemomaster 6162 manufactured by Kanomax Japan Inc.).

(Pulverization and classification)

Subsequently, the total amount of a dried polymer (1) obtained after the drying was fed to a three-stage roll mill so as to be pulverized. Thereafter, the dried polymer (1) thus pulverized was classified with use of JIS standard sieves having respective mesh sizes of 710 μm and 175 μm. Thus, a water-absorbing resin powder (1) having a non-uniformly pulverized shape was obtained. The water-absorbing resin powder (1) had a mass average particle diameter (D50) of 348 μm, a logarithmic standard deviation (σζ) of a particle size distribution of 0.32, a CRC of 42.1 g/g, and a proportion of particles having a particle diameter of less than 150 μm (a proportion of particles passing through a sieve having a mesh size of 150 μm) of 0.5 mass%.

(Surface crosslinking and additive addition)

Next, to 100 parts by mass of the water-absorbing resin powder (1), a surface-crosslinking agent solution (1) containing 0.025 parts by mass of ethyleneglycoldiglycidyl ether, 0.4 parts by mass of 1,4-butanediol, 0.6 parts by mass of propylene glycol, and 3.0 parts by mass of deionized water was added. Then, a resultant mixture was mixed until the mixture was made uniform. Thereafter, the mixture was heat-treated at 190°C for approximately 30 minutes so that resulting water-absorbing resin particles (1) would have a CRC of 35 g/g. Then, the mixture was force-cooled to 60°C.
Subsequently, the water-absorbing resin particles (1) obtained through the above operations was subjected to a paint shaker test (described earlier), and damage equivalent to that caused during a production process was caused to the water-absorbing resin particles (1). Thereafter, to 100 parts by mass of the water-absorbing resin powder (1), 1.01 parts by mass of an aqueous chelating agent solution containing 0.01 parts by mass of trisodium diethylenetriamine pentaacetate and 1 part by mass of deionized water was added. Then, a resultant mixture was mixed until the mixture was made uniform. Thereafter, the mixture was dried at 60°C for 1 hour, and a resultant product was passed through a JIS standard sieve having a mesh size of 710 μm. Then, to the product thus having been passed through the JIS standard sieve, 0.4 parts by mass of silicon dioxide (product name: Aerosil 200, manufactured by Nippon Aerosil Co., Ltd.) was added. Then, a resultant mixture was mixed until the mixture was made uniform.
Through the above operations, a particulate water-absorbing agent (1) was obtained. Tables 3 through 5 show physical properties of the particulate water-absorbing agent (1).
Note that an amount of an increase in 150 μm passing particles (particles passing through a sieve having a mesh size of 150 pm), the amount being obtained after the paint shaker test (PS), means an amount of an increase in 150 μm passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper.

[Production Example 2]

The strip-shaped hydrogel (b), which had been obtained in Production Example b, was subjected to gel-crushing similar to that carried out in Production Example 1, except for the following change in condition. Thus, a particulate hydrogel (2) was obtained.
In Production Example 2, the pore diameter of the porous plate of the screw extruder was changed from 9.5 mm to 8 mm. The change caused the porous plate to have an aperture ratio of 25.6%. In Production Example 2, gel-grinding energy (GGE) was 31.9 J/g, and gel-grinding energy (2) (GGE (2)) was 17.5 J/g. The hydrogel (b) which had not been subjected to the gel-crushing had a temperature of 80°C, and the particulate hydrogel (2) had a temperature of 84°C after the gel-crushing.
The particulate hydrogel (2) obtained after the gel-crushing had a moisture content of 52.5 mass%, a mass average particle diameter (D50) of 860 μm, and a logarithmic standard deviation (σζ) of a particle size distribution of 0.95. Table 2 shows gel-crushing conditions and physical properties of the particulate hydrogel (2).
Subsequently, the particulate hydrogel (2) was subjected to drying, pulverization, and classification similar to those carried out in Production Example 1, so that water-absorbing resin powder (2) having a non-uniformly pulverized shape was obtained. The water-absorbing resin powder (2) had a mass average particle diameter (D50) of 355 μm, a logarithmic standard deviation (σζ) of a particle size distribution of 0.32, a CRC of 48.2 g/g, and a proportion of particles having a particle diameter of less than 150 μm (a proportion of particles passing through a sieve having a mesh size of 150 μm) of 0.4 mass%.
Next, to 100 parts by mass of the water-absorbing resin powder (2), a surface-crosslinking agent solution (2) containing 0.025 parts by mass of ethyleneglycoldiglycidyl ether, 0.4 parts by mass of ethylene carbonate, 0.6 parts by mass of propylene glycol, and 3.0 parts by mass of deionized water was added. Then, a resultant mixture was mixed until the mixture was made uniform. Thereafter, the mixture was heat-treated at 190°C for approximately 30 minutes so that resulting water-absorbing resin particles (2) would have a CRC of 38 g/g. Then, the mixture was force-cooled to 60°C.

Thereafter, operations similar to those carried out in Production Example 1 were carried out, so that a particulate water-absorbing agent (2) was obtained. Tables 3 through 5 show physical properties of the particulate water-absorbing agent (2).

[Production Example 3]

A dried polymer (2) obtained in Production Example 2 was subjected to pulverization and classification similar to those carried out in Production Example 2, except for the following change in condition. Thus, water-absorbing resin powder (3) having a non-uniformly pulverized shape was obtained. Specifically, in the classification carried out in Production Example 3, the sieve having a mesh size of 710 $\mu$m was changed to a sieve having a mesh size of 850 $\mu$m.

The water-absorbing resin powder (3) had a mass average particle diameter (D50) of 431 $\mu$m, a logarithmic standard deviation ($\sigma\zeta$) of a particle size distribution of 0.35, a CRC of 48.2 g/g, and a proportion of particles having a particle diameter of less than 150 $\mu$m (a proportion of particles passing through a sieve having a mesh size of 150 $\mu$m) of 0.3 mass%.

Thereafter, the water-absorbing resin powder (3) was subjected to surface crosslinking, similar to that carried out in Production Example 2, so as to be passed through the sieve having a mesh size of 850 $\mu$m. Then, the water-absorbing resin powder (3) was further subjected to a treatment similar to that carried out in Production Example 2, so that a particulate water-absorbing agent (3) was obtained. Tables 3 through 5 show physical properties of the particulate water-absorbing agent (3).

[Production Example 4]

To 100 parts by mass of the water-absorbing resin powder (1), which had been obtained in Production Example 1, a surface-crosslinking agent solution (4) containing 0.3 parts by mass of 1,4-butanediol, 0.5 parts by mass of propylene glycol, and 2.0 parts by mass of deionized water was added. Then, a resultant mixture was mixed until the mixture was made uniform. Thereafter, the mixture was heat-treated at 200°C for approximately 30 minutes so that resulting water-absorbing resin particles (4) would have a CRC of 35 g/g. Then, the mixture was force-cooled to 60°C.

Subsequently, the water-absorbing resin particles (4) obtained through the above operations was subjected to a paint shaker test (described earlier), and damage equivalent to that caused during a production process was caused to the water-absorbing resin particles (4). Thereafter, to 100 parts by mass of water-absorbing resin powder (4), an aqueous solution (4-1) containing 0.05 parts by mass of polyethylene glycol (having an average molecular weight of 400) and 0.5 parts by mass of deionized water was added. Then, a resultant mixture was mixed until the mixture was made uniform. Thereafter, to the mixture, an aqueous solution (4-2) containing 0.01 parts by mass of trisodium diethylenetriamine pentaacetate, 0.6 parts by mass of a 27.5 mass% aqueous aluminum sulfate solution (8 mass% based on aluminum oxide), 0.1 parts by mass of a 60 mass% aqueous sodium lactate solution, and 0.02 parts by mass of propylene glycol was further added. Then, a resultant mixture was mixed until the mixture was made uniform. Thereafter, the mixture was dried at 60°C for 1 hour, and a resultant product was passed through a JIS standard sieve having a mesh size of 710 $\mu$m.

Through the above operations, a particulate water-absorbing agent (4) was obtained. Tables 3 through 5 show physical properties of the particulate water-absorbing agent (4).

[Production Example 5]

To 100 parts by mass of the water-absorbing resin powder (1), which had been obtained in Production Example 1, a surface-crosslinking agent solution (5) containing 0.3 parts by mass of ethylene carbonate, 0.5 parts by mass of propylene glycol, 0.001 parts by mass of polyoxyethylene (20) sorbitan monostearate, and 2.0 parts by mass of deionized water was added. Then, a resultant mixture was mixed until the mixture was made uniform. Thereafter, the mixture was heat-treated at 200°C for approximately 45 minutes so that resulting water-absorbing resin particles (5) would have a CRC of 32 g/g.

Then, operations similar to those carried out in Production Example 4 were carried out, so that a particulate water-absorbing agent (5) was obtained. Tables 3 through 5 show physical properties of the particulate water-absorbing agent (5).

[Production Example 6]

The strip-shaped hydrogel (d), which had been obtained in Production Example d, was subjected to gel-crushing similar to that carried out in Production Example 1, except for the following change in condition. Thus, a particulate hydrogel (6) was obtained.

In Production Example 6, the pore diameter of the porous plate of the screw extruder was changed from 9.5 mm to 6.4 mm, and the number of pores was changed from 40 to 83. The change caused the porous plate to have an aperture ratio of 41.4%. In Production Example 6, gel-grinding energy (GGE) was 29.5 J/g, and gel-grinding energy (2) (GGE (2)) was 15.7 J/g. The hydrogel (d) which had not been subjected to the gel-crushing had a temperature of 80°C, and the particulate hydrogel (6) had a temperature of 86°C after the gel-crushing.

The particulate hydrogel (6) obtained after the gel-crushing had a moisture content of 53.5 mass%, a mass average particle diameter (D50) of 360 μm, and a logarithmic standard deviation ($\sigma\zeta$) of a particle size distribution of 0.99. Table 2 shows gel-crushing conditions and physical properties of the particulate hydrogel (6).

Subsequently, the particulate hydrogel (6) was subjected to drying, pulverization, and classification similar to those carried out in Production Example 1, so that water-absorbing resin powder (6) having a non-uniformly pulverized shape was obtained. The water-absorbing resin powder (6) had a mass average particle diameter (D50) of 351 μm, a logarithmic standard deviation ($\sigma\zeta$) of a particle size distribution of 0.32, a CRC of 39.3 g/g, and a proportion of particles having a particle diameter of less than 150 μm (a proportion of particles passing through a sieve having a mesh size of 150 μm) of 0.4 mass%.

Subsequently, to 100 parts by mass of the water-absorbing resin powder (6), a surface-crosslinking agent solution (6) containing 0.025 parts by mass of ethyleneglycoldiglycidyl ether, 0.4 parts by mass of ethylene carbonate, 0.6 parts by mass of propylene glycol, and 3.0 parts by mass of deionized water was added. Then, a resultant mixture was mixed until the mixture was made uniform. Thereafter, the mixture was heat-treated at 190°C for approximately 30 minutes so that resulting water-absorbing resin particles (6) would have a CRC of 34 g/g to 35 g/g. Then, the mixture was force-cooled to 60°C.

Thereafter, operations similar to those carried out in Production Example 1 were carried out, so that a particulate water-absorbing agent (6) was obtained. Tables 3 through 5 show physical properties of the particulate water-absorbing agent (6).

[Production Example 7]

The strip-shaped hydrogel (e), which had been obtained in Production Example e, was subjected to gel-crushing similar to that carried out in Production Example 6. Thus, a particulate hydrogel (7) was obtained. In Production Example 7, gel-grinding energy (GGE) was 34.5 J/g, and gel-grinding energy (2) (GGE (2)) was 19.6 J/g. The hydrogel (e) which had not been subjected to the gel-crushing had a temperature of 80°C, and the particulate hydrogel (7) had a temperature of 87°C after the gel-crushing.

The particulate hydrogel (7) obtained after the gel-crushing had a moisture content of 53.4 mass%, a mass average particle diameter (D50) of 627 μm, and a logarithmic standard deviation ($\sigma\zeta$) of a particle size distribution of 1.02. Table 2 shows gel-crushing conditions and physical properties of the particulate hydrogel (7).

Subsequently, the particulate hydrogel (7) was subjected to drying, pulverization, and classification similar to those carried out in Production Example 1, so that water-absorbing resin powder (7) having a non-uniformly pulverized shape was obtained. The water-absorbing resin powder (7) had a mass average particle diameter (D50) of 366 μm, a logarithmic standard deviation ($\sigma\zeta$) of a particle size distribution of 0.32, a CRC of 49.4 g/g, and a proportion of particles having a particle diameter of less than 150 μm (a proportion of particles passing through a sieve having a mesh size of 150 μm) of 0.4 mass%.

Subsequently, to 100 parts by mass of the water-absorbing resin powder (7), a surface-crosslinking agent solution (7) containing 0.025 parts by mass of ethyleneglycoldiglycidyl ether, 0.4 parts by mass of 1,3-propanediol, 0.6 parts by mass of propylene glycol, and 3.0 parts by mass of deionized water was added. Then, a resultant mixture was mixed until the mixture was made uniform. Thereafter, the mixture was heat-treated at 190°C for approximately 30 minutes so that resulting water-absorbing resin particles (7) would have a CRC of 39 g/g to 40 g/g.

Thereafter, operations similar to those carried out in Production Example 6 were carried out, so that a particulate water-absorbing agent (7) was obtained. Tables 3 through 5 show physical properties of the particulate water-absorbing agent (7).

[Production Example 8]

A dried polymer (6) obtained in Production Example 6 was subjected to pulverization and classification similar to those carried out in Production Example 6, except for the following change in condition. Thus, water-absorbing resin powder (8) having a non-uniformly pulverized shape was obtained. Specifically, in the classification carried out in Production Example 6, the sieve having a mesh size of 710 μm was changed to a sieve having a mesh size of 850 μm.

The water-absorbing resin powder (8) had a mass average particle diameter (D50) of 450 μm, a logarithmic standard deviation ($\sigma\zeta$) of a particle size distribution of 0.32, a CRC of 39.5 g/g, and a proportion of particles having a particle diameter of less than 150 μm (a proportion of particles passing through a sieve having a mesh size of 150 μm) of 0.1 mass%.

Thereafter, operations similar to those carried out in Production Example 6 were carried out, so that a particulate water-absorbing agent (8) was obtained. Tables 3 through 5 show physical properties of the particulate water-absorbing agent (8).

[Production Example 9]

A dried polymer (7) obtained in Production Example 7 was subjected to pulverization and classification similar to those carried out in Production Example 7, except for the following change in condition. Thus, water-absorbing resin powder (9) having a non-uniformly pulverized shape was obtained. Specifically, in the classification carried out in Production Example 7, the sieve having a mesh size of 710 $\mu$m was changed to a sieve having a mesh size of 850 $\mu$m.

The water-absorbing resin powder (9) had a mass average particle diameter (D50) of 448 $\mu$m, a logarithmic standard deviation ($\sigma\zeta$) of a particle size distribution of 0.3 1, a CRC of 49.6 g/g, and a proportion of particles having a particle diameter of less than 150 $\mu$m (a proportion of particles passing through a sieve having a mesh size of 150 $\mu$m) of 0.3 mass%.

Thereafter, operations similar to those carried out in Production Example 7 were carried out, so that a particulate water-absorbing agent (9) was obtained. Tables 3 through 5 show physical properties of the particulate water-absorbing agent (9).

[Production Example 10]

The dried polymer (6) obtained in Production Example 6 was subjected to pulverization and classification similar to those carried out in Production Example 6, except for the following change in condition. Thus, water-absorbing resin powder (10) having a non-uniformly pulverized shape was obtained. Specifically, in the classification carried out in Production Example 6, the sieve having a mesh size of 710 $\mu$m was changed to a sieve having a mesh size of 750 $\mu$m.

The water-absorbing resin powder (10) had a mass average particle diameter (D50) of 392 $\mu$m, a logarithmic standard deviation ($\sigma\zeta$) of a particle size distribution of 0.36, a CRC of 39.5 g/g, and a proportion of particles having a particle diameter of less than 150 $\mu$m (a proportion of particles passing through a sieve having a mesh size of 150 $\mu$m) of 0.3 mass%.

To 100 parts by mass of the water-absorbing resin powder (10), a surface-crosslinking agent solution (10) containing 0.3 parts by mass of ethylene carbonate, 0.5 parts by mass of propylene glycol, and 2.0 parts by mass of deionized water was added. Then, a resultant mixture was mixed until the mixture was made uniform. Thereafter, the mixture was heat-treated at 200°C for approximately 45 minutes so that resulting water-absorbing resin particles (10) would have a CRC of 31 g/g to 32 g/g. Then, the mixture was force-cooled to 60°C.

Subsequently, the water-absorbing resin particles (10) obtained through the above operations was subjected to a paint shaker test (described earlier), and damage equivalent to that caused during a production process was caused to the water-absorbing resin particles (10). Thereafter, to 100 parts by mass of water-absorbing resin powder, an aqueous solution (10-1) containing 0.05 parts by mass of polyethylene glycol (having an average molecular weight of 400) and 0.5 parts by mass of water was added. Then, a resultant mixture was mixed until the mixture was made uniform. Thereafter, to the mixture, an aqueous solution (10-2) containing 0.01 parts by mass of trisodium diethylenetriamine pentaacetate, 0.6 parts by mass of a 27.5 mass% aqueous aluminum sulfate solution (8 mass% based on aluminum oxide), 0.1 parts by mass of a 60 mass% aqueous sodium lactate solution, and 0.02 parts by mass of propylene glycol was further added. Then, a resultant mixture was mixed until the mixture was made uniform. Thereafter, the mixture was dried at 60°C for 1 hour, and a resultant product was passed through a sieve having a mesh size of 750 $\mu$m.

Through the above operations, a particulate water-absorbing agent (10) was obtained. Tables 3 through 5 show physical properties of the particulate water-absorbing agent (10).

[Comparative Production Example 1]

The strip-shaped hydrogel (c), which had been obtained in Production Example c, was subjected to gel-crushing similar to that carried out in Production Example 1, except for the following change in condition. Thus, a comparative particulate hydrogel (1) was obtained.

In Comparative Production Example 1, the pore diameter of the porous plate of the screw extruder was changed from 9.5 mm to 12.5 mm. The change caused the porous plate to have an aperture ratio of 62.5%. In Comparative Production Example 1, gel-grinding energy (GGE) was 19.4 J/g, and gel-grinding energy (2) (GGE (2)) was 7.6 J/g. The hydrogel (c) which had not been subjected to the gel-crushing had a temperature of 82°C, and the comparative particulate hydrogel (1) had a temperature of 84°C after the gel-crushing.

The comparative particulate hydrogel (1) obtained after the gel-crushing had a moisture content of 47.4 mass%, a mass average particle diameter (D50) of 1322 $\mu$m, and a logarithmic standard deviation ($\sigma\zeta$) of a particle size distribution of 1.32. Table 2 shows gel-crushing conditions and physical properties of the comparative particulate hydrogel (1).

Subsequently, the comparative particulate hydrogel (1) was subjected to drying, pulverization, and classification similar to those carried out in Production Example 1, so that comparative water-absorbing resin powder (1) having a non-uniformly pulverized shape was obtained. The comparative water-absorbing resin powder (1) had a mass average particle diameter (D50) of 350 $\mu$m, a logarithmic standard deviation ($\sigma\zeta$) of a particle size distribution of 0.32, a CRC of 41.9 g/g, and a proportion of particles having a particle diameter of less than 150 $\mu$m (a proportion of particles passing through a sieve having a mesh size of 150 $\mu$m) of 0.5 mass%.

Thereafter, operations similar to those carried out in Production Example 1 were carried out, so that a comparative particulate water-absorbing agent (1) was obtained. Tables 3 through 5 show physical properties of the comparative particulate water-absorbing agent (1).

[Comparative Production Example 2]

The comparative water-absorbing resin powder (1), which had been obtained in Comparative Production Example 1, was subjected to surface crosslinking and additive addition similar to those carried out in Production Example 5, so that a comparative particulate water-absorbing agent (2) was obtained. Tables 3 through 5 show physical properties of the comparative particulate water-absorbing agent (2).

[Comparative Production Example 3]

A comparative dried polymer (1) obtained in Comparative Production Example 1 was subjected to pulverization and classification similar to those carried out in Comparative Production Example 1, except for the following change in condition. Thus, comparative water-absorbing resin powder (3) having a non-uniformly pulverized shape was obtained. Specifically, in Comparative Production Example 1, the sieve having a mesh size of 710 $\mu$m was changed to a sieve having a mesh size of 850 $\mu$m.

The comparative water-absorbing resin powder (3) had a mass average particle diameter (D50) of 431 $\mu$m, a logarithmic standard deviation ($\sigma\zeta$) of a particle size distribution of 0.35, a CRC of 42.2 g/g, and a proportion of particles having a particle diameter of less than 150 $\mu$m (a proportion of particles passing through a sieve having a mesh size of 150 $\mu$m) of 0.3 mass%.

Thereafter, operations similar to those carried out in Comparative Production Example 1 were carried out, so that a comparative particulate water-absorbing agent (3) was obtained. Tables 3 through 5 show physical properties of the comparative particulate water-absorbing agent (3).

[Table 2]

| | | Gel-grinding energy (GGE) (J/g) | Gel-grinding energy (2) (GGE(2)) (J/g) | Physical properties of particulate hydrogel | | |
|---|---|---|---|---|---|---|
| | | | | Moisture content (wt%) | Mass average particle diameter (D50) ($\mu$m) | Logarithmic standard deviation ($\sigma\zeta$) of particle size distribution |
| Production Example 1 | Particulate hydrogel (1) | 26.9 | 13.6 | 50.9 | 994 | 1.01 |
| Production Example 2 | Particulate hydrogel (2) | 31.9 | 17.5 | 52.5 | 860 | 0.95 |
| Production Example 6 | Particulate hydrogel (6) | 29.5 | 15.7 | 53.5 | 360 | 0.99 |
| Production Example 7 | Particulate hydrogel (7) | 34.5 | 19.6 | 53.4 | 627 | 1.02 |
| Comparative Production Example 1 | Comparative particulate hydrogel (1) | 19.4 | 7.6 | 47.4 | 1322 | 1.32 |

[Table 3-1]

| | CRC (g/g) | AAP 2.06 kPa (g/g) | Diff. abs. 60 min (g/g) | Diff. abs. 10 min (g/g) | SFC | DRC 5min (g/g) | DRC index | Bulk spec. grav. (g/cm3) | Surface tension (mN/m) | Int. gas bubble ratio (%) | Water -soluble cont. (Ext) (wt%) | Deg. sol. comp. (wt%) | Incr. after PS (wt%) | B.R. (% ) |
|---|---|---|---|---|---|---|---|---|---|---|---|---|---|---|
| PWAA (0) | 33.7 | 25.6 | 25.1 | 16.5 | 5 | 44.0 | 14.3 | - | 72 | - | - | - | - | 0 |
| PWAA (1) | 35.3 | 28.6 | 27.3 | 18.1 | 13 | 43.2 | 16.3 | 0.60 | 72 | 1.2 | 19 | 14 | 3.4 | 0 |
| PWAA (2) | 38.6 | 28.1 | 27.0 | 13.5 | 4 | 40.7 | 23.1 | 0.61 | 72 | 1.2 | 21 | 20 | 3.5 | 0 |
| PWAA (3) | 39.1 | 27.1 | 25.3 | 11.9 | 6 | 38.2 | 25.4 | 0.60 | 72 | 1.3 | 22 | 21 | 3.3 | 0 |
| PWAA (4) | 34.6 | 34.0 | 32.5 | 17.7 | 12 | 41.8 | 20.2 | 0.60 | 72 | 1.1 | 18 | 14 | 3.4 | 0 |
| PWAA (5) | 32.1 | 32.4 | 31.0 | 21.4 | 22 | 40.0 | 25.3 | 0.60 | 72 | 1.1 | 17 | 19 | 3.3 | 0 |
| PWAA (6) | 34.3 | 29.8 | 28.8 | 21.1 | 12 | 47.1 | 5.3 | 0.59 | 72 | 1.1 | - | - | 3.5 | 0 |
| PWAA (7) | 39.5 | 28.0 | 27.1 | 12.8 | 5 | 45.5 | 9.6 | 0.60 | 72 | 1.1 | - | - | 3.4 | 0 |
| PWAA (8) | 34.6 | 29.1 | 27.9 | 18.8 | 13 | 43.8 | 11.5 | 0.59 | 72 | 1.2 | - | - | 3.4 | 0 |
| PWAA (9) | 39.8 | 27.2 | 26.9 | 11.9 | 13 | 42.7 | 14.0 | 0.60 | 72 | 1.2 | - | - | 3.3 | 0 |
| PWAA (10) | 31.8 | 31.5 | 30.5 | 21.8 | 21 | 44.0 | 12.7 | 0.60 | 72 | 1.1 | - | - | 3.3 | 0 |
| PWAA (11-1) | 38.6 | 33.3 | 23.8 | 7.5 | 3 | 39.8 | 25.6 | 0.61 | 72 | 1.2 | - | - | 3.3 | 0 |
| PWAA (11-2) | 38.5 | 32.5 | 24.4 | 7.6 | 4 | 39.8 | 25.6 | 0.60 | 72 | 1.2 | - | - | 3.2 | 0 |
| PWAA (11-3) | 38.5 | 32.8 | 24.1 | 7.3 | 3 | 39.6 | 26.3 | 0.60 | 72 | 1.2 | - | - | 3.3 | 0 |
| PWAA (11-4) | 38.3 | 33.8 | 23.2 | 8.2 | 3 | 39.1 | 27.7 | 0.61 | 72 | 1.2 | - | - | 3.1 | 0 |
| PWAA (11-5) | 35.6 | 31.5 | 28.1 | 11.7 | 3 | 38.7 | 28.2 | 0.60 | 72 | 1.1 | - | - | 1.4 | 0 |
| PWAA (11-6) | 35.4 | 31.0 | 28.8 | 11.5 | 4 | 39.1 | 27.2 | 0.61 | 72 | 1.1 | - | - | 1.5 | 0 |
| PWAA (11-7) | 35.5 | 31.2 | 28.4 | 11.5 | 3 | 38.8 | 27.9 | 0.60 | 72 | 1.1 | - | - | 1.1 | 0 |
| PWAA (11-8) | 35.2 | 31.8 | 27.4 | 12.0 | 3 | 38.3 | 29.4 | 0.60 | 72 | 1.1 | - | - | 1.3 | 0 |

Abbreviations in the above table include the following.

PWAA: Particulate water-absorbing agent; Comp. PWAA: Comparative particulate water-absorbing agent; Diff. abs. 60 min: Diffusing absorbency after 60 minutes; Diff. abs. 10 min: Diffusing absorbency after 10 minutes; Bulk spec. grav.: Bulk specific gravity; Int. gas bubble ratio: Internal gas bubble ratio; Water-soluble cont.: Water-soluble content; Deg. sol. comp.: Degradable soluble component; Incr. after PS: Increase after PS.

[Table 3-2]

| | CRC (g/g) | AAP 2.06 kPa (g/g) | Diff. abs. 60 min (g/g) | Diff. abs. 10 min (g/g) | SFC | DRC 5min (g/g) | DRC index | Bulk spec. grav. (g/cm3) | Surface tension (mN/m) | Int. gas bubble ratio (%) | Water-soluble cont. (Ext) (wt%) | Deg. sol. comp. (wt%) | Incr. after PS (wt%) | B.R. (%) |
|---|---|---|---|---|---|---|---|---|---|---|---|---|---|---|
| PWAA (12-1) | 39.5 | 32.8 | 23.2 | 7.7 | 3 | 43.2 | 15.8 | 0.59 | 72 | 1.2 | - | - | 3.2 | 0 |
| PWAA (12-2) | 39.4 | 32.0 | 23.8 | 7.8 | 4 | 43.8 | 14.2 | 0.60 | 72 | 1.2 | - | - | 3.4 | 0 |
| PWAA (12-3) | 39.4 | 32.4 | 23.5 | 8.1 | 3 | 43.5 | 15.0 | 0.60 | 72 | 1.2 | - | - | 3.3 | 0 |
| PWAA (12-4) | 39.2 | 33.4 | 22.4 | 7.4 | 3 | 42.8 | 16.8 | 0.60 | 72 | 1.2 | - | - | 3.5 | 0 |
| PWAA (12-5) | 35.8 | 31.1 | 22.5 | 12.9 | 3 | 42.5 | 17.5 | 0.59 | 72 | 1.1 | - | - | 1.2 | 0 |
| PWAA (12-6) | 35.7 | 30.8 | 23.1 | 12.3 | 4 | 43.1 | 15.9 | 0.60 | 72 | 1.1 | - | - | 1.5 | 0 |
| PWAA (12-7) | 35.7 | 30.9 | 22.9 | 12.1 | 3 | 42.7 | 16.9 | 0.59 | 72 | 1.1 | - | - | 1.3 | 0 |
| PWAA (12-8) | 35.3 | 31.5 | 22.1 | 12.4 | 3 | 42.2 | 18.2 | 0.60 | 72 | 1.1 | - | - | 1.4 | 0 |
| PWAA (13-1) | 35.2 | 33.9 | 27.9 | 10.6 | 4 | 42.5 | 18.6 | 0.61 | 72 | 1.2 | - | - | 3.3 | 0 |
| PWAA (13-2) | 33.1 | 32.1 | 28.8 | 13.9 | 12 | 41.9 | 19.6 | 0.60 | 72 | 1.2 | - | - | 1.2 | 0 |
| PWAA (14) | 35.3 | 32.1 | 28.6 | 10.8 | 4 | 38.0 | 25.5 | 0.60 | 72 | 1.2 | - | - | 1.1 | 0 |
| PWAA (15) | 36.1 | 32.2 | 27.5 | 11.1 | 4 | 40.7 | 21.4 | 0.60 | 72 | 1.2 | - | - | 1.1 | 0 |
| PWAA (16) | 33.1 | 32.0 | 29.0 | 12.7 | 3 | 39.4 | 22.1 | 0.60 | 72 | 1.2 | - | - | 1.1 | 0 |
| PWAA (17) | 32.5 | 32.1 | 27.9 | 14.5 | 3 | 40.1 | 22.9 | 0.60 | 72 | 1.2 | - | - | 1.1 | 0 |
| PWAA (18) | 38.5 | 28.1 | 27.1 | 13.7 | 4 | 40.8 | 22.8 | 0.60 | 72 | 1.1 | - | - | 3.6 | 0 |
| PWAA (19) | 38.9 | 27.1 | 25.4 | 11.3 | 5 | 38.1 | 24.3 | 0.60 | 72 | 1.2 | - | - | 3.3 | 0 |
| PWAA (20) | 39.4 | 28.0 | 27.2 | 11.8 | 4 | 45.7 | 9.0 | 0.60 | 72 | 1.2 | - | - | 3.5 | 0 |

Abbreviations in the above table include the following.

PWAA: Particulate water-absorbing agent; Comp. PWAA: Comparative particulate water-absorbing agent; Diff. abs. 60 min: Diffusing absorbency after 60 minutes; Diff. abs. 10 min: Diffusing absorbency after 10 minutes; Bulk spec. grav.: Bulk specific gravity; Int. gas bubble ratio: Internal gas bubble ratio; Water-soluble cont.: Water-soluble content; Deg. sol. comp.: Degradable soluble component; Incr. after PS: Increase after PS.

[Table 3-3]

| | CRC (g/g) | AAP 2.06 kPa (g/g) | Diff. abs. 60 min (g/g) | Diff. abs. 10 min (g/g) | SFC | DRC 5min (g/g) | DRC index | Bulk spec. grav. (g/cm3) | Surface tension (mN/m) | Int. gas bubble ratio (%) | Water-soluble cont. (Ext) (wt%) | Deg. sol. comp. (wt%) | Incr. after PS (wt%) | B.R. (%) |
|---|---|---|---|---|---|---|---|---|---|---|---|---|---|---|
| PWAA (21-1) | 38.7 | 33.4 | 23.9 | 8.8 | 3 | 39.8 | 25.6 | 0.61 | 72 | 1.2 | - | - | 3.3 | 0 |
| PWAA (21-2) | 38.5 | 32.4 | 24.3 | 8.5 | 4 | 39.8 | 25.6 | 0.60 | 72 | 1.2 | - | - | 3.2 | 0 |
| PWAA (21-3) | 38.6 | 32.9 | 24.2 | 8.6 | 3 | 39.6 | 26.3 | 0.60 | 72 | 1.2 | - | - | 3.3 | 0 |
| PWAA (21-4) | 38.2 | 33.8 | 23.3 | 7.7 | 3 | 39.1 | 27.7 | 0.61 | 72 | 1.2 | - | - | 3.1 | 0 |
| PWAA (22-1) | 35.8 | 31.0 | 22.6 | 12.6 | 3 | 42.6 | 17.2 | 0.59 | 72 | 1.1 | - | - | 1.3 | 0 |
| PWAA (22-2) | 35.6 | 30.9 | 23.0 | 12.0 | 4 | 43.2 | 15.5 | 0.60 | 72 | 1.1 | - | - | 1.5 | 0 |
| PWAA (22-3) | 35.7 | 31.0 | 23.1 | 11.8 | 3 | 42.8 | 16.7 | 0.59 | 72 | 1.1 | - | - | 1.4 | 0 |
| PWAA (22-4) | 35.4 | 31.3 | 22.2 | 11.3 | 3 | 42.1 | 18.5 | 0.60 | 72 | 1.1 | - | - | 1.3 | 0 |
| PWAA (23) | 33.0 | 29.5 | 28.6 | 21.9 | 20 | 43.0 | 16.9 | 0.60 | 72 | 1.2 | - | - | 3.4 | 0 |
| PWAA (24) | 38.5 | - | 27.0 | - | - | 40.7 | 23.3 | 0.61 | 66 | 1.2 | 21 | 20 | 3.0 | 0 |
| Comp. PWAA (1) | 34.9 | 27.9 | 26.3 | 13.5 | 13 | 33.1 | 44.7 | 0.65 | 72 | 1.2 | 19 | 15 | 3.1 | 0 |
| Comp. PWAA (2) | 32.4 | 31.5 | 29.5 | 12.3 | 21 | 32.5 | 46.5 | 0.66 | 72 | 1.2 | 17 | 20 | 3.3 | 0 |
| Comp. PWAA (3) | 35.1 | 27.0 | 26.5 | 13.7 | 11 | 30.5 | 43.4 | 0.65 | 72 | 1.3 | - | - | 3.2 | 0 |
| Comp. PWAA (4) | 34.9 | 32.9 | 27.5 | 6.2 | 4 | 31.1 | 50.7 | 0.66 | 72 | 1.2 | - | - | 3.5 | 0 |
| Comp. PWAA (5) | 34.8 | 32.4 | 28.1 | 5.3 | 4 | 31.5 | 49.7 | 0.65 | 72 | 1.2 | - | - | 3.3 | 0 |
| Comp. PWAA (6) | 34.8 | 32.6 | 28.1 | 4.9 | 4 | 31.3 | 50.1 | 0.65 | 72 | 1.2 | - | - | 3.1 | 0 |
| Comp. PWAA (7) | 34.6 | 33.1 | 27.0 | 6.0 | 4 | 30.8 | 51.6 | 0.66 | 72 | 1.2 | - | - | 3.4 | 0 |
| Comp. PWAA (8) | 29.1 | 28.1 | 27.2 | 17.0 | 40 | 28.7 | 47.1 | 0.65 | 72 | 1.2 | - | - | 3.7 | 0 |

Abbreviations in the above table include the following.

PWAA: Particulate water-absorbing agent; Comp. PWAA: Comparative particulate water-absorbing agent; Diff. abs. 60 min: Diffusing absorbency after 60 minutes; Diff. abs. 10 min: Diffusing absorbency after 10 minutes; Bulk spec. grav.: Bulk specific gravity; Int. gas bubble ratio: Internal gas bubble ratio; Water-soluble cont.: Water-soluble content; Deg. sol. comp.: Degradable soluble component; Incr. after PS: Increase after PS.

[Table 4-1]

| | | Particle size distribution (PSD) | | | | | | Mass average particle diameter (D50) (μm) | Logarithmic standard deviation (σζ) of particle size distribution |
|---|---|---|---|---|---|---|---|---|---|---|
| | | Particles remaining on 850 μm sieve (wt%) | 850-600 (wt%) | 600-500 (wt%) | 500-425 (wt%) | 425-300 (wt%) | 300-150 (wt%) | Particles which passed through 150 μm sieve (wt%) | | |
| PWAA (0) | | 0.0 | 1 | 8.8 | 18.2 | 40.0 | 30.0 | 2.0 | 350 | 0.34 |
| PWAA (1) | | 0.0 | 1.0 | 11.3 | 17.1 | 40.3 | 29.6 | 0.7 | 356 | 0.32 |
| PWAA (2) | | 0.0 | 0.9 | 12.1 | 17.6 | 40.2 | 28.5 | 0.7 | 359 | 0.32 |
| PWAA (3) | | 0.0 | 10.9 | 19.4 | 20.1 | 29.6 | 19.5 | 0.5 | 426 | 0.35 |
| PWAA (4) | | 0.0 | 0.8 | 10.9 | 17.5 | 41.5 | 28.7 | 0.6 | 357 | 0.31 |
| PWAA (5) | | 0.0 | 1.1 | 11.4 | 17.2 | 39.8 | 29.8 | 0.7 | 356 | 0.32 |
| PWAA (6) | | 0.0 | 1.0 | 11.5 | 16.5 | 41.2 | 29.3 | 0.5 | 356 | 0.32 |
| PWAA (7) | | 0.0 | 1.5 | 13.2 | 18.1 | 39.3 | 27.5 | 0.4 | 366 | 0.32 |
| PWAA (8) | | 0.0 | 15.1 | 22.3 | 19.8 | 29.4 | 13.2 | 0.2 | 451 | 0.32 |
| PWAA (9) | | 0.0 | 8.8 | 27.7 | 20.4 | 28.2 | 14.5 | 0.4 | 449 | 0.31 |
| PWAA (10) | | 0.0 | 8.9 | 18.2 | 14.5 | 34.8 | 23.1 | 0.5 | 393 | 0.36 |
| PWAA (11-1) | | 0.0 | 0.8 | 13.5 | 16.7 | 39.2 | 29.3 | 0.5 | 359 | 0.33 |
| PWAA (11-2) | | 0.0 | 0.9 | 13.4 | 16.6 | 39.1 | 29.4 | 0.6 | 359 | 0.33 |
| PWAA (11-3) | | 0.0 | 0.8 | 13.6 | 16.5 | 39.0 | 29.5 | 0.6 | 358 | 0.33 |
| PWAA (11-4) | | 0.0 | 0.7 | 13.3 | 16.6 | 39.1 | 29.6 | 0.7 | 358 | 0.33 |
| PWAA (11-5) | | 0.0 | 1.0 | 13.8 | 17.4 | 40.0 | 27.5 | 0.3 | 365 | 0.32 |
| PWAA (11-6) | | 0.0 | 0.8 | 13.7 | 17.5 | 40.1 | 27.6 | 0.3 | 364 | 0.31 |
| PWAA (11-7) | | 0.0 | 1.0 | 13.9 | 17.3 | 40.0 | 27.5 | 0.3 | 365 | 0.32 |
| PWAA (11-8) | | 0.0 | 0.9 | 14.0 | 17.4 | 39.5 | 27.8 | 0.4 | 364 | 0.32 |

(continued)

| | Particle size distribution (PSD) | | | | | | | Mass average particle diameter (D50) (μm) | Logarithmic standard deviation (σζ) of particle size distribution |
|---|---|---|---|---|---|---|---|---|---|
| | Particles remaining on 850 μm sieve (wt%) | 850-600 (wt%) | 600-500 (wt%) | 500-425 (wt%) | 425-300 (wt%) | 300-150 (wt%) | Particles which passed through 150 μm sieve (wt%) | | |

Abbreviations in the above table include the following.
PWAA: Particulate water-absorbing agent; Comp. PWAA: Comparative particulate water-absorbing agent.

[Table 4-2]

| | Particle size distribution (PSD) | | | | | | | Mass average particle diameter (D50) (μm) | Logarithmic standard deviation (σζ) of particle size distribution |
|---|---|---|---|---|---|---|---|---|---|
| | Particles remaining on 850 μm sieve (wt%) | 850-600 (wt%) | 600-500 (wt%) | 500-425 (wt%) | 425-300 (wt%) | 300-150 (wt%) | Particles which passed through 150 μm sieve (wt%) | | |
| PWAA (21-1) | 0.0 | 0.9 | 13.5 | 16.7 | 39.2 | 29.3 | 0.4 | 360 | 0.32 |
| PWAA (21-2) | 0.0 | 1.0 | 13.5 | 16.6 | 39.0 | 29.4 | 0.5 | 359 | 0.33 |
| PWAA (21-3) | 0.0 | 0.8 | 13.4 | 16.7 | 39.0 | 29.6 | 0.5 | 358 | 0.33 |
| PWAA (21-4) | 0.0 | 0.8 | 13.3 | 16.7 | 39.1 | 29.4 | 0.7 | 358 | 0.33 |
| PWAA (22-1) | 0.0 | 2.1 | 14.7 | 18.6 | 37.8 | 26.6 | 0.2 | 373 | 0.32 |
| PWAA (22-2) | 0.0 | 2.0 | 14.9 | 18.9 | 37.0 | 26.9 | 0.3 | 373 | 0.32 |
| PWAA (22-3) | 0.0 | 2.3 | 14.6 | 18.5 | 37.1 | 27.1 | 0.4 | 372 | 0.33 |
| PWAA (22-4) | 0.0 | 2.3 | 15.1 | 18.7 | 36.2 | 27.1 | 0.6 | 373 | 0.33 |
| PWAA (23) | 0.0 | 1.0 | 11.3 | 17.1 | 40.3 | 29.6 | 0.7 | 356 | 0.32 |
| PWAA (24) | 0.0 | 1.1 | 11.2 | 17.2 | 40.2 | 29.7 | 0.6 | 356 | 0.32 |
| Comp. PWAA (1) | 0.0 | 1.0 | 11.0 | 17.3 | 40.5 | 29.4 | 0.8 | 356 | 0.32 |
| Comp. PWAA (2) | 0.0 | 1.1 | 11.2 | 17.2 | 39.9 | 29.7 | 0.9 | 355 | 0.33 |
| Comp. PWAA (3) | 0.0 | 10.9 | 19.4 | 20.1 | 29.6 | 19.5 | 0.5 | 426 | 0.35 |

(continued)

| | Particle size distribution (PSD) | | | | | | | Mass average particle diameter (D50) (μm) | Logarithmic standard deviation (σζ) of particle size distribution |
|---|---|---|---|---|---|---|---|---|---|
| | Particles remaining on 850 μm sieve (wt%) | 850-600 (wt%) | 600-500 (wt%) | 500-425 (wt%) | 425-300 (wt%) | 300-150 (wt%) | Particles which passed through 150 μm sieve (wt%) | | |
| Comp. PWAA (4) | 0.0 | 0.7 | 10.5 | 16.8 | 41.3 | 29.8 | 0.9 | 353 | 0.32 |
| Comp. PWAA (5) | 0.0 | 0.7 | 10.4 | 16.7 | 41.6 | 29.7 | 0.9 | 352 | 0.32 |
| Comp. PWAA (6) | 0.0 | 0.8 | 10.6 | 16.9 | 40.9 | 29.9 | 0.9 | 353 | 0.32 |
| Comp. PWAA (7) | 0.0 | 0.8 | 10.9 | 17.1 | 40.2 | 30.0 | 1.0 | 353 | 0.33 |
| Comp. PWAA (8) | 0.0 | 12.0 | 18.8 | 21.2 | 29.0 | 18.5 | 0.5 | 431 | 0.35 |
| Abbreviations in the above table include the following. PWAA: Particulate water-absorbing agent; Comp. PWAA: Comparative particulate water-absorbing agent. | | | | | | | | | |

[Table 5]

| | | DRC5min | | | | |
|---|---|---|---|---|---|---|
| | | 850-600 (g/g) | 600-500 (g/g) | 500-425 (g/g) | 425-300 (g/g) | 300-150 (g/g) |
| Prod. Ex. 1 | PWAA (1) | 35 | 34 | 39 | 44 | 49 |
| Prod. Ex. 2 | PWAA (2) | 31 | 32 | 35 | 41 | 47 |
| Prod. Ex. 3 | PWAA (3) | 31 | 32 | 35 | 41 | 47 |
| Prod. Ex. 4 | PWAA (4) | 35 | 36 | 38 | 42 | 46 |
| Prod. Ex. 5 | PWAA (5) | 33 | 35 | 36 | 41 | 44 |
| Prod. Ex. 6 | PWAA (6) | 38 | 37 | 43 | 48 | 53 |
| Prod. Ex. 7 | PWAA (7) | 37 | 36 | 41 | 47 | 52 |
| Prod. Ex. 8 | PWAA (8) | 38 | 37 | 43 | 48 | 53 |
| Prod. Ex. 9 | PWAA (9) | 37 | 36 | 41 | 47 | 52 |
| Prod. Ex. 10 | PWAA (10) | 37 | 38 | 42 | 47 | 48 |
| Prod. Ex. 23 | PWAA (23) | 34 | 34 | 39 | 44 | 48 |
| Comp. Prod. Ex. 1 | Comp. PWAA (1) | 21 | 25 | 28 | 33 | 40 |
| Comp. Prod. Ex. 2 | Comp. PWAA (2) | 21 | 25 | 29 | 33 | 38 |
| Comp. Prod. Ex. 3 | Comp. PWAA (3) | 21 | 25 | 28 | 33 | 40 |

(continued)

| | | DRC5min | | | | |
|---|---|---|---|---|---|---|
| | | 850-600 (g/g) | 600-500 (g/g) | 500-425 (g/g) | 425-300 (g/g) | 300-150 (g/g) |

Abbreviations in the above table include the following.
Prod. Ex.: Production Example; Comp. Prod. Ex.: Comparative Production Example; PWAA: Particulate water-absorbing agent; Comp. PWAA: Comparative particulate water-absorbing agent.

(Recap)

A comparison between Production Example 1 and Comparative Production Example 1 shows that according to an example of the production method of the present disclosure, a DRC index of 43 or less can be achieved by subjecting a hydrogel having a CRC of 33 g/g or more to gel-crushing under the condition that gel-grinding energy (GGE) is 20 J/g or more, or gel-grinding energy (2) (GGE(2)) is 9 J/g or more.

[Example 1]

First, 100 parts by mass of the particulate water-absorbing agent (2) as obtained in Production Example 2 was mixed uniformly with 20 parts by mass of ethylene-vinyl acetate copolymer (EVA; melting point: 95°C) as an adhesive, so that a mixture (1) was obtained.

Next, the mixture (1) was dispersed evenly onto a polypropylene nonwoven fabric (mass per unit area: 15 $g/m^2$; such a fabric hereinafter also referred to as "nonwoven fabric (1)") having a width of 30 cm. This produced a layered body (1) constituted by the nonwoven fabric, the particulate water-absorbing agent (2), and the adhesive. At this time, the mass per unit area of the mixture (1) (i.e., the amount of the mixture (1) dispersed per unit area of the nonwoven fabric) was 187 $g/m^2$.

Next, the layered body (1) obtained as above was placed on another polypropylene nonwoven fabric (1) so that the particulate water-absorbing agent (2) and the adhesive were sandwiched by nonwoven fabric. Thereafter, pressure was applied to the materials at 130°C, so that the materials bonded. Through these operations, a water-absorbing sheet (1) was obtained.

In order to evaluate the performance of the water-absorbing sheet (1) thus obtained, the water-absorbing sheet (1) was cut to a size of 8 cm by 16 cm and then subjected to an absorbent body evaluation (measurement of liquid absorption speed and re-wet). Table 6-1 shows the evaluation results.

[Example 2]

Operations were carried out similarly to Example 1, except that the mass per unit area of the mixture (1) (i.e., the amount of the mixture (1) dispersed per unit area of the nonwoven fabric) was changed to 300 $g/m^2$. This produced a water-absorbing sheet (2).

In order to evaluate the performance of the water-absorbing sheet (2) thus obtained, the water-absorbing sheet (2) was cut to a size of 8 cm by 16 cm and then subjected to an absorbent body evaluation (measurement of liquid absorption speed and re-wet). Table 6-1 shows the evaluation results.

[Example 3]

Operations were carried out similarly to Example 1, except that the particulate water-absorbing agent (23) was used. This produced a water-absorbing sheet (3).

In order to evaluate the performance of the water-absorbing sheet (3) thus obtained, the water-absorbing sheet (3) was cut to a size of 8 cm by 16 cm and then subjected to an absorbent body evaluation (measurement of liquid absorption speed and re-wet). Table 6-1 shows the evaluation results.

[Example 4]

In the operations of Example 2, the particulate water-absorbing agent (23) was used. Except for this, operations were carried out similarly to Example 1. This produced a water-absorbing sheet (4).

In order to evaluate the performance of the water-absorbing sheet (4) thus obtained, the water-absorbing sheet (4) was cut to a size of 8 cm by 16 cm and then subjected to an absorbent body evaluation (measurement of liquid absorption speed and re-wet). Table 6-1 shows the evaluation results.

[Comparative Example 1]

Operations were carried out similarly to Example 1, except that the comparative particulate water-absorbing agent (1) was used. This produced a comparative water-absorbing sheet (1). In order to evaluate the performance of the comparative water-absorbing sheet (1) thus obtained, the comparative water-absorbing sheet (1) was cut to a size of 8 cm by 16 cm and then subjected to an absorbent body evaluation (measurement of liquid absorption speed and re-wet). Table 6-1 shows the evaluation results.

[Example 5]

First, 100 parts by mass of the particulate water-absorbing agent (23) was mixed uniformly with 20 parts by mass of ethylene-vinyl acetate copolymer (EVA; melting point: 95°C) as an adhesive, so that a mixture (5) was obtained.

Next, the mixture (5) was dispersed evenly onto a polypropylene nonwoven fabric (mass per unit area: 15 g/m$^2$; such a fabric hereinafter also referred to as "nonwoven fabric (5)") having a width of 30 cm. This produced a layered body (5-1) constituted by the nonwoven fabric, the particulate water-absorbing agent (23), and the adhesive. At this time, the mass per unit area of the mixture (5) (i.e., the amount of the mixture (5) dispersed per unit area of the nonwoven fabric) was 94 g/m$^2$.

Next, the layered body (5-1) obtained as above was placed on another polypropylene nonwoven fabric (mass per unit area: 25 g/m$^2$; such a fabric hereinafter also referred to as "nonwoven fabric (2)") so that the particulate water-absorbing agent (23) and the adhesive were sandwiched by nonwoven fabric. Thereafter, pressure was applied to the materials at 130°C, so that the materials bonded. Through these operations, an intermediate sheet (5) was obtained.

Next, the mixture (1) as obtained in Example 1 was dispersed evenly onto a nonwoven fabric (1). This produced a layered body (5-2) constituted by the nonwoven fabric, the particulate water-absorbing agent (2), and the adhesive. At this time, the mass per unit area of the mixture (1) (i.e., the amount of the mixture (1) dispersed per unit area of the nonwoven fabric) was 94 g/m$^2$.

The intermediate sheet (5) was then placed onto the layered body (5-2) so that a surface of the nonwoven fabric (2) of the intermediate sheet (5) faced a surface of the layered body (5-2) on which the mixture (1) was provided. Pressure was then applied at 130°C, so that the intermediate sheet (5) and the layered body (5-2) were bonded.

These operations produced a water-absorbing sheet (5) in which: the particulate water-absorbing agent (23) was provided on an upper-layer side (side which comes in contact with liquid first) of the water-absorbing sheet (5); and the particulate water-absorbing agent (2) was provided on a lower-layer side of the water-absorbing sheet (5).

In order to evaluate the performance of the water-absorbing sheet (5) thus obtained, the water-absorbing sheet (5) was cut to a size of 8 cm by 16 cm and then subjected to an absorbent body evaluation (measurement of liquid absorption speed and re-wet). Table 6-2 shows the evaluation results.

[Example 6]

Operations were carried out similarly to Example 5, except that the particulate water-absorbing agent (2) was replaced with the particulate water-absorbing agent (23). This produced a water-absorbing sheet (6).

In the water-absorbing sheet (6), the particulate water-absorbing agent (23) was provided to both an upper-layer side (side which comes in contact with liquid first) and a lower-layer side of the water-absorbing sheet (6).

In order to evaluate the performance of the water-absorbing sheet (6) thus obtained, the water-absorbing sheet (6) was cut to a size of 8 cm by 16 cm and then subjected to an absorbent body evaluation (measurement of liquid absorption speed and re-wet). Table 6-2 shows the evaluation results.

[Example 7]

Operations were carried out similarly to Example 5, except that the particulate water-absorbing agent (23) was replaced with the particulate water-absorbing agent (2). This produced a water-absorbing sheet (7).

In the water-absorbing sheet (7), the particulate water-absorbing agent (2) was provided to both an upper-layer side (side which comes in contact with liquid first) and a lower-layer side of the water-absorbing sheet (7).

In order to evaluate the performance of the water-absorbing sheet (7) thus obtained, the water-absorbing sheet (7) was cut to a size of 8 cm by 16 cm and then subjected to an absorbent body evaluation (measurement of liquid absorption speed and re-wet). Table 6-2 shows the evaluation results.

[Comparative Example 2]

Operations were carried out similarly to Example 5, except that the particulate water-absorbing agent (2) and the particulate water-absorbing agent (23) were both replaced with the comparative particulate water-absorbing agent (1). This produced a comparative water-absorbing sheet (2). In order to evaluate the performance of the comparative water-absorbing sheet (2) thus obtained, the comparative water-absorbing sheet (2) was cut to a size of 8 cm by 16 cm and then subjected to an absorbent body evaluation (measurement of liquid absorption speed and re-wet). Table 6-2 shows the evaluation results.

[Example 8]

First, 2.10 g of the particulate water-absorbing agent (7) (corresponding to the second particulate water-absorbing agent) was dispersed uniformly (in an amount of 164 g/m$^2$) onto a surface of a polypropylene nonwoven fabric (2) (corresponding to the intermediate base material; mass per unit area: 50.6 g/m$^2$; has a degree thickness, the thickness being approximately 4 mm to 6 mm without load).
Next, 0.1 g to 0.2 g of an adhesive ("Spray Nori 77", manufactured by 3M Japan Limited) containing styrene butadiene rubber was dispersed (in an amount of 7.8 g/m$^2$ to 15.6 g/m$^2$) onto a surface of a pulp fiber nonwoven fabric (1) (corresponding to the second base material; mass per unit area: 42 g/m$^2$) which had been cut to a size of 8 cm by 16 cm.
Next, the polypropylene nonwoven fabric (2) was placed on the pulp fiber nonwoven fabric (1) such that the surface of the polypropylene nonwoven fabric (2) on which the particulate water-absorbing agent was dispersed faced (was in contact with) the surface of the pulp fiber nonwoven fabric (1) on which the adhesive was dispersed. Pressure was then applied so that the polypropylene nonwoven fabric (2) and the pulp fiber nonwoven fabric (1) were bonded.
Next, 0.700 g of the particulate water-absorbing agent (6) (corresponding to the first particulate water-absorbing agent) was dispersed uniformly (in an amount 55 g/m$^2$) onto the surface of the polypropylene nonwoven fabric (2) on which the particulate water-absorbing agent (7) was not provided.
Next, a pulp fiber nonwoven fabric (1) (corresponding to the first base material; mass per unit area: 15 g/m$^2$) onto which 0.1 g to 0.2 g (7.8 g/m$^2$ to 15.6 g/m$^2$) of an adhesive ("Spray Nori 77", manufactured by 3M Japan Limited) containing styrene butadiene rubber had been dispersed was placed on the polypropylene nonwoven fabric (2), such that the surface of the polypropylene nonwoven fabric (2) on which the particulate water-absorbing agent (6) was dispersed faced (was in contact with) the surface of the pulp fiber nonwoven fabric (1) on which the adhesive was dispersed. Pressure was then applied so that the polypropylene nonwoven fabric (2) and the pulp fiber nonwoven fabric (1) were bonded. In this way, a water-absorbing sheet (8) was obtained.
Note that the pulp fiber nonwoven fabrics (1) and the polypropylene nonwoven fabric (2) used in the present Example are water permeable.
The water-absorbing sheet (8) obtained as above was subjected to an absorbent body evaluation (measurement of liquid absorption speed and re-wet). Table 6-3 shows the evaluation results.

[Example 9]

Operations were carried out similarly to Example 8, except that the particulate water-absorbing agent (6) was used as the first particulate water-absorbing agent, and the particulate water-absorbing agent (3) was used as the second particulate water-absorbing agent. This produced a water-absorbing sheet (9). The water-absorbing sheet (9) thus obtained was subjected to an absorbent body evaluation (measurement of liquid absorption speed and re-wet). Table 6-3 shows the evaluation results.

[Example 10]

Operations were carried out similarly to Example 8, except that the particulate water-absorbing agent (2) was used as the first particulate water-absorbing agent, and the particulate water-absorbing agent (7) was used as the second particulate water-absorbing agent. This produced a water-absorbing sheet (10). The water-absorbing sheet (10) thus obtained was subjected to an absorbent body evaluation (measurement of liquid absorption speed and re-wet). Table 6-3 shows the evaluation results.

[Example 11]

Operations were carried out similarly to Example 8, except that the comparative particulate water-absorbing agent (1) was used as the first particulate water-absorbing agent, and the particulate water-absorbing agent (7) was used as the second particulate water-absorbing agent. This produced a water-absorbing sheet (11). The water-absorbing sheet (11) thus obtained was subjected to an absorbent body evaluation (measurement of liquid absorption speed and re-wet). Table 6-3 shows the evaluation results.

[Example 12]

Operations were carried out similarly to Example 8, except that the particulate water-absorbing agent (23) was used as the first particulate water-absorbing agent, and the particulate water-absorbing agent (3) was used as the second particulate water-absorbing agent. This produced a water-absorbing sheet (12). The water-absorbing sheet (12) thus obtained was subjected to an absorbent body evaluation (measurement of liquid absorption speed and re-wet). Table 6-3 shows the evaluation results.

[Example 13]

Operations were carried out similarly to Example 8, except that the comparative particulate water-absorbing agent (2) was used as the first particulate water-absorbing agent, and the particulate water-absorbing agent (3) was used as the second particulate water-absorbing agent. This produced a water-absorbing sheet (13). The water-absorbing sheet (13) thus obtained was subjected to an absorbent body evaluation (measurement of liquid absorption speed and re-wet). Table 6-3 shows the evaluation results.

[Example 14]

Operations were carried out similarly to Example 8, except that the particulate water-absorbing agent (23) was used as the first particulate water-absorbing agent, and the particulate water-absorbing agent (24) was used as the second particulate water-absorbing agent. This produced a water-absorbing sheet (14). The water-absorbing sheet (14) thus obtained was subjected to an absorbent body evaluation (measurement of liquid absorption speed and re-wet). Table 6-3 shows the evaluation results.

[Example 15]

Operations were carried out similarly to Example 8, except that the particulate water-absorbing agent (23) was used as the first particulate water-absorbing agent, and the particulate water-absorbing agent (2) was used as the second particulate water-absorbing agent. This produced a water-absorbing sheet (15). The water-absorbing sheet (15) thus obtained was subjected to an absorbent body evaluation (measurement of liquid absorption speed and re-wet). Table 6-3 shows the evaluation results.

[Comparative Example 3]

Operations were carried out similarly to Example 8, except that the comparative particulate water-absorbing agent (1) was used as both the first particulate water-absorbing agent and the second particulate water-absorbing agent. This produced a comparative water-absorbing sheet (3). The comparative water-absorbing sheet (3) thus obtained was subjected to an absorbent body evaluation (measurement of liquid absorption speed and re-wet). Table 6-3 shows the evaluation results.

[Curved surface evaluation 1, flat surface evaluation 2, curved surface evaluation 2, liquid flow evaluation]

The water-absorbing sheet (1) obtained in Example 1 and the water-absorbing sheet (3) obtained in Example 3 were each subjected to the curved surface evaluation 1, the flat surface evaluation 2, the curved surface evaluation 2, and the liquid flow evaluation. The results are shown in Table 8, Table 9, and Table 10.

[Table 6-1]

| | | 1st PWAA localized in vicinity of 1st base material | DRC index of 1st PWAA | 2nd PWAA localized in vicinity of 2nd base material | DRC index of 2nd PWAA | Total mass per unit area of PWAA [g/m²] | Is intermediate base material present? | Flat surface evaluation 1 | | | |
|---|---|---|---|---|---|---|---|---|---|---|---|
| | | | | | | | | Amount of liquid per round of introduction | t1 | t2 | Re-wet |
| | | | | | | | | [ml] | [sec] | [sec] | [g] |
| Ex. 1 | WAS (1) | PWAA (2) | 23.1 | PWAA (2) | 23.1 | 156 | No | 30 | 104 | 148 | 0.35 |
| Ex. 2 | WAS (2) | PWAA (2) | 23.1 | PWAA (2) | 23.1 | 250 | No | 40 | 99 | 98 | 0.16 |
| Ex. 3 | WAS (3) | PWAA (23) | 16.9 | PWAA (23) | 16.9 | 156 | No | 30 | 87 | 62 | 0.76 |
| Ex. 4 | WAS (4) | PWAA (23) | 16.9 | PWAA (23) | 16.9 | 250 | No | 40 | 60 | 45 | 0.26 |
| Comp. Ex. 1 | Comp. WAS (1) | Comp. PWAA (1) | 44.1 | Comp. PWAA (1) | 44.7 | 156 | No | 30 | 121 | 153 | 1.06 |

Abbreviations in the above table include the following.

Ex.: Example; Comp. Ex.: Comparative Example; WAS: Water-absorbing sheet; Comp. WAS: Comparative water-absorbing sheet; PWAA: Particulate water-absorbing agent; Comp. PWAA: Comparative particulate water-absorbing agent.

[Table 6-2]

| | | 1st PWAA localized in vicinity of 1st base material | DRC index of 1st PWAA | 2nd PWAA localized in vicinity of 2nd base material | DRC index of 2nd PWAA | Total mass per unit area of PWAA $[g/m^2]$ | Is intermediate base material present? | Flat surface evaluation 1 | | | |
|---|---|---|---|---|---|---|---|---|---|---|---|
| | | | | | | | | Amount of liquid per round of introduction | t1 | t2 | Re-wet |
| | | | | | | | | [ml] | [sec] | [sec] | [g] |
| Ex. 5 | WAS (5) | PWAA (23) | 17.0 | PWAA (2) | 23.4 | 157 | Yes | 30 | 46 | 47 | 0.40 |
| Ex. 6 | WAS (6) | PWAA (23) | 17.0 | PWAA (23) | 17.0 | 157 | Yes | 30 | 46 | 39 | 0.99 |
| Ex. 7 | WAS (7) | PWAA (2) | 23.4 | PWAA (2) | 23.4 | 157 | Yes | 30 | 51 | 78 | 0.29 |
| Comp. Ex. 2 | Comp. WAS (2) | Comp. PWAA (1) | 44.7 | Comp. PWAA (1) | 44.1 | 157 | Yes | 30 | 52 | 83 | 1.25 |

Abbreviations in the above table include the following.

Ex.: Example; Comp. Ex.: Comparative Example; WAS: Water-absorbing sheet; Comp. WAS: Comparative water-absorbing sheet; PWAA: Particulate water-absorbing agent; Comp. PWAA: Comparative particulate water-absorbing agent.

[Table 6-3]

| | | 1st PWAA localized in vicinity of 1st base material | DRC index of 1st PWAA | 2nd PWAA localized in vicinity of 2nd base material | DRC index of 2nd PWAA | Total mass per unit area of PWAA [g/m²] | Is intermediate base material present? | Flat surface evaluation 1 | | | |
|---|---|---|---|---|---|---|---|---|---|---|---|
| | | | | | | | | Amount of liquid per round of introduction | t1 | t2 | Re-wet |
| | | | | | | | | [ml] | [sec] | [sec] | [g] |
| Ex. 8 | WAS (8) | PWAA (6) | 5.3 | PWAA (7) | 9.6 | 219 | Yes | 30 | 17 | 15 | 1.53 |
| Ex. 9 | WAS (9) | PWAA (6) | 5.3 | PWAA (3) | 25.4 | 219 | Yes | 30 | 18 | 16 | 1.62 |
| Ex. 10 | WAS (10) | PWAA (2) | 23.1 | PWAA (7) | 9.6 | 219 | Yes | 30 | 18 | 19 | 1.33 |
| Ex. 11 | WAS (11) | Comp. PWAA (1) | 44.7 | PWAA (7) | 9.6 | 219 | Yes | 30 | 19 | 18 | 1.83 |
| Ex. 12 | WAS (12) | PWAA (23) | 16.9 | PWAA (3) | 25.4 | 219 | Yes | 30 | 19 | 16 | 1.89 |
| Ex. 13 | WAS (13) | Comp. PWAA (2) | 46.5 | PWAA (3) | 25.4 | 219 | Yes | 30 | 20 | 21 | 1.95 |
| Ex. 14 | WAS (14) | PWAA (23) | 16.9 | PWAA (24) | 23.3 | 219 | Yes | 30 | 21 | 18 | 1.96 |
| Ex. 15 | WAS (15) | PWAA (23) | 16.9 | PWAA (2) | 23.1 | 219 | Yes | 30 | 20 | 16 | 1.81 |
| Comp. Ex. 3 | Comp. WAS (3) | Comp. PWAA (1) | 44.7 | Comp. PWAA (1) | 44.7 | 219 | Yes | 30 | 20 | 28 | 3.10 |

Abbreviations in the above table include the following.

Ex.: Example; Comp. Ex.: Comparative Example; WAS: Water-absorbing sheet; Comp. WAS: Comparative water-absorbing sheet; PWAA: Particulate water-absorbing agent; Comp. PWAA: Comparative particulate water-absorbing agent.

[Table 7]

| | | Curved surface evaluation 1 | | | |
|---|---|---|---|---|---|
| | | Amount of liquid per round of introduction (ml) | t1 (Sec) | t2 (Sec) | Re-wet (g) |
| Example 1 | Water-absorbing sheet (1) | 30 | 101 | 81 | 0.37 |
| Example 3 | Water-absorbing sheet (3) | 30 | 99 | 66 | 1.32 |

[Table 8]

| | | Flat surface evaluation 2 | | |
|---|---|---|---|---|
| | | Amount of liquid introduced (ml) | t1 (Sec) | Re-wet (g) |
| Example 1 | Water-absorbing sheet (1) | 30 | 70 | 0.20 |
| Example 3 | Water-absorbing sheet (3) | 30 | 50 | 0.03 |

[Table 9]

| | | Curved surface evaluation 2 | | |
|---|---|---|---|---|
| | | Amount of liquid introduced (ml) | t1 (Sec) | Re-wet (g) |
| Example 1 | Water-absorbing sheet (1) | 30 | 39 | 0.03 |
| Example 3 | Water-absorbing sheet (3) | 30 | 38 | 0.01 |
| Example 1 | Water-absorbing sheet (1) | 40 | 56 | 1.41 |
| Example 3 | Water-absorbing sheet (3) | 40 | 56 | 1.54 |

[Table 10]

| | | Liquid flow evaluation | |
|---|---|---|---|
| | | Amount of liquid introduced (ml) | Absorption amount (g) |
| Example 1 | Water-absorbing sheet (1) | 15 | 9 |
| Example 3 | Water-absorbing sheet (3) | 15 | 11 |

(Production device)

As a device for producing a polyacrylic acid (salt)-based water-absorbing resin powder in the following Production Examples, there was prepared a continuous production device for carrying out a polymerization step, a gel-crushing step, a drying step, a pulverization step, a classification step, a surface-crosslinking step, a cooling step, a particle sizing step, and a transportation step for linking the above individual steps. The continuous production device had a production capacity of 3500 kg/hr. The above steps can each include a single line or two or more lines. In a case where the above steps include two or more lines, the production capacity is shown as the sum of the respective production amounts of the two or more lines. The continuous production device was used to continuously produce a polyacrylic acid (salt)-based water-absorbing resin powder.

[Production Example f]

First, there was prepared an aqueous monomer solution (6) containing 300 parts by weight of acrylic acid, 100 parts by weight of a 48 weight% aqueous sodium hydroxide solution, 0.78 parts by weight of polyethylene glycol diacrylate (average n number: 9), 16.4 parts by weight of a 0.1 weight% aqueous trisodium diethylenetriamine pentaacetate solution, 336.5 parts by weight of deionized water, and 2.2 parts by weight of liquid malic acid (DL-malic acid, a 50 weight% aqueous solution, manufactured by FUSO CHEMICAL CO., LTD., food additive grade).

Next, the aqueous monomer solution (6) whose temperature had been adjusted to 38°C was continuously fed by a metering pump, and then 150.6 parts by weight of a 48 weight% aqueous sodium hydroxide solution was further continuously line-mixed with the aqueous monomer solution (6). At this stage, the temperature of the aqueous monomer solution (6) was raised to 83°C due to heat of neutralization.

Furthermore, 14.6 parts by weight of a 4 weight% aqueous sodium persulfate solution was continuously line-mixed with the aqueous monomer solution (6), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (6) was obtained. The belt-shaped hydrogel (6) obtained was continuously cut at regular intervals in the width direction relative to the traveling direction of the polymerization belt so that the cut length was 300 mm. Thus, a hydrogel (6) was obtained. The hydrogel (6) had a CRC of 36.1 g/g and a resin solid content of 48.0 weight%.

[Production Example g]

First, there was prepared an aqueous monomer solution (7) containing 300 parts by weight of acrylic acid, 100 parts by weight of a 48 weight% aqueous sodium hydroxide solution, 0.87 parts by weight of polyethylene glycol diacrylate (average n number: 9), 16.4 parts by weight of a 0.1 weight% aqueous trisodium diethylenetriamine pentaacetate solution, 360.2 parts by weight of deionized water, and 1.8 parts by weight of liquid malic acid (powder, manufactured by FUSO CHEMICAL CO., LTD., food additive grade).

Next, the aqueous monomer solution (7) whose temperature had been adjusted to 38°C was continuously fed by a metering pump, and then 150.6 parts by weight of a 48 weight% aqueous sodium hydroxide solution was further continuously line-mixed with the aqueous monomer solution (7). At this stage, the temperature of the aqueous monomer solution (7) was raised to 81°C due to heat of neutralization.

Furthermore, 14.6 parts by weight of a 4 weight% aqueous sodium persulfate solution was continuously line-mixed with the aqueous monomer solution (6), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (7) was obtained. The belt-shaped hydrogel (7) obtained was continuously cut at regular intervals in the width direction relative to the traveling direction of the polymerization belt so that the cut length was 300 mm. Thus, a hydrogel (7) was obtained. The hydrogel (7) had a CRC of 36.8 g/g and a resin solid content of 47.0 weight%.

[Production Example h]

First, there was prepared an aqueous monomer solution (8) containing 300 parts by weight of acrylic acid, 100 parts by weight of a 48 weight% aqueous sodium hydroxide solution, 1.42 parts by weight of polyethylene glycol diacrylate (average n number: 9), 16.4 parts by weight of a 0.1 weight% aqueous trisodium diethylenetriamine pentaacetate solution, 273.2 parts by weight of deionized water.

Next, the aqueous monomer solution (8) whose temperature had been adjusted to 38°C was continuously fed by a metering pump, and then 150.6 parts by weight of a 48 weight% aqueous sodium hydroxide solution was further continuously line-mixed with the aqueous monomer solution (8). At this stage, the temperature of the aqueous monomer solution (8) was raised to 87°C due to heat of neutralization.

Furthermore, 14.6 parts by weight of a 4 weight% aqueous sodium persulfate solution was continuously line-mixed with the aqueous monomer solution (6), and then a resultant mixture was continuously fed into a continuous polymerization device, having a planar polymerization belt with dams at both ends, so that the fed mixture had a thickness of 10 mm. Thereafter, polymerization (polymerization time: 3 minutes) was continuously carried out, so that a belt-shaped hydrogel (8) was obtained. The belt-shaped hydrogel (8) obtained was continuously cut at regular intervals in the width direction relative to the traveling direction of the polymerization belt so that the cut length was 300 mm. Thus, a hydrogel (8) was obtained. The hydrogel (8) had a CRC of 32.1 g/g and a resin solid content of 53.2 weight%.

[Production Example 11-1]

With 100 parts by weight of the water-absorbing resin particles (2), which had been obtained in Production Example 2, a (covalently bonding) surface-crosslinking agent solution containing 0.025 parts by weight of ethyleneglycoldiglycidyl ether, 0.4 parts by weight of ethylene carbonate, 0.6 parts by weight of propylene glycol, and 3.0 parts by weight of deionized water was uniformly mixed. Then, a resultant mixture was heat-treated at 175°C for approximately 40 minutes. In this case, the mixture was heat-treated so that resultant water-absorbing resin powder (11-1) would have a CRC of 38 g/g to 40 g/g. Thereafter, the mixture was cooled, water-absorbing resin particles obtained through the above operations were subjected to the paint shaker test (described earlier), and damage equivalent to that caused during a production process was caused to the water-absorbing resin particles. Then, with 100 parts by weight of the water-absorbing resin particles, an aqueous solution containing 1 part by weight of water and 0.01 parts by weight of trisodium diethylenetriamine pentaacetate was uniformly mixed. A resultant mixture was dried at 60°C for 1 hour and then passed through a JIS standard sieve having a mesh size of 710 $\mu$m. With the mixture, 0.3 parts by weight of hydrotalcite (product name: DHT-6, manufactured by Kyowa Chemical Industry Co., Ltd., $Mg_6Al_2(OH)_{16}CO_3 \cdot 4H_2O$ [x=0.25 and m=0.50 in the general formula], having a volume average particle diameter of 0.5 $\mu$m) was uniformly mixed. Thus, a particulate water-absorbing agent (11-1) was obtained. Tables 3, 4, and 13 show physical properties of the particulate water-absorbing agent (11-1).

Note that an amount of an increase in 150 $\mu$m passing particles, the amount being obtained after the paint shaker test, means an amount of an increase in 150 $\mu$m passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper. The hydrotalcite was contained in the particulate water-absorbing agent (11-1) in an amount, obtained by XRD measurement, of 0.3 weight%. Furthermore, the hydrotalcite which was present on a surface of the particulate water-absorbing agent (11-1) had an average particle diameter, obtained by particle size measurement, of 0.5 $\mu$m.

Note that Tables 3, 4, and 11 through 13 show not only physical properties of the particulate water-absorbing agents of Production Examples 1 through 11 but also physical properties of, for example, the comparative particulate water-absorbing agents of Comparative Production Examples 1 through 3.

[Production Example 11-2]

Operations similar to those carried out in Production Example 11-1 were carried out except that 0.3 parts by weight of hydrotalcite (product name: HT-1-NC, manufactured by SAKAI CHEMICAL INDUSTRY CO., LTD., chemical formula $Mg_4Al_2(OH)_{12}CO_3 \cdot 3H_2O$ [x=0.33 and m=0.5 in the general formula], having a volume average particle diameter of 0.58 $\mu$m) instead of the hydrotalcite (product name: DHT-6) of Production Example 11-1 was mixed. Thus, a particulate water-absorbing agent (11-2) was obtained. Tables 3, 4, and 13 show physical properties of the particulate water-absorbing agent (11-2). The hydrotalcite was contained in the particulate water-absorbing agent (11-2) in an amount, obtained by XRD measurement, of 0.3 weight%. Furthermore, the hydrotalcite which was present on a surface of the particulate water-absorbing agent (11-2) had an average particle diameter, obtained by particle size measurement, of 0.58 $\mu$m.

[Production Example 11-3]

Operations similar to those carried out in Production Example 11-1 were carried out except that 0.3 parts by weight of hydrotalcite (product name: HT-P, manufactured by SAKAI CHEMICAL INDUSTRY CO., LTD., chemical formula $Mg_{4.5}Al_2(OH)_{13}CO_3 \cdot 3.5H_2O$ [x=0.69 and m=0.54 in the general formula], having a volume average particle diameter of 0.45 $\mu$m) instead of the hydrotalcite (product name: DHT-6) of Production Example 11-1 was mixed. Thus, a particulate water-absorbing agent (11-3) was obtained. Tables 3, 4, and 13 show physical properties of the particulate water-absorbing agent (11-3). The hydrotalcite was contained in the particulate water-absorbing agent (11-3) in an amount, obtained by XRD measurement, of 0.3 weight%. Furthermore, the hydrotalcite which was present on a surface of the particulate water-absorbing agent (11-3) had an average particle diameter, obtained by particle size measurement, of 0.45 $\mu$m.

[Production Example 11-4]

Operations similar to those carried out in Production Example 11-1 were carried out except that 0.5 parts by weight of tricalcium phosphate (manufactured by Wako Pure Chemical Industries, Ltd., CAS No. 7758-87-4) instead of the hydrotalcite (product name: DHT-6) of Production Example 11-1 was mixed. Thus, a particulate water-absorbing agent (11-4) was obtained. Tables 3, 4, and 13 show physical properties of the particulate water-absorbing agent (11-4). Furthermore, the tricalcium phosphate which was present on a surface of the particulate water-absorbing agent (11-4) had a crystallite diameter, obtained by particle size measurement, of 0.04 $\mu$m, and an average primary particle diameter of 0.04 $\mu$m.

[Production Example 11-5]

Conditions under which to carry out a surface treatment in Production Example 11-1 were changed as below.

With 100 parts by weight of the water-absorbing resin particles (2), a (covalently bonding) surface-crosslinking agent solution containing 0.030 parts by weight of ethyleneglycoldiglycidyl ether, 1.0 part by weight of propylene glycol, and 3.0 parts by weight of deionized water was uniformly mixed. Then, a resultant mixture was heat-treated at 100°C for approximately 45 minutes. In this case, the mixture was heat-treated so that resultant water-absorbing resin powder (11-5) would have a CRC of 35 g/g to 36 g/g. Thereafter, the mixture was cooled, water-absorbing resin particles obtained through the above operations were subjected to the paint shaker test (described earlier), and damage equivalent to that caused during a production process was caused to the water-absorbing resin particles. Then, with 100 parts by weight of the water-absorbing resin particles, an aqueous solution containing 1 part by weight of water and 0.01 parts by weight of trisodium diethylenetriamine pentaacetate was uniformly mixed. A resultant mixture was dried at 60°C for 1 hour and then passed through a JIS standard sieve having a mesh size of 710 $\mu$m. With the mixture, 0.3 parts by weight of hydrotalcite (product name: DHT-6, manufactured by Kyowa Chemical Industry Co., Ltd., $Mg_6Al_2(OH)_{16}CO_3 \cdot 4H_2O$ [x=0.25 and m=0.50 in the general formula], having a volume average particle diameter of 0.5 $\mu$m) was uniformly mixed. Thus, a particulate water-absorbing agent (11-5) was obtained. Tables 3, 4, and 13 show physical properties of the particulate water-absorbing agent (11-5). Note that an amount of an increase in 150 $\mu$m passing particles, the amount being obtained after the paint shaker test, means an amount of an increase in 150 $\mu$m passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper.

[Production Example 11-6]

Operations similar to those carried out in Production Example 11-5 were carried out except that 0.3 parts by weight of hydrotalcite (product name: HT-1-NC, manufactured by SAKAI CHEMICAL INDUSTRY CO., LTD., chemical formula $Mg_4Al_2(OH)_{12}CO_3 \cdot 3H_2O$ [x=0.33 and m=0.5 in the general formula], having a volume average particle diameter of 0.58 $\mu$m) instead of the hydrotalcite (product name: DHT-6) of Production Example 11-5 was mixed. Thus, a particulate water-absorbing agent (11-6) was obtained. Tables 3, 4, and 13 show physical properties of the particulate water-absorbing agent (11-6).

[Production Example 11-7]

Operations similar to those carried out in Production Example 11-5 were carried out except that 0.3 parts by weight of hydrotalcite (product name: HT-P, manufactured by SAKAI CHEMICAL INDUSTRY CO., LTD., chemical formula $Mg_{4.5}Al_2(OH)_{13}CO_3 \cdot 3.5H_2O$ [x=0.69 and m=0.54 in the general formula], having a volume average particle diameter of 0.45 $\mu$m) instead of the hydrotalcite (product name: DHT-6) of Production Example 11-5 was mixed. Thus, a particulate water-absorbing agent (11-7) was obtained. Tables 3, 4, and 13 show physical properties of the particulate water-absorbing agent (11-7).

[Production Example 11-8]

Operations similar to those carried out in Production Example 11-5 were carried out except that 0.5 parts by weight of tricalcium phosphate (manufactured by Wako Pure Chemical Industries, Ltd., CAS No.7758-87-4) instead of the hydrotalcite (product name: DHT-6) of Production Example 11-5 was mixed. Thus, a particulate water-absorbing agent (11-8) was obtained. Tables 3, 4, and 13 show physical properties of the particulate water-absorbing agent (11-8).

[Production Example 12-1]

Surface treatment and additive addition operations similar to those carried out in Production Example 11-1 were carried out with respect to 100 parts by weight of the water-absorbing resin particles (7), which had been obtained in Production Example 7. Thus, a particulate water-absorbing agent (12-1) was obtained. Tables 3, 4, and 13 show physical properties of the particulate water-absorbing agent (12-1).

[Production Example 12-2]

Operations similar to those carried out in Production Example 12-1 were carried out except that 0.3 parts by weight of hydrotalcite (product name: HT-1-NC, manufactured by SAKAI CHEMICAL INDUSTRY CO., LTD., chemical formula $Mg_4Al_2(OH)_{12}CO_3 \cdot 3H_2O$ [x=0.33 and m=0.5 in the general formula], having a volume average particle diameter of 0.58 $\mu$m) instead of the hydrotalcite (product name: DHT-6) of Production Example 12-1 was mixed. Thus, a particulate water-absorbing agent (12-2) was obtained. Tables 3, 4, and 13 show physical properties of the particulate water-absorbing agent (12-2).

[Production Example 12-3]

Operations similar to those carried out in Production Example 12-1 were carried out except that 0.3 parts by weight of hydrotalcite (product name: HT-P, manufactured by SAKAI CHEMICAL INDUSTRY CO., LTD., chemical formula $Mg_{4.5}Al_2(OH)_{13}CO_3 \cdot 3.5H_2O$ [x=0.69 and m=0.54 in the general formula], having a volume average particle diameter of 0.45 $\mu$m) instead of the hydrotalcite (product name: DHT-6) of Production Example 12-1 was mixed. Thus, a particulate water-absorbing agent (12-3) was obtained. Tables 3, 4, and 13 show physical properties of the particulate water-absorbing agent (12-3).

[Production Example 12-4]

Operations similar to those carried out in Production Example 12-1 were carried out except that 0.5 parts by weight of tricalcium phosphate (manufactured by Wako Pure Chemical Industries, Ltd., CAS No.7758-87-4) instead of the hydrotalcite (product name: DHT-6) of Production Example 12-1 was mixed. Thus, a particulate water-absorbing agent (12-4) was obtained. Tables 3, 4, and 13 show physical properties of the particulate water-absorbing agent (12-4).

[Production Example 12-5]

Conditions under which to carry out a surface treatment in Production Example 12-1 were changed as below.

With 100 parts by weight of the water-absorbing resin particles (7), a (covalently bonding) surface-crosslinking agent solution containing 0.030 parts by weight of ethyleneglycoldiglycidyl ether, 1.0 part by weight of propylene glycol, and 3.0 parts by weight of deionized water was uniformly mixed. Then, a resultant mixture was heat-treated at 100°C for approximately 45 minutes. In this case, the mixture was heat-treated so that resultant water-absorbing resin powder (12-5) would have a CRC of 35 g/g to 36 g/g. Thereafter, the mixture was cooled, water-absorbing resin particles obtained through the above operations were subjected to the paint shaker test (described earlier), and damage equivalent to that caused during a production process was caused to the water-absorbing resin particles. Then, with 100 parts by weight of the water-absorbing resin particles, an aqueous solution containing 1 part by weight of water and 0.01 parts by weight of trisodium diethylenetriamine pentaacetate was uniformly mixed. A resultant mixture was dried at 60°C for 1 hour and then passed through a JIS standard sieve having a mesh size of 710 $\mu$m. With the mixture, 0.3 parts by weight of hydrotalcite (product name: DHT-6, manufactured by Kyowa Chemical Industry Co., Ltd., $Mg_6Al_2(OH)_{16}CO_3 \cdot 4H_2O$ [x=0.25 and m=0.50 in the general formula], having a volume average particle diameter of 0.5 $\mu$m) was uniformly mixed. Thus, a particulate water-absorbing agent (12-5) was obtained. Tables 3, 4, and 13 show physical properties of the particulate water-absorbing agent (12-5). Note that an amount of an increase in 150 $\mu$m passing particles, the amount being obtained after the paint shaker test, means an amount of an increase in 150 $\mu$m passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper.

[Production Example 12-6]

Operations similar to those carried out in Production Example 12-5 were carried out except that 0.3 parts by weight of hydrotalcite (product name: HT-1-NC, manufactured by SAKAI CHEMICAL INDUSTRY CO., LTD., chemical formula $Mg_4Al_2(OH)_{12}CO_3 \cdot 3H_2O$ [x=0.33 and m=0.5 in the general formula], having a volume average particle diameter of 0.58 $\mu$m) instead of the hydrotalcite (product name: DHT-6) of Production Example 12-5 was mixed. Thus, a particulate water-absorbing agent (12-6) was obtained. Tables 3, 4, and 13 show physical properties of the particulate water-absorbing agent (12-6).

[Production Example 12-7]

Operations similar to those carried out in Production Example 12-5 were carried out except that 0.3 parts by weight of hydrotalcite (product name: HT-P, manufactured by SAKAI CHEMICAL INDUSTRY CO., LTD., chemical formula $Mg_{4.5}Al_2(OH)_{13}CO_3 \cdot 3.5H_2O$ [x=0.69 and m=0.54 in the general formula], having a volume average particle diameter of 0.45 $\mu$m) instead of the hydrotalcite (product name: DHT-6) of Production Example 12-5 was mixed. Thus, a particulate water-absorbing agent (12-7) was obtained. Tables 3, 4, and 13 show physical properties of the particulate water-absorbing agent (12-7).

[Production Example 12-8]

Operations similar to those carried out in Production Example 12-5 were carried out except that 0.5 parts by weight of tricalcium phosphate (manufactured by Wako Pure Chemical Industries, Ltd., CAS No.7758-87-4) instead of the hydrotalcite (product name: DHT-6) of Production Example 12-5 was mixed. Thus, a particulate water-absorbing agent (12-8) was obtained. Tables 3, 4, and 13 show physical properties of the particulate water-absorbing agent (12-8).

[Production Example 13-1]

Surface treatment and additive addition operations similar to those carried out in Production Example 11-4 were carried out with respect to 100 parts by weight of the water-absorbing resin particles (1), which had been obtained in Production Example 1. Thus, a particulate water-absorbing agent (13-1) was obtained. Tables 3, 4, and 13 show physical properties of the particulate water-absorbing agent (13-1).

[Production Example 13-2]

Conditions under which to carry out a surface treatment in Production Example 13-1 were changed as below.
Surface treatment and additive addition operations similar to those carried out in Production Example 11-5 were carried out with respect to 100 parts by weight of the water-absorbing resin particles (1). Thus, a particulate water-absorbing agent (13-2) was obtained. Tables 3, 4, and 13 show physical properties of the particulate water-absorbing agent (13-2).

[Production Example 14]

Surface treatment and additive addition operations similar to those carried out in Production Example 11-5 were carried out with respect to 100 parts by weight of the water-absorbing resin particles (3), which had been obtained in Production Example 3. Thus, a particulate water-absorbing agent (14) was obtained. Tables 3, 4, and 13 show physical properties of the particulate water-absorbing agent (14).

[Production Example 15]

Gel-crushing, drying, pulverization, and classification operations similar to those carried out in Production Example 11-1 were carried out except that the sieve having a mesh size of 710 $\mu$m and used in Production Example 11-1 was replaced with a sieve having a 750 $\mu$m. Water-absorbing resin particles (15) thus obtained had a weight average particle diameter (D50) of 385 $\mu$m, a logarithmic standard deviation ($\sigma\zeta$) of a particle size distribution of 0.35, a CRC of 48.3 g/g, and a proportion of 150 $\mu$m passing particles (a proportion of particles passing through a sieve having a mesh size of 150 $\mu$m) of 0.3 weight%.
Next, surface treatment and additive addition operations similar to those carried out in Production Example 11-6 were carried out with respect to 100 parts by weight of the water-absorbing resin particles (15). Thus, a particulate water-absorbing agent (15) was obtained. Tables 3, 4, and 13 show physical properties of the particulate water-absorbing agent (15).

[Production Example 16]

Gel-crushing, drying, pulverization, and classification operations similar to those carried out in Production Example 13-1 were carried out except that the sieve having a mesh size of 710 $\mu$m and used in Production Example 13-1 was replaced with a sieve having a mesh size of 850 $\mu$m. Water-absorbing resin particles (16) thus obtained had a weight average particle diameter (D50) of 428 $\mu$m, a logarithmic standard deviation ($\sigma\zeta$) of a particle size distribution of 0.35, a CRC of 42.8 g/g, and a proportion of 150 $\mu$m passing particles (a proportion of particles passing through a sieve having a mesh size of 150 $\mu$m) of 0.3 weight%.
Next, surface treatment and additive addition operations similar to those carried out in Production Example 11-7 were carried out with respect to 100 parts by weight of the water-absorbing resin particles (16). Thus, a particulate water-absorbing agent (16) was obtained. Tables 3, 4, and 13 show physical properties of the particulate water-absorbing agent (16).

[Production Example 17]

Gel-crushing, drying, pulverization, and classification operations similar to those carried out in Production Example 13-1 were carried out except that the sieve having a mesh size of 710 μm and used in Production Example 13-1 was replaced with a sieve having a 750 μm. Water-absorbing resin particles (17) thus obtained had a weight average particle diameter (D50) of 386 μm, a logarithmic standard deviation (σζ) of a particle size distribution of 0.35, a CRC of 42.6 g/g, and a proportion of 150 μm passing particles (a proportion of particles passing through a sieve having a mesh size of 150 μm) of 0.3 weight%.

Next, surface treatment and additive addition operations similar to those carried out in Production Example 11-8 were carried out with respect to 100 parts by weight of the water-absorbing resin particles (17). Thus, a particulate water-absorbing agent (17) was obtained. Tables 3, 4, and 13 show physical properties of the particulate water-absorbing agent (17).

[Production Example 18]

(Gel-crushing)

The hydrogel (6), which had been obtained in Production Example 6, was fed to a screw extruder so as to be subjected to gel-crushing. As the screw extruder, a meat chopper including a porous plate and a screw shaft was used. The porous plate was provided at a tip of the meat chopper and had a diameter of 100 mm, a pore diameter of 8 mm, 54 pores, and a thickness of 10 mm, and the screw shaft had an outer diameter of 86 mm. While the screw shaft of the meat chopper was being rotated at 130 rpm, the hydrogel (6) was fed at 4640 g per minute, and at the same time, water vapor was fed at 83 g per minute. In this case, gel-grinding energy (GGE) was 32.3 J/g, and GGE (2) was 17.8 J/g. The hydrogel (6) which had not been subjected to the gel-crushing had a temperature of 80°C, and the temperature was raised to 84°C in a crushed gel obtained after the gel-crushing, i.e., a particulate hydrogel (18).

The particulate hydrogel (18) obtained through the above gel-crushing step had a resin solid content of 47.5 weight%, a weight average particle diameter (D50) of 820 μm, and a logarithmic standard deviation (σζ) of a particle size distribution of 0.94. Table 11 shows conditions under which to carry out the gel-crushing step, and Table 12 shows physical properties of the particulate hydrogel (18).

(Drying)

Next, the particulate hydrogel (18) was dispersed onto a through-flow plate within 1 minute of the end of the gel-crushing (at this stage, the particulate hydrogel (18) had a temperature of 80°C), and dried at 185°C for 30 minutes, so that a dried polymer (18) was obtained. Hot air had an average air velocity of 1.0 m/s in the direction perpendicular to the traveling direction of the through-flow belt. The air velocity of the hot air was measured with use of Anemomaster 6162, which is a constant temperature thermal anemometer manufactured by Kanomax Japan Inc.

(Pulverization and classification)

Subsequently, the total amount of a dried polymer (18) obtained through the above drying step was fed to a three-stage roll mill so as to be pulverized (subjected to a pulverizing step). Thereafter, the dried polymer thus pulverized was further classified with use of JIS standard sieves having respective mesh sizes of 710 μm and 175 μm. Thus, water-absorbing resin particles (18) having a non-uniformly pulverized shape were obtained. The water-absorbing resin particles (18) had a weight average particle diameter (D50) of 356 μm, a logarithmic standard deviation (σζ) of a particle size distribution of 0.32, a CRC of 48.3 g/g, and a proportion of 150 μm passing particles (a proportion of particles passing through a sieve having a mesh size of 150 μm) of 0.4 weight%.

(Surface treatment and additive addition)

Next, with 100 parts by weight of the water-absorbing resin particles (18), a (covalently bonding) surface-crosslinking agent solution containing 0.025 parts by weight of ethyleneglycoldiglycidyl ether, 0.4 parts by weight of ethylene carbonate, 0.6 parts by weight of propylene glycol, and 3.0 parts by weight of deionized water was uniformly mixed. Then, a resultant mixture was heat-treated at 190°C for approximately 30 minutes so that resultant water-absorbing resin powder (18) would have a CRC of 38 g/g to 39 g/g. Thereafter, the mixture was cooled, water-absorbing resin particles obtained through the above operations were subjected to the paint shaker test (described earlier), and damage equivalent to that caused during a production process was caused to the water-absorbing resin particles. Then, with 100 parts by weight of the water-absorbing resin particles, an aqueous solution containing 1 part by weight of water and 0.01 parts by weight of trisodium diethylenetriamine pentaacetate was uniformly mixed. A resultant mixture was dried at 60°C for 1 hour and then passed through a JIS standard sieve having a mesh size of 710 μm. To the mixture, 0.4 parts by weight of silicon dioxide (product name: Aerosil 200, manufactured by Nippon Aerosil Co., Ltd.) was uniformly added. Thus, a particulate water-absorbing agent (18) was obtained. Tables 3, 4, and 13 show physical properties of the particulate water-absorbing agent (18). Note that an amount of an increase in 150 $\mu$m passing particles, the amount being obtained after the paint shaker test, means an amount of an increase in 150 $\mu$m passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper.

[Production Example 19]

The total amount of the dried polymer (18), which had been obtained in Production Example 18, was fed to a three-stage roll mill so as to be pulverized (subjected to a pulverizing step), and thereafter was further classified with use of sieves having respective mesh sizes of 850 $\mu$m and 256 $\mu$m. Thus, water-absorbing resin particles (19) having a non-uniformly pulverized shape were obtained. The water-absorbing resin particles (19) had a weight average particle diameter (D50) of 447 $\mu$m, a logarithmic standard deviation ($\sigma\zeta$) of a particle size distribution of 0.29, a CRC of 48.8 g/g, and a proportion of 150 $\mu$m passing particles (a proportion of particles passing through a sieve having a mesh size of 150 $\mu$m) of 0.2 weight%.

Next, operations similar to those carried out with respect to the water-absorbing resin particles (18) of Production Example 18 were carried out with respect to the water-absorbing resin particles (19). Thus, a particulate water-absorbing agent (19) was obtained. Tables 3, 4, and 13 show physical properties of the particulate water-absorbing agent (19). Note that an amount of an increase in 150 $\mu$m passing particles, the amount being obtained after the paint shaker test, means an amount of an increase in 150 $\mu$m passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper.

[Production Example 20]

Operations similar to those carried out in Production Example 7 were carried out with use of the hydrogel (7), which had been obtained in Production Example g. In this case, gel-grinding energy (GGE) was 35.2 J/g, and GGE (2) was 20.1 J/g. The hydrogel (7) which had not been subjected to the gel-crushing had a temperature of 80°C, and the temperature was raised to 86°C in a crushed gel obtained after the gel-crushing, i.e., a particulate hydrogel (20).

The particulate hydrogel (20) obtained through the above gel-crushing step had a resin solid content of 46.6 weight%, a weight average particle diameter (D50) of 601 $\mu$m, and a logarithmic standard deviation ($\sigma\zeta$) of a particle size distribution of 0.97. Table 11 shows conditions under which to carry out the gel-crushing step, and Table 12 shows physical properties of the particulate hydrogel (20).

Water-absorbing resin particles (20) obtained by drying, pulverizing, and classifying the particulate hydrogel (20) had a weight average particle diameter (D50) of 360 $\mu$m, a logarithmic standard deviation ($\sigma\zeta$) of a particle size distribution of 0.32, a CRC of 49.6 g/g, and a proportion of 150 $\mu$m passing particles (a proportion of particles passing through a sieve having a mesh size of 150 $\mu$m) of 0.3 weight%.

Surface treatment and additive addition similar to those carried out in Production Example 7 were carried out with respect to the water-absorbing resin particles (20), so that a particulate water-absorbing agent (20) was obtained.

Tables 3, 4, and 13 show physical properties of the particulate water-absorbing agent (20). Note that an amount of an increase in 150 $\mu$m passing particles, the amount being obtained after the paint shaker test, means an amount of an increase in 150 $\mu$m passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper.

[Production Example 21-1]

With 100 parts by weight of the water-absorbing resin particles (18), which had been obtained in Production Example 18, a (covalently bonding) surface-crosslinking agent solution containing 0.025 parts by weight of ethyleneglycoldiglycidyl ether, 0.4 parts by weight of ethylene carbonate, 0.6 parts by weight of propylene glycol, and 3.0 parts by weight of deionized water was uniformly mixed. Then, a resultant mixture was heat-treated at 175°C for approximately 40 minutes. In this case, the mixture was heat-treated so that resultant water-absorbing resin powder (21) would have a CRC of 38 g/g to 39 g/g. Thereafter, the mixture was cooled, water-absorbing resin particles obtained through the above operations were subjected to the paint shaker test (described earlier), and damage equivalent to that caused during a production process was caused to the water-absorbing resin particles. Then, with 100 parts by weight of the water-absorbing resin particles, an aqueous solution containing 1 part by weight of water and 0.01 parts by weight of trisodium diethylenetriamine pentaacetate was uniformly mixed. A resultant mixture was dried at 60°C for 1 hour and then passed through a JIS standard sieve having a mesh size of 710 μm. With the mixture, 0.3 parts by weight of hydrotalcite (product name: DHT-6, manufactured by Kyowa Chemical Industry Co., Ltd., $Mg_6Al_2(OH)_{16}CO_3 \cdot 4H_2O$ [x=0.25 and m=0.50 in the general formula], having a volume average particle diameter of 0.5 μm) was uniformly mixed. Thus, a particulate water-absorbing agent (21-1) was obtained. Tables 3, 4, and 13 show physical properties of the particulate water-absorbing agent (21-1).

Note that an amount of an increase in 150 μm passing particles, the amount being obtained after the paint shaker test, means an amount of an increase in 150 μm passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper. The hydrotalcite was contained in the particulate water-absorbing agent (21-1) in an amount, obtained by XRD measurement, of 0.3 weight%. Furthermore, the hydrotalcite which was present on a surface of the particulate water-absorbing agent (21-1) had an average particle diameter, obtained by particle size measurement, of 0.5 μm.

[Production Example 21-2]

Operations similar to those carried out in Production Example 21-1 were carried out except that 0.3 parts by weight of hydrotalcite (product name: HT-1-NC, manufactured by SAKAI CHEMICAL INDUSTRY CO., LTD., chemical formula $Mg_4Al_2(OH)_{12}CO_3 \cdot 3H_2O$ [x=0.33 and m=0.5 in the general formula], having a volume average particle diameter of 0.58 μm) instead of the hydrotalcite (product name: DHT-6) of Production Example 21-1 was mixed. Thus, a particulate water-absorbing agent (21-2) was obtained. Tables 3, 4, and 13 show physical properties of the particulate water-absorbing agent (21-2). The hydrotalcite was contained in the particulate water-absorbing agent (21-2) in an amount, obtained by XRD measurement, of 0.3 weight%. Furthermore, the hydrotalcite which was present on a surface of the particulate water-absorbing agent (21-2) had an average particle diameter, obtained by particle size measurement, of 0.58 μm.

[Production Example 21-3]

Operations similar to those carried out in Production Example 21-1 were carried out except that 0.3 parts by weight of hydrotalcite (product name: HT-P, manufactured by SAKAI CHEMICAL INDUSTRY CO., LTD., chemical formula $Mg_{4.5}Al_2(OH)_{13}CO_3 \cdot 3.5H_2O$ [x=0.69 and m=0.54 in the general formula], having a volume average particle diameter of 0.45 μm) instead of the hydrotalcite (product name: DHT-6) of Production Example 21-1 was mixed. Thus, a particulate water-absorbing agent (21-3) was obtained. Tables 3, 4, and 13 show physical properties of the particulate water-absorbing agent (21-3). The hydrotalcite was contained in the particulate water-absorbing agent (21-3) in an amount, obtained by XRD measurement, of 0.3 weight%. Furthermore, the hydrotalcite which was present on a surface of the particulate water-absorbing agent (21-3) had an average particle diameter, obtained by particle size measurement, of 0.45 μm.

[Production Example 21-4]

Operations similar to those carried out in Production Example 21-1 were carried out except that 0.5 parts by weight of tricalcium phosphate (manufactured by Wako Pure Chemical Industries, Ltd., CAS No.7758-87-4) instead of the hydrotalcite (product name: DHT-6) of Production Example 21-1 was mixed. Thus, a particulate water-absorbing agent (21-4) was obtained. Tables 3, 4, and 13 show physical properties of the particulate water-absorbing agent (21-4). Furthermore, the tricalcium phosphate which was present on a surface of the particulate water-absorbing agent (21-4) had a crystallite diameter, obtained by particle size measurement, of 0.04 μm, and an average primary particle diameter of 0.04 μm.

[Production Example 22-1]

Operations similar to those carried out in Production Example 21-1 were carried out by changing water-absorbing resin particles and conditions under which to carry out a surface treatment. Specifically, the water-absorbing resin particles (18) were replaced with the water-absorbing resin particles (20), which had been obtained in Production Example 20. Furthermore, the conditions under which to carry out a surface treatment were changed as below. With 100 parts by weight of the water-absorbing resin particles (20), a (covalently bonding) surface-crosslinking agent solution containing 0.030 parts by weight of ethyleneglycoldiglycidyl ether, 1.0 part by weight of propylene glycol, and 3.0 parts by weight of deionized water was uniformly mixed. Then, a resultant mixture was heat-treated at 100°C for approximately 45 minutes. In this case, the mixture was heat-treated so that resultant water-absorbing resin powder (22) would have a CRC of 35 g/g to 36 g/g. Thereafter, the mixture was cooled, water-absorbing resin particles obtained through the above operations were subjected to the paint shaker test (described earlier), and damage equivalent to that caused during a production process was caused to the water-absorbing resin particles. Then, with 100 parts by weight of the water-absorbing resin particles, an aqueous solution containing 1 part by weight of water and 0.01 parts by weight of trisodium diethylenetriamine pentaacetate was uniformly mixed. A resultant mixture was dried at 60°C for 1 hour and then passed through a JIS standard sieve having a mesh size of 710 $\mu$m. With the mixture, 0.3 parts by weight of hydrotalcite (product name: DHT-6, manufactured by Kyowa Chemical Industry Co., Ltd., $Mg_6Al_2(OH)_{16}CO_3 \cdot 4H_2O$ [x=0.25 and m=0.50 in the general formula], having a volume average particle diameter of 0.5 $\mu$m) was uniformly mixed. Thus, a particulate water-absorbing agent (22-1) was obtained. Tables 3, 4, and 13 show physical properties of the particulate water-absorbing agent (22-1). Note that an amount of an increase in 150 $\mu$m passing particles, the amount being obtained after the paint shaker test, means an amount of an increase in 150 $\mu$m passing particles, the amount being obtained in a case where the paint shaker test is further carried out with respect to the particulate water-absorbing agent. It is assumed that damage caused, by this paint shaker test, to the particulate water-absorbing agent is process damage caused during production of an absorbent body such as a disposable diaper.

[Production Example 22-2]

Operations similar to those carried out in Production Example 22-1 were carried out except that 0.3 parts by weight of hydrotalcite (product name: HT-1-NC, manufactured by SAKAI CHEMICAL INDUSTRY CO., LTD., chemical formula $Mg_4Al_2(OH)_{12}CO_3 \cdot 3H_2O$ [x=0.33 and m=0.5 in the general formula], having a volume average particle diameter of 0.58 $\mu$m) instead of the hydrotalcite (product name: DHT-6) of Production Example 22-1 was mixed. Thus, a particulate water-absorbing agent (22-2) was obtained. Tables 3, 4, and 13 show physical properties of the particulate water-absorbing agent (22-2).

[Production Example 22-3]

Operations similar to those carried out in Production Example 22-1 were carried out except that 0.3 parts by weight of hydrotalcite (product name: HT-P, manufactured by SAKAI CHEMICAL INDUSTRY CO., LTD., chemical formula $Mg_{4.5}Al_2(OH)_{13}CO_3 \cdot 3.5H_2O$ [x=0.69 and m=0.54 in the general formula], having a volume average particle diameter of 0.45 $\mu$m) instead of the hydrotalcite (product name: DHT-6) of Production Example 22-1 was mixed. Thus, a particulate water-absorbing agent (22-3) was obtained. Tables 3, 4, and 13 show physical properties of the particulate water-absorbing agent (22-3).

[Production Example 22-4]

Operations similar to those carried out in Production Example 22-1 were carried out except that 0.5 parts by weight of tricalcium phosphate (manufactured by Wako Pure Chemical Industries, Ltd., CAS No.7758-87-4) instead of the hydrotalcite (product name: DHT-6) of Production Example 22-1 was mixed. Thus, a particulate water-absorbing agent (22-4) was obtained. Tables 3, 4, and 13 show physical properties of the particulate water-absorbing agent (22-4).

[Production Example 23]

The water-absorbing resin powder (1), which had been obtained in Production Example 1, was subjected to surface crosslinking and additive addition similar to those carried out in Production Example 1, except for the following change in condition. Thus, a particulate water-absorbing agent (23) was obtained. Specifically, a heating treatment time of Production Example 1 was changed from 30 minutes to 45 minutes.
Thereafter, operations similar to those carried out in Production Example 1 were carried out, so that the particulate water-absorbing agent (23) was obtained. Tables 3 through 5 mentioned above show physical properties of the particulate water-absorbing agent (23).

[Production Example 24]

Operations similar to those carried out in Production Example 2 were carried out except that 1.01 parts by mass of the aqueous chelating agent solution used in Production Example 2 and containing 0.01 parts by mass of trisodium diethylenetriamine pentaacetate and 1 part by mass of deionized water was replaced with an aqueous chelating agent solution (2) containing 0.01 parts by mass of trisodium diethylenetriamine pentaacetate, 0.002 parts by mass of polyoxyethylene (20) sorbitan monostearate, and 1 part by mass of deionized water. Thus, a particulate water-absorbing agent (24) was obtained. Tables 3 and 4 mentioned above show physical properties of the particulate water-absorbing agent (24).

[Comparative Production Example 4]

The comparative particulate hydrogel (1), which had been obtained in Comparative Production Example 1, was used to be subjected to drying, pulverization, and classification operations similar to those carried out in Production Example 11-1, so that comparative water-absorbing resin particles (4) having a non-uniformly pulverized shape were obtained. The comparative water-absorbing resin particles (4) had a weight average particle diameter (D50) of 350 $\mu$m, a logarithmic standard deviation ($\sigma\zeta$) of a particle size distribution of 0.32, a CRC of 41.9 g/g, and a proportion of 150 $\mu$m passing particles (a proportion of particles passing through a sieve having a mesh size of 150 $\mu$m) of 0.5 weight%.

Next, the comparative water-absorbing resin particles (4) were used to be subjected to surface treatment and additive addition similar to those carried out in Production Example 11-1. Thus, a comparative particulate water-absorbing agent (4) was obtained. Tables 3, 4, and 13 show physical properties of the comparative particulate water-absorbing agent (4).

[Comparative Production Example 5]

Operations similar to those carried out in Comparative Production Example 4 were carried out except that 0.3 parts by weight of hydrotalcite (product name: HT-1-NC, manufactured by SAKAI CHEMICAL INDUSTRY CO., LTD., chemical formula $Mg_4Al_2(OH)_{12}CO_3 \cdot 3H_2O$ [x=0.33 and m=0.5 in the general formula], having a volume average particle diameter of 0.58 $\mu$m) instead of the hydrotalcite (product name: DHT-6) of Comparative Production Example 4 was mixed. Thus, a comparative particulate water-absorbing agent (5) was obtained. Tables 3, 4, and 13 show physical properties of the comparative particulate water-absorbing agent (5).

[Comparative Production Example 6]

Operations similar to those carried out in Comparative Production Example 4 were carried out except that 0.3 parts by weight of hydrotalcite (product name: HT-P, manufactured by SAKAI CHEMICAL INDUSTRY CO., LTD., chemical formula $Mg_{4.5}Al_2(OH)_{13}CO_3 \cdot 3.5H_2O$ [x=0.69 and m=0.54 in the general formula], having a volume average particle diameter of 0.45 $\mu$m) instead of the hydrotalcite (product name: DHT-6) of Comparative Production Example 4 was mixed. Thus, a comparative particulate water-absorbing agent (6) was obtained. Tables 3, 4, and 13 show physical properties of the comparative particulate water-absorbing agent (6).

[Comparative Production Example 7]

Operations similar to those carried out in Comparative Production Example 4 were carried out except that 0.5 parts by weight of tricalcium phosphate (manufactured by Wako Pure Chemical Industries, Ltd., CAS No.7758-87-4) instead of the hydrotalcite (product name: DHT-6) of Comparative Production Example 4 was mixed. Thus, a comparative particulate water-absorbing agent (7) was obtained. Tables 3, 4, and 13 show physical properties of the comparative particulate water-absorbing agent (7).

[Comparative Production Example 8]

Operations similar to those carried out in Production Example 1 were carried out, except for the operations described below. The hydrogel (8), which had been obtained in Production Example h, was used instead of the hydrogel (1). The pore diameter of the porous plate provided at the tip of the screw extruder was changed to 12.5 mm. In this case, gel-grinding energy (GGE) was 19.1 J/g, and GGE (2) was 7.4 J/g. The hydrogel (8) which had not been subjected to the gel-crushing had a temperature of 82°C, and the temperature was raised to 84°C in a crushed gel obtained after the gel-crushing, i.e., a comparative particulate hydrogel (8).

The comparative particulate hydrogel (8) obtained through the above gel-crushing step had a resin solid content of 52.6 weight%, a weight average particle diameter (D50) of 1223 $\mu$m, and a logarithmic standard deviation ($\sigma\zeta$) of a particle size distribution of 1.28. Table 1 shows conditions under which to carry out the gel-crushing step, and Table 12 shows physical properties of the comparative particulate hydrogel (8).

Subsequently, the comparative particulate hydrogel (8) was subjected to drying, pulverization, and classification operations similar to those carried out in Production Example 3, so that comparative water-absorbing resin particles (8) having a non-uniformly pulverized shape were obtained. The comparative water-absorbing resin particles (8) had a weight average particle diameter (D50) of 426 $\mu$m, a logarithmic standard deviation ($\sigma\zeta$) of a particle size distribution of 0.34, a CRC of 36.2 g/g, and a proportion of 150 $\mu$m passing particles (a proportion of particles passing through a sieve having a mesh size of 150 $\mu$m) of 0.3 weight%.

Next, the comparative water-absorbing resin particles (8) were used to be subjected to surface treatment and additive addition similar to those carried out in Production Example 1. Thus, a comparative particulate water-absorbing agent (8) was obtained. Tables 3, 4, and 13 show physical properties of the comparative particulate water-absorbing agent (8).

[Table 11]

| | Hydrogel used | CRC of hydrogel (g/g) | Moisture content of hydrogel (wt%) | Gel-grinding energy (GGE) (J/g) | Gel-grinding energy (2) (GGE(2)) (J/g) |
|---|---|---|---|---|---|
| Prod. Ex. 1 | Hydrogel (1) | 33.5 | 50.5 | 26.9 | 13.6 |
| Prod. Ex. 2 | Hydrogel (2) | 36.0 | 51.9 | 31.9 | 17.5 |
| Prod. Ex. 3 | Hydrogel (2) | 36.0 | 51.9 | 31.9 | 17.5 |
| Prod. Ex. 4 | Hydrogel (1) | 33.5 | 50.5 | 26.9 | 13.6 |
| Prod. Ex. 5 | Hydrogel (1) | 33.5 | 50.5 | 26.9 | 13.6 |
| Prod. Ex. 6 | Hydrogel (4) | 33.3 | 52.9 | 29.5 | 15.7 |
| Prod. Ex. 7 | Hydrogel (5) | 36.7 | 52.8 | 34.5 | 19.6 |
| Prod. Ex. 8 | Hydrogel (4) | 33.3 | 52.9 | 29.5 | 15.7 |
| Prod. Ex. 9 | Hydrogel (5) | 36.7 | 52.8 | 34.5 | 19.6 |
| Prod. Ex. 10 | Hydrogel (4) | 33.3 | 52.9 | 29.5 | 15.7 |
| Prod. Ex. 11-1 to 11-8 | Hydrogel (2) | 36.0 | 51.9 | 31.9 | 17.5 |
| Prod. Ex. 12-1 to 12-8 | Hydrogel (5) | 36.7 | 52.8 | 34.5 | 19.6 |
| Prod. Ex. 13-1 to 13-2 | Hydrogel (1) | 33.5 | 50.5 | 26.9 | 13.6 |
| Prod. Ex. 14 | Hydrogel (2) | 36.0 | 51.9 | 31.9 | 17.5 |
| Prod. Ex. 15 | Hydrogel (2) | 36.0 | 51.9 | 31.9 | 17.5 |
| Prod. Ex. 16 | Hydrogel (1) | 33.5 | 50.5 | 26.9 | 13.6 |
| Prod. Ex. 17 | Hydrogel (1) | 33.5 | 50.5 | 26.9 | 13.6 |
| Prod. Ex. 18 | Hydrogel (6) | 36.1 | 52.0 | 32.3 | 17.8 |
| Prod. Ex. 19 | Hydrogel (6) | 36.1 | 52.0 | 32.3 | 17.8 |
| Prod. Ex. 20 | Hydrogel (7) | 36.8 | 53.0 | 35.2 | 20.1 |
| Prod. Ex. 21-1 to 21-4 | Hydrogel (6) | 36.1 | 52.0 | 32.3 | 17.8 |
| Prod. Ex. 22-1 to 22-4 | Hydrogel (7) | 36.8 | 53.0 | 35.2 | 20.1 |
| Prod. Ex. 23 | Hydrogel (1) | 33.5 | 50.5 | 26.9 | 13.6 |
| Comp. Prod. Ex. 1 to 7 | Hydrogel (3) | 33.6 | 46.9 | 19.4 | 7.6 |
| Comp. Prod. Ex. 8 | Hydrogel (8) | 32.1 | 46.8 | 19.1 | 7.4 |
| Abbreviations in the above table include the following. Prod. Ex.: Production Example; Comp. Prod. Ex.: Comparative Production Example. | | | | | |

[Table 12]

| | | Moisture content of part. hydrogel (wt%) | Mass average particle diameter (D50) ($\mu$m) | Logarithmic standard deviation ($\sigma\zeta$) of particle size distribution |
|---|---|---|---|---|
| Prod. Ex. 1 | Part. hydrogel (1) | 50.9 | 994 | 1.01 |
| Prod. Ex. 2 | Part. hydrogel (2) | 52.5 | 860 | 0.95 |
| Prod. Ex. 3 | Part. hydrogel (2) | 52.5 | 860 | 0.95 |
| Prod. Ex. 4 | Part. hydrogel (1) | 50.9 | 994 | 1.01 |
| Prod. Ex. 5 | Part. hydrogel (1) | 50.9 | 994 | 1.01 |
| Prod. Ex. 6 | Part. hydrogel (6) | 53.5 | 360 | 0.99 |
| Prod. Ex. 7 | Part. hydrogel (7) | 53.4 | 627 | 1.02 |
| Prod. Ex. 8 | Part. hydrogel (6) | 53.5 | 360 | 0.99 |
| Prod. Ex. 9 | Part. hydrogel (7) | 53.4 | 627 | 1.02 |
| Prod. Ex. 10 | Part. hydrogel (6) | 53.5 | 360 | 0.99 |
| Prod. Ex. 11-1 to 11-8 | Part. hydrogel (2) | 52.5 | 860 | 0.95 |
| Prod. Ex. 12-1 to 12-8 | Part. hydrogel (7) | 53.4 | 627 | 1.02 |
| Prod. Ex. 13-1 to 13-2 | Part. hydrogel (1) | 50.9 | 994 | 1.01 |
| Prod. Ex. 14 | Part. hydrogel (2) | 52.5 | 860 | 0.95 |
| Prod. Ex. 15 | Part. hydrogel (2) | 52.5 | 860 | 0.95 |
| Prod. Ex. 16 | Part. hydrogel (1) | 50.9 | 994 | 1.01 |
| Prod. Ex. 17 | Part. hydrogel (1) | 50.9 | 994 | 1.01 |
| Prod. Ex. 18 | Part. hydrogel (18) | 52.5 | 820 | 0.94 |
| Prod. Ex. 19 | Part. hydrogel (18) | 52.5 | 820 | 0.94 |
| Prod. Ex. 20 | Part. hydrogel (20) | 53.4 | 601 | 0.97 |
| Prod. Ex. 21-1 to 21-4 | Part. hydrogel (18) | 52.5 | 820 | 0.94 |
| Prod. Ex. 22-1 to 22-4 | Part. hydrogel (20) | 53.4 | 601 | 0.97 |
| Prod. Ex. 23 | Part. hydrogel (1) | 50.9 | 994 | 1.01 |
| Comp. Prod. Ex. 1 to 7 | Comp. part. hydrogel (1) | 47.4 | 1322 | 1.32 |
| Comp. Prod. Ex. 8 | Comp. part. hydrogel (8) | 47.4 | 1223 | 1.28 |

Abbreviations in the above table include the following.
Prod. Ex.: Production Example; Comp. Prod. Ex.: Comparative Production Example; Part. hydrogel: Particulate hydrogel; Comp. part. hydrogel: Comparative particulate hydrogel.

[Table 13-1]

| | | DRC index | Absorbent body evaluation re-wet (g) |
|---|---|---|---|
| Prod. Ex. 1 | PWAA (1) | 16.0 | 4.0 |
| Prod. Ex. 2 | PWAA (2) | 23.4 | 3.2 |
| Prod. Ex. 3 | PWAA (3) | 25.2 | 4.2 |
| Prod. Ex. 4 | PWAA (4) | 20.5 | 4.1 |
| Prod. Ex. 5 | PWAA (5) | 25.1 | 4.9 |
| Prod. Ex. 6 | PWAA (6) | 4.9 | 2.9 |
| Prod. Ex. 7 | PWAA (7) | 9.1 | 2.1 |

(continued)

| | | DRC index | Absorbent body evaluation re-wet (g) |
|---|---|---|---|
| Prod. Ex. 8 | PWAA (8) | 11.8 | 3.9 |
| Prod. Ex. 9 | PWAA (9) | 14.4 | 2.9 |
| Prod. Ex. 10 | PWAA (10) | 12.8 | 3.6 |
| Prod. Ex. 11-1 | PWAA (11-1) | 25.6 | 3.3 |
| Prod. Ex. 11-2 | PWAA (11-2) | 25.7 | 3.2 |
| Prod. Ex. 11-3 | PWAA (11-3) | 26.2 | 3.4 |
| Prod. Ex. 11-4 | PWAA (11-4) | 27.7 | 3.5 |
| Prod. Ex. 11-5 | PWAA (11-5) | 28.2 | 4.2 |
| Prod. Ex. 11-6 | PWAA (11-6) | 27.2 | 4.1 |
| Prod. Ex. 11-7 | PWAA (11-7) | 28.0 | 4.3 |
| Prod. Ex. 11-8 | PWAA (11-8) | 29.4 | 4.6 |
| Prod. Ex. 12-1 | PWAA (12-1) | 15.8 | 2.5 |
| Prod. Ex. 12-2 | PWAA (12-2) | 14.2 | 2.6 |
| Prod. Ex. 12-3 | PWAA (12-3) | 15.0 | 2.4 |
| Prod. Ex. 12-4 | PWAA (12-4) | 16.9 | 2.9 |
| Prod. Ex. 12-5 | PWAA (12-5) | 17.5 | 3.4 |
| Prod. Ex. 12-6 | PWAA (12-6) | 15.9 | 3.5 |
| Prod. Ex. 12-7 | PWAA (12-7) | 16.9 | 3.4 |
| Prod. Ex. 12-8 | PWAA (12-8) | 18.2 | 3.8 |

Abbreviations in the above table include the following. Prod. Ex.: Production Example; Comp. Prod. Ex.: Comparative Production Example; PWAA: Particulate water-absorbing agent; Comp. PWAA: Comparative particulate water-absorbing agent.

[Table 13-2]

| | | DRC index | Absorbent body evaluation re-wet (g) |
|---|---|---|---|
| Prod. Ex. 13-1 | PWAA (13-1) | 18.6 | 3.9 |
| Prod. Ex. 13-2 | PWAA (13-2) | 19.6 | 4.5 |
| Prod. Ex. 14 | PWAA (14) | 25.4 | 4.1 |
| Prod. Ex. 15 | PWAA (15) | 21.4 | 3.9 |
| Prod. Ex. 16 | PWAA (16) | 22.1 | 4.3 |
| Prod. Ex. 17 | PWAA (17) | 22.9 | 4.5 |
| Prod. Ex. 18 | PWAA (18) | 22.8 | 3.2 |
| Prod. Ex. 19 | PWAA (19) | 24.3 | 3.3 |
| Prod. Ex. 20 | PWAA (20) | 9.0 | 2.1 |
| Prod. Ex. 21-1 | PWAA (21-1) | 25.6 | 3.3 |
| Prod. Ex. 21-2 | PWAA (21-2) | 25.6 | 3.2 |
| Prod. Ex. 21-3 | PWAA (21-3) | 26.2 | 3.1 |
| Prod. Ex. 21-4 | PWAA (21-4) | 27.7 | 3.2 |
| Prod. Ex. 22-1 | PWAA (22-1) | 17.2 | 3.4 |
| Prod. Ex. 22-2 | PWAA (22-2) | 15.5 | 3.5 |

(continued)

| | | DRC index | Absorbent body evaluation re-wet (g) |
|---|---|---|---|
| Prod. Ex. 22-3 | PWAA (22-3) | 16.7 | 3.2 |
| Prod. Ex. 22-4 | PWAA (22-4) | 18.5 | 3.1 |
| Prod. Ex. 23 | PWAA (23) | 16.9 | 4.8 |
| Comp. Prod. Ex. 1 | Comp. PWAA (1) | 44.1 | 14.5 |
| Comp. Prod. Ex. 2 | Comp. PWAA (2) | 45.6 | 16.1 |
| Comp. Prod. Ex. 3 | Comp. PWAA (3) | 43.1 | 15.8 |
| Comp. Prod. Ex. 4 | Comp. PWAA (4) | 50.8 | 14.8 |
| Comp. Prod. Ex. 5 | Comp. PWAA (5) | 49.6 | 14.9 |
| Comp. Prod. Ex. 6 | Comp. PWAA (6) | 50.2 | 15.0 |
| Comp. Prod. Ex. 7 | Comp. PWAA (7) | 51.5 | 15.6 |
| Comp. Prod. Ex. 8 | Comp. PWAA (8) | 47.1 | 14.7 |

Abbreviations in the above table include the following. Prod. Ex.: Production Example; Comp. Prod. Ex.: Comparative Production Example; PWAA: Particulate water-absorbing agent; Comp. PWAA: Comparative particulate water-absorbing agent.

(Analysis)

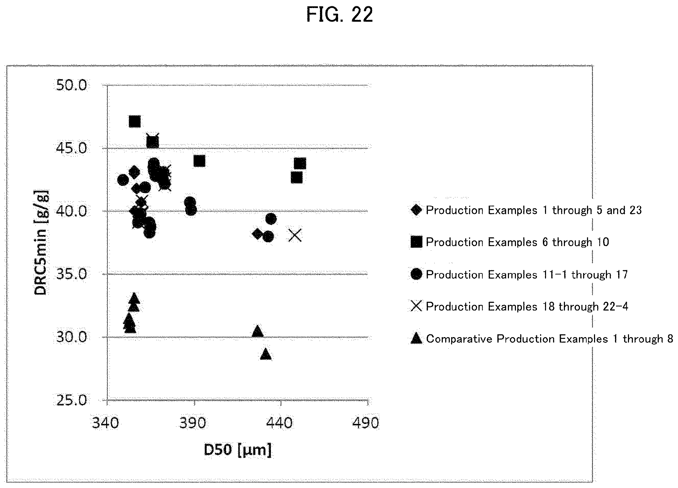

Plotting the DRC5min and weight average particle diameter (D50) (as shown in Tables 3 and 4, respectively) of Production Examples 1 through 24 and Comparative Production Examples 1 through 8 provides a graph as shown in Fig. 22. In examining Production Examples 6 through 10, it can be seen that the relationship between DRC5min and D50 exhibits linearity. Similar linearity is observed in Comparative Production Examples 1 through 3 and Production Examples 1 through 5. From this data, it can be understood that an increase in D50 correlates to a decrease in DRC5min.

In a comparison between Production Examples 6 through 10 and Comparative Production Examples 1 through 3, it can be seen that Production Examples 6 through 10 have, overall, a higher value of DRC5min.

The above-described general index of DRC was derived as a means of distinguishing between a particulate water-absorbing agent having an overall higher value of DRC5min (such as Production Examples 1 through 10) and a particulate water-absorbing agent having an overall lower value of DRC5min (such as Comparative Production Examples 1 through 3).

General index of DRC (Index of DRC) = (K - DRC5min(g/g))/(D50($\mu$m)/ 1000)

where K is any constant.

As one typical example, in a case where K = 49, the DRC index can be represented by the following formula.

In a case where K = 49, DRC index (Index of DRC) = (49 - DRC5min(g/g))/(D50($\mu$m)/ 1000).

The DRC indexes of Production Examples 6 through 10 fall within a range 5 to 14. In contrast, the DRC indexes of Comparative Production Examples 1 through 3 fall within a range of 43 to 46. This clearly indicates the difference in physical properties of the particulate water-absorbing agents of the Production Examples and the particulate water-absorbing agents of the Comparative Production Examples. The DRC indexes of Production Examples 1 through 5 fall within a range of 16 to 25. The DRC index makes it possible to easily determine whether or not a particulate water-absorbing agent has preferable physical properties.

The DRC indexes of Production Examples 11-1 through 17 fall within a range 14 to 30. In contrast, the DRC indexes of Comparative Production Examples 4 through 7 fall within a range of 49 to 52. This clearly indicates the difference in physical properties of the particulate water-absorbing agents of the Production Examples and the particulate water-absorbing agents of the Comparative Production Examples. In the case of these examples as well, the DRC index makes it possible to easily determine whether or not a particulate water-absorbing agent has preferable physical properties.

As seen in Production Examples 11-1 through 17, a particulate water-absorbing agent for use in the present invention has a high CRC value, a high AAP value, a low DRC index, and a B.R. value of 0 weight%. In contrast, it can be seen that the Comparative Production Examples 4 through 7 have DRC indexes exceeding 49 and do not have a high water absorption speed.

As seen in Production Examples 18 through 22-4, even in a case where malic acid is included at the time of polymerization, a particulate water-absorbing agent for use in the present invention has a high CRC value, a high AAP value, a low DRC index, and a B.R. value of 0 weight%. In contrast, it can be seen that the Comparative Production Example 8 has a DRC index exceeding 47 and does not have a high water absorption speed.

Production Example 23 differed from Production Example 1 in that, in Production Example 23, after the covalent bonding surface-crosslinking agent solution of Production Example 1 was mixed uniformly, the heating treatment was carried out for 45 minutes instead of 30 minutes. In comparison to Production Example 1, Production Example 23 had a CRC which was 2.3 g/g lower, but the values of AAP, diffusing absorbency after 60 minutes, diffusing absorbency after 10 minutes, and SFC were increased in Production Example 23. A person skilled in the art can set conditions appropriately so that a particulate water-absorbing agent having intended physical properties can be obtained.

Table 13 shows the re-wet values of absorbent bodies produced using various particulate water-absorbing agents. In cases where the particulate water-absorbing agents of the Production Examples were used, re-wet values fell in a range of 2.1 g to 4.9 g. In contrast, in cases where the particulate water-absorbing agents of the Comparative Production Examples were used, re-wet values were 14.5 g or higher. This indicates that a particulate water-absorbing agent for use in the present invention has a re-wet which is low and which has been improved.

The particulate water-absorbing agent for use in the present invention, which is produced by using a high gel-grinding energy to crush a hydrogel having a high moisture content, has excellent physical properties. The centrifuge retention capacity (CRC) thereof is 30 g/g to 50 g/g, and thus fluid retention capacity is high. The value of dunk retention capacity 5minutes (DRC5min) is high, and thus a high water absorption speed is also achieved. At each particle size, the particulate water-absorbing agent for use in the present invention has an overall high DRC5min, as can be seen in Table 3, which shows DRC5min by particle size. The particulate water-absorbing agent for use in the present invention further has excellent physical properties in terms of one or more of, and preferably all of, the following: surface tension, particle shape, YI value, YI value after colorations acceleration test, moisture absorption fluidity (B.R.), water-soluble content (Ext), degradable soluble content, fluid retention capacity under pressure (AAP), gel capillary absorption (GCA), internal gas bubble ratio, damage resistance paint shaker test, bulk specific gravity, diffusing absorbency after 60 minutes, diffusing absorbency after 10 minutes, and re-wet.

The particulate water-absorbing agent for use in the present invention has a high AAP value and a low DRC index. This indicates that the particulate water-absorbing agent for use in the present invention makes it possible to provide a particulate water-absorbing agent which has both a high fluid retention capacity and a high water absorption speed, and which is also excellent in terms of absorption amount under pressure. Furthermore, the particulate water-absorbing agent for use in the present invention has a low B.R. value, which indicates excellent moisture absorption fluidity. With conventional art, it was impossible to obtain a particulate water-absorbing agent which has both a high fluid retention capacity and a high water absorption speed, and which exhibits excellent blocking prevention (moisture absorption fluidity) under highly humid conditions and/or excellent absorption amount under pressure. The present invention, however, makes it possible to provide such a particulate water-absorbing agent.

In the above descriptions, preferred embodiments of the present invention were used as illustrative examples of the present invention. It is to be understood, however, that the scope of the present invention shall be construed only from the scope of the claims.

Industrial Applicability

A sanitary product (particularly disposable diaper), in which a water-absorbing sheet for use in the present invention is used, exhibits water absorption performance superior to those of conventional sanitary products. Therefore, the present invention can be used in the various fields such as the fields of hygienic materials such as disposable diapers and sanitary napkins and the fields of sheets for pets and waterproofing agents.

Reference Signs List

100:    Plastic supporting cylinder
101:    400-mesh metal gauze made of stainless steel
102:    Swollen gel
103:    Petri dish
104:    Glass filter

105:    Filter paper
106:    0.90-mass% saline

**Claims**

**1.** An absorbent article comprising a water-absorbing sheet, a liquid-permeable sheet, and a liquid-impermeable sheet, wherein the water-absorbing sheet is sandwiched between the liquid-permeable sheet and the liquid-impermeable sheet, and wherein
the water-absorbing sheet comprises:

a first base material;
a second base material; and
a particulate water-absorbing agent sandwiched between the first base material and the second base material, at least one of said first base material and said second base material being a water-permeable base material, said particulate water-absorbing agent containing a first particulate water-absorbing agent and a second particulate water-absorbing agent, the first particulate water-absorbing agent being localized in the vicinity of the first base material and the second particulate water-absorbing agent being localized in the vicinity of the second base material, and
said second particulate water-absorbing agent satisfying the following physical properties (1), (2), and (3):

(1) a centrifuge retention capacity (CRC) is 30 g/g to 50 g/g;
(2) a mass average particle diameter (D50) is 200 $\mu$m to 600 $\mu$m; and
(3) a DRC index defined by the following Formula (a) is 34 or less:

$$DRC\ index=(49-DRC5min)/(D50/1000) \quad ...$$

Formula (a),

wherein a method for measuring the mass average particulate diameter (D50) is as described in paragraphs [0398] to [0400] of the publication EP 3 586 957 A1 of the present application, and the DRC5min is a value measured by a method as described in paragraphs [0404] to [0406] of the publication EP 3 586 957 A1 of the present application.

**2.** The absorbent article according to claim 1, wherein:

said first particulate water-absorbing agent satisfies the conditions (1) and (2) above; and
said first particulate water-absorbing agent has a DRC index of 50 or less, which is defined by the Formula (a) of the condition (3).

**3.** The absorbent article according to claim 1, wherein
said first particulate water-absorbing agent has a DRC index of 43 or less.

**4.** The absorbent article according to claim 1, wherein
said first particulate water-absorbing agent has a DRC index of more than 0 but 43 or less.

**5.** The absorbent article according to any one of claims 1 through 4, wherein
said first base material is a water-permeable base material.

**6.** The absorbent article according to any one of claims 1 through 5, wherein
said first base material is provided on a side so as to come into contact with a human body wearing the absorbent article which is a sanitary product.

**7.** The absorbent article according to any one of claims 1 through 6, wherein
said particulate water-absorbing agent is fixed to a base material with use of an adhesive.

**8.** The absorbent article according to claim 7, wherein
said adhesive is a hot melt adhesive.

**9.** The absorbent article according to claim 8, wherein
an amount of said hot melt adhesive used is 0.01 times to 2.0 times as much by mass as an amount of an entire mass of a particulate water-absorbing agent used per water-absorbing sheet.

**10.** The absorbent article according to any one of claims 7 through 9, wherein
said adhesive is at least one selected from the group consisting of an ethylene-vinyl acetate copolymer adhesive, a styrene elastomer adhesive, a polyolefin-based adhesive, and a polyester-based adhesive.

**11.** The absorbent article according to any one of claims 1 through 10, wherein
a particulate water-absorbing agent used per water-absorbing sheet is contained in an amount of 100 g/m$^2$ to 1000 g/m$^2$ per unit area of the water-absorbing sheet.

**12.** The absorbent article according to any one of claims 1 through 11, wherein
an amount of said first particulate water-absorbing agent contained per unit area of the first base material is equal to or less than an amount of said second particulate water-absorbing agent contained per unit area of the second base material.

**13.** The absorbent article according to any one of claims 1 through 12, wherein
said water-permeable base material has a water permeability index of 20 to 100, and the water permeability index is a value measured by a method as described in Japanese Patent No. 5711974.

**14.** The absorbent article according to any one of claims 1 through 13, wherein
said water-permeable base material is hydrophilic nonwoven fabric.

**15.** The absorbent article according to claim 14, wherein
said hydrophilic nonwoven fabric has a basis weight of 25 g/m$^2$ or more.

**16.** The absorbent article according to claim 14 or 15, wherein
said hydrophilic nonwoven fabric is at least one nonwoven fabric selected from the group consisting of rayon fibers, polyolefin fibers, polyester fibers, and pulp fibers.

**17.** The absorbent article according to any one of claims 1 through 16, wherein the water-absorbing sheet further comprises:

at least one intermediate base material,
said particulate water-absorbing agent containing a first particulate water-absorbing agent and a second particulate water-absorbing agent, the first particulate water-absorbing agent being localized in the vicinity of the first base material and the second particulate water-absorbing agent being localized in the vicinity of the second base material,
said first particulate water-absorbing agent being present in the first base material, in the intermediate base material, and in the vicinity of respective surfaces of the first base material and of the intermediate base material facing each other, and
said second particulate water-absorbing agent being present in the second base material, in the intermediate base material, and in the vicinity of respective surfaces of the second base material and of the intermediate base material facing each other.

**18.** The absorbent article according to claim 17, wherein
said particulate water-absorbing agent recited in claim 1 is present in the second base material of the water-absorbing sheet, in the intermediate base material of the water-absorbing sheet, and in the vicinity of the respective surfaces of the second base material and of the intermediate base material facing each other.

**19.** The absorbent article according to any one of claims 1 through 18, further comprising:
another particulate water-absorbing agent which is different in particle shape or water absorbent property from said particulate water-absorbing agent recited in claim 1.

**20.** The absorbent article according to any one of claims 17 through 19, wherein:

said particulate water-absorbing agent recited in claim 1 is present in the second base material of the water-absorbing sheet, in the intermediate base material of the water-absorbing sheet, and in the vicinity of respective surfaces of the second base material and of the intermediate base material facing each other; and
said another particulate water-absorbing agent is present in the first base material of the water-absorbing sheet, in the intermediate base material of the water-absorbing sheet, and in the vicinity of respective surfaces of the first base material and of the intermediate base material facing each other.

21. The absorbent article according to any one of claims 1 through 20, wherein:

said particulate water-absorbing agent contains a first particulate water-absorbing agent and a second particulate water-absorbing agent, the first particulate water-absorbing agent being localized in the vicinity of the first base material and the second particulate water-absorbing agent being localized in the vicinity of the second base material;
said first particulate water-absorbing agent has a non-uniformly pulverized shape; and
said second particulate water-absorbing agent has a spherical shape or is a granulated material of spherical particles.

22. The absorbent article according to any one of claims 1 through 21, wherein
said water-absorbing sheet has a thickness of 5 mm or less in a dry state.

23. The absorbent article according to any one of claims 1 through 22, wherein
said water-absorbing sheet has a surface having an embossed region.

24. The absorbent article according to any one of claims 1 through 23, wherein
said water-absorbing sheet has a region in which the particulate water-absorbing agent is not present and which extends along a length of the water-absorbing sheet.

25. The absorbent article according to any one of claims 1 through 24, wherein
said particulate water-absorbing agent recited in claim 1 has a DRC index of 30 or less.

26. The absorbent article according to any one of claims 1 through 25, wherein
said particulate water-absorbing agent recited in claim 1 has a DRC index of 20 or less.

27. The absorbent article according to any one of claims 1 through 26, wherein
said particulate water-absorbing agent recited in claim 1 has a saline flow conductivity (SFC) of less than 30 $(\times 10^{-7} \cdot cm^3 \cdot s \cdot g^{-1})$.

28. The absorbent article according to any one of claims 1 through 27, wherein
said particulate water-absorbing agent recited in claim 1 has a surface tension of 65 mN/m or more, and a method for measuring the surface tension is as described in paragraphs [0407] and [0408] of the publication EP 3 586 957 A1 of the present application.

29. The absorbent article according to any one of claims 1 through 28, wherein
a particle shape of said particulate water-absorbing agent recited in claim 1 is a non-uniformly pulverized shape.

30. The absorbent article according to any one of claims 1 through 29, wherein
said particulate water-absorbing agent recited in claim 1 has a moisture absorption fluidity (B.R.) of 50 mass% or less, and the moisture absorption fluidity (B.R.) is a value measured by a method as described paragraph [0412] of the publication EP 3 586 957 A1 of the present application.

31. the absorbent article according to any one of claims 1 through 30, wherein
said particulate water-absorbing agent recited in claim 1 has a water-soluble content (Ext) of 25 mass% or less, and the water-soluble content (Ext) is measured in accordance with the EDANA method ERT 470.2-02.

32. The absorbent article according to any one of claims 1 through 31, wherein
said particulate water-absorbing agent recited in claim 1 has a degradable soluble content of 30 mass% or less, and the degradable soluble content is a value measured by a method as described in paragraphs [0413] to [0417] of the publication EP 3 586 957 A1 of the present application.

**33.** The absorbent article according to any one of claims 1 through 32, wherein
said particulate water-absorbing agent recited in claim 1 has an absorption against pressure (AAP 2.06 kPa) of 18 g/g or more.

**34.** The absorbent article according to any one of claims 1 through 33, wherein
said particulate water-absorbing agent recited in claim 1 has an absorption against pressure (AAP 2.06 kPa) of 26 g/g or more.

**35.** The absorbent article according to any one of claims 1 through 34, wherein
said particulate water-absorbing agent recited in claim 1 contains a polyacrylic acid (salt)-based water-absorbing resin as a main component.

**36.** The absorbent article according to any one of claims 1 through 35, wherein
said particulate water-absorbing agent recited in claim 1 has a diffusing absorbency under pressure (DAP) of 16 g/g or more, and the diffusing absorbency under pressure (DAP) is a value measured by a method as described in Japanese Patent Application Publication No. 2010-142808.

**37.** The absorbent article according to any one of claims 1 to 36, wherein:
the water-absorbing sheet is provided so that in a case where the absorbent article is used, the first base material comes into contact with a liquid before the second base material comes into contact with the liquid.

**Patentansprüche**

**1.** Absorbierender Artikel, der eine Wasser-absorbierende Schicht, eine Flüssigkeits-durchlässige Schicht und eine Flüssigkeits-undurchlässige Schicht umfasst, wobei die Wasser-absorbierende Schicht zwischen die Flüssigkeits-durchlässige Schicht und die Flüssigkeits-undurchlässige Schicht eingelegt ist und wobei
die Wasser-absorbierende Schicht folgendes umfasst:

ein erstes Basismaterial;
ein zweites Basismaterial; und
ein partikelförmiges Wasser-absorbierendes Mittel, das zwischen das erste Basismaterial und das zweite Basismaterial eingelegt ist, wobei
mindestens eines von dem ersten Basismaterial und dem zweiten Basismaterial ein Wasser-durchlässiges Basismaterial ist,
das partikelförmige Wasser-absorbierende Mittel ein erstes partikelförmiges Wasser-absorbierendes Mittel und ein zweites partikelförmiges Wasser-absorbierendes Mittel enthält, wobei sich das erste partikelförmige Wasser-absorbierende Mittel in der Nähe des ersten Basismaterials befindet und sich das zweite partikelförmige Wasser-absorbierende Mittel in der Nähe des zweiten Basismaterials befindet, und
das zweite partikelförmige Wasser-absorbierende Mittel die folgenden physikalischen Eigenschaften (1), (2) und (3) erfüllt:

(1) eine Zentrifugenretentionskapazität (centrifuge retention capacity, CRC) ist 30 g/g bis 50 g/g;
(2) ein massengemittelter Partikeldurchmesser (D50) ist 200 $\mu$m bis 600 $\mu$m; und
(3) ein durch die folgende Formel (a) definierter DRC-Index ist 34 oder weniger:

$$DRC\text{-}Index = (49 - DRC5min) / (D50/1000) \ldots \qquad \text{Formel (a)},$$

wobei das Verfahren zur Messung des massengemittelten Partikeldurchmessers (D50) wie in den Paragraphen [0398] bis [0400] der Veröffentlichung EP 3 586 957 A1 der vorliegenden Anmeldung beschrieben ist und der DRC5min ein Wert ist, der mit einem Verfahren gemessen wird, das in den Paragraphen [0404] bis [0406] der Veröffentlichung EP 3 586 957 A1 der vorliegenden Anmeldung beschrieben ist.

**2.** Absorbierender Artikel gemäß Anspruch 1, wobei das erste partikelförmige Wasser-absorbierende Mittel die Bedingungen (1) und (2) oben erfüllt; und
das erste partikelförmige Wasser-absorbierende Mittel einen DRC-Index von 50 oder weniger hat, der durch die Formel (a) der Bedingung (3) definiert ist.

3. Absorbierender Artikel gemäß Anspruch 1, wobei das erste partikelförmige Wasser-absorbierende Mittel einen DRC-Index von 43 oder weniger hat.

4. Absorbierender Artikel gemäß Anspruch 1, wobei das erste partikelförmige Wasser-absorbierende Mittel einen DRC-Index von mehr als 0, jedoch 43 oder weniger hat.

5. Absorbierender Artikel gemäß mindestens einem der Ansprüche 1 bis 4, wobei das erste Basismaterial ein Wasser-durchlässiges Basismaterial ist.

6. Absorbierender Artikel gemäß mindestens einem der Ansprüche 1 bis 5, wobei das erste Basismaterial so auf einer Seite vorgesehen ist, dass es in Kontakt mit dem Körper eines Menschen kommt, der den absorbierenden Artikel, der ein Hygieneprodukt ist, trägt.

7. Absorbierender Artikel gemäß mindestens einem der Ansprüche 1 bis 6, wobei das partikelförmige Wasser-absorbierende Mittel unter Verwendung eines Klebstoffs an einem Basismaterial befestigt ist.

8. Absorbierender Artikel gemäß Anspruch 7, wobei der Klebstoff ein Schmelzklebstoff ist.

9. Absorbierender Artikel gemäß Anspruch 8, wobei eine verwendete massenbezogene Menge des Schmelzklebstoffs 0,01- bis 2,0-mal so viel ist wie die Menge der gesamten Masse eines partikelförmigen Wasser-absorbierenden Mittels, das pro Wasser-absorbierende Schicht verwendet wird.

10. Absorbierender Artikel gemäß mindestens einem der Ansprüche 7 bis 9, wobei der Klebstoff mindestens einer ist, ausgewählt aus der Gruppe, bestehend aus einem Ethylen-Vinylacetat-Copolymer-Klebstoff, einem Styrol-Elastomer-Klebstoff, einem Klebstoff auf Polyolefinbasis und einem Klebstoff auf Polyesterbasis.

11. Absorbierender Artikel gemäß mindestens einem der Ansprüche 1 bis 10, wobei ein pro Wasser-absorbierender Schicht verwendetes, partikelförmiges Wasser-absorbierendes Mittel in einer Menge von $100\,g/m^2$ bis $1.000\,g/m^2$ pro Einheitsfläche der Wasser-absorbierenden Schicht enthalten ist.

12. Absorbierender Artikel gemäß mindestens einem der Ansprüche 1 bis 11, wobei eine Menge des pro Einheitsfläche des ersten Basismaterials enthaltenen ersten partikelförmigen Wasser-absorbierenden Mittels gleich oder weniger ist als eine Menge des pro Einheitsfläche des zweiten Basismaterials enthaltenen zweiten partikelförmigen Wasser-absorbierenden Mittels.

13. Absorbierender Artikel gemäß mindestens einem der Ansprüche 1 bis 12, wobei das Wasser-durchlässige Basismaterial einen Wasserdurchlässigkeitsindex von 20 bis 100 hat und der Wasserdurchlässigkeitsindex ein Wert ist, der mit einem Verfahren gemessen wird, wie es in dem japanischen Patent Nr. 5711974 beschrieben ist.

14. Absorbierender Artikel gemäß mindestens einem der Ansprüche 1 bis 13, wobei das Wasser-durchlässige Basismaterial ein hydrophiles Faservlies ist.

15. Absorbierender Artikel gemäß Anspruch 14, wobei das hydrophile Faservlies ein Flächengewicht von $25\,g/m^2$ oder mehr hat.

16. Absorbierender Artikel gemäß Anspruch 14 oder 15, wobei das hydrophile Faservlies mindestens ein Faservlies ist, das ausgewählt ist aus der Gruppe, bestehend aus RayonFasern, Polyolefin-Fasern, Polyester-Fasern und Zellstoff-Fasern.

17. Absorbierender Artikel gemäß mindestens einem der Ansprüche 1 bis 16, wobei die Wasser-absorbierende Schicht ferner umfasst:

mindestens ein Zwischen-Basismaterial,
wobei das partikelförmige Wasser-absorbierende Mittel ein erstes partikelförmiges Wasser-absorbierendes Mittel und ein zweites partikelförmiges Wasser-absorbierendes Mittel enthält, wobei das erste partikelförmige Wasser-absorbierende Mittel sich in der Nähe des ersten Basismaterials befindet und das zweite partikelförmige Wasser-absorbierende Mittel sich in der Nähe des zweiten Basismaterials befindet,
das erste partikelförmige Wasser-absorbierende Mittel in dem ersten Basismaterial, in dem Zwischen-Basismaterial und in der Nähe der entsprechenden Oberflächen des ersten Basismaterials und des Zwischen-Basismaterials, die einander gegenüberliegen, vorliegt, und

das zweite partikelförmige Wasser-absorbierende Mittel in dem zweiten Basismaterial, in dem Zwischen-Basismaterial und in der Nähe der entsprechenden Oberflächen des zweiten Basismaterials und des Zwischen-Basismaterials, die einander gegenüberliegen, vorliegt.

18. Absorbierender Artikel gemäß Anspruch 17, wobei das in Anspruch 1 definierte, partikelförmige Wasser-absorbierende Mittel in dem zweiten Basismaterial der Wasser-absorbierenden Schicht, in dem Zwischen-Basismaterial der Wasser-absorbierenden Schicht und in der Nähe der entsprechenden Oberflächen des zweiten Basismaterials und des Zwischen-Basismaterials, die einander gegenüberliegen, vorliegt.

19. Absorbierender Artikel gemäß mindestens einem der Ansprüche 1 bis 18, der ferner ein weiteres partikelförmiges Wasser-absorbierendes Mittel umfasst, das sich in der Partikelform oder den Wasser-absorbierenden Eigenschaften von dem partikelförmigen Wasser-absorbierenden Mittel, das in Anspruch 1 definiert ist, unterscheidet.

20. Absorbierender Artikel gemäß mindestens einem der Ansprüche 17 bis 19, wobei:

das in Anspruch 1 definierte, partikelförmige Wasser-absorbierende Mittel in dem zweiten Basismaterial der Wasser-absorbierenden Schicht, in dem Zwischen-Basismaterial der Wasser-absorbierenden Schicht und in der Nähe der entsprechenden Oberflächen des zweiten Basismaterials und des Zwischen-Basismaterials, die einander gegenüberliegen, vorliegt; und

das weitere partikelförmige Wasser-absorbierende Mittel in dem ersten Basismaterial der Wasser-absorbierenden Schicht, in dem Zwischen-Basismaterial der Wasser-absorbierenden Schicht und in der Nähe der entsprechenden Oberflächen des ersten Basismaterials und des Zwischen-Basismaterials, die einander gegenüberliegen, vorliegt.

21. Absorbierender Artikel gemäß mindestens einem der Ansprüche 1 bis 20, wobei das partikelförmige Wasser-absorbierende Mittel ein erstes partikelförmiges Wasser-absorbierendes Mittel und ein zweites partikelförmiges Wasser-absorbierendes Mittel enthält, wobei sich das erste partikelförmige Wasser-absorbierende Mittel in der Nähe des ersten Basismaterials befindet und das zweite partikelförmige Wasser-absorbierende Mittel sich in der Nähe des zweiten Basismaterials befindet;

das erste partikelförmige Wasser-absorbierende Mittel eine ungleichförmig pulverisierte Form hat; und

das zweite partikelförmige Wasser-absorbierende Mittel eine sphärische Form hat oder ein granuliertes Material sphärischer Partikel ist.

22. Absorbierender Artikel gemäß mindestens einem der Ansprüche 1 bis 21, wobei die Wasser-absorbierende Schicht eine Dicke von 5 mm oder weniger in einem trockenen Zustand hat.

23. Absorbierender Artikel gemäß mindestens einem der Ansprüche 1 bis 22, wobei die Wasser-absorbierende Schicht eine Oberfläche mit einem geprägten Bereich aufweist.

24. Absorbierender Artikel gemäß mindestens einem der Ansprüche 1 bis 23, wobei die Waser-absorbierende Schicht einen Bereich hat, in dem das partikelförmige Wasser-absorbierende Mittel nicht vorliegt und der sich in Richtung einer Länge der Wasser-absorbierenden Schicht erstreckt.

25. Absorbierender Artikel gemäß mindestens einem der Ansprüche 1 bis 24, wobei das in Anspruch 1 definierte, partikelförmige Wasser-absorbierende Mittel einen DRC-Index von 30 oder weniger hat.

26. Absorbierender Artikel gemäß mindestens einem der Ansprüche 1 bis 25, wobei das in Anspruch 1 definierte, partikelförmige Wasser-absorbierende Mittel einen DRC-Index von 20 oder weniger hat.

27. Absorbierender Artikel gemäß mindestens einem der Ansprüche 1 bis 26, wobei das in Anspruch 1 definierte, partikelförmige Wasser-absorbierende Mittel eine Saline Flow Conductivity (SFC) von weniger als 30 ($\times$ $10^{-7} \cdot cm^3 \cdot s \cdot g^{-1}$) hat.

28. Absorbierender Artikel gemäß mindestens einem der Ansprüche 1 bis 27, wobei das in Anspruch 1 definierte, partikelförmige Wasser-absorbierende Mittel eine Oberflächenspannung von 65 mN/m oder mehr hat und das Verfahren zur Messung der Oberflächenspannung wie in den Paragraphen [0407] und [0408] der Veröffentlichung EP 3 586 957 A1 der vorliegenden Anmeldung beschrieben ist.

29. Absorbierender Artikel gemäß mindestens einem der Ansprüche 1 bis 28, wobei eine Partikelform des in Anspruch 1 definierten, partikelförmigen Wasser-absorbierenden Mittelseine ungleichförmig pulverisierte Form ist.

30. Absorbierender Artikel gemäß mindestens einem der Ansprüche 1 bis 29, wobei das in Anspruch 1 definierte, partikelförmige Wasser-absorbierende Mittel eine Feuchtigkeitsabsorptionsfluidität (moisture absorption fluidity, B.R.) von 50 Masse-% oder weniger hat und die Feuchtigkeitsabsorptionsfluidität (moisture absorption fluidity, B.R.) ein Wert ist, der mit einem Verfahren gemessen wird, das in Paragraph [0412] der Veröffentlichung EP 3 586 957 A1 der vorliegenden Anmeldung beschrieben ist.

31. Absorbierender Artikel gemäß mindestens einem der Ansprüche 1 bis 30, wobei das in Anspruch 1 definierte, partikelförmige Wasser-absorbierende Mittel einen wasserlöslichen Anteil (Ext) von 25 Masse-% oder weniger hat, wobei der wasserlösliche Anteil (Ext) in Übereinstimmung mit der EDANA-Methode ERT 470.2-02 gemessen wird.

32. Absorbierender Artikel gemäß mindestens einem der Ansprüche 1 bis 31, wobei das in Anspruch 1 definierte, partikelförmige Wasser-absorbierende Mittel einen abbaubaren löslichen Anteil von 30 Masse-% oder weniger hat und der abbaubare lösliche Anteil ein Wert ist, der mit einem Verfahren gemessen wird, wie es in den Paragraphen [0413] bis [0417] der Veröffentlichung EP 3 586 957 A1 der vorliegenden Anmeldung beschrieben ist.

33. Absorbierender Artikel gemäß mindestens einem der Ansprüche 1 bis 32, wobei das in Anspruch 1 definierte, partikelförmige Wasser-absorbierende Mittel eine Absorption unter Druck (absorption against pressure, AAP 2,06 kPa) von 18 g/g oder mehr hat.

34. Absorbierender Artikel gemäß mindestens einem der Ansprüche 1 bis 33, wobei das in Anspruch 1 definierte, partikelförmige Wasser-absorbierende Mittel eine Absorption unter Druck (absorption against pressure, AAP 2,06 kPa) von 26 g/g oder mehr hat.

35. Absorbierender Artikel gemäß mindestens einem der Ansprüche 1 bis 34, wobei das in Anspruch 1 definierte, partikelförmige Wasser-absorbierende Mittel ein Wasser-absorbierendes Harz auf Basis von Polyacrylsäure(salz) als eine Hauptkomponente enthält.

36. Absorbierender Artikel gemäß mindestens einem der Ansprüche 1 bis 35, wobei das in Anspruch 1 definierte, partikelförmige Wasser-absorbierende Mittel eine Diffusionsabsorptionsvermögen unter Druck (diffusing absorbency under pressure, DAP) von 16 g/g oder mehr hat, wobei das Diffusionsabsorptionsvermögen unter Druck (DAP) ein Wert ist, der mit einem Verfahren gemessen wird, wie es in der japanischen Patentanmeldung Nr. 2010-142808 beschrieben ist.

37. Absorbierender Artikel gemäß mindestens einem der Ansprüche 1 bis 36, in dem die Wasser-absorbierende Schicht so angeordnet ist, dass bei Verwendung des absorbierenden Artikels das erste Basismaterial in Kontakt mit einer Flüssigkeit kommt, bevor das zweite Basismaterial in Kontakt mit der Flüssigkeit kommt.

**Revendications**

1. Article absorbant comprenant une feuille absorbant l'eau, une feuille perméable au liquide et une feuille imperméable au liquide, dans lequel la feuille absorbant l'eau est prise en sandwich entre la feuille perméable au liquide et la feuille imperméable au liquide, et dans lequel
la feuille absorbant l'eau comprend :

un premier matériau de base ;
un deuxième matériau de base ; et
un agent absorbant l'eau particulaire pris en sandwich entre le premier matériau de base et le deuxième matériau de base,
au moins un dudit premier matériau de base et dudit deuxième matériau de base étant un matériau de base perméable à l'eau,
ledit agent absorbant l'eau particulaire contenant un premier agent absorbant l'eau particulaire et un deuxième agent absorbant l'eau particulaire, le premier agent absorbant l'eau particulaire étant localisé à proximité du premier matériau de base et le deuxième agent absorbant l'eau particulaire étant localisé à proximité du deuxième matériau de base, et

ledit deuxième agent absorbant l'eau particulaire satisfaisant aux propriétés physiques suivantes (1), (2) et (3) :

(1) une capacité de rétention centrifuge (CRC) va de 30 g/g à 50 g/g ;
(2) un diamètre moyen de particule en masse (D50) va de 200 $\mu$m à 600 $\mu$m ; et
(3) un indice de DRC défini par la formule suivante (a) s'élève à 34 ou moins :

$$\text{Indice de DRC} = (49 - DRC5min)/(D50/1000) \ \ldots \text{formule (a)}$$

dans lequel un procédé de mesure du diamètre moyen de particule en masse (D50) est comme décrit dans les paragraphes [0398] à [0400] de la publication EP 3 586 957 A1 de la présente demande, et le DRC5min est une valeur mesurée par un procédé comme décrit dans les paragraphes [0404] à [0406] de la publication EP 3 586 957 A1 de la présente demande.

2. Article absorbant selon la revendication 1, dans lequel :

ledit premier agent absorbant l'eau particulaire satisfait aux conditions (1) et (2) ci-dessus ; et
ledit premier agent absorbant l'eau particulaire présente un indice de DRC de 50 ou moins, qui est défini par la formule (a) de la condition (3).

3. Article absorbant selon la revendication 1, dans lequel ledit premier agent absorbant l'eau particulaire présente un indice de DRC de 43 ou moins.

4. Article absorbant selon la revendication 1, dans lequel ledit premier agent absorbant l'eau particulaire présente un indice de DRC de plus de 0 mais inférieur ou égal à 43.

5. Article absorbant selon l'une quelconque des revendications 1 à 4, dans lequel ledit premier matériau de base est un matériau de base perméable à l'eau.

6. Article absorbant selon l'une quelconque des revendications 1 à 5, dans lequel ledit premier matériau de base est prévu sur un côté de sorte à venir en contact avec un corps humain portant l'article absorbant qui est un produit sanitaire.

7. Article absorbant selon l'une quelconque des revendications 1 à 6, dans lequel ledit agent absorbant l'eau particulaire est fixé à un matériau de base avec utilisation d'un adhésif.

8. Article absorbant selon la revendication 7, dans lequel ledit adhésif est un adhésif thermofusible.

9. Article absorbant selon la revendication 8, dans lequel une quantité dudit adhésif thermofusible utilisé est 0,01 fois à 2,0 fois plus grande en masse qu'une quantité d'une masse entière d'un agent absorbant l'eau particulaire utilisé par feuille absorbant l'eau.

10. Article absorbant selon l'une quelconque des revendications 7 à 9, dans lequel ledit adhésif est au moins un élément sélectionné à partir du groupe constitué d'un adhésif de copolymère d'éthylène-vinyle acétate, un adhésif d'élastomère de styrène, un adhésif à base de polyoléfine et un adhésif à base de polyester.

11. Article absorbant selon l'une quelconque des revendications 1 à 10, dans lequel un agent absorbant l'eau particulaire utilisé par feuille absorbant l'eau est contenu dans une quantité de 100 g/m$^2$ à 1000 g/m$^2$ par unité de surface de la feuille absorbant l'eau.

12. Article absorbant selon l'une quelconque des revendications 1 à 11, dans lequel une quantité dudit premier agent absorbant l'eau particulaire contenu par unité de surface du premier matériau de base est égale à ou inférieure à une quantité dudit deuxième agent absorbant l'eau particulaire contenu par unité de surface du deuxième matériau de base.

**13.** Article absorbant selon l'une quelconque des revendications 1 à 12, dans lequel
ledit matériau de base perméable à l'eau présente un indice de perméabilité à l'eau de 20 à 100, et l'indice de perméabilité à l'eau est une valeur mesurée par un procédé comme décrit dans le brevet japonais n° 5711974.

**14.** Article absorbant selon l'une quelconque des revendications 1 à 13, dans lequel
ledit matériau de base perméable à l'eau est un non-tissé hydrophile.

**15.** Article absorbant selon la revendication 14, dans lequel
ledit non-tissé hydrophile présente un poids de base de 25 g/m$^2$ ou plus.

**16.** Article absorbant selon la revendication 14 ou la revendication 15, dans lequel
ledit non-tissé hydrophile est au moins un non-tissé sélectionné à partir du groupe constitué de fibres de rayonne, fibres de polyoléfine, fibres de polyester et fibres de pulpe.

**17.** Article absorbant selon l'une quelconque des revendications 1 à 16, dans lequel la feuille absorbant l'eau comprend en outre :

au moins un matériau de base intermédiaire,
ledit agent absorbant l'eau particulaire contenant un premier agent absorbant l'eau particulaire et un deuxième agent absorbant l'eau particulaire, le premier agent absorbant l'eau particulaire étant localisé à proximité du premier matériau de base et le deuxième agent absorbant l'eau particulaire étant localisé à proximité du deuxième matériau de base,
ledit premier agent absorbant l'eau particulaire étant présent dans le premier matériau de base, dans le matériau de base intermédiaire, et à proximité des surfaces respectives du premier matériau de base et du matériau de base intermédiaire se faisant face l'un l'autre, et
ledit deuxième agent absorbant l'eau particulaire étant présent dans le deuxième matériau de base, dans le matériau de base intermédiaire, et à proximité de surfaces respectives du deuxième matériau de base et du matériau de base intermédiaire se faisant face l'un l'autre.

**18.** Article absorbant selon la revendication 17, dans lequel
ledit agent absorbant l'eau particulaire selon la revendication 1 est présent dans le deuxième matériau de base de la feuille absorbant l'eau, dans le matériau de base intermédiaire de la feuille absorbant l'eau, et à proximité des surfaces respectives du deuxième matériau de base et du matériau de base intermédiaire se faisant face l'un l'autre.

**19.** Article absorbant selon l'une quelconque des revendications 1 à 18, comprenant en outre :
un autre agent absorbant l'eau particulaire qui est différent en forme particulaire ou propriété d'absorption d'eau dudit agent absorbant l'eau particulaire selon la revendication 1.

**20.** Article absorbant selon l'une quelconque des revendications 17 à 19, dans lequel :

ledit agent absorbant l'eau particulaire selon la revendication 1 est présent dans le deuxième matériau de base de la feuille absorbant l'eau, dans le matériau de base intermédiaire de la feuille absorbant l'eau, et à proximité de surfaces respectives du deuxième matériau de base et du matériau de base intermédiaire se faisant face l'un l'autre ; et
ledit autre agent absorbant l'eau particulaire est présent dans le premier matériau de base de la feuille absorbant l'eau, dans le matériau de base intermédiaire de la feuille absorbant l'eau, et à proximité des surfaces respectives du premier matériau de base et du matériau de base intermédiaire se faisant face l'un l'autre.

**21.** Article absorbant selon l'une quelconque des revendications 1 à 20, dans lequel :

ledit agent absorbant l'eau particulaire contient un premier agent absorbant l'eau particulaire et un deuxième agent absorbant l'eau particulaire, le premier agent absorbant l'eau particulaire étant localisé à proximité du premier matériau de base et le deuxième agent absorbant l'eau particulaire étant localisé à proximité du deuxième matériau de base ;
ledit premier agent absorbant l'eau particulaire présente une forme pulvérisée de manière non uniforme ; et
ledit deuxième agent absorbant l'eau particulaire présente une forme sphérique ou est un matériau granulé de particules sphériques.

**106**

**22.** Article absorbant selon l'une quelconque des revendications 1 à 21, dans lequel
ladite feuille absorbant l'eau présente une épaisseur de 5 mm ou moins dans un état sec.

**23.** Article absorbant selon l'une quelconque des revendications 1 à 22, dans lequel
ladite feuille absorbant l'eau présente une surface présentant une région embossée.

**24.** Article absorbant selon l'une quelconque des revendications 1 à 23, dans lequel
ladite feuille absorbant l'eau présente une région dans laquelle l'agent absorbant l'eau particulaire n'est pas présent et
qui s'étend le long d'une longueur de la feuille absorbant l'eau.

**25.** Article absorbant selon l'une quelconque des revendications 1 à 24, dans lequel
ledit agent absorbant l'eau particulaire selon la revendication 1 présente un indice de DRC de 30 ou moins.

**26.** Article absorbant selon l'une quelconque des revendications 1 à 25, dans lequel
ledit agent absorbant l'eau particulaire selon la revendication 1 présente un indice de DRC de 20 ou moins.

**27.** Article absorbant selon l'une quelconque des revendications 1 à 26, dans lequel
ledit agent absorbant l'eau particulaire selon la revendication 1 présente une conductivité de flux salin (SFC) de moins
de 30 (x$10^{-7}$·cm$^3$·s·g$^{-1}$).

**28.** Article absorbant selon l'une quelconque des revendications 1 à 27, dans lequel
ledit agent absorbant l'eau particulaire selon la revendication 1 présente une tension de surface de 65 mN/m ou plus,
et un procédé de mesure de la tension de surface est comme décrit dans les paragraphes [0407] et [0408] de la
publication EP 3 586 957 A1 de la présente demande.

**29.** Article absorbant selon l'une quelconque des revendications 1 à 28, dans lequel
une forme particulaire dudit agent absorbant l'eau particulaire selon la revendication 1 est une forme pulvérisée de
manière non uniforme.

**30.** Article absorbant selon l'une quelconque des revendications 1 à 29, dans lequel
ledit agent absorbant l'eau particulaire selon la revendication 1 présente une fluidité d'absorption d'humidité (B.R.) de
50 % en masse ou moins, et la fluidité d'absorption d'humidité (B.R.) est une valeur mesurée par un procédé selon le
paragraphe [0412] décrit de la publication EP 3 586 957 A1 de la présente demande.

**31.** Article absorbant selon l'une quelconque des revendications 1 à 30, dans lequel
ledit agent absorbant l'eau particulaire selon la revendication 1 présente un contenu soluble dans l'eau (Ext) de 25 %
en masse ou moins, et le contenu soluble dans l'eau (Ext) est mesuré selon le procédé EDANA ERT 470.2-02.

**32.** Article absorbant selon l'une quelconque des revendications 1 à 31, dans lequel
ledit agent absorbant l'eau particulaire selon la revendication 1 présente un contenu soluble dégradable de 30 % en
masse ou moins, et le contenu soluble dégradable est une valeur mesurée par un procédé comme décrit dans les
paragraphes [0413] à [0417] de la publication EP 3 586 957 A1 de la présente demande.

**33.** Article absorbant selon l'une quelconque des revendications 1 à 32, dans lequel
ledit agent absorbant l'eau particulaire selon la revendication 1 présente une absorption sous pression (AAP 2,06
kPa) de 18 g/g ou plus.

**34.** Article absorbant selon l'une quelconque des revendications 1 à 33, dans lequel
ledit agent absorbant l'eau particulaire selon la revendication 1 présente une absorption sous pression (AAP 2,06
kPa) de 26 g/g ou plus.

**35.** Article absorbant selon l'une quelconque des revendications 1 à 34, dans lequel
ledit agent absorbant l'eau particulaire selon la revendication 1 contient une résine d'absorption d'eau à base de (sel)
d'acide polyacrylique comme composant principal.

**36.** Article absorbant selon l'une quelconque des revendications 1 à 35, dans lequel
ledit agent absorbant l'eau particulaire selon la revendication 1 présente une absorbance de diffusion sous pression
(DAP) de 16 g/g ou plus, et l'absorbance de diffusion sous pression (DAP) est une valeur mesurée par un procédé comme décrit dans la publication de demande de brevet japonaise n° 2010-142808.

**37.** Article absorbant selon l'une quelconque des revendications 1 à 36, dans lequel :
la feuille absorbant l'eau est fournie de sorte que, dans un cas où l'article absorbant est utilisé, le premier matériau de base entre en contact avec un liquide avant que le deuxième matériau de base n'entre en contact avec le liquide.

## FIG. 1

## FIG. 2

INNER DIAMETER:20mm

78mm

20mm

70mm

80mm

10mm

158mm

FIG. 3

INTRODUCTION OF LIQUID

WEIGHT

LIQUID INJECTION TUBE

WATER-ABSORBING SHEET

CONTAINER

## FIG. 4

FIG. 5

INNER DIAMETER:20mm

39mm

78mm

39mm

118mm

59mm

59mm

59mm

20mm

FIG. 6

INTRODUCTION OF LIQUID

WEIGHT

LIQUID INJECTION TUBE

WATER-ABSORBING SHEET

CONTAINER

## FIG. 7

INNER DIAMETER:
23.5mm

WEIGHT

100mm

23.5mm

170mm

220mm

WEIGHT

100mm

FIG. 8

INTRODUCTION OF LIQUID

LIQUID INJECTION TUBE

WEIGHT

WATER-ABSORBING
SHEET

## FIG. 9

FIG. 10

FIG. 11

EP 3 586 957 B2

FIG. 12

GUIDE

WATER-ABSORBING SHEET

CONTAINER

FIG. 13

GUIDE

INTRODUCTION OF LIQUID

WATER-ABSORBING SHEET

CONTAINER

# FIG. 14

WATER-ABSORBING
SHEET

FUNNEL

VAT

280mm

WATER-ABSORBING
SHEET

VAT

20mm

φ2mm

10°

350mm

FIG. 15

FIG. 16

FIG. 17

INTRODUCTION OF LIQUID

LIQUID INJECTION TUBE

WEIGHT

CONTAINER

WATER-ABSORBING SHEET

VINYL TAPE

FIG. 18

100

100

101

102

105 104

106

103

## FIG. 19

FIG. 20

PARTICULATE
WATER-ABSORBING
AGENT

FIG. 21

FIG. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2011126079 A
- EP 2015030129 A
- EP 2015030130 A
- EP 2015129917 A
- EP 2016204302 A
- EP 0872491 A
- EP 2007004529 A
- EP 0712659 A
- EP 2004096304 A
- EP 2005016393 A
- US 5147342 A
- US 5149335 A
- EP 0532002 A
- US 5601542 A
- US 5669894 A
- WO 2012174026 A
- WO 2013078109 A
- WO 2015041784 A
- WO 2011117187 A
- WO 2012001117 A
- WO 2012024445 A
- WO 2010004894 A
- WO 2010004895 A
- JP 2010115406 A
- WO 2010076857 A
- WO 2010082373 A
- WO 2010113754 A
- WO 2010143635 A
- WO 2011043256 A
- WO 2011086841 A
- WO 2011086842 A
- WO 2011086843 A
- WO 2011086844 A
- WO 2011117997 A
- WO 2011118409 A
- WO 2011136087 A
- WO 2012043546 A
- WO 2013099634 A
- US 2013099635 A
- US 2016104962 A
- US 2016104374 A
- WO 2016103872 A
- US 6586549 B
- US 20140058346

- US 7638570 B
- WO 2011126079 A
- WO 2011034146 A
- WO 2015129917 A
- JP 2016194921 A
- US 7108916 B
- WO 2009005114 A
- WO 2004096304 A
- WO 2016204302 A
- WO 2011040530 A
- JP 5711974 B
- JP 2008183159 A
- JP 2002345883 A
- JP 6315501 A
- JP 6190003 A
- JP 6190002 A
- JP 6190001 A
- JP 2252558 A
- JP 2252560 A
- JP 2252561 A
- US 20080161512
- US 2009123197 A
- US 20080194863
- US 20050215734
- US 6241928 B
- US 7265190 B
- US 4893999 A
- US 6987151 B
- US 6710141 B
- WO 2006100300 A
- WO 2011025012 A
- WO 2011025013 A
- WO 2011111657 A
- US 7183456 B
- WO 2005075070 A
- WO 2015152299 A
- EP 942014 A
- EP 1108745 A
- JP 8057311 A
- JP 2009531158 W
- US 2006204755
- JP 2010142808 A
- JP 9235378 A

**Non-patent literature cited in the description**

- *CHEMICAL ABSTRACTS*, 7758-87-4